# EUROPEAN PATENT APPLICATION

(11) **EP 1 958 631 A1**
(43) Date of publication of application: **20.08.2008**
(21) Application number: 07102360.0
(22) Date of filing: 14.02.2007
(51) Int. Cl.: A61K 31/404, A61K 31/4196, A61K 31/4427, A61K 31/4523, A61K 31/497, A61P 5/06, A61P 5/08

(54) **Novel triazole derivatives as ligands of G-protein coupled receptors**

(71) Applicant: AEterna Zentaris GmbH, 60314 Frankfurt (DE); AEterna Zentaris Inc., Quebec G1P 4P5 (CA)
(72) Inventor: Perrissoud, Daniel, 61348 Bad Homburg (DE); Ong, Huy, H3R 2Y3 Ville mont-Royal, Quebec (CA); Mulumba, Mukandila, J4L 4V7 Longueuil, Quebec (CA); Jossart, Christian, H2J 3N3 Montreal, Quebec (CA); Lachance, Yves, G7A 3K7 St-Nicolas, Quebec (CA)

(57) **Abstract**

The present invention provides novel triazole derivatives according to formula (I) as ligands of G-protein coupled receptors (GPCR), which are useful in the treatment or prophylaxis of physiological and/or pathophysiological conditions in mammals, preferably humans, which are mediated by GPCR. The present invention further provides GPCR antagonists and agonists that can be used for modulation of these receptors and are useful for treating above conditions, in particular adipogenesis, adiposity, cachexia, diabetes, diabetes type I, diabetes type II, energy balance, energy homeostasis, food intake, hyperlipidemia, hyperphagia, obesity, short-, medium- and/or long-term regulation of energy balance, short-, medium- and/or long-term regulation (stimulation and/or inhibition) of food intake.

## Description

### Technical field

The invention relates to novel triazole derivatives that act as ligands of G-protein coupled receptors. These compounds are useful in the treatment or prophylaxis of various physiological and pathophysiological conditions, such as adipogenesis, adiposity, obesity, alteration of food intake and energy balance, cachexia, hyperlipidemia and/or hyperphagia.

### Prior art

Many medically significant biological processes are mediated by signal transduction pathways that involve G proteins (Lefkowitz RJ, Nature 1992, 358(6385):372). The family of G protein-coupled receptors (GPCR) includes receptors for hormones, neurotransmitters, growth factors, and viruses. Specific examples of GPCRs include receptors for such diverse agents as calcitonin, adrenergic hormones, endothelin, cAMP, adenosine, acetylcholine, serotonin, dopamine, histamine, thrombin, kinin, follicle stimulating hormone, opsins, endothelial differentiation gene-1, rhodopsins, odorants, cytomegalovirus, G proteins themselves, effector proteins such as phospholipase C, adenyl cyclase, and phosphodiesterase, and actuator proteins such as protein kinase A and protein kinase C.

The GPCR protein superfamily now contains over 250 types of paralogs, receptors that represent variants generated by gene duplications (or other processes), as opposed to orthologs, the same receptor from different species. The superfamily can be broken down into five families:
Family I, receptors typified by rhodopsin and the beta-adrenergic receptor and currently represented by over 200 unique members (reviewed by Dohlman HG et al., Ann. Rev. Biochem. 1991, 60: 653-88, and references cited therein);
Family II, the parathyroid hormone/calcitonin/-secretin receptor family (Juppner H et al., Science 1991, 254: 1024-26; Lin HY et al., Science 1991, 254: 1022-24);
Family III, the metabotropic glutamate receptor family in mammals (Nakanishi S, Science 1992, 258: 597-603);
Family IV, the cAMP receptor family, important in the chemotaxis and development of Dictyostelium discoideum (Klein PS et al., Science 1988, 241: 1467-72); and
Family V, the fingal mating pheromone receptors such as STE2 (reviewed by Kurjan J, Ann. Rev. Biochem. 1992, 61: 1097-1129).

GPCRs possess seven conserved membrane-spanning domains connecting at least eight divergent hydrophilic loops. GPCRs (also known as 7TM receptors) have been characterized as including these seven conserved hydrophobic stretches of about 20 to 30 amino acids, connecting at least eight divergent hydrophilic loops. Most GPCRs have single conserved cysteine residues in each of the first two extracellular loops, which form disulfide bonds that are believed to stabilize functional protein structure. The seven transmembrane regions are designated as TM1, TM2, TM3, TM4, TM5, TM6, and TM7. TM3 has been implicated in signal transduction.

Phosphorylation and lipidation (palmitylation or farnesylation) of cysteine residues can influence signal transduction of some GPCRs. Most GPCRs contain potential phosphorylation sites within the third cytoplasmic loop and/or the carboxy terminus. For several GPCRs, phosphorylation by protein kinase A and/or specific receptor kinases mediates receptor desensitization.

For some receptors, the ligand binding sites of GPCRs are believed to comprise hydrophilic sockets formed by several GPCR transmembrane domains. The hydrophilic sockets are surrounded by hydrophobic residues of the GPCRs. The hydrophilic side of each GPCR transmembrane helix is postulated to face inward and form a polar ligand binding site. TM3 has been implicated in several GPCRs as having a ligand binding site, such as the TM3 aspartate residue. TM5 serines, a TM6 asparagine, and TM6 or TM7 phenylalanines or tyrosines also are implicated in ligand binding.

GPCRs are coupled inside the cell by heterotrimeric G proteins to various intracellular enzymes, ion channels, and transporters (see Johnson GL et al., Endocr. Rev. 1989, 10: 317-31). Different G protein alpha subunits preferentially stimulate particular effectors to modulate various biological functions in a cell. Phosphorylation of cytoplasmic residues of GPCRs is an important mechanism for the regulation of some GPCRs. For example, in one form of signal transduction, the effect of hormone binding is the activation inside the cell of the enzyme, adenylate cyclase. Enzyme activation by hormones is dependent on the presence of the nucleotide GTP. GTP also influences hormone binding. A G protein connects the hormone receptor to adenylate cyclase. G protein exchanges GTP for bound GDP when activated by a hormone receptor. The GTP-carrying form then binds to activated adenylate cyclase. Hydrolysis of GTP to GDP, catalyzed by the G protein itself, returns the G protein to its basal, inactive form. Thus, the G protein serves a dual role, as an intermediate that relays the signal from receptor to effector, and as a clock that controls the duration of the signal.

Over the past decades, many hundreds of therapeutic agents targeting GPCRs receptors have been successfully introduced into the market. This indicates that these receptors have an established, proven history as therapeutic targets.

Further references dealing with G-protein coupled receptors comprise: WO 02/42458, WO 2004/000883.

Obesity and overweight are defined as an excess of body fat relative to lean body mass. An increase in caloric intake or a decrease in energy expenditure or both can bring about this imbalance leading to surplus energy being stored as fat. Obesity is associated with important medical morbidities and an increase in mortality. The causes of obesity are poorly understood and may be due to genetic factors, environmental factors or a combination of the two to cause a positive energy balance. In contrast, anorexia and cachexia are characterized by an imbalance in energy intake versus energy expenditure leading to a negative energy balance and weight loss. An interesting commentary review was published not long ago in Nature Medicine (Friedman JM, Nature Medicine 2004, 10(6): 563-569).

Agents that either increase energy expenditure and/or decrease energy intake, absorption or storage would be useful for treating obesity, overweight, and associated comorbidities. Agents that either increase energy intake and/or decrease energy expenditure or increase the amount of lean tissue would be useful for treating cachexia, anorexia and wasting disorders. In general the following exemplary physiological and/or pathophysiological states can be targeted: obesity, overweight, overweight-associated comorbidities including hypertension, type 2 diabetes, coronary artery disease, hyperlipidemia, stroke, gallbladder disease, gout, osteoarthritis, sleep apnea and respiratory problems, anorexia, cachexia, wasting disorders, appetite suppression, appetite enhancement, increases or decreases in satiety, modulation of body weight, and/or other eating disorders such as bulimia.

As pointed out supra, G protein-coupled receptors (GPCRs) are integral membrane proteins characterized by seven transmembrane spanning helical domains that mediate the actions of many extracellular signals. GPCRs interact with heterotrimeric guanine nucleotide binding regulatory proteins (G proteins) that modulate a variety of second messenger systems or ionic conductances to effect physiological responses. In fact, almost 50% of 30 currently marketed drugs elicit their therapeutic effects by interacting with GPCRs (Kirkpatrick, Nat. Rev. Drug Disc. 2002, 1 (7): 488).

A number of peripherally and centrally acting signaling molecules produce a sense of hunger/satiety or produce elevation in lipid mobilization/oxidation through their interactions with GPCRs. There are numerous examples of neurotransmitters and hormones acting on central satiety pathways. Endocannabinoids, melanin concentrating hormone, serotonin, dopamine, NPY, alpha-MSH, GLP-I, ghrelin and orexin serve as few examples of neurotransmitters/hormones that modulate satiety and/or energy expenditure through GPCRs (Di Marzo V et al., Nature 2001, 410: 822-25; Marsh DJ et al., Proc. Natl. Acad. Sci. USA 2002, 99(5): 3240-45; Nonogaki K et al., Nat. Med. 1998, 4(10): 1152-56; Gadde KM et al., Obes. Res. 2001, 9: 544-51; Daniels AJ et al., Peptides 2001, 22: 483-91; Hinney A et al., J. Clin. Endocrin. Metabol. 1999, 84: 1483-86; Meier JJ et al., Eur. J. Pharmacol. 2002, 440: 269-79; Nakazato M et al., Nature 2001, 409: 194-98; Haynes AC et al., Regul. Pept. 2002, 104: 153-59).

In addition to modulation of central pathways, GPCRs also play a critical role in regulating energy expenditure in the periphery. For example, selective agonist ligands of beta3-adrenergic receptors (AR) induce increase in lipolysis and lipid oxidation in rodents resulting in a decrease in body weight (Arch JRS, Eur. J. Pharmacol. 2002, 440: 99-107).

Further relevant literature concerning GPCRs and obesity as well as related physiological states comprise: Melanin-concentrating-hormone (MCH) receptor (SLC-1; Chambers J et al., Nature 1999, 400: 261-265; Saito Y et al., Nature 1999, 400: 265-269; Saito Y et al., TEM 2000, 11 (8): 299-303); NPY receptors (Fetissov SO et al., Neuropeptides 2004, 38: 175-188); general ovierview about GPCRs involved in metabolic diseases (Bagnol D, Curr. Opin. Drug. Disc. Dev. 2004, 7(5): 665-682); GPR103 receptors (AQ27, SP9155, Fukusumi S et al., J. Biol. Chem. 2003, 278(47): 46387-46395; Moriya R et al., Endocrinology 2006, 147(6): 2916-2922; Takayasu S et al., PNAS 2006, 103(19): 7438-7443; Kampe J et al., Brain Res. 2006, 1119(1): 133-149; Jiang Y et al., J. Biol. Chem. 2003, 278(30): 27652-27657; EP 1 207 201; Egido EM et al., "26RFa, a novel orexigenic neuropeptide, inhibits insulin secretion in the rat pancreas.", Peptides, in press, available online 13 June 2006; Thuau R et al., Peptides 2005, 26(5):779-89; Baribault H et al., Mol Cell Biol 2006, 26(2):709-17; Lee DK et al., Gene 2001, 275(1):83-91), cortistatin receptor (MrgX2, Robas N et al., J. Biol. Chem. 2003, 278(45): 44400-44404 ; Allia E et al., J. Pathol. 2005, 207(3): 336-345).

Compounds containing triazole moieties have been widely recognized in the medicinal chemistry due to their various biological activities. The following patent families are all directed to heterocyclic compounds that are said to show certain biological action for use in different medicinal indications. Triazole moieties are implicitly or explicitly contained.

WO 2004/111015 discloses modulators of the glucocorticoid receptor. WO 2004/052280 describes anti-agiogenic compounds as inhibitors of tyrosine kinase activity of VEGF receptors and their use in cancer. WO 2004/096795 also discloses tyrosine kinase inhibitors, preferably C-FMS inhibitors. WO 03/011831 and WO 03/011210 both describe heteroarylheteroalkylamine derivatives as inhibitors of nitric oxide synthase. WO 02/00651 is directed to Factor XA inhibitors for use in thromboembolic disorders. WO 01/94318 and WO 01/94317 both describe chemical libraries of substituted azole derivatives and methods of their synthesis for use in drug discovery high-throughput screening. However, they fail to provide any biological activity or any medicinal use nor do they name specific compounds. WO 00/76971 and WO 00/76970 both claim serine protease inhibitors useful as antithrombotic agents. WO 01/36395 discloses triazole derivatives as farnesyl transferase inhibitors. WO 96/33176 and US 5,703,092 are directed to hydroxamic acid compounds as metal-loprotease and TNF inhibitors. WO 93/09095 describes 2-heterocyclicethylamine derivatives and their use in neurological and neurodegenerative disorders. WO 2004/103270 claims compounds for the treatment of thrombosis, in particular Factor Xla inhibitors. WO 98/38177, US 6,506,782, US 6,849,650 and US 2003/0130188 all describe heterocyclic compounds as inhibitors of beta-amyloid peptide release or its synthesis for use in Alzheimer's disease. WO 00/54729 discloses heterocyclic aromatic compounds as GH secretagogues which are said to stimulate endogenous production and/or release of GH and can also contain triazole moieties. In addition, a method for increasing levels of endogenous GH or increasing the endogenous production or release of GH administering such GHS is described. Furthermore, a method is provided for preventing or treating osteoporosis (improving bone density and/or strength), or treating obesity, or increasing muscle mass and/or muscle strength and function in elderly humans, or reversal or prevention of frailty in elderly humans administering such GHS. US 6,525,203, US 6,518,292 US 6,660,760 are members of the same patent family as WO 00/54729. WO 2004/021984 describes heterocyclic aromatic compounds GH secretagogues which are said to be useful in stimulating endogenous production or release of GH. However, claimed compounds consists of bi- to tetracylic aromatic rings and do not contain triazoles. WO 97/23508 claims compounds of peptide mimetic nature as GHS and are said to act directly on pituitary cells in vitro to release GH therefrom and show improved properties, such as improved resistance to proteolytic degradation and improved bioavailability. In addition, claimed compounds could also be administered in vivo to increase GH release. The compounds are peptide derivatives and do not explicitly contain triazole moieties. US 6,127,391, US 5,977,178 and US 6,555,570 are members of the same patent family as WO 97/23508. WO 01/36395 discloses triazole compounds for the treatment of tumor cell proliferation.

### Description of the invention

The present invention has the object to provide novel compounds which can be employed for the treatment or prophylaxis of physiological and/or pathophysiological conditions in mammals, in particular humans, that are mediated by G-protein coupled receptors (GPCR). It is another object of the underlying invention to provide compounds for the above treatment where the treatment is achieved by modulation of GPCR receptors. A further object of the present invention is to provide antagonists of GPCR receptors for those treatments. It is yet another object of the underlying invention to provide agonists of GPCR receptors for those treatments.

The object of the invention has been surprisingly solved in one aspect by providing compounds according to formula (1) 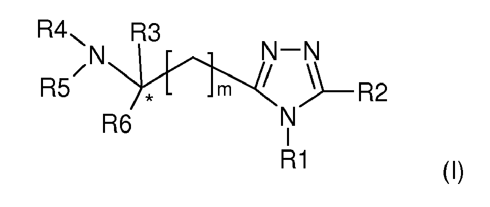 wherein:
R1 and R2 are independently of one another selected from the group consisting of "hydrogen atom, alkyl, alkenyl, alkynyl, cycloalkyl, cycloalkylalkyl, aryl, heteroaryl, arylalkyl, heteroarylalkyl, heterocyclyl, heterocyclylalkyl, alkylsulfonyl, arylsulfonyl, arylalkylsulfonyl" which are optionally substituted in the alkyl, cycloalkyl, cycloalkylalkyl, aryl, heteroaryl, arylalkyl, heteroarylalkyl, heterocyclyl and/or heterocyclylalkyl group by up to 3 substituents independently selected from the group consisting of "halogen, -F, -Cl, -Br, -I, -N₃, -CN, -NR7R8, -OH, -NO₂, alkyl, aryl, arylalkyl, -O-alkyl, -O-aryl, -O-arylalkyl"; and preferably are selected from the group consisting of "alkyl, aryl, heteroaryl, arylalkyl, heteroarylalkyl" optionally being substituted by up to 3 substituents independently selected from the group consisting of "halogen, -F, -Cl, -Br, -I, -N₃, -CN, -NR7R8, -OH, -NO₂, alkyl, aryl, arylalkyl, - O-alkyl, -O-aryl, -O-arylalkyl";
one of radicals R3 and R4 is a hydrogen atom, whereas the other radical is selected from the group consisting of "hydrogen atom, alkyl, aryl, heteroaryl, arylalkyl, heteroarylalkyl, heterocyclyl, heterocyclylalkyl, -alkyl-O-aryl, -alkyl-O-arylalkyl, - alkyl-O-heteroaryl, -alkyl-O-heteroarylalkyl, -alkyl-O-heterocyclyl, alkyl-O-heterocyclylalkyl, -alkyl-CO-aryl, -alkyl-CO-arylalkyl, -alkyl-CO-heteroaryl, - alkyl-CO-heteroarylalkyl, -alkyl-CO-heterocyclyl, -alkyl-CO-heterocyclylalkyl, - alkyl-C(O)O-aryl, -alkyl-C(O)O-arylalkyl, -alkyl-C(O)O-heteroaryl, -alkyl-C(O)O-heteroarylalkyl, -alkyl-C(O)O-heterocyclyl, -alkyl-C(O)O-heterocyclylalkyl, -alkyl-CO-NH₂, -alkyl-CO-OH, -alkyl-NH₂, -alkyl-NH-C(NH)-NH₂, alkylsulfonyl, arylsulfonyl, arylalkylsulfonyl, alkyl-S-alkyl, alkyl-S-H" which are optionally substituted in the aryl, heteroaryl, arylalkyl, heteroarylalkyl, heterocyclyl and/or heterocyclylalkyl group by up to 3 substituents independently selected from the group consisting of "halogen, -F, -Cl, -Br, -I, -N₃, -CN, -NR7R8, -OH, - NO₂, alkyl, aryl, arylalkyl, -O-alkyl, -O-aryl, -O-arylalkyl"; and preferably are selected from the group consisting of "arylalkyl, heteroarylalkyl, heterocyclylalkyl, - alkyl-O-aryl, -alkyl-O-arylalkyl, -alkyl-O-heteroaryl, -alkyl-O-heteroarylalkyl, - alkyl-O-heterocyclyl, alkyl-O-heterocyclylalkyl, -alkyl-CO-aryl, -alkyl-CO-arylalkyl, -alkyl-CO-heteroaryl, -alkyl-CO-heteroarylalkyl, -alkyl-CO-heterocyclyl, alkyl-CO-heterocyclylalkyl, -alkyl-C(O)O-aryl, -alkyl-C(O)O-arylalkyl, -alkyl-C(O)O-heteroaryl, -alkyl-C(O)O-heteroarylalkyl, -alkyl-C(O)O-heterocyclyl, -alkyl-C(O)O-heterocyclylalkyl, -alkyl-CO-NH₂, -alkyl-CO-OH, - alkyl-NH₂, -alkyl-NH-C(NH)-NH₂," optionally being substituted in the aryl, heteroaryl, arylalkyl, heteroarylalkyl, heterocyclyl and/or heterocyclylalkyl group by up to 3 substituents independently selected from the group consisting of "halogen, -F, -Cl, -Br, -I, -N₃, -CN, -NR7R8, -OH, -NO₂, alkyl, aryl, arylalkyl, -O-alkyl, -O-aryl, -O-arylalkyl";
R5 is selected from the group consisting of "hydrogen atom, alkyl, cycloalkyl, cycloalkylalkyl, aryl, heteroaryl, arylalkyl, heteroarylalkyl, heterocyclyl, heterocyclylalkyl, -CO-alkyl, -CO-cycloalkyl, -CO-cycloalkylalkyl, -CO-aryl, -CO-arylalkyl, -CO-heteroaryl, -CO-heteroarylalkyl, -CO-heterocyclyl, -CO-heterocyclylalkyl, - CO-C*(R9R10)-NH₂, -CO-CH₂-C*(R9R10)-NH₂, -CO-C*(R9R10)-CH₂-NH₂, alkylsulfonyl, arylsulfonyl, arylalkylsulfonyl" which are optionally substituted by up to 3 substituents independently selected from the group consisting of "halogen, -F, - Cl, -Br, -I, -N₃, -CN, -NR7R8, -OH, -NO₂, alkyl, aryl, arylalkyl, -O-alkyl, -O-aryl, -O-arylalkyl"; and preferably is selected from the group consisting of "hydrogen atom, -CO-alkyl, -CO-cycloalkyl, -CO-aryl, -CO-heteroaryl, -CO-arylalkyl, - CO-heteroarylalkyl, -CO-heterocyclyl, -CO-C*(R9R10)-NH₂, -CO-CH₂-C*(R9R10)-NH₂, -CO-C*(R9R10)-CH₂-NH₂, optionally being substituted by up to 3 substituents independently selected from the group consisting of "halogen, -F, - Cl, -Br, -I, -N₃, -CN, -NR7R8, -OH, -NO₂, alkyl, aryl, arylalkyl, -O-alkyl, -O-aryl, -O-arylalkyl";
R6 is selected from the group consisting of "hydrogen atom, alkyl, cycloalkyl, cycloalkylalkyl" and preferably is a hydrogen atom;
R7 and R8 are independently of one another selected from the group consisting of "hydrogen atom, alkyl, cycloalkyl, cycloalkylalkyl" and preferably are a hydrogen atom;
R9 and R10 are independently of one another selected from the group consisting of "hydrogen atom, alkyl, natural alpha-amino acid side chain, unnatural alpha-amino acid side chain" and preferably are selected from the group consisting of "hydrogen atom, alkyl";
m is 0, 1 or 2 and preferably is 0; and
* means a carbon atom of R or S configuration when chiral;
that can be used for the manufacture of a medicament for the treatment or prophylaxis of physiological and/or pathophysiological conditions in mammals, where the physiological and/or pathophysiological conditions are mediated by at least one G-protein coupled receptor (GPCR);
with the proviso that the following G-protein coupled receptors (GPCR) are excluded: "GHS receptors, GHS type 1 receptor, GHS-R 1a, GHS-R 1b, motilin receptor, motilin receptor 1a, neurotensin receptor, TRH receptor, GPR38 (FM1), GPR39 (FM2), FM3, GHS-R subtype, GHS binding site, cardiac GHS-R, mammary G H S-R".

In a preferred embodiment compounds according to above formula (I) are provided, where
R3 is selected from the group consisting of "-alkyl-CO-aryl, -alkyl-CO-arylalkyl, - alkyl-CO-heteroaryl, -alkyl-CO-heteroarylalkyl, -alkyl-CO-heterocyclyl, alkyl-CO-heterocyclylalkyl, -alkyl-C(O)O-aryl, -alkyl-C(O)O-arylalkyl, -alkyl-C(O)O-heteroaryl, -alkyl-C(O)O-heteroarylalkyl, -alkyl-C(O)O-heterocyclyl, -alkyl-C(O)O-heterocyclylalkyl, -alkyl-CO-NH₂, -alkyl-CO-OH, -alkyl-NH-C(NH)-NH₂, alkyl-S-alkyl, alkyl-S-H", and preferably is selected from the group consisting of "-alkyl-CO-arylalkyl, -alkyl-C(O)O-arylalkyl, -alkyl-CO-NH₂, -alkyl-CO-OH";
that can be used for the manufacture of a medicament for the treatment or prophylaxis of physiological and/or pathophysiological conditions in mammals, where the physiological and/or pathophysiological conditions are mediated by at least one G-protein coupled receptor (GPCR);
with the proviso that the following G-protein coupled receptors (GPCR) are excluded: "GHS receptors, GHS type 1 receptor, GHS-R 1 a, GHS-R 1b, motilin receptor, motilin receptor 1 a, neurotensin receptor, TRH receptor, GPR38 (FM1), GPR39 (FM2), FM3, GHS-R subtype, GHS binding site, cardiac GHS-R, mammary GHS-R".

In another preferred embodiment compounds according to above formula (I) are provided, where
R4 is a hydrogen atom;
R5 is selected from the group consisting of "hydrogen atom, alkyl, cycloalkyl, cycloalkylalkyl, aryl, heteroaryl, arylalkyl, heteroarylalkyl, heterocyclyl, heterocyclylalkyl, alkylsulfonyl, arylsulfonyl, arylalkylsulfonyl, -CO-cycloalkyl, -CO-cycloalkylalkyl, -CO-aryl, -CO-arylalkyl, -CO-heteroaryl, -CO-heteroarylalkyl, -CO-heterocyclyl, -CO-heterocyclylalkyl";
with the proviso that if R5 is "-CO-heteroarylalkyl", "heteroaryl" is not imidazole; and
with the proviso that if R5 is "-CO-heterocyclyl" and "heterocyclyl" contains only nitrogen atoms as heteroatoms, that at least two nitrogen atoms are contained in "heterocyclyl"; and
with the proviso that if R5 is "-CO-heterocyclylalkyl" and "heterocyclyl" contains only nitrogen atoms as heteroatoms that in the case that one or two nitrogen atoms are contained in "heterocyclyl" no nitrogen atom is positioned at position 1 of "heterocyclyl" that is the atom directly linking "heterocyclyl" to the carbonyl group "-CO-";
where "alkyl, cycloalkyl, cycloalkylalkyl, aryl, heteroaryl, arylalkyl, heteroarylalkyl, heterocyclyl, heterocyclylalkyl, alkylsulfonyl, arylsulfonyl, arylalkylsulfonyl, -CO-cycloalkyl, -CO-cycloalkylalkyl, -CO-aryl, -CO-arylalkyl, -CO-heteroaryl, -CO-heteroarylalkyl, -CO-heterocyclyl, and/or -CO-heterocyclylalkyl" are optionally substituted by up to 3 substituents independently selected from the group consisting of "halogen, -F, -Cl, -Br, -I, -N₃, -CN, -NR7R8, -OH, -NO₂, alkyl, aryl, arylalkyl, -O-alkyl, -O-aryl, -O-arylalkyl";
with the proviso that if R5 is "-CO-cycloalkyl" or "-CO-cycloalkylalkyl", R5 is not substituted with NR7R8 at position 1 of "cycloalkyl", that is the C atom directly linking "cycloalkyl" to the carbonyl group "-CO-" in case of R5 = "-CO-cycloalkyl" or to the "alkyl" in case R5 = "-CO-cycloalkylalkyl"; and
with the proviso that if R5 is "-CO-aryl" or "-CO-arylalkyl" and "aryl" is phenyl/benzene and is only substituted with one substituent, this one substituent is not -NR7R8;
R6 is a hydrogen atom;
R7 and R8 are independently of one another selected from the group consisting of "hydrogen atom, alkyl, cycloalkyl, cycloalkylalkyl" and preferably are a hydrogen atom; and
m is 0, 1 or 2, and more preferably is 0;
that can be used for the manufacture of a medicament for the treatment or prophylaxis of physiological and/or pathophysiological conditions in mammals, where the physiological and/or pathophysiological conditions are mediated by at least one G-protein coupled receptor (GPCR);
with the proviso that the following G-protein coupled receptors (GPCR) are excluded: "GHS receptors, GHS type 1 receptor, GHS-R 1a, GHS-R 1b, motilin receptor, motilin receptor 1a, neurotensin receptor, TRH receptor, GPR38 (FM1), GPR39 (FM2), FM3, GHS-R subtype, GHS binding site, cardiac GHS-R, mammary GHS-R".

In a further aspect, the object of the invention has surprisingly been achieved by providing compounds according to formula (I), where
R1 is selected from the group consisting of "hydrogen, methyl, (2-methoxyphenyl)-methyl, (3-methoxyphenyl)-methyl, (4-methoxyphenyl)-methyl, (3-methoxyphenyl)-ethyl, (4-methoxyphenyl)-ethyl, phenyl, phenyl-methyl, phenyl-ethyl, (4-ethylphenyl)-methyl, (4-methylphenyl)-methyl, (4-fluorophenyl)-methyl, (4-bromophenyl)-methyl, (2,4-dimethoxyphenyl)-methyl, (3,5-dimethoxyphenyl)-methyl, 2,2-diphenyl-ethyl, naphthaline-1-yl-methyl, 1*H*-indole-3-yl-methyl, 2-(1*H* indole-3-yl)-ethyl, 3-(1*H*-indole-3-yl)-propyl, 4-methyl-phenyl, 4-ethyl-phenyl, n-hexyl, (3,4-dichlorophenyl)-methyl, (4-nitro-phenyl)-methyl, (pyridine-2-yl)-methyl, (pyridine-3-yl)-methyl, (pyridine-4-yl)-methyl, (thiophene-2-yl)-methyl, (thiophene-3-yl)-methyl, (furan-2-yl)-methyl, (furan-3-yl)-methyl";
R2 is selected from the group consisting of "methyl, 1 H indole-3-yl-methyl, 2-(1*H-*indole-3-yl)-ethyl, 3-(1*H*-indole-3-yl)-propyl, 2-phenyl-ethyl, 3-phenyl-propyl, 4-phenyl-butyl, 2-methoxy-phenylmethyl, 3-methoxy-phenylmethyl, 4-methoxy-phenylmethyl, 2-methoxy-phenylethyl, 3-methoxy-phenylethyl, 4-methoxy-phenylethyl";
R3 is selected from the group consisting of "hydrogen atom, methyl, propan-2-yl, 2-methyl-propan-1-yl, butan-2-yl, butan-1-yl, -CH₂-SH, -(CH₂)₂-S-CH₃, 1*H*-indole-3-yl-methyl, phenyl-methyl, 2-phenyl-ethyl, -CH₂-O-CH₂-phenyl, -CH₂-CO-CH₂-phenyl, -(CH₂)₂-CO-CH₂-phenyl, -CH₂-C(O)O-phenyl, -(CH₂)₂-C(O)O-phenyl, hydroxy-methyl, 1 -hydroxy-ethan-1-yl, -CH₂-CO-NH₂, -(CH₂)₂-CO-NH₂, (1-hydroxy-benzene-4-yl)-methyl, -CH₂-CO-OH, -(CH₂)₂-CO-OH, -(CH₂)₄-NH₂, (1 H-imidazol-5-yl)-methyl, -(CH₂)₃-NH-C(NH)-NH₂, -(CH₂)₃-NH₂, -(CH₂)₃-NH-CO-NH₂", and preferably is selected from the group consisting of "1*H*-indole-3-yl-methyl, -CH₂-CO-CH₂-phenyl, -(CH₂)₂-CO-CH₂-phenyl, -CH₂-C(O)O-phenyl, - (CH₂)₂-C(O)O-phenyl";
R4 is a hydrogen atom;
R5 is selected from the group consisting of "hydrogen atom, -CO-CH₂-NH₂ (Gly residue), -CO-CH₂-CH₂-NH₂ (beta-Ala residue), -CO-CHCH₃-NH₂ (D- and/or L-alpha-Ala residue), -CO-(pyrrolidine-2-yl) (D- and/or L-Pro residue), 2-amino-2-carbonyl-propane (2-amino-isobutyric acid/Aib residue), 4-carbonyl-1*H*-piperidine, 3-carbonyl-1*H*-piperidine, R-(3-carbonyl-1*H*-piperidine), S-(3-carbonyl-1*H*piperidine), 2-carbonyl-1*H* piperidine, R-(2-carbonyl-1*H*-piperidine), S-(2-carbonyl-1*H-*piperidine), 1-amino-2-carbonyl-benzene, carbonyl-cyclohexane, 2-acetyl-pyridine, 3-acetyl-pyridine, 4-acetyl-pyridine, 2-propionyl-pyridine, 3-propionyl-pyridine, 4-propionyl-pyridine, (R-1-amino)-2-carbonyl-cyclohexane, (S-1-amino)-2-carbonyl-cyclohexane, 2-carbonyl-1*H*-imidazole, 2-carbonyl-pyridine, 3-carbonyl-pyridine, 4-carbonyl-pyridine, 2-amino-3-carbonyl-pyridine, 2-carbonyl-pyrazine, 2-carbonyl-4-hydroxy-1*H*-pyrrolidine, 4-carbonyl-1*H*,3*H*-diazacyclohexane, methylsulfonyl, phenylsulfonyl, 1-carbonyl-1-amino-2-phenylethane, phenylmethyl, 1-carbonyl-4-azide-benzene, 2-carbonyl-2,5-dihydro-1H-pyrrole, 2-carbonyl-piperazine, 2-carbonyl-1H-pyrrolidine, 2-aminoethane, carbonyl-benzene, 2-carbonyl-pyrazine, 3-carbonyl-pyrazine, 4-carbonyl-oxacyclohexane, 4-methylphenylsulfonyl, phenylmethyl-sulfonyl";
R6 is a hydrogen atom; and
m is 0;
that can be used for the manufacture of a medicament for the treatment or prophylaxis of physiological and/or pathophysiological conditions in mammals, where the physiological and/or pathophysiological conditions are mediated by at least one G-protein coupled receptor (GPCR);
with the proviso that the following G-protein coupled receptors (GPCR) are excluded: "GHS receptors, GHS type 1 receptor, GHS-R 1a, GHS-R 1b, motilin receptor, motilin receptor 1a, neurotensin receptor, TRH receptor, GPR38 (FM1), GPR39 (FM2), FM3, GHS-R subtype, GHS binding site, cardiac GHS-R, mammary GHS-R".

In a preferred embodiment compounds according to above formula (I) are provided, where
R3 is selected from the group consisting of "-CH₂-CO-CH₂-phenyl, -(CH₂)₂-CO-CH₂-phenyl, -CH₂-CO-NH₂, -(CH₂)₂-CO-NH₂, -CH₂-CO-OH, -(CH₂)₂-CO-OH, -(CH₂)₃-NH-C(NH)-NH₂, -CH₂-SH, -(CH₂)₂-S-CH₃";
that can be used for the manufacture of a medicament for the treatment or prophylaxis of physiological and/or pathophysiological conditions in mammals, where the physiological and/or pathophysiological conditions are mediated by at least one G-protein coupled receptor (GPCR);
with the proviso that the following G-protein coupled receptors (GPCR) are excluded: "GHS receptors, GHS type 1 receptor, GHS-R 1a, GHS-R 1b, motilin receptor, motilin receptor 1 a, neurotensin receptor, TRH receptor, GPR38 (FM1), GPR39 (FM2), FM3, GHS-R subtype, GHS binding site, cardiac GHS-R, mammary GHS-R".

In another preferred embodiment compounds according to above formula (I) are provided, where
R5 is selected from the group consisting of "hydrogen atom, methylsulfonyl, phenylsulfonyl, carbonyl-cyclohexane, (R-1-amino)-2-carbonyl-cyclohexane, (S-1-amino)-2-carbonyl-cyclohexane, 2-carbonyl-pyridine, 3-carbonyl-pyridine, 4-carbonyl-pyridine, 2-acetyl-pyridine, 3-acetyl-pyridine, 4-acetyl-pyridine, 2-propionyl-pyridine, 3-propionyl-pyridine, 4-propionyl-pyridine, 2-amino-3-carbonyl-pyridine, 2-carbonyl-1 H-imidazole, 2-carbonyl-pyrazine, 4-carbonyl-1*H*,3*H* diazacyclohexane";
that can be used for the manufacture of a medicament for the treatment or prophylaxis of physiological and/or pathophysiological conditions in mammals, where the physiological and/or pathophysiological conditions are mediated by at least one G-protein coupled receptor (GPCR);
with the proviso that the following G-protein coupled receptors (GPCR) are excluded: "GHS receptors, GHS type 1 receptor, GHS-R 1 a, GHS-R 1b, motilin receptor, motilin receptor 1a, neurotensin receptor, TRH receptor, GPR38 (FM1), GPR39 (FM2), FM3, GHS-R subtype, GHS binding site, cardiac GHS-R, mammary GHS-R".

In a further aspect, the object of the invention has surprisingly been achieved by providing novel triazole compounds selected from the group consisting of:
**compound 1** (*R*)-*N*-(1-(5-(2-(1*H*-indol-3-yl)ethyl)-4-(2,4-dimethoxybenzyl)-4*H-*1,2,4-triazol-3-yl)-2-(1*H*-indol-3-yl)ethyl)-2-amino-2-methylpropanamide, 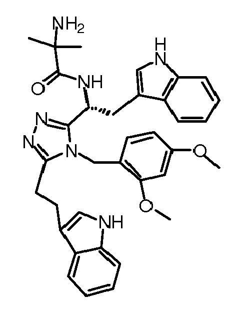
**compound 2** (*R*)-*N*-(1-(5-(2-(1*H*-indol-3-yl)ethyl)-4-phenethyl-4*H*-1,2,4-triazol-3-yl)-2-(1*H*-indol-3-yl)ethyl)-2-amino-2-methylpropanamide, 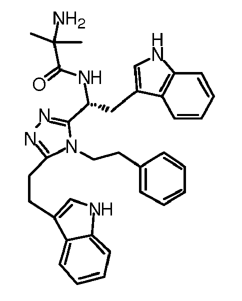
compound 3 (*R*)-*N*-(1-(5-(3-(1*H*-indol-3-yl)propyl)-4-phenethyl-4*H-*1,2,4-triazol-3-yl)-2-(1*H*-indol-3-yl)ethyl)-2-amino-2-methylpropanamide, 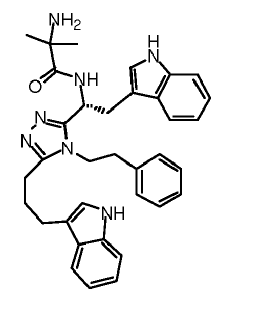
**compound 4** (*R*)-*N*-(1-(5-benzyl-4-(naphthalen-1-ylmethyl)-4*H*-1,2,4-triazol-3-yl)-2-(1*H*-indol-3-yl)ethyl)-2-amino-2-methylpropanamide, 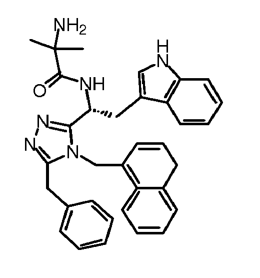
**compound 5** (*R*)-*N*-(1-(5-(2-(1*H*-indol-3-yl)ethyl)-4-(naphthalen-1-ylmethyl)-4*H-*1,2,4-triazol-3-yl)-2-(1*H*-indol-3-yl)ethyl)-2-amino-2-methylpropanamide, 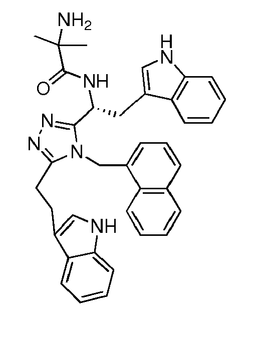
**compound 6** (*R*)-*N*-(1-(5-(2-(1*H*-indol-3-yl)ethyl)-4-(3-methoxybenzyl)-4*H*-1,2,4-triazol-3-yl)-2-(1*H*-indol-3-yl)ethyl)-2-amino-2-methylpropanamide, 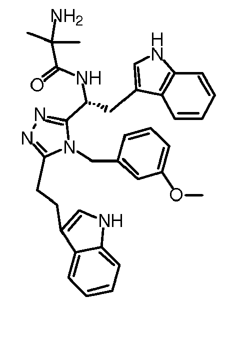
**compound 7** (*R*)-*N*-(1-(4-(3-methoxybenzyl)-5-benzyl-4*H*-1,2,4-triazol-3-yl)-2-(1*H*-indol-3-yl)ethyl)-2-amino-2-methylpropanamide, 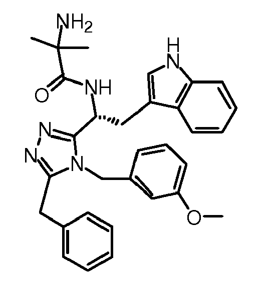
**compound 8** (*R*)-*N*-(1-(5-(2-(1*H*-indol-3-yl)ethyl)-4-benzyl-4*H*-1,2,4-triazol-3-yl)-2-(1*H*-indol-3-yl)ethyl)-2-amino-2-methylpropanamide, 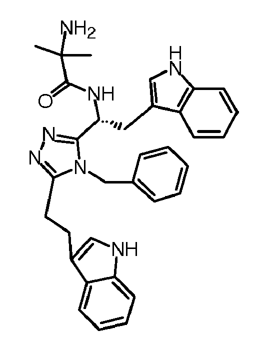
**compound 9** (*R*)-*N*-(1-(5-(3-(1*H-*indol-3-yl)propyl)-4-benzyl-4*H*-1,2,4-triazol-3-yl)-2-(1*H*-indol-3-yl)ethyl)-2-amino-2-methylpropanamide, 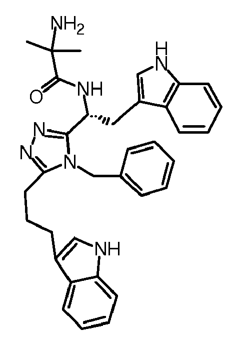
**compound 10** (*R*)*-N-*(1-(5-(3-(1*H*-indol-3-yl)propyl)-4-(3-methoxybenzyl)-4*H-*1,2,4-triazol-3-yl)-2-(1*H* indol-3-yl)ethyl)-2-amino-2-methylpropanamide, 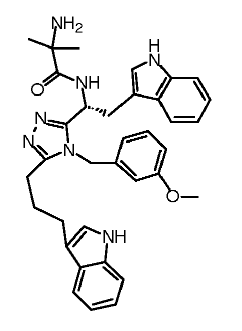
**compound 11** *(R)-N*-(1-(5-(3-(1*H*-indol-3-yl)propyl)-4-(naphthalen-1-ylmethyl)-4*H* 1,2,4-triazol-3-yl)-2-(1*H*-indol-3-yl)ethyl)-2-amino-2-methylpropanamide, 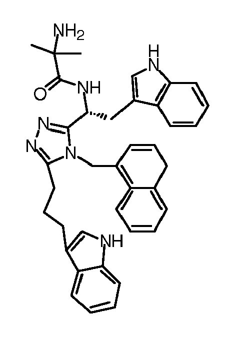
**compound 12** *(R)-N*-(1-(5-(2-(1*H*-indol-3-yl)ethyl)-4-(4-methoxybenzyl)-4*H*-1,2,4-triazol-3-yl)-2-(1*H*-indol-3-yl)ethyl)-2-amino-2-methylpropanamide, 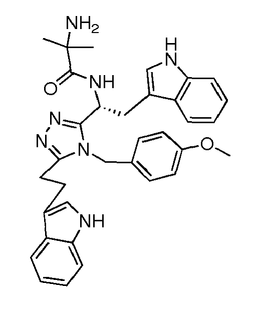
**compound 13** (*R*)-*N*-(1-(4-(4-methoxybenzyl)-5-benzyl-4*H*-1,2,4-triazol-3-yl)-2-(1*H*-indol-3-yl)ethyl)-2-amino-2-methylpropanamide, 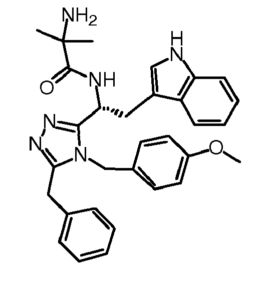
**compound 14** *(R)-N*-(1-(5-(3-(1*H*-indol-3-yl)propyl)-4-(4-bromobenzyl)-4*H*-1,2,4-triazol-3-yl)-2-(1*H*-indol-3-yl)ethyl)-2-amino-2-methylpropanamide, 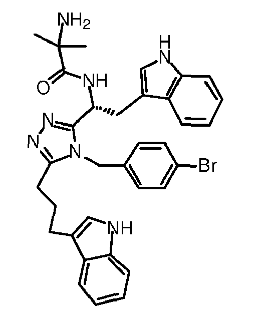
**compound 15** (*R*)*-N-*(1-(5-(2-(1*H*-indol-3-yl)ethyl)-4-hexyl-4*H*-1,2,4-triazol-3-yl)-2-(1*H*-indol-3-yl)ethyl)-2-amino-2-methylpropanamide, 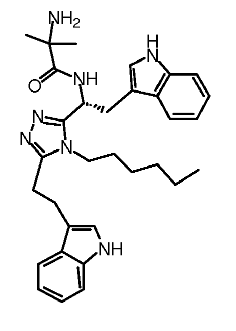
compound 16 *(R)-N*-(1-(5-(3-(1*H* indol-3-yl)propyl)-4-hexyl-4H 1,2,4-triazol-3-yl)-2-(1*H*-indol-3-yl)ethyl)-2-amino-2-methylpropanamide, 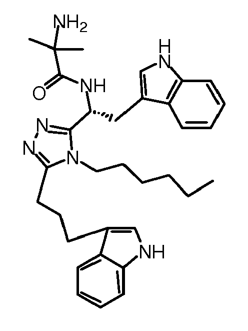
**compound 17** (*R*)-*N*-(1-(4,5-bis(2-(1*H*-indol-3-yl)ethyl)-4H-1,2,4-triazol-3-yl)-2-(1*H*-indol-3-yl)ethyl)-2-amino-2-methylpropanamide, 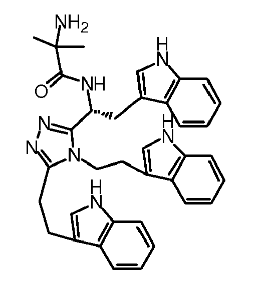
**compound 18** (*S*)-*N*-(1-(5-(2-(1*H*-indol-3-yl)ethyl)-4-(2,4-dimethoxybenzyl)-4*H-*1,2,4-triazol-3-yl)-2-(1*H*-indol-3-yl)ethyl)-2-amino-2-methylpropanamide, 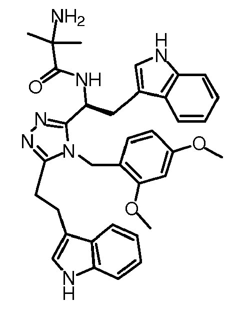
**compound 19** *(R)-N-*(1-(4-(3-methoxybenzyl)-5-phenethyl-4*H*-1,2,4-triazol-3-yl)-2-(1*H-*indol-3-yl)ethyl)-2-amino-2-methylpropanamide, 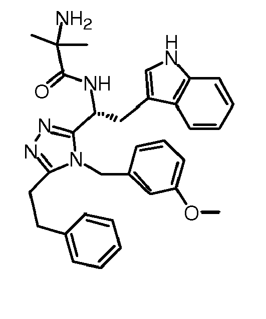
**compound 20** (*R*)*-N-*(1-(4-(4-methoxybenzyl)-5-phenethyl-4*H*-1,2,4-triazol-3-yl)-2-(1*H*-indol-3-yl)ethyl)-2-amino-2-methylpropanamide, 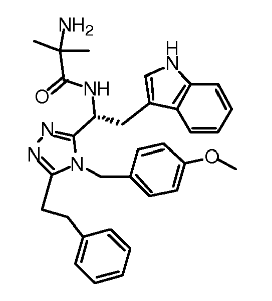
**compound 21** *(R)-N-*(1-(5-(2-(1*H*-indol-3-yl)ethyl)-4-(3,5-dimethoxybenzyl)-4*H-*1,2,4-triazol-3-yl)-2-(1*H*-indol-3-yl)ethyl)-2-amino-2-methylpropanamide, 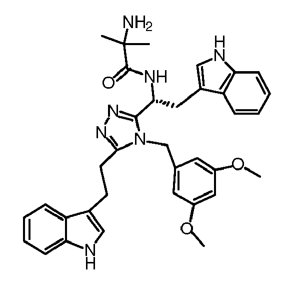
**compound 22** *(R)-N*-(1-(4-(4-methoxybenzyl)-5-(3-phenylpropyl)-4*H*-1,2,4-triazol-3-yl)-2-(1*H*-indol-3-yl)ethyl)-2-amino-2-methylpropanamide, 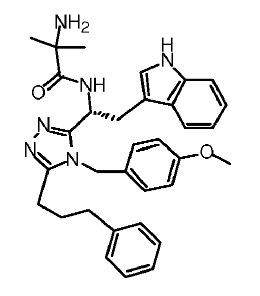
**compound 23** *(R)-N-(*1-(5-(3-(1*H*-indol-3-yl)propyl)-4-(4-methoxybenzyl)-4*H* 1,2,4-triazol-3-yl)-2-(1*H*-indol-3-yl)ethyl)-2-amino-2-methylpropanamide, 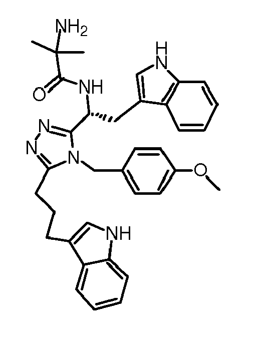
**compound 24** *(R)-N-(*1-(4-(2-(1*H*-indol-3-yl)ethyl)-5-(3-(1*H*-indol-3-yl)propyl)-4*H-*1,2,4-triazol-3-yl)-2-(1*H*-indol-3-yl)ethyl)-2-amino-2-methylpropanamide, 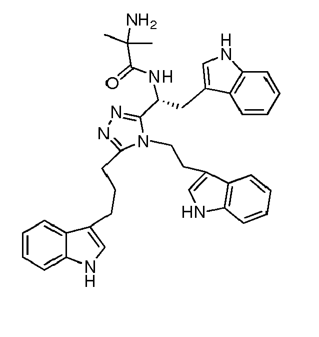
**compound 25** *(R)N-*(1-(5-(2-(1*H*-indol-3-yl)ethyl)-4-(2-methoxy)benzyl)-4*H-*1,2,4-triazol-3-yl)-2-(1*H*-indol-3-yl)ethyl)-2-amino-2-methylpropanamide, 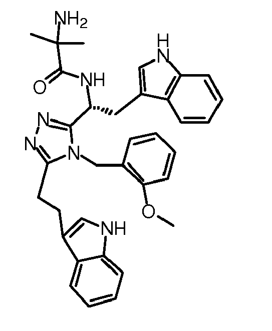
**compound 26** *(R)-N-*(1-(4-(2-methoxybenzyl)-5-phenethyl-4*H*-1,2,4-triazol-3-yl)-2-(1*H*-indol-3-yl)ethyl)-2-amino-2-methylpropanamide, 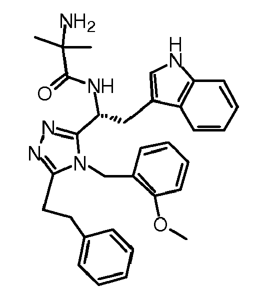
**compound 27** (*R*)-*N*-(2-(1*H*-indol-3-yl)-1-(4-(naphthalen-1-ylmethyl)-5-phenethyl-4*H*-1,2,4-triazol-3-yl)ethyl)-2-amino-2-methylpropanamide, 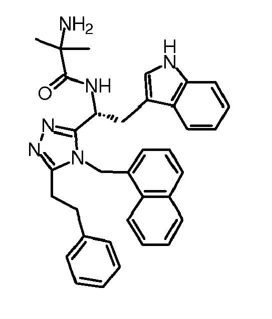
**compound 28** *(R)-N-*(1-(5-(2-(1*H*-indol-3-yl)ethyl)-4-(3,4-dichlorobenzyl)-4*H-*1,2,4-triazol-3-yl)-2-(1*H*-indol-3-yl)ethyl)-2-amino-2-methylpropanamide, 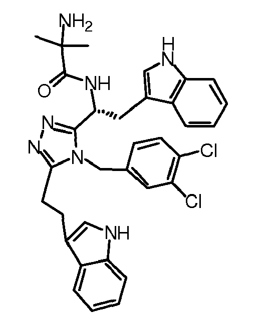
**compound 29** (*R*)*-N-*(1-(5-(2-(1*H-*indol-3-yl)ethyl)-4-(4-fluorobenzyl)-4*H*-1,2,4-triazol-3-yl)-2-(1*H*-indol-3-yl)ethyl)-2-amino-2-methylpropanamide, 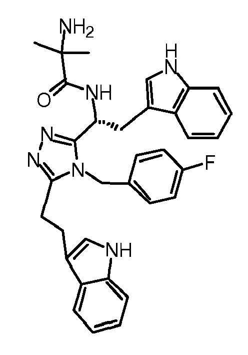
**compound 30** (*R*)-*N*-(1-(4-(4-fluorobenzyl)-5-benzyl-4*H*-1,2,4-triazol-3-yl)-2-(1*H-*indol-3-yl)ethyl)-2-amino-2-methylpropanamide, 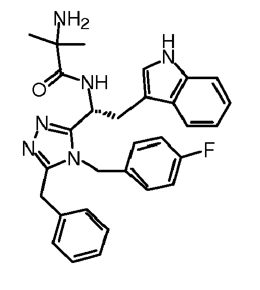
**compound 31** *(R)-N-*(1-(5-(2-(1*H-*indol-3-yl)ethyl)-4-(2,4-dimethoxybenzyl)-4*H-*1,2,4-triazol-3-yl)-2-(1*H*-indol-3-yl)ethyl)piperidine-4-carboxamide, 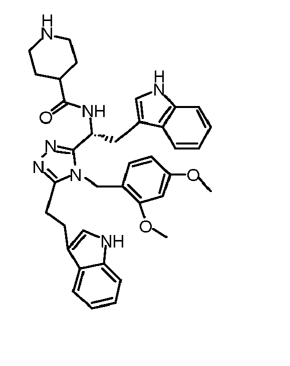
**compound 32** (*R*)*-N-*(1-(5-(2-(1*H*-indol-3-yl)ethyl)-4-(2,4-dimethoxybenzyl)-4*H-*1,2,4-triazol-3-yl)-2-(1*H*-indol-3-yl)ethyl)piperidine-3-carboxamide, 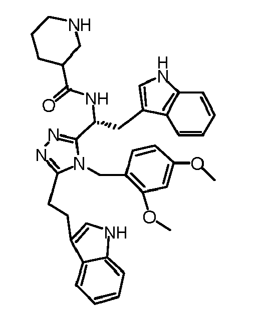
**compound 33** (*R*)-*N*-(1-(4-(4-methylbenzyl)-5-(3-phenylpropyl)-4*H*-1,2,4-triazol-3-yl)-2-(1*H*-indol-3-yl)ethyl)-2-amino-2-methylpropanamide, 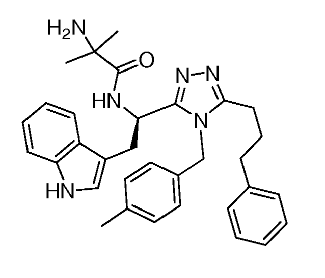
**compound 34** (*R*)-*N*-(1-(5-(2-(1*H*-indol-3-yl)ethyl)-4-(4-methylbenzyl)-4*H-*1,2,4-triazol-3-yl)-2-(1*H*-indol-3-yl)ethyl)-2-amino-2-methylpropanamide, 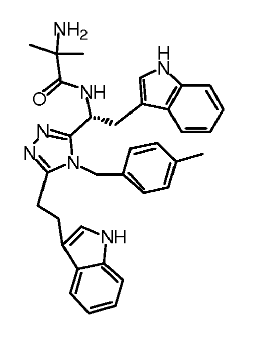
**compound 36** (*R*)*-N-*(1-(5-(2-(1*H*-indol-3-yl)ethyl)-4-(2,4-dimethoxybenzyl)-4*H-*1,2,4-triazol-3-yl)-2-(1*H*-indol-3-yl)ethyl)piperidine-2-carboxamide, 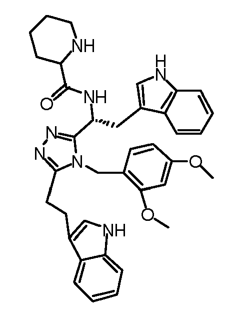
**compound 37** (*R*)-*N*-(1-(4-(4-methylbenzyl)-5-phenethyl-4H-1,2,4-triazol-3-yl)-2-(1*H*-indol-3-yl)ethyl)-2-amino-2-methylpropanamide, 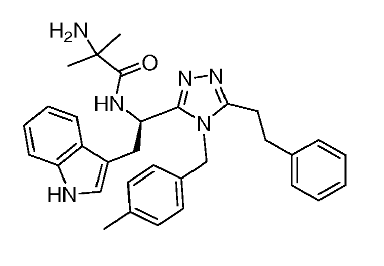
**compound 38** (*R*)*-N*-(1-(5-(2-(1*H*-indol-3-yl)ethyl)-4-(2,4-dimethoxybenzyl)-4*H-*1,2,4-triazol-3-yl)-2-(1*H*-indol-3-yl)ethyl)-2-aminobenzamide, 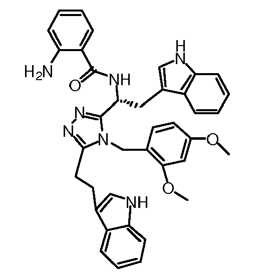
**compound 39** (*R*)-*N*-(1-(5-benzyl-4-(pyridin-2-ylmethyl)-4*H*-1,2,4-triazol-3-yl)-2-(1*H-*indol-3-yl)ethyl)-2-amino-2-methylpropanamide, 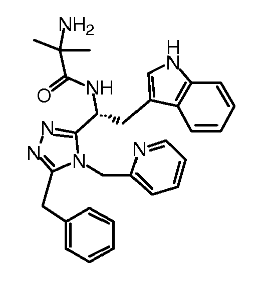
**compound 40** (2*S*,4*R*)-*N*-((*R*)-1-(4-(4-methoxybenzyl)-5-phenethyl-4*H*-1,2,4-triazol-3-yl)-2-(1*H*-indol-3-yl)ethyl)-4-hydroxypyrrolidine-2-carboxamide, 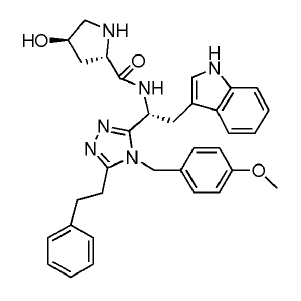
**compound 41** (*S*)-*N*-((*R*)-1-(4-(4-methoxybenzyl)-5-phenethyl-4*H*-1,2,4-triazol-3-yl)-2-(1*H*-indol-3-yl)ethyl)piperidine-3-carboxamide, 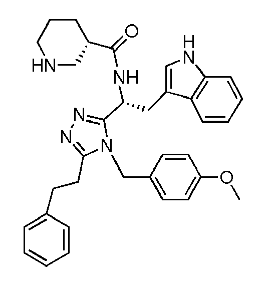
**compound 42** (*R*)-*N*-((*H*)-1-*(*4-(4-methoxybenzyl)-5-phenethyl-4*H*-1,2,4-triazol-3-yl)-2-(1*H*-indol-3-yl)ethyl)piperidine-3-carboxamide, 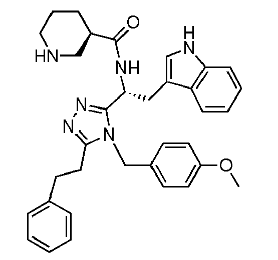
**compound 43** (*R*)-*N*-(1-(4-(4-ethylbenzyl)-5-phenethyl-4*H*-1,2,4-triazol-3-yl)-2-(1*H*-indol-3-yl)ethyl)-2-amino-2-methylpropanamide, 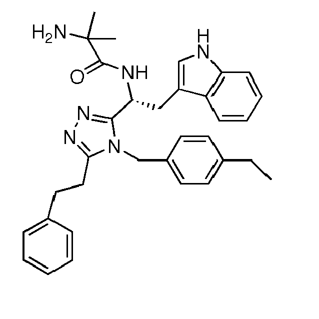
**compound 44** *(R)-N-*(1-(4-(4-methoxybenzyl)-5-phenethyl-4*H-*1,2,4-triazol-3-yl)-2-(1*H*-indol-3-yl)ethyl)piperidine-4-carboxamide, 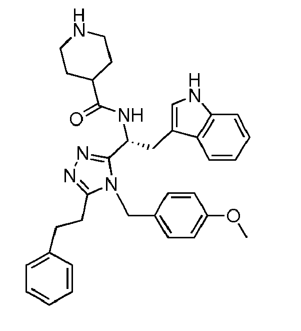
**compound 45** (*R*)-*N*-(1-(5-(2-(1*H-*indol-3-yl)ethyl)-4-(4-methoxybenzyl)-4*H-*1,2,4-triazol-3-yl)-2-(1*H*-indol-3-yl)ethyl)piperidine-4-carboxamide, 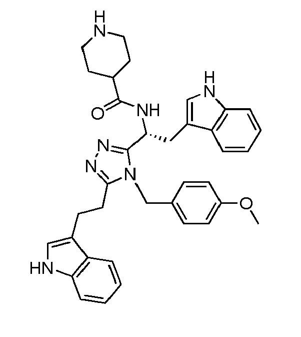
**compound 46** (*S*)-*N*-((*R*)-1-(4-(4-methoxybenzyl)-5-phenethyl-4*H*-1,2,4-triazol-3-yl)-2-(1*H*-indol-3-yl)ethyl)pyrrolidine-2-carboxamide, 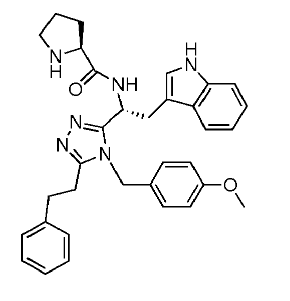
**compound 47** (*R*)-*N*-((*R*)-1-(4-(4-methoxybenzyl)-5-phenethyl-4*H*-1,2,4-triazol-3-yl)-2-(1*H*-indol-3-yl)ethyl)pyrrolidine-2-carboxamide, 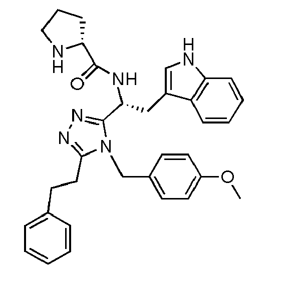
**compound 48** (*S*)-*N*-((*R*)-1-(4-(4-methoxybenzyl)-5-phenethyl-4*H*-1,2,4-triazol-3-yl)-2-(1*H*-indol-3-yl)ethyl)piperidine-2-carboxamide, 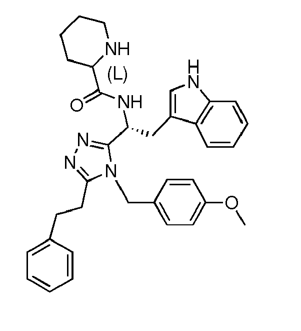
**compound 49** (*R*)-*N*-((*R*)-1-(4-(4-methoxybenzyl)-5-phenethyl-4*H*-1,2,4-triazol-3-yl)-2-(1*H*-indol-3-yl)ethyl)piperidine-2-carboxamide, 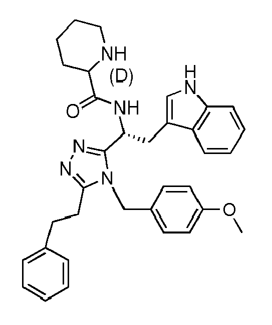
**compound 50** (*R*)-*N*-(1-(4-(4-methoxybenzyl)-5-phenethyl-4*H*-1,2,4-triazol-3-yl)-2-(1*H*-indol-3-yl)ethyl)-2-aminoacetamide, 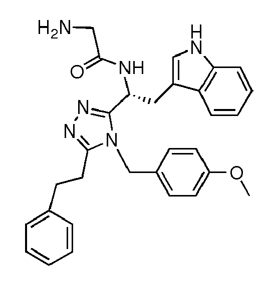
**compound 51** (*R*)-*N*-(1-(4-(4-methoxybenzyl)-5-phenethyl-4*H*-1,2,4-triazol-3-yl)-2-(1*H*-indol-3-yl)ethyl)-2-(pyridin-2-yl)acetamide, 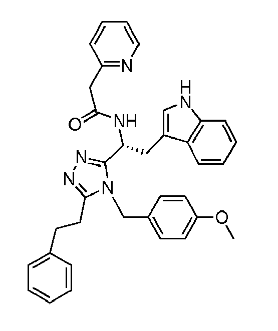
**compound 52** (*R*)-*N*-(1-(4-(4-methoxybenzyl)-5-phenethyl-4*H*-1,2,4-triazol-3-yl)-2-(1*H*-indol-3-yl)ethyl)-2-(pyridin-4-yl)acetamide, 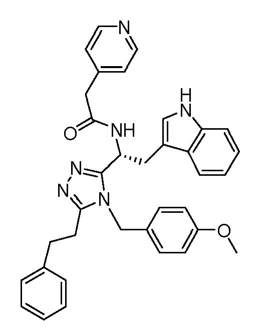
**compound 53** (*R*)-*N*-(1-(4-(4-methoxybenzyl)-5-phenethyl-4*H*-1,2,4-triazol-3-yl)-2-(1*H*-indol-3-yl)ethyl)cyclohexanecarboxamide, 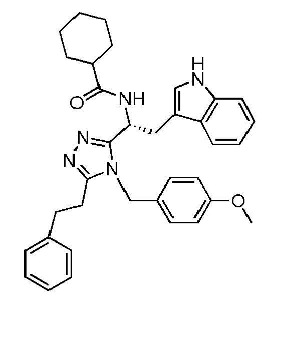
**compound 54** (*R*)-*N*-(1-(5-(2-(1*H*-indol-3-yl)ethyl)-4-benzyl-4*H*-1,2,4-triazol-3-yl)-2-(1*H*-indol-3-yl)ethyl)piperidine-4-carboxamide, 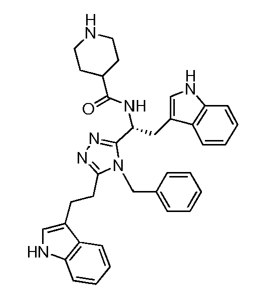
**compound 55** (*R*)-*N*-(1-(5-(2-(1*H*-indol-3-yl)ethyl)-4-benzyl-4*H*-1,2,4-triazol-3-yl)-2-(1*H*-indol-3-yl)ethyl)piperidine-3-carboxamide, 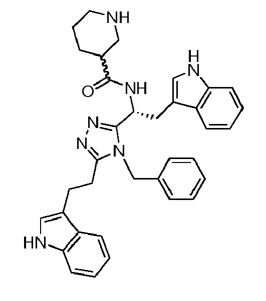
**compound 56** (*R*)-*N*-(1-(4-(4-methoxybenzyl)-5-phenethyl-4*H*-1,2,4-triazol-3-yl)-2-(1*H*-indol-3-yl)ethyl)-3-aminopropanamide, 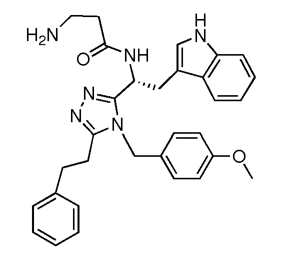
**compound 57** (*S*)-*N*-((*R*)-1-(4-(4-methoxybenzyl)-5-phenethyl-4*H-*1,2,4-triazol-3-yl)-2-(1*H*-indol-3-yl)ethyl)-2-aminopropanamide, 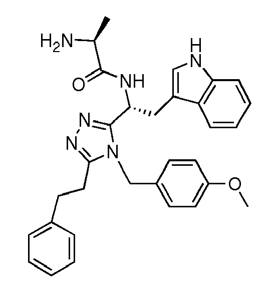
**compound 58** (*R*)*-N-*(1-(4-(4-methoxybenzyl)-5-phenethyl-4*H*-1,2,4-triazol-3-yl)-2-(1*H*-indol-3-yl)ethyl)-2-(pyridin-3-yl)acetamide, 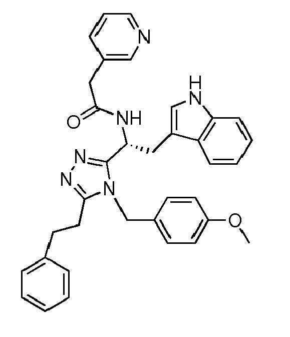
**compound 59** (*R*)*-N-*(1-(4-(4-methoxybenzyl)-5-phenethyl-4*H*-1,2,4-triazol-3-yl)-2-(1*H*-indol-3-yl)ethyl)-3-(pyridin-3-yl)propanamide, 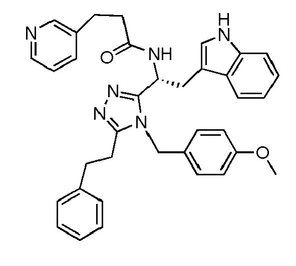
**compound 60** (*R*)*-N-*(1-(5-(2-(1*H*-indol-3-yl)ethyl)-4-benzyl-4*H*-1,2,4-triazol-3-yl)-2-(1*H*-indol-3-yl)ethyl)-2-(pyridin-2-yl)acetamide, 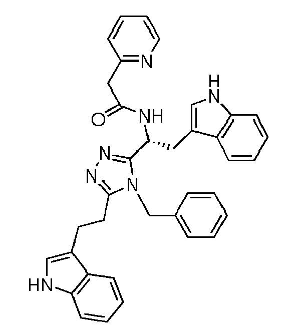
**compound 61** (*R*)*-N*-(1-(5-(2-(1*H*-indol-3-yl)ethyl)-4-(2,4-dimethoxybenzyl)-4*H-*1,2,4-triazol-3-yl)-2-(1*H*-indol-3-yl)ethyl)-2-(pyridin-2-yl)acetamide, 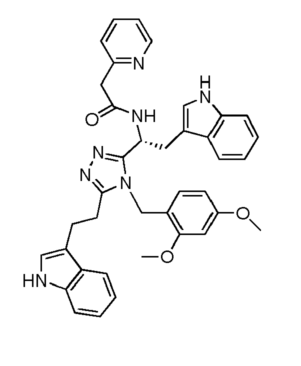
**compound 62** (*R*)-*N*-(1-(4-(2,4-dimethoxybenzyl)-5-phenethyl-4*H*-1,2,4-triazol-3-yl)-2-(1*H*-indol-3-yl)ethyl)piperidine-4-carboxamide, 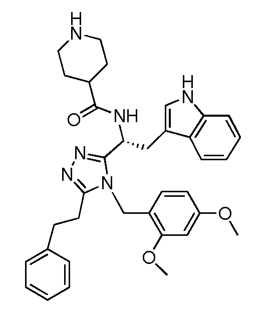
**compound 63** (*R*)-*N*-((*R*)-1-(4-(2,4-dimethoxybenzyl)-5-phenethyl-4*H*-1,2,4-triazol-3-yl)-2-(1*H*-indol-3-yl)ethyl)piperidine-2-carboxamide, 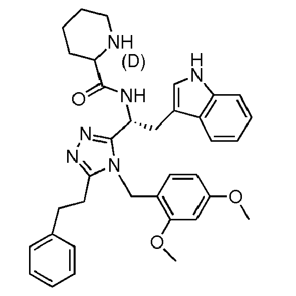
**compound 64** (*R*)-*N*-(1-(4-(2,4-dimethoxybenzyl)-5-phenethyl-4*H*-1,2,4-triazol-3-yl)-2-(1*H*-indol-3-yl)ethyl)picolinamide, 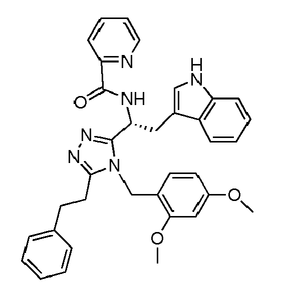
**compound 65** (*R*)-*N*-(1-(4-(2,4-dimethoxybenzyl)-5-phenethyl-4*H*-1,2,4-triazol-3-yl)-2-(1*H*-indol-3-yl)ethyl)isonicotinamide, 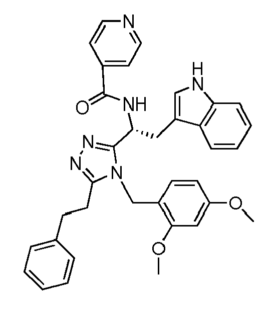
**compound 66** (*R*)-*N*-(1-(4-(2,4-dimethoxybenzyl)-5-phenethyl-4*H*-1,2,4-triazol-3-yl)-2-(1*H*-indol-3-yl)ethyl)pyrazine-2-carboxamide, 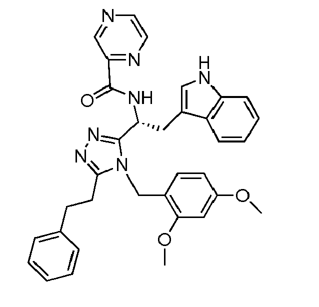
**compound 67** (*R*)-*N*-1-(4-(2,4-dimethoxybenzyl)-5-phenethyl-4*H*-1,2,4-triazol-3-yl)-2-(1*H*-indol-3-yl)ethyl)piperazine-2-carboxamide, 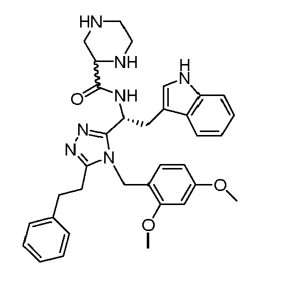
**compound 68** (*S*)-*N*-((*R*)-1-(4-(2,4-dimethoxybenzyl)-5-phenethyl-4*H*-1,2,4-triazol-3-yl)-2-(1*H*-indol-3-yl)ethyl)pyrrolidine-2-carboxamide, 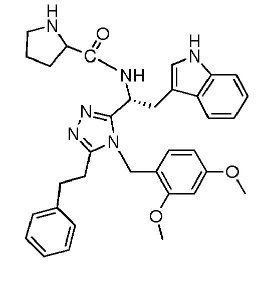
**compound 69** (*R*)-*N*-(1-(5-(2-(1*H-*indol-3-yl)ethyl)-4-(2,4-dimethoxybenzyl)-4*H-*1,2,4-triazol-3-yl)-2-(1*H*-indol-3-yl)ethyl)-2-aminoacetamide, 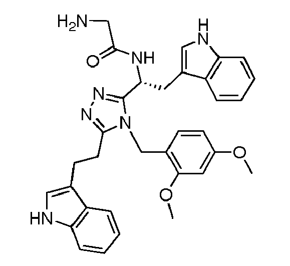
**compound 70** (*S*)-*N-*((*R*)-1-(5-(2-(1*H*-indol-3-yl)ethyl)-4-(2,4-dimethoxybenzyl)-4*H-*1,2,4-triazol-3-yl)-2-(1*H*-indol-3-yl)ethyl)pyrrolidine-2-carboxamide, 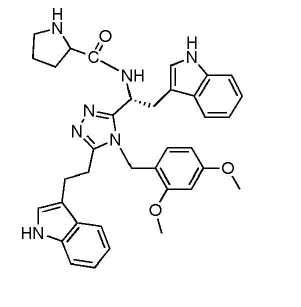
**compound 71** (*R*)-*N*-(1-(5-(2-(1*H-*indol-3-yl)ethyl)-4-(2,4-dimethoxybenzyl)-4*H-*1,2,4-triazol-3-yl)-2-(1*H*-indol-3-yl)ethyl)pyrazine-2-carboxamide, 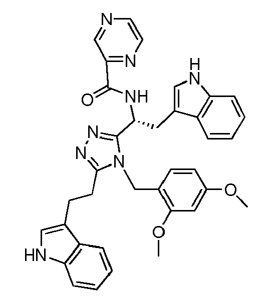
**compound 72** (*R*)-*N*-1-(5-(2-(1*H*-indol-3-yl)ethyl)-4-(2,4-dimethoxybenzyl)-4*H-*1,2,4-triazol-3-yl)-2-(1*H*-indol-3-yl)ethyl)piperazine-2-carboxamide, 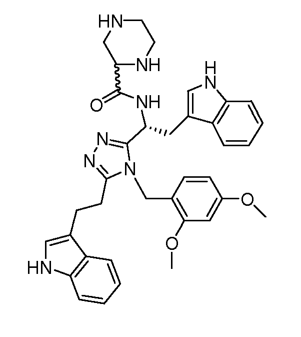
**compound 73** (*R*)-*N*-(1-(5-(2-(1*H-*indol-3-yl)ethyl)-4-(2,4-dimethoxybenzyl)-4*H-*1,2,4-triazol-3-yl)-2-(1*H*-indol-3-yl)ethyl)picolinamide, 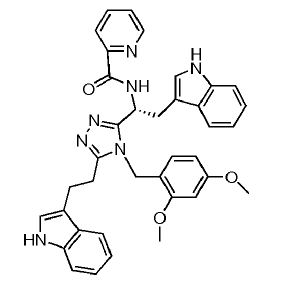
**compound 74** (*R*)-1-(5-(2-(1*H*-indol-3-yl)ethyl)-4-(2,4-dimethoxybenzyl)-4*H*-1,2,4-triazol-3-yl)-2-(1*H*-indol-3-yl)ethanamine, 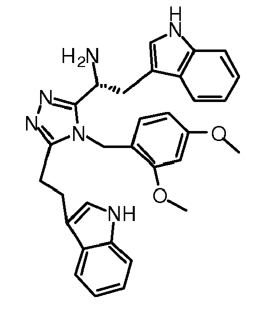
**compound 75** (*R*)-*N*-(1-(4-(2,4-dimethoxybenzyl)-5-phenethyl-4*H*-1,2,4-triazol-3-yl)-2-(1*H*-indol-3-yl)ethyl)-2-aminoacetamide, 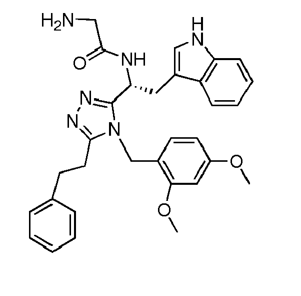
**compound 76** (*R*)-*N*-(1-(4-(4-methoxybenzyl)-5-phenethyl-4*H*-1,2,4-triazol-3-yl)-2-(1*H*-indol-3-yl)ethyl)pyrazine-2-carboxamide, 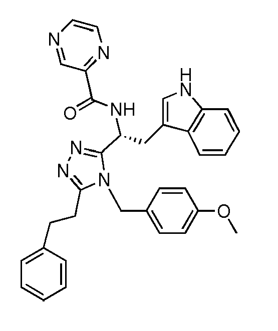
**compound 77** *(R)-N-*(1-(4-(4-methoxybenzyl)-5-phenethyl-4*H*-1,2,4-triazol-3-yl)-2-(1*H*-indol-3-yl)ethyl)isonicotinamide, 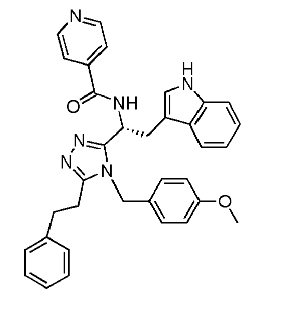
**compound 78** (*R*)-*N*-1-(4-(4-methoxybenzyl)-5-phenethyl-4*H*-1,2,4-triazol-3-yl)-2-(1*H*-indol-3-yl)ethyl)piperazine-2-carboxamide, 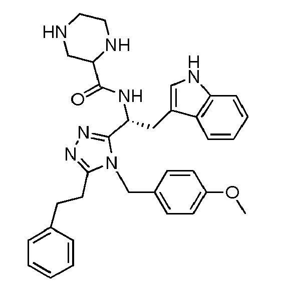
**compound 79** (*R*)-*N*-(1-(4-(4-methoxybenzyl)-5-phenethyl-4*H*-1,2,4-triazol-3-yl)-2-(1*H*-indol-3-yl)ethyl)picolinamide, 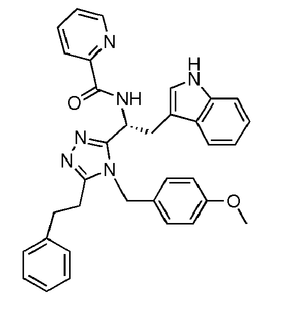
**compound 80** (*R*)-*N*-(1-(5-(2-(1*H*-indol-3-yl)ethyl)-4-(4-methoxybenzyl)-4*H-*1,2,4-triazol-3-yl)-2-(1*H*-indol-3-yl)ethyl)picolinamide, 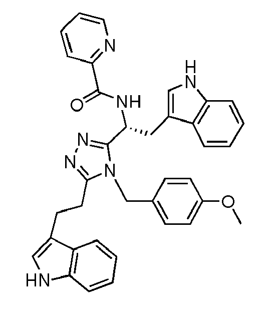
**compound 81** (*R*)-*N*-1-(5-(2-(1*H*-indol-3-yl)ethyl)-4-(4-methoxybenzyl)-4*H*-1,2,4-triazol-3-yl)-2-(1*H*-indol-3-yl)ethyl)piperazine-2-carboxamide, 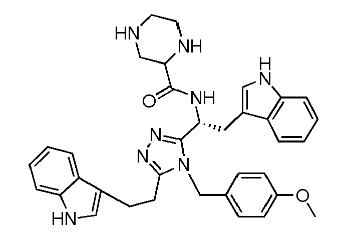
**compound 82** (*R*)-*N*-(1-(4-(4-ethylbenzyl)-5-phenethyl-4*H*-1,2,4-triazol-3-yl)-2-(1*H*-indol-3-yl)ethyl)-2-(pyridin-2-yl)acetamide, 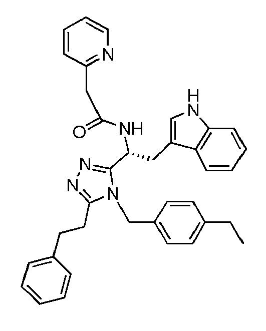
**compound 83** (*R*)-*N*-(1-(4-(4-ethylbenzyl)-5-phenethyl-4*H*-1,2,4-triazol-3-yl)-2-(1*H*-indol-3-yl)ethyl)piperidine-4-carboxamide, 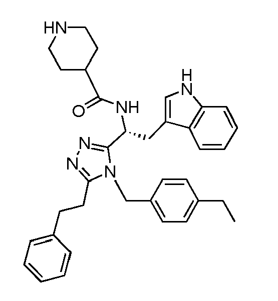
**compound 84** (*R*)-*N*-1-(4-(4-ethylbenzyl)-5-phenethyl-4*H*-1,2,4-triazol-3-yl)-2-(1*H-*indol-3-yl)ethyl)piperazine-2-carboxamide, 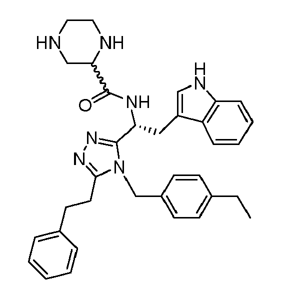
**compound 85** (*R*)-*N*-(1-(4-(4-ethylbenzyl)-5-phenethyl-4*H*-1,2,4-triazol-3-yl)-2-(1*H*-indol-3-yl)ethyl)pyrazine-2-carboxamide, 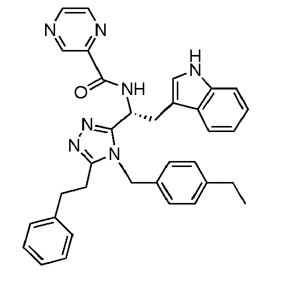
**compound 86** (*R*)*-N-*(1-(5-(2-(1*H-*indol-3-yl)ethyl)-4-(2,4-dimethoxybenzyl)-4*H-*1,2,4-triazol-3-yl)-2-(1*H*-indol-3-yl)ethyl)-2-cis-aminocyclohexanecarboxamide, 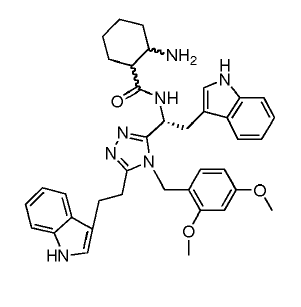
**compound 87** (*S*)-*N*-((*R*)-1-(5-(2-(1*H*-indol-3-yl)ethyl)-4-(4-methoxybenzyl)-4*H-*1,2,4-triazol-3-yl)-2-(1*H*-indol-3-yl)ethyl)piperidine-3-carboxamide, 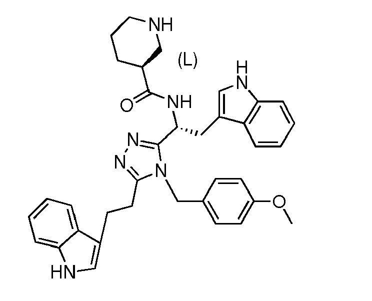
**compound 88** (*R*)-*N*-((*R*)-1-(5-(2-(1*H*-indol-3-yl)ethyl)-4-(4-methoxybenzyl)-4*H-*1,2,4-triazol-3-yl)-2-(1*H*-indol-3-yl)ethyl)piperidine-2-carboxamide, 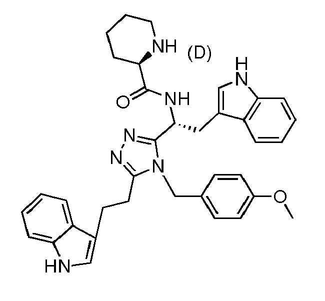
**compound 89** (*S*)-*N*-((*R*)-1-(5-(2-(1*H*-indol-3-yl)ethyl)-4-(4-methoxybenzyl)-4*H-*1,2,4-triazol-3-yl)-2-(1*H*-indol-3-yl)ethyl)pyrrolidine-2-carboxamide, 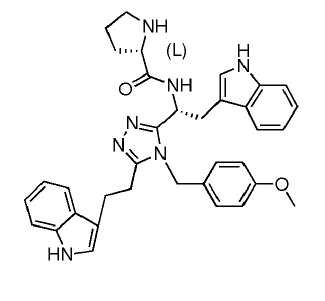
**compound 90** (*R*)-*N*-((*R*)-1-(5-(2-(1*H*-indol-3-yl)ethyl)-4-(4-methoxybenzyl)-4*H-*1,2,4-triazol-3-yl)-2-(1*H*-indol-3-yl)ethyl)pyrrolidine-2-carboxamide, 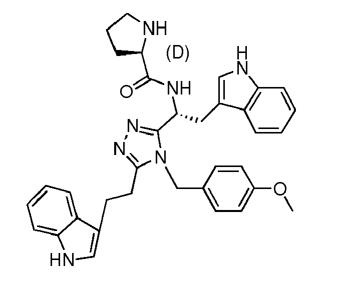
**compound 91** (*R*)-*N*-(1-(5-(2-(1*H*-indol-3-yl)ethyl)-4-(4-methoxybenzyl)-4*H*-1,2,4-triazol-3-yl)-2-(1*H*-indol-3-yl)ethyl)-2-(pyridin-2-yl)acetamide, 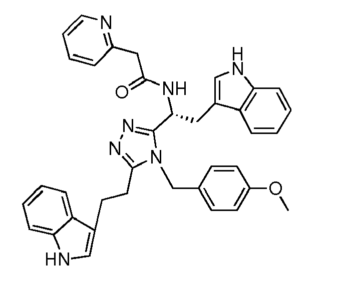
**compound 92** *(R)-N-*(1-(5-(2-(1*H*-indol-3-yl)ethyl)-4-(4-bromobenzyl)-4*H*-1,2,4-triazol-3-yl)-2-(1*H-*indol-3-yl)ethyl)-2-amino-2-methylpropanamide, 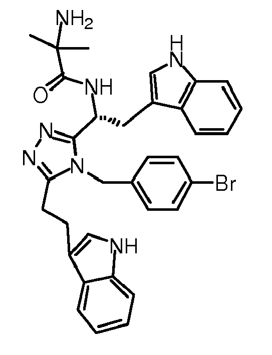
**compound 93** *(R)-N-*(1-(5-(2-(1*H-*indol-3-yl)ethyl)-4-(4-methoxybenzyl)-4*H-*1,2,4-triazol-3-yl)-2-phenylethyl)-2-amino-2-methylpropanamide, 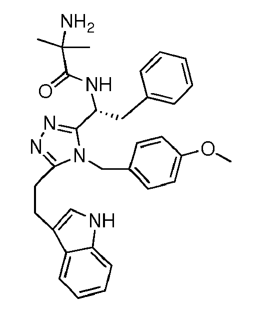
**compound 94** (*R*)-*N*-(2-(1*H*-indol-3-yl)-1-(5-phenethyl-4-(thiophen-2-ylmethyl)-4*H*-1,2,4-triazol-3-yl)ethyl)piperidine-4-carboxamide, 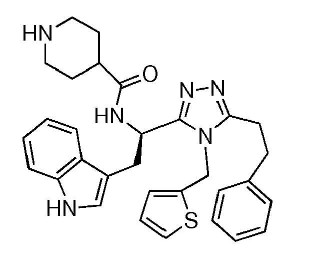
**compound 95** (*R*)*-N-*(1-(4-(2-(1*H*-indol-3-yl)ethyl)-4*H*-1,2,4-triazol-3-yl)-2-(1*H-*indol-3-yl)ethyl)-2-amino-2-methylpropanamide, 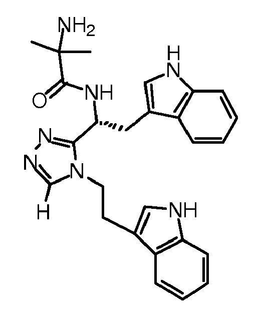
**compound 96** (*R*)-*N*-(1-(5-((1*H*-indol-3-yl)methyl)-4-methyl-4*H*-1,2,4-triazol-3-yl)-2-(1*H*-indol-3-yl)ethyl)-2-amino-2-methylpropanamide, 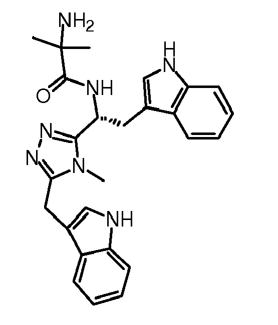
**compound 97** (*R*)-*N*-(1-(5-(2-(1*H*-indol-3-yl)ethyl)-4-methyl-4*H*-1,2,4-triazol-3-yl)-2-(1*H*-indol-3-yl)ethyl)-2-amino-2-methylpropanamide, 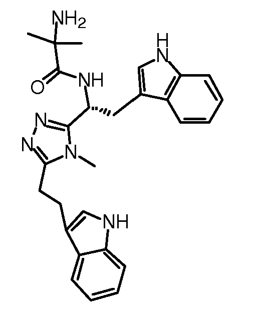
**compound 98** (*R*)-*N*-(1-(5-((1*H*-indol-3-yl)methyl)-4*H*-1,2,4-triazol-3-yl)-2-(1*H-*indol-3-yl)ethyl)-2-amino-2-methylpropanamide, 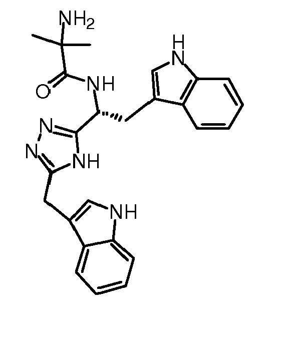
**compound 99** (*R*)-*N*-(1-(5-((1*H*-indol-3-yl)methyl)-4-(2,4-dimethoxybenzyl)-4*H-*1,2,4-triazol-3-yl)-2-(1*H*-indol-3-yl)ethyl)-2-amino-2-methylpropanamide, 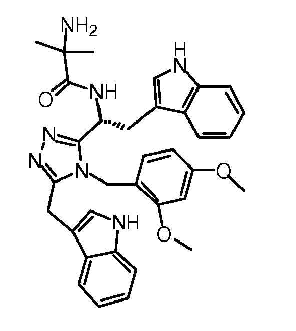
**compound 100** (*R*)-*N-*(1-(4-(2,4-dimethoxybenzyl)-5-methyl-4H 1,2,4-triazol-3-yl)-2-(1*H*-indol-3-yl)ethyl)-2-amino-2-methylpropanamide, 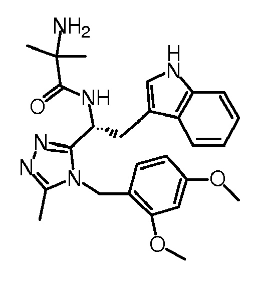
**compound 101** (*R*)-*N*-(1-(5-((1*H-*indol-3-yl)methyl)-4-(4-methoxybenzyl)-4*H*-1,2,4-triazol-3-yl)-2-(1*H*-indol-3-yl)ethyl)-2-amino-2-methylpropanamide, 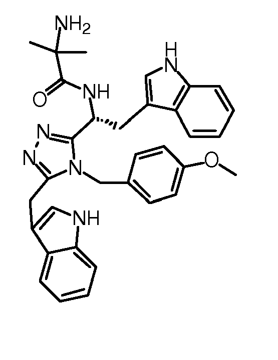
**compound 102** (*R*)-*N*-(1-(4-(2,4-dimethoxybenzyl)-5-benzyl-4*H*-1,2,4-triazol-3-yl)-2-(1*H*-indol-3-yl)ethyl)-2-amino-2-methylpropanamide, 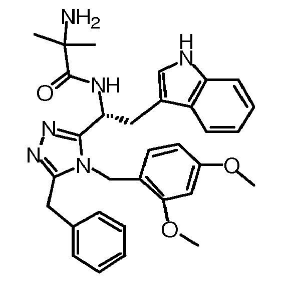
**compound 103** *(R)-N-*(1-(5-(3-(1*H*-indol-3-yl)propyl)-4-(2,4-dimethoxybenzyl)-4*H-*1,2,4-triazol-3-yl)-2-(1*H*-indol-3-yl)ethyl)-2-amino-2-methylpropanamide, 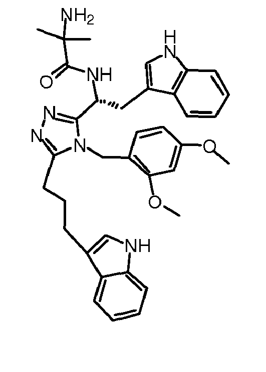
**compound 104** (*R*)*-N-*(1-(5-((1*H*-indol-3-yl)methyl)-4-phenethyl-4*H*-1,2,4-triazol-3-yl)-2-(1*H*-indol-3-yl)ethyl)-2-amino-2-methylpropanamide, 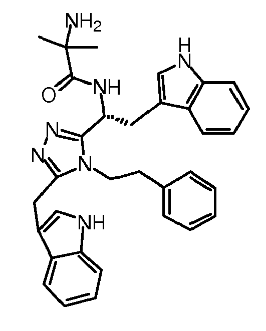
**compound 105** (*R*)-*N*-(1-(5-benzyl-4-phenethyl-4*H*-1,2,4-triazol-3-yl)-2-(1*H*-indol-3-yl)ethyl)-2-amino-2-methylpropanamide, 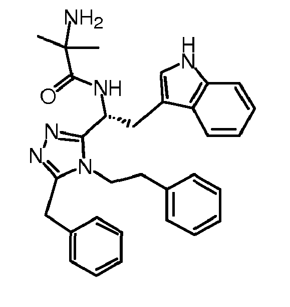
**compound 106** (*R*)-*N*-(1-(5-benzyl-4-(2,2-diphenylethyl)-4*H*-1,2,4-triazol-3-yl)-2-(1*H*-indol-3-yl)ethyl)-2-amino-2-methylpropanamide, 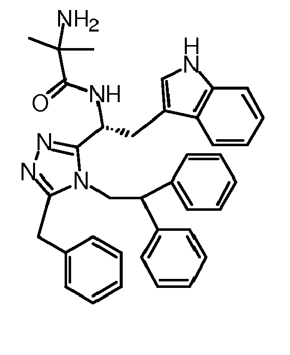
**compound 107** (*R*)*-N-*(1-(5-(2-(1*H-*indol-3-yl)ethyl)-4-(2,2-diphenylethyl)-4*H-*1,2,4-triazol-3-yl)-2-(1*H*-indol-3-yl)ethyl)-2-amino-2-methylpropanamide, 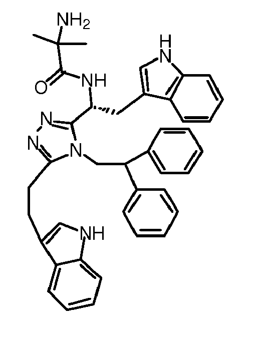
**compound 108** *(R)-N*-(1-(4-(3,5-dimethoxybenzyl)-5-benzyl-4*H*-1,2,4-triazol-3-yl)-2-(1*H*-indol-3-yl)ethyl)-2-amino-2-methylpropanamide, 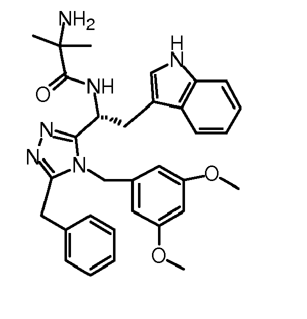
**compound 109** *(R)-N-*(1-(4,5-dibenzyl-4*H-*1,2,4-triazol-3-yl)-2-(1*H*-indol-3-yl)ethyl)-2-amino-2-methylpropanamide, 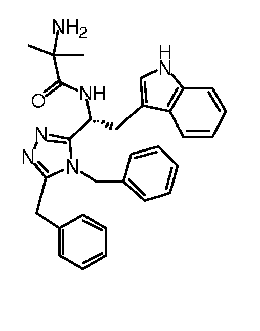
**compound 110** *(R)-*N-(1-(5-benzyl-4-hexyl-4*H*-1,2,4-triazol-3-yl)-2-(1*H*-indol-3-yl)ethyl)-2-amino-2-methylpropanamide, 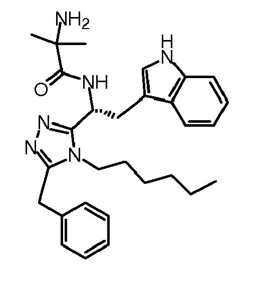
**compound 111** (*R*)-*N*-(1-(4-(2-(1*H*-indol-3-yl)ethyl)-5-benzyl-4*H*-1,2,4-triazol-3-yl)-2-(1*H*-indol-3-yl)ethyl)-2-amino-2-methylpropanamide, 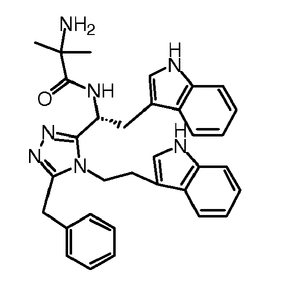
**compound 112** (*S*)-*N*-(1-(4-(2,4-dimethoxybenzyl)-5-benzyl-4*H*-1,2,4-triazol-3-yl)-2-(1*H*-indol-3-yl)ethyl)-2-amino-2-methylpropanamide, 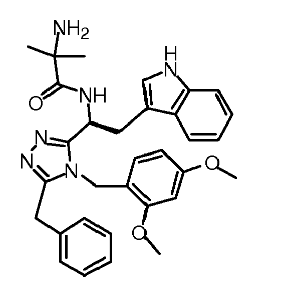
**compound 113** (*R*)-*N*-(1-(4-(3,5-dimethoxybenzyl)-5-phenethyl-4*H*-1,2,4-triazol-3-yl)-2-(1*H*-indol-3-yl)ethyl)-2-amino-2-methylpropanamide, 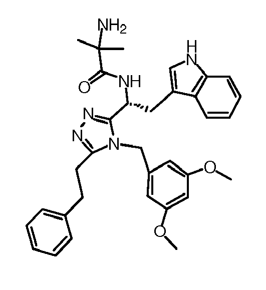
**compound 114** (*R*)*-N-*(1-(4-(4-bromobenzyl)-5-benzyl-4*H*-1,2,4-triazol-3-yl)-2-(1*H-*indol-3-yl)ethyl)-2-amino-2-methylpropanamide, 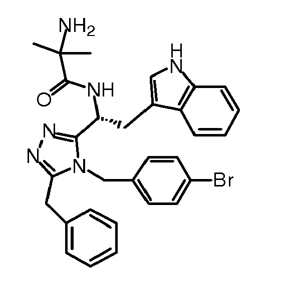
**compound 115** (*R*)-*N*-(1-(4-(2-methoxybenzyl)-5-benzyl-4*H*-1,2,4-triazol-3-yl)-2-(1*H*-indol-3-yl)ethyl)-2-amino-2-methylpropanamide, 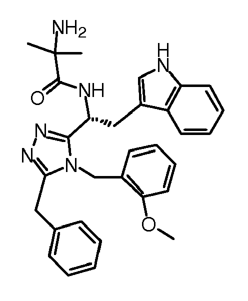
**compound 116** (*S*)-*N*-(1-(4-(2,4-dimethoxybenzyl)-5-phenethyl-4*H*-1,2,4-triazol-3-yl)-2-(1*H*-indol-3-yl)ethyl)-2-amino-2-methylpropanamide, 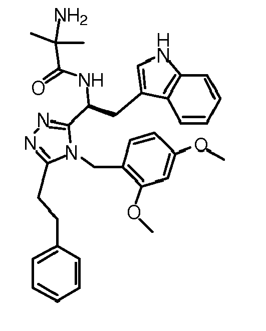
**compound 117** (*R*)-*N*-(1-(4,5-diphenethyl-4*H-*1,2,4-triazol-3-yl)-2-(1*H*-indol-3-yl)ethyl)-2-amino-2-methylpropanamide, 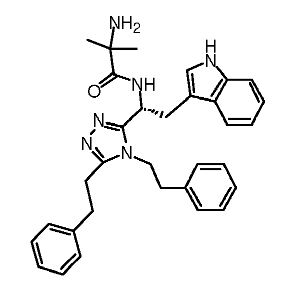
**compound 118** (*R)-N-*(1-(4-(3,4-dichlorobenzyl)-5-benzyl-4*H*-1,2,4-triazol-3-yl)-2-(1*H*-indol-3-yl)ethyl)-2-amino-2-methylpropanamide, 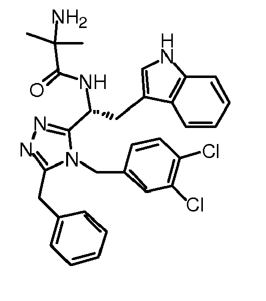
**compound 119** (*R*)*-*1-(5-(2-(1*H*-indol-3-yl)ethyl)-4-(2,4-dimethoxybenzyl)-4*H*-1,2,4-triazol-3-yl)-2-(1*H*-indol-3-yl)ethanamine, 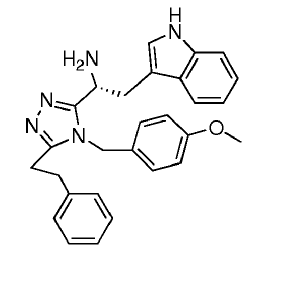
**compound 120** (*R*)-*N*-(1-(4-(4-methoxybenzyl)-5-benzyl-4*H*-1,2,4-triazol-3-yl)-2-phenylethyl)-2-amino-2-methylpropanamide, 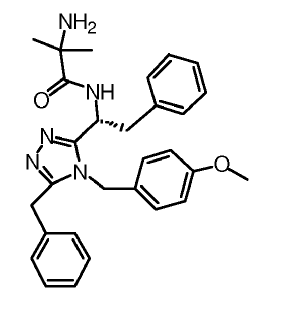
**compound 121** (*R*)-*N*-(1-(4-(4-f!uorobenzy!)-5-phenethyl-4*H*-1,2,4-triazol-3-yi)-2-(1*H*-indol-3-yl)ethyl)-2-amino-2-methylpropanamide, 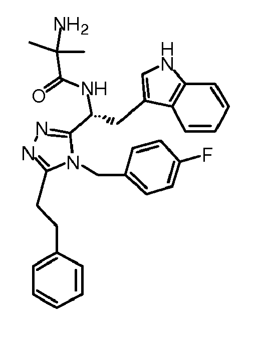
**compound 122** *(R)-N*-(1-(4-(3,4-dichlorobenzyl)-5-phenethyl-4*H*-1,2,4-triazol-3-yl)-2-(1*H*-indol-3-yl)ethyl)-2-amino-2-methylpropanamide, 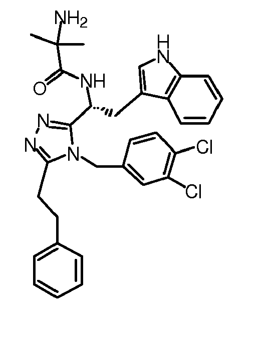
**compound 124** (*R*)*-N*-(1-(4-(4-methylbenzyl)-5-benzyl-4*H*-1,2,4-triazol-3-yl)-2-(1*H-*indol-3-yl)ethyl)-2-amino-2-methylpropanamide, 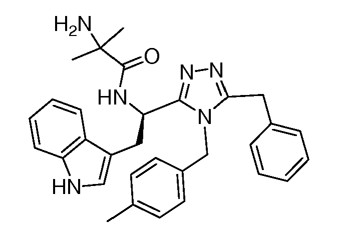
**compound 125** (*S*)-*N*-(1-(4-(4-methoxybenzyl)-5-(3-phenylpropyl)-4*H-*1,2,4-triazol-3-yl)-2-(1*H*-indol-3-yl)ethyl)-2-amino-2-methylpropanamide, 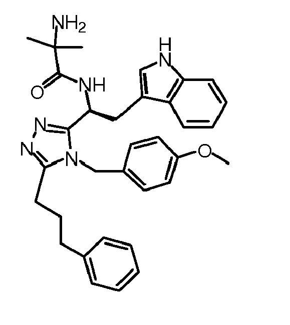
**compound 126** (*S*)-*N*-(1-(4-(4-methoxybenzyl)-5-benzyl-4*H*-1,2,4-triazol-3-yl)-2-(1*H*-indol-3-yl)ethyl)-2-amino-2-methylpropanamide, 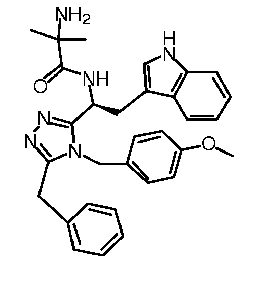
**compound 128** *N*-((*R*)-1-(4-(4-nitrobenzyl)-5-phenethyl-4*H*-1,2,4-triazol-3-yl)-2-(1*H*-indol-3-yl)ethyl)-2-amino-2-methylpropanamide, 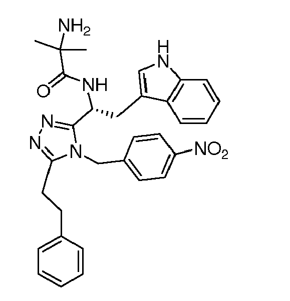
**compound 129** (*S*)-*N*-(1-(4-(4-methoxybenzyl)-5-phenethyl-4*H-*1,2,4-triazol-3-yl)-2-(1*H*-indol-3-yl)ethyl)-2-amino-2-methylpropanamide, 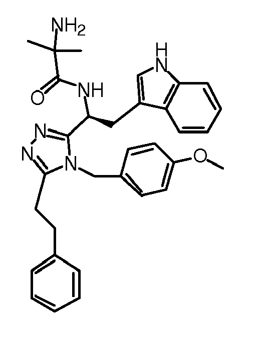
**compound 130** (*R*)-*N*-(1-(4-(4-methoxyphenethyl)-5-phenethyl-4*H*-1,2,4-triazol-3-yl)-2-(1*H*-indol-3-yl)ethyl)-2-amino-2-methylpropanamide, 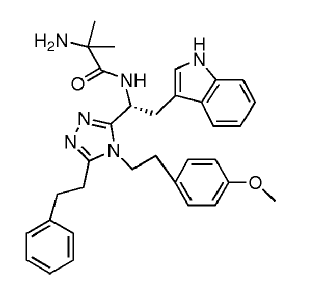
**compound 131** (*R*)-*N*-(2-(1*H*-indol-3-yl)-1-(5-phenethyl-4-(thiophen-2-ylmethyl)-4*H*-1,2,4-triazol-3-yl)ethyl)-2-amino-2-methylpropanamide, 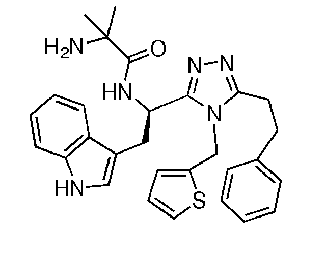
**compound 132** (*R*)*-N*-(2-(1*H*-indol-3-yl)-1-(5-phenethyl-4-(pyridin-2-ylmethyl)-4*H-*1,2,4-triazol-3-yl)ethyl)-2-amino-2-methylpropanamide, 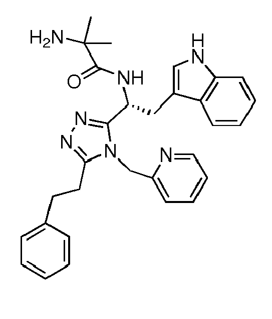
**compound 133** (*R*)-*N*-(2-(1*H*-indol-3-yl)-1-(5-phenethyl-4-(pyridin-2-ylmethyl)-4*H-*1,2,4-triazol-3-yl)ethyl)piperidine-3-carboxamide, 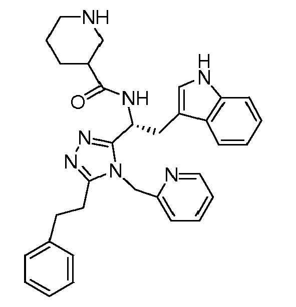
**compound 134** (*S*)-*N*-((*R*)-1-(4-(4-ethylbenzyl)-5-phenethyl-4*H*-1,2,4-triazol-3-yl)-2-(1*H*-indol-3-yl)ethyl)pyrrolidine-2-carboxamide, 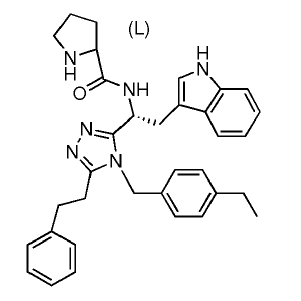
**compound 135** *N*-((*R*)-1-(4-(4-ethylbenzyl)-5-phenethyl-4*H*-1,2,4-triazol-3-yl)-2-(1*H*-indol-3-yl)ethyl)-2-aminoacetamide, 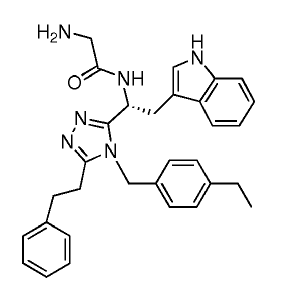
**compound 136** *N*-((*R*)-1-(5-(2-(1*H*-indol-3-yl)ethyl)-4-(4-methoxybenzyl)-4*H*-1,2,4-triazol-3-yl)-2-(1*H*-indol-3-yl)ethyl)-2-(pyridin-4-yl)acetamide, 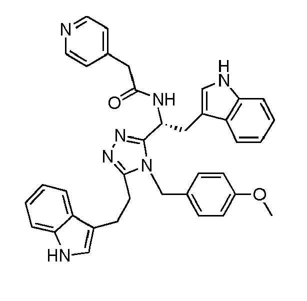
**compound 137** (2*R*)-*N*-((*R*)-1-(4-(4-ethylbenzyl)-5-phenethyl-4*H*-1,2,4-triazol-3-yl)-2-(1*H*-indol-3-yl)ethyl)piperidine-2-carboxamide, 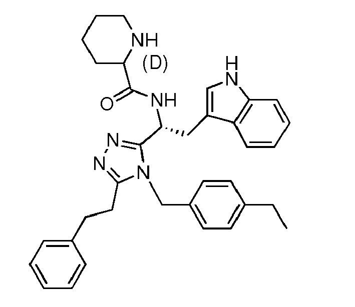
**compound 138** *N*-((*R*)-1-(4-(4-ethylbenzyl)-5-phenethyl-4*H*-1,2,4-triazol-3-yl)-2-(1*H*-indol-3-yl)ethyl)picolinamide, 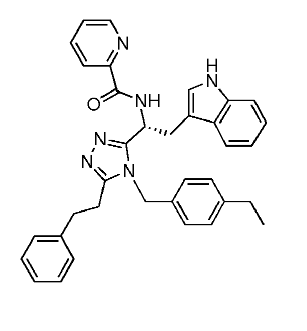
**compound 139** *N*-((*R*)-1-(4-(4-ethylbenzyl)-5-phenethyl-4*H*-1,2,4-triazol-3-yl)-2-(1*H*-indol-3-yl)ethyl)-2-aminopyridine-3-carboxamide, 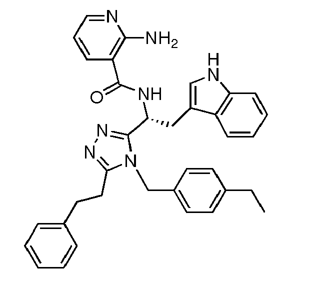
**compound 140** (2*S*)-*N*-((*R*)-1-(4-(4-ethylbenzyl)-5-phenethyl-4*H*-1,2,4-triazol-3-yl)-2-(1*H*-indol-3-yl)ethyl)-2-aminopropanamide, 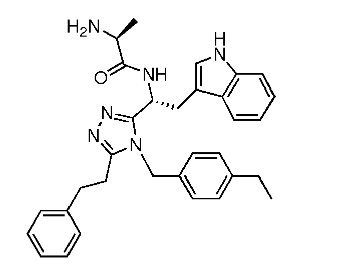
**compound 141** *N*-((*R*)-1-(4-(4-ethylbenzyl)-5-phenethyl-4*H*-1,2,4-triazol-3-yl)-2-(1*H*-indol-3-yl)ethyl)isonicotinamide, 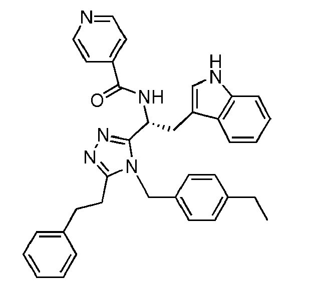
**compound 142** *N*-((*R*)-2-(1*H*-indol-3-yl)-1-(5-phenethyl-4-phenyl-4*H*-1,2,4-triazol-3-yl)ethyl)piperidine-4-carboxamide, 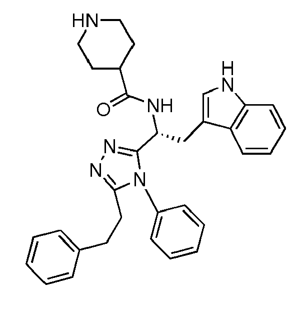
**compound 143** (2*S*)-*N*-((*R*)-2-(1*H*-indol-3-yl)-1-(5-phenethyl-4-phenyl-4*H*-1,2,4-triazol-3-yl)ethyl)pyrrolidine-2-carboxamide, 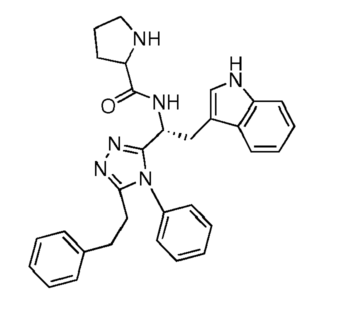
**compound 144** *N*-((*R*)-2-(1*H*-indol-3-yl)-1-(5-phenethyl-4-phenyl-4*H*-1,2,4-triazol-3-yl)ethyl)-2-aminoacetamide, 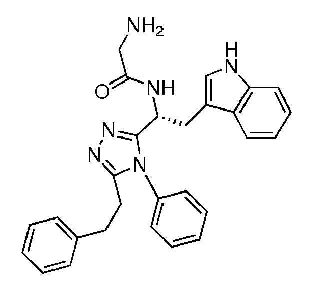
**compound 145** *N-*((*R*)-2-(1*H*-indol-3-yl)-1-(5-phenethyl-4-phenyl-4*H*-1,2,4-triazol-3-yl)ethyl)-2-(pyridin-2-yl)acetamide, 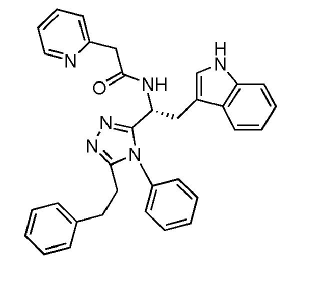
**compound 146** *N*-((*R*)-2-(1*H*-indol-3-yl)-1-(5-phenethyl-4-phenyl-4*H*-1,2,4-triazol-3-yl)ethyl)picolinamide, 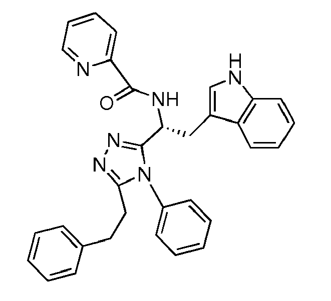
**compound 147** *N*-((*R*)-1-(5-(2-(1*H*-indol-3-yl)ethyl)-4-(4-ethylphenyl)-4*H*-1,2,4-triazol-3-yl)-2-(1*H*-indol-3-yl)ethyl)picolinamide, 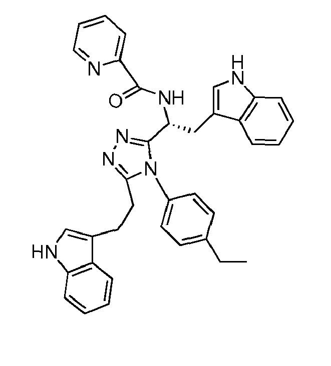
**compound 148** *N*-((*R*)-1-(5-(2-(1*H-*indol-3-yl)ethyl)-4-(4-ethylphenyl)-4*H-*1,2,4-triazol-3-yl)-2-(1*H*-indol-3-yl)ethyl)-2-(pyridin-2-yl)acetamide, 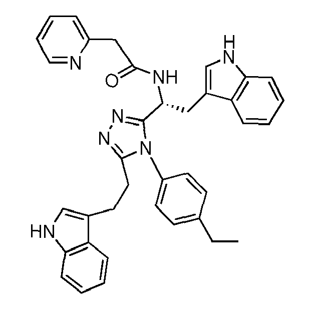
**compound 149** *N*-((*R*)-1-(5-(2-(1*H-*indol-3-yl)ethyl)-4-(4-ethylphenyl)-4*H*-1,2,4-triazol-3-yl)-2-(1*H*-indol-3-yl)ethyl)-2-aminoacetamide, 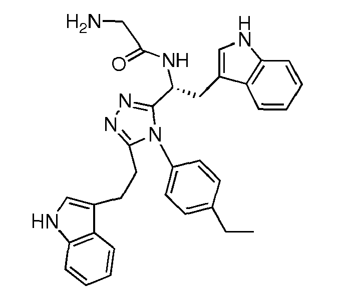
**compound 150** (2*S*)-*N*-((*R*)-1-(5-(2-(1*H*-indol-3-yl)ethyl)-4-(4-ethylphenyl)-4*H-*1,2,4-triazol-3-yl)-2-(1*H*-indol-3-yl)ethyl)pyrrolidine-2-carboxamide, 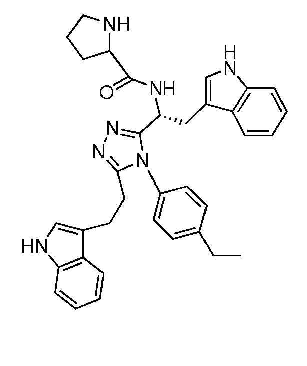
**compound 152** *N*-((*R*)-1-(5-(2-(1*H*-indol-3-yl)ethyl)-4-(4-methoxybenzyl)-4*H*-1,2,4-triazol-3-yl)-2-(1*H*-indol-3-yl)ethyl)-2-aminoacetamide, 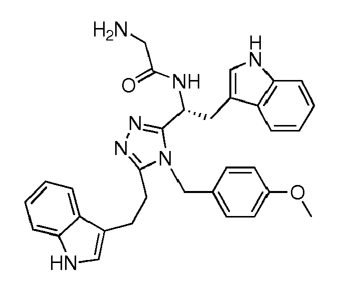
**compound 153** *N*-((*R*)-1-(4-(4-methoxybenzyl)-5-phenethyl-4*H*-1,2,4-triazol-3-yl)-2-(1*H*-indol-3-yl)ethyl)-2-trans-aminocyclohexanecarboxamide, 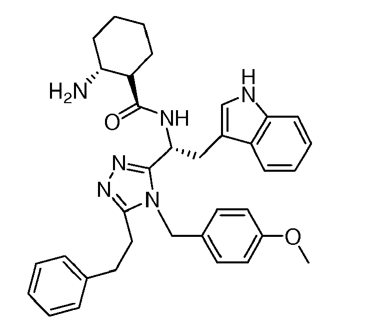
**compound 154** *N*-((*R*)-1-(4-(4-ethylbenzyl)-5-phenethyl-4*H*-1,2,4-triazol-3-yl)-2-(1*H*-indol-3-yl)ethyl)-2-(pyridin-3-yl)acetamide, 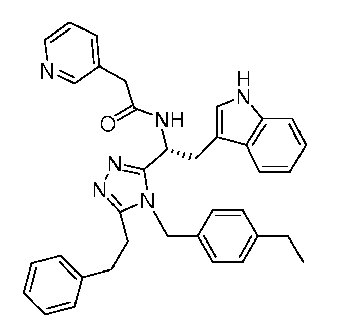
**compound 155** (3*S*)-*N*-((*R*)-1-(4-(4-ethylbenzyl)-5-phenethyl-4*H*-1,2,4-triazol-3-yl)-2-(1*H*-indol-3-yl)ethyl)piperidine-3-carboxamide, 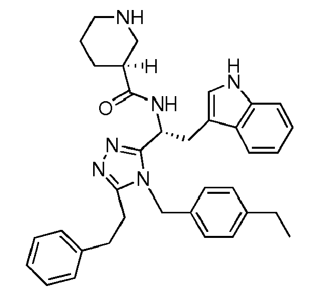
**compound 156** *N*-((*R*)-1-(4-(4-ethylbenzyl)-5-phenethyl-4*H*-1,2,4-triazol-3-yl)-2-(1*H*-indol-3-yl)ethyl)-2-aminobenzamide, 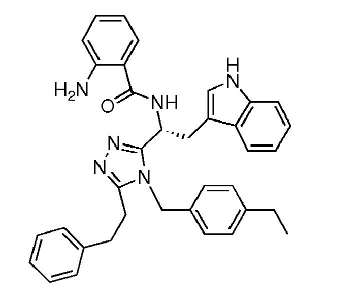
**compound 157** *N*-((*R*)-1-(5-(2-(1*H*-indol-3-yl)ethyl)-4-phenyl-4*H*-1,2,4-triazol-3-yl)-2-(1*H*-indol-3-yl)ethyl)picolinamide, 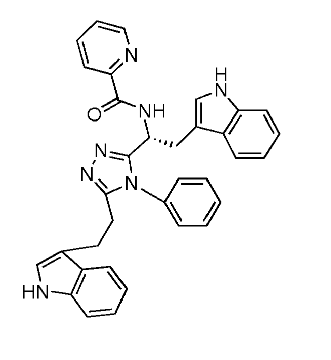
**compound 158** *N*-((*R*)-1-(5-(2-(1*H*-indol-3-yl)ethyl)-4-phenyl-4*H*-1,2,4-triazol-3-yl)-2-(1*H*-indol-3-yl)ethyl)piperidine-4-carboxamide, 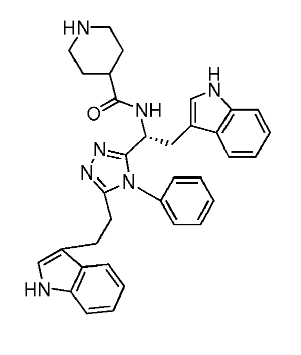
**compound 159** *N*-((*R*)-2-(1*H*-indol-3-yl)-1-(4-(2,4-dimethoxyphenyl)-5-phenethyl-4*H*-1,2,4-triazol-3-yl)ethyl)picolinamide, 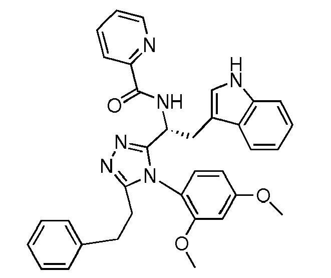
**compound 160** *N*-((*R*)-2-(1*H*-indol-3-yl)-1-(4-(2,4-dimethoxyphenyl)-5-phenethyl-4*H*-1,2,4-triazol-3-yl)ethyl)-2-(pyridin-2-yl)acetamide, 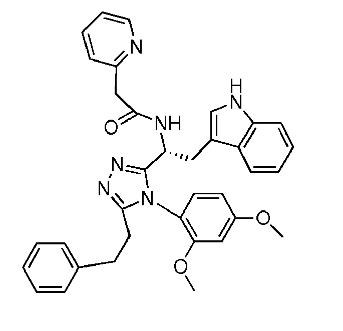
**compound 161** *N*-((*R*)-2-(1*H* indol-3-yl)-1-(4-(2,4-dimethoxyphenyl)-5-phenethyl-4*H*-1,2,4-triazol-3-yl)ethyl)pyrazine-2-carboxamide, 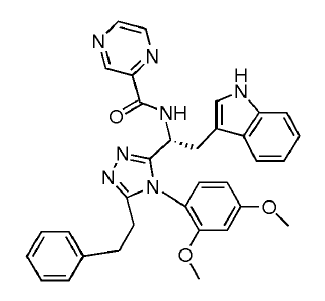
**compound 162** *N*-((*R*)-2-(1*H*-indol-3-yl)-1-(4-(2,4-dimethoxyphenyl)-5-phenethyl-4*H*-1,2,4-triazol-3-yl)ethyl)-2-aminoacetamide, 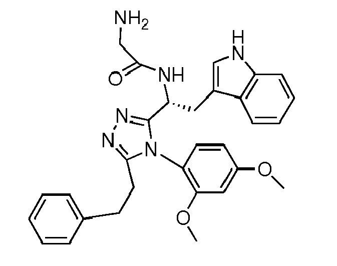
**compound 163** *N*-((*R*)-2-(1 *H*-indol-3-yl)-1-(4-(2,4-dimethoxyphenyl)-5-phenethyl-4*H*-1,2,4-triazol-3-yl)ethyl)piperidine-4-carboxamide, 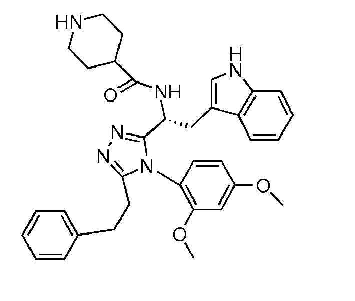
**compound 164** *N*-((*R*)-1-(5-benzyl-4-((pyridin-2-yl)methyl)-4*H*-1,2,4-triazol-3-yl)-2-(1*H*-indol-3-yl)ethyl)picolinamide, 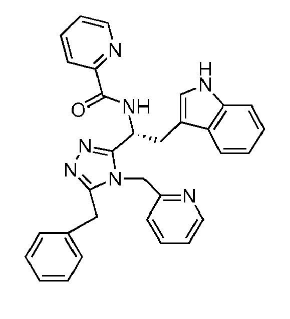
**compound 165** *N*-((*R*)-1-(5-benzyl-4-((pyridin-2-yl)methyl)-4*H*-1,2,4-triazol-3-yl)-2-(1*H*-indol-3-yl)ethyl)-2-amino-acetamide, 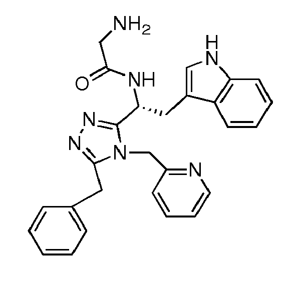
**compound 166** *N*-((*R*)-1-(5-benzyl-4-((pyridin-2-yl)methyl)-4*H*-1,2,4-triazol-3-yl)-2-(1*H*-indol-3-yl)ethyl)piperidine-4-carboxamide, 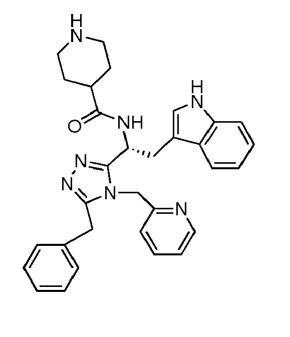
**compound 167** *N*-((*R*)-1-(5-benzy!-4-((pyhd!n-4-yl)methyl)-4*H*-1,2,4-triazol-3-yl)-2-(1*H*-indol-3-yl)ethyl)-2-amino-2-methylpropanamide, 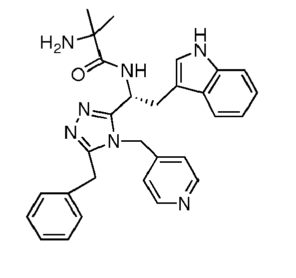
**compound 168** *N***-**((*R*)-1-(5-(4-methoxybenzyl)-4-phenethyl-4*H*-1,2,4-triazol-3-yl)-2-(1*H*-indol-3-yl)ethyl)-2-amino-2-methylpropanamide, 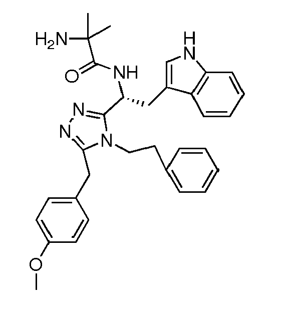
**compound 169** *N*-((*R*)-1-(5-benzyl-4-((pyridin-4-yl)methyl)-4*H*-1,2,4-triazol-3-yl)-2-(1*H*-indol-3-yl)ethyl)picolinamide, 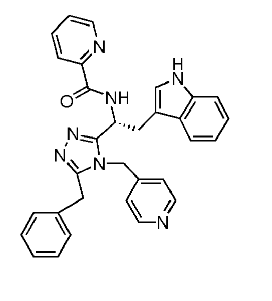
**compound 170** *N*-((*R*)-1-(5-benzyl-4-((pyridin-4-yl)methyl)-4*H*-1,2,4-triazol-3-yl)-2-(1*H*-indol-3-yl)ethyl)2-amino-acetamide, 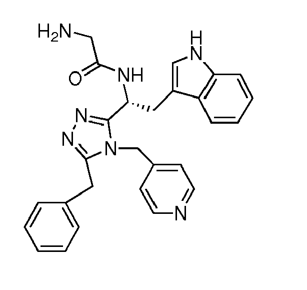
**compound 171** (*R*)-benzyl-3-(2-aminoisobutyramido)-3-(5-(2-(1*H*-indol-3-yl)ethyl)-4-(4-methoxybenzyl)-4*H-*1,2,4-triazol-3-yl)-propanoate, 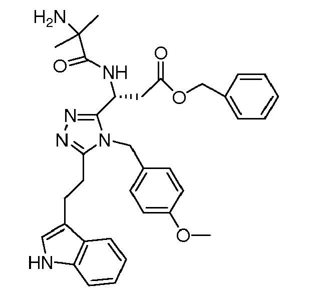
**compound 172** *N*-((*R*)-1-(5-benzyl-4-((pyridin-3-yl)methyl)-4*H*-1,2,4-triazol-3-yl)-2-(1*H*-indol-3-yl)ethyl)-2-amino-2-methylpropanamide, 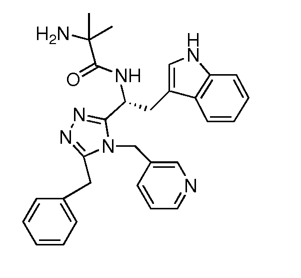
**compound 173** *N*-((*R*)-1-(4-benzyl-5-phenethyl-4*H*-1,2,4-triazol-3-yl)-2-(1*H-*indol-3-yl)ethyl)-2-amino-2-methylpropanamide, 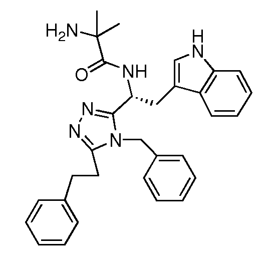
**compound 174** *N*-((*R*)-2-(1*H*-indol-3-yl)-1-(4-methyl-5-phenethyl-4*H*-1,2,4-triazol-3-yl)ethyl)picolinamide, 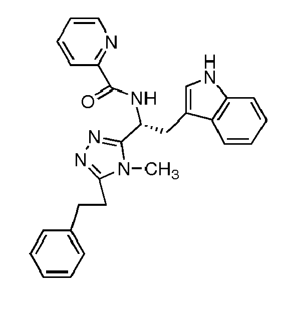
**compound 175** *N*-((*R*)-1-(5-(2-(1*H*-indol-3-yl)ethyl)-4-phenyl-4*H*-1,2,4-triazol-3-yl)-2-(1*H*-indol-3-yl)ethyl)-2-amino-2-methylpropanamide, 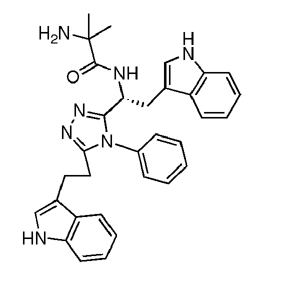
**compound 176** *N*-((*R*)-1*-*(4-(4-methoxybenzyl)-5-phenethyl-4*H*-1,2,4-triazol-3-yl)-2-(1*H*-indol-3-yl)ethyl)benzamide, 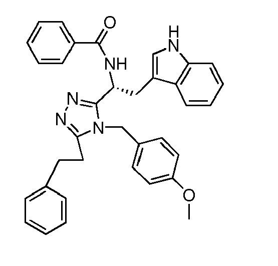
**compound 177** (*R*)-1-(4-(2,4-dimethoxybenzyl)-5-phenethyl-4*H*-1,2,4-triazol-3-yl)-2-(1*H*-indol-3-yl)-N-phenylmethanesulfonylamine, 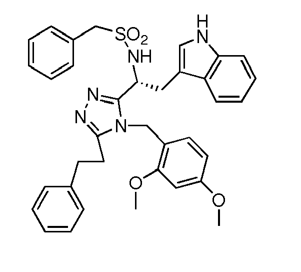
**compound 178** (*R*)-1-(4-(2,4-dimethoxybenzyl)-5-phenethyl-4*H*-1,2,4-triazol-3-yl)-2-(1*H*-indol-3-yl)-N-tosylethanamine, 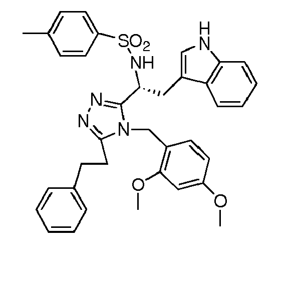
**compound 179** *N*-((*R*)-1-(4-(2,4-dimethoxybenzyl)-5-phenethyl-4*H-*1,2,4-triazol-3-yl)-2-(1*H*-indol-3-yl)ethyl)-2-amino-2-methylpropanamide, 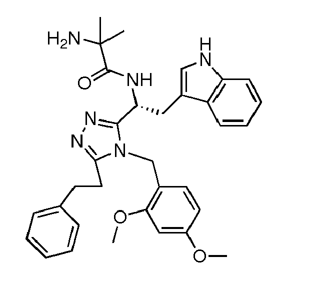
**compound 180** *N*-1-((*R*)-1-(4-(2,4-dimethoxybenzyl)-5-phenethyl-4*H*-1,2,4-triazol-3-yl)-2-(1*H*-indol-3-yl)ethyl)ethane-1,2-diamine, 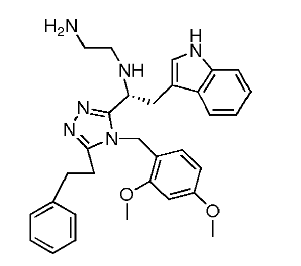
**compound 181** *N-*((*R*)*-*1-(4-((furan-2-yl)methyl)-5-phenethyl-4*H*-1,2,4-triazol-3-yl)-2-(1*H*-indol-3-yl)ethyl)-2-amino-2-methylpropanamide, 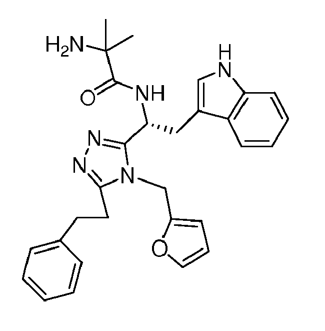
**compound 182** *N*-((*R*)-1-(4-((furan-2-yl)methyl)-5-phenethyl-4*H-*1,2,4-triazol-3-yl)-2-(1*H*-indol-3-yl)ethyl)picolinamide, 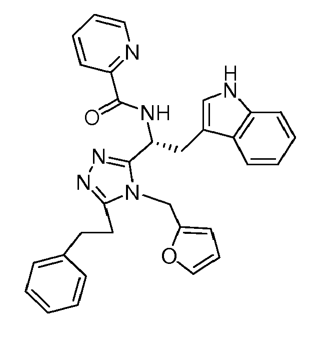
**compound 183** *N*-((*R*)-1-(4-((furan-2-yl)methyl)-5-phenethyl-4*H*-1,2,4-triazol-3-yl)-2-(1*H*-indol-3-yl)ethyl)piperidine-4-carboxamide, 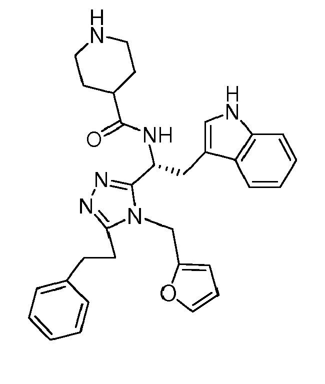
**compound 184** *N*-((*R*)-1-(4-(4-methoxybenzyl)-5-phenethyl-4*H*-1,2,4-triazol-3-yl)-2-(1*H*-indol-3-yl)ethyl)-tetrahydro-2H-pyran-4-carboxamides 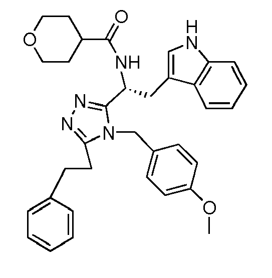
**compound 185** *N*-((*R*)-1*-*(5-((1*H*-indol-3-yl)methyl)-4-(3-methoxybenzyl)-4*H*-1,2,4-triazol-3-yl)-2-(1*H*-indol-3-yl)ethyl)-2-amino-2-methylpropanamide, 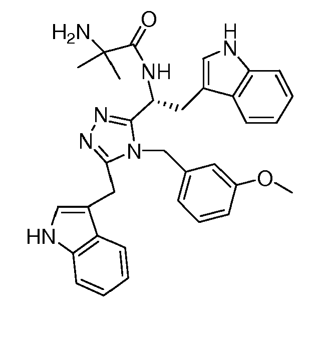
**compound 186** (2*S*)-*N*-((*R*)-1-(4-(4-methoxybenzyl)-5-phenethyl-4*H*-1,2,4-triazol-3-yl)-2-(1*H*-indol-3-yl)ethyl)-2-amino-3-phenylpropanamide, 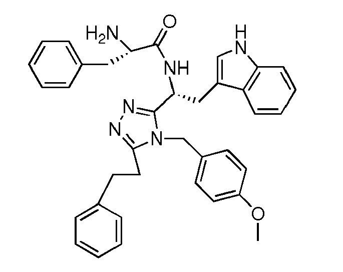
**compound 187** (*R*)-1-(5-(2-(1*H*-indol-3-yl)ethyl)-4-(2,4-dimethoxybenzyl)-4*H*-1,2,4-triazol-3-yl)-2-(1*H*-indol-3-yl)-N-tosylethanamine, 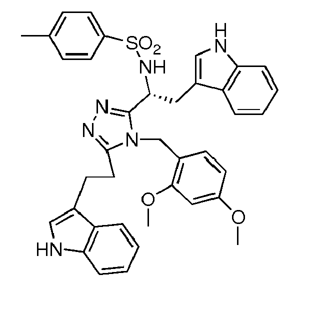
**compound 188** *N*-((*R*)-1-(4-(2,4-dimethoxybenzyl)-5-phenethyl-4*H*-1,2,4-triazol-3-yl)-2-(1*H*-indol-3-yl)ethyl)-4-azidobenzamide, 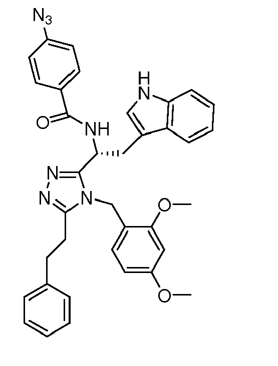
**compound 189** *N*-benzyl-(*R*)-1-(4-(2,4-dimethoxybenzyl)-5-phenethyl-4*H*-1,2,4-triazol-3-yl)-2-(1*H*-indol-3-yl)ethanamine, 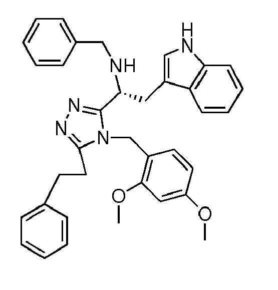
**compound 190** (2*S*)-*N*-((*R*)-1-(5-(2-(1*H*-indol-3-yl)ethyl)-4-(4-methoxybenzyl)-4*H-*1,2,4-triazol-3-yl)-2-(1*H*-indol-3-yl)ethyl)-2,5-dihydro-1*H*-pyrrole-2-carboxamide,

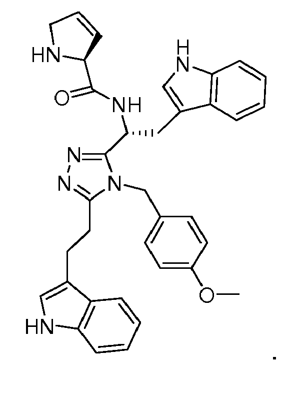

For the avoidance of doubt, if chemical name and chemical structure of the above illustrated compounds do not correspond by mistake, the chemical structure is regarded to unambigously define the compound.

In a preferred embodiment these compounds can be used for the manufacture of a medicament for the treatment or prophylaxis of physiological and/or pathophysiological conditions in mammals, where the physiological and/or pathophysiological conditions are mediated by at least one G-protein coupled receptor (GPCR); with the proviso that the following G-protein coupled receptors (GPCR) are excluded: "GHS receptors, GHS type 1 receptor, GHS-R 1a, GHS-R 1b, motilin receptor, motilin receptor 1a, neurotensin receptor, TRH receptor, GPR38 (FM1), GPR39 (FM2), FM3, GHS-R subtype, GHS binding site, cardiac GHS-R, mammary GHS-R".

All compounds of formula (I) as shown above, the preferred embodiments and the triazole compounds 1 to 190 mentioned explicitly are referred to collectively hereinafter as "compounds of the invention".

The terms indicated for explanation of the above compounds of formula (I) and preferred embodiments always, unless indicated otherwise in the description or in the claims, have the following meanings:

The term "substituted" means that the corresponding radical or group has one or more substituents. Where a radical has a plurality of substituents, and a selection of various substituents is specified, the substituents are selected independently of one another and need not be identical. The term "unsubstituted" means that the corresponding group has no substituent. The term "optionally substituted" means that the corresponding group is either unsubstituted or substituted by one or more substituents. The term "substituted by up to 3 substituents" means that the corresponding radical or group is substituted either by one or by two or three substituents.

The term "alkyl" includes for the purposes of this invention acyclic saturated hydrocarbons having C1-C12 carbon atoms, which may be straight-chain or branched. The term "alkyl" preferably stands for alkyl chains of 1 to 8, particularly preferably 1 to 6, carbon atoms. Examples of suitable alkyl radicals are methyl, ethyl, n-propyl, isopropyl, n-butyl, isobutyl. sec-butyl, tert-butyl, n-pentyl, tert-pentyl, iso-Pentyl, neo-Pentyl, n-hexyl, isohexyl, 2-Hexyl, n-heptyl, n-octyl, n-nonyl, n-decyl, n-undecyl, n-dodecyl.

The term "alkenyl" includes for the purposes of this invention acyclic unsaturated or partially unsaturated hydrocarbons having C2-C12 carbon atoms, i.e. C₂-12-alkenyls,which may be straight-chain or branched and contain one or more C-C double bonds. The term "alkenyl" preferably stands for alkenyl chains of 2 to 8, particularly preferably 2 to 6, carbon atoms. Examples are ethylenyl (vinyl), propenyl (-CH₂CH=CH₂; -CH=CH-CH₃, -C(=CH₂)-CH₃), butenyl, pentenyl, hexenyl, heptenyl, octenyl, octadienyl.

The term "alkynyl" refers to acyclic unsaturated or partially unsaturated hydrocarbons having C2-C12 carbon atoms, i.e. C₂₋₈-alkynyls, which may be straight-chain or branched and contain one or more C-C triple bonds. Alkynyls may additionally also contain at least one C-C double bond. The term "alkynyl" preferably stands for alkynyl chains of 2 to 8, particularly preferably 2 to 6, carbon atoms. Examples are ethynyl, propynyl (-CH₂-C≡CH, -C≡C-CH₃), butynyl, pentynyl, hexynyl, heptynyl, octynyl.

The term "cycloalkyl" stands for a saturated or partially unsaturated non-aromatic cyclic hydrocarbon group/radical, containing 1 to 3 rings, including monocyclic alkyl, bicyclic alkyl and tricyclic alkyl, and containing a total of 3 to 20 carbon atoms forming the rings, preferably 3 to 12, most preferably (C3-C8)-cycloalkyl. The cycloalkyl radical may also be part of a bi- or polycyclic system, where, for example, the cycloalkyl radical is fused to an aryl, heteroaryl or heterocyclyl radical as defined herein by any possible and desired ring member(s). The bonding to the compounds of formula (I) can be effected via any possible ring member of the cycloalkyl radical. Examples of suitable cycloalkyl radicals are cyclopropyl, cyclobutyl, cyclopentyl, cyclohexyl, cycloheptyl, cyclooctyl, cyclodecyl, cyclohexenyl, cyclopentenyl, cyclooctadienyl.

The term "cycloalkylalkyl" refers to a radical in which the cycloalkyl group is linked via an alkyl, alkenyl or alkynyl group, where the alkyl, alkenyl, alkynyl and cycloalkyl groups have the meanings defined herein, preferably a (C3-C8)-cycloalkyl-(C1-C4)-alkyl radical. Examples thereof are cyclopropylmethyl, cyclohexylmethyl, cyclopentylethyl, cyclohexenylethyl.

The term "heterocyclyl" refers to a 3- to 14-membered, preferably 3-, 4-, 5-, 6-, 7-or 8-membered, cyclic organic radical which contains at least 1 heteroatom, optionally 2, 3, 4 or 5 heteroatoms, especially nitrogen, oxygen and/or sulphur, the heteroatoms being the same or different and the cyclic radical being saturated or unsaturated, but not aromatic. The heterocyclyl radical may also be part of a bi- or polycyclic system, where, for example, the heterocyclyl radical is fused to an aryl, heteroaryl or cycloalkyl radical as defined herein by any possible and desired ring member(s). The bonding to the compounds of formula (I) can be effected via any possible ring member of the heterocyclyl radical. Examples are tetrahydrofuryl, pyrrolidinyl, imidazolidinyl, thiazolidinyl, tetrahydropyranyl, piperidinyl, piperazinyl, morpholinyl, thiapyrrolidinyl, oxapiperazinyl, oxapiperidinyl and oxadiazolyl.

The term "heterocyclylalkyl" refers to radicals in which the heterocyclyl group is linked via an alkyl, alkenyl or alkynyl group, where the alkyl, alkenyl, alkynyl and heterocyclyl groups have the meanings defined herein.

The term "aryl" refers to aromatic hydrocarbon systems having 3 to 14, preferably 5 to 14, carbon atoms. The aryl radical may also be part of a bi- or polycyclic system, where, for example, the aryl radical is fused to a heterocyclyl, heteroaryl or cycloalkyl radical as defined herein by any possible and desired ring member. The bonding to the compounds of formula (I) can be effected via any possible ring member of the aryl radical. Preferred aryl radicals are phenyl, biphenyl, naphthyl and anthracenyl, but likewise indanyl, indenyl or 1,2,3,4-tetrahydronaphthyl.

The term "heteroaryl" refers to a 5-, 6- or 7-membered cyclic aromatic radical which comprises at least 1, where appropriate also 2, 3, 4 or 5 heteroatoms, preferably nitrogen, oxygen and/or sulfur, where the heteroatoms are identical or different. The number of nitrogen atoms is preferably between 0 and 3, and that of the oxygen and sulfur atoms is between 0 and 1. The heteroaryl radical may also be part of a bi- or polycyclic system, where, for example, the heteroaryl radical is fused to a heterocyclyl, aryl or cycloalkyl radical as defined herein by any possible and desired ring member. The bonding to the compounds of formula (I) can be effected via any possible ring member of the heteroaryl radical. Examples are pyrrolyl, furyl, thienyl, thiazolyl, isothiazolyl, oxazolyl, oxadiazolyl, isoxazolyl, pyrazolyl, imidazolyl, triazole, tetrazole, pyridinyl, pyrimidinyl, pyridazinyl, pyrazinyl, triazinyl, phthalazinyl, indolyl, indazolyl, indolizinyl, quinolinyl, isoquinolinyl, quinoxalinyl, quinazolinyl, pteridinyl, carbazolyl, phenazinyl, phenoxazinyl, phenothiazinyl, and acridinyl.

The terms "arylalkyl" and "heteroarylalkyl" refer to radicals in which the aryl or heteroaryl radical is linked via an alkyl, alkenyl or alkynyl group, where the alkyl, alkenyl, alkynyl, aryl and heteroaryl groups have the meanings defined herein. Preferred "arylalkyl" groups are phenyl-(C₁-C₄)-alkyl radicals, preferably benzyl or phenylethyl radicals. Preferred "heteroarylalkyl" groups are indolyl-(C₁-C₄)-alkyl radicals, preferably 1*H-*indole-3-yl-methyl or 2(1*H*-indole-3-yl)-ethyl.

The terms "alkylsulfonyl", "arylsulfonyl" and "arylalkylsulfonyl" refer to radicals in which the alkyl, aryl or arylalkyl group is linked via a -SO₂- group, where the alkyl, aryl and arylalkyl groups have the meanings defined herein. Examples are methylsulfonyl and phenylsulfonyl.

The term "halogen", "halogen atom" or "halogen substituent" (Hal-) refers to one, where appropriate, a plurality of fluorine (F, fluoro), bromine (Br, bromo), chlorine (Cl, chloro), or iodine (I, iodo) atoms. The designations "dihalogen", "trihalogen" and "perhalogen" refer respectively to two, three and four substituents, where each substituent can be selected independently from the group consisting of fluorine, chlorine, bromine and iodine. "Halogen" preferably means a fluorine, chlorine or bromine atom.

The term "natural alpha-amino acid side chain" for the purpose of the present invention refers to all side chains of the known 20 proteinogenic alpha-amino acids as well as to side chains of naturally occurring (i.e. in any biological systems) alpha-amino acids, such as for instance selenocystein, pyrrolysine, citrulline, ornithine, homocysteine, N-methylariginine, N-acetyllysine, gamma-carboxyglutamate, 5-hydroxylysine, 3-methylhistidine and/or N,N,N,-trimethyllysine. In this connection "side chain" refers to the residue that is attached to the alpha-carbon atom, e.g. methyl in case of an Ala side chain or benzyl in case of a Phe side chain.

The term "unnatural alpha amino acid side chain" for the purpose of the present invention refers to all side chains of known alpha-amino acids that are not proteinogenic nor are known to occur naturally (i.e. in any biological systems). Examples are norleucine, cyclohexylglycine, 2-naphthylalanine, substituted alpha-amino acids (e.g. halogen substituted Tyr or Phe) as well as protected alpha-amino acid side chains, where a protection group such as Fmoc, Boc, Z, CBZ, Aloc, trityl, acetyl and/or benzyl is directly attached/reacted to a functionalization (e.g. amino, hydroxy and/or carboxy residue). In this connection "side chain" is referred to as for "natural alpha amino acid side chains".

Above embodiments of radicals R1 to R10 that possess functionalization (e.g. amino, hydroxy and/or carboxy residues), such as alkyl-CO-NH₂, -alkyl-CO-OH, - alkyl-NH₂, -alkyl-NH-C(NH)-NH₂,-CO-C*(R9R10)-NH₂, -CO-CH₂-C*(R9R10)-NH₂, -CO-C*(R9R10)-CH₂-NH₂ and/or 2-amino-2-carbonyl-propane (2-amino-isobutyric acid/Aib residue), may be protected with protection groups as mentioned above. Such protection group carrying embodiments are regarded as belonging to/ within the scope and spirit of the invention.

All stereoisomers of the compounds of the invention are contemplated, either in a mixture or in pure or substantially pure form. The compounds of the present invention can have asymmetric centers at any of the carbon atoms including any one of the R radicals. Consequently, compounds of the invention can exist in the form of their racemates, in the form of the pure enantiomers and/or diastereomers or in the form of mixtures of these enantiomers and/or diastereomers. The mixtures may have any desired mixing ratio of the stereoisomers. All these different stereochemical forms and mixtures are within the scope of the present invention.

Thus, for example, the compounds of the invention which have one or more centers of chirality and which occur as racemates or as diastereomer mixtures can be fractionated by methods known per se into their optical pure isomers, i.e. enantiomers or diastereomers. The separation of the compounds of the invention can take place by column separation on chiral or nonchiral phases or by recrystallization from an optionally optically active solvent or with use of an optically active acid or base or by derivatization with an optically active reagent such as, for example, an optically active alcohol, and subsequent elimination of the radical.

The compounds of the inventivon may be present in the form of their double bond isomers as "pure" E or Z isomers, or in the form of mixtures of these double bond isomers.

Where possible, the compounds of the invention may be in the form of the tautomers.

It is likewise possible for the compounds of the invention to be in the form of any desired prodrugs such as, for example, esters, carbonates, carbamates, ureas, amides or phosphates, in which cases the actually biologically active form is released only through metabolism. Any compound that can be converted in vivo to provide the bioactive agent (i.e. compounds of the invention) is a prodrug within the scope and spirit of the invention.

Various forms of prodrugs are well known in the art and are described for instance in:
(i) Wermuth CG et al., Chapter 31: 671-696, The Practice of Medicinal Chemistry, Academic Press 1996;
(ii) Bundgaard H, Design of Prodrugs, Elsevier 1985; and
(iii) Bundgaard H, Chapter 5: 131-191, A Textbook of Drug Design and Development, Harwood Academic Publishers 1991.

Said references are incorporated herein by reference.
It is further known that chemical substances are converted in the body into metabolites which may where appropriate likewise elicit the desired biological effect - in some circumstances even in more pronounced form.
Any biologically active compound that was converted in vivo by metabolism from any of the compounds of the invention is a metabolite within the scope and spirit of the invention.

The compounds of the invention can, if they have a sufficiently basic group such as, for example, a primary, secondary or tertiary amine, be converted with inorganic and organic acids into salts. The pharmaceutically acceptable salts of the compounds of the invention are preferably formed with hydrochloric acid, hydrobromic acid, iodic acid, sulfuric acid, phosphoric acid, methanesulfonic acid, p-toluenesulfonic acid, carbonic acid, formic acid, acetic acid, sulfoacetic acid, trifluoroacetic acid, oxalic acid, malonic acid, maleic acid, succinic acid, tartaric acid, racemic acid, malic acid, embonic acid, mandelic acid, fumaric acid, lactic acid, citric acid, taurocholic acid, glutaric acid, stearic acid, glutamic acid or aspartic acid. The salts which are formed are, inter alia, hydrochlorides, chlorided, hydrobromides, bromides, iodides, sulfates, phosphates, methanesulfonates, tosylates, carbonates, bicarbonates, formates, acetates, sulfoacetates, triflates, oxalates, malonates, maleates, succinates, tartrates, malates, embonates, mandelates, fumarates, lactates, citrates, glutarate, stearate, aspartates and glutamates. The stoichiometry of the salts formed from the compounds of the invention may moreover be an integral or non-integral multiple of one.

The compounds of the invention can, if they contain a sufficiently acidic group such as, for example, the carboxy, sulfonic acid, phosphoric acid or a phenolic group, be converted with inorganic and organic bases into their physiologically tolerated salts. Examples of suitable inorganic bases are ammonium, sodium hydroxide, potassium hydroxide, calcium hydroxide, and of organic bases are ethanolamine, diethanolamine, triethanolamine, ethylenediamine, t-butylamine, t-octylamine, dehydroabietylamine, cyclohexylamine, dibenzylethylene-diamine and lysine. The stoichiometry of the salts formed from the compounds of the invention can moreover be an integral or non-integral multiple of one.

It is likewise possible for the compounds of the invention to be in the form of their solvates and, in particular, hydrates which can be obtained for example by crystallization from a solvent or from aqueous solution. It is moreover possible for one, two, three or any number of solvate or water molecules to combine with the compounds of the invention to give solvates and hydrates.

It is known that chemical substances form solids which exist in different order states which are referred to as polymorphic forms or modifications. The various modifications of a polymorphic substance may differ greatly in their physical properties. The compounds of the invention can exist in various polymorphic forms, and certain modifications may moreover be metastable. All these polymorphic forms of the compounds of the invention are to be regarded as belonging to the invention.

In a further preferred embodiment, the compounds of the invention can be used for the manufacture of a medicament for the treatment or prophylaxis of physiological and/or pathophysiological conditions in mammals.

In yet a further preferred embodiment, the compounds of the invention can be used for the manufacture of a medicament for the treatment or prophylaxis of physiological and/or pathophysiological conditions in mammals, where the treatment is achieved by modulation of one or more G-protein coupled receptors (GPCR) as described and defined herein.

In another preferred embodiment, in the course of the herein described treatments the compounds of the invention are G-protein coupled receptor (GPCR) antagonists.

In another preferred embodiment, in the course of the herein described treatments the compounds of the invention are G-protein coupled receptor (GPCR) agonists.

The triazole derivatives (compounds of the invention) as illustrated herein are ligands of GPCRs as described and defined herein. Thus, the aforementioned compounds of the invention are suitable for the treatment or prophylaxis of physiological and/or pathophysiological conditions mediated by GPCR receptors as described and defined herein and/or physiological and/or pathophysiological conditions which can be influenced by modulation of these receptors, and thus prevented, treated and/or alleviated.

For the purpose of the present invention, the term "treatment" is also intended to include prophylactic treatment or alleviation.

The term "ligand" is intended to refer for the purposes of the present invention to every compound which binds in any way to a receptor and induces either activation, inhibition and/or another conceivable effect (e.g. allosteric effects) at this receptor. The term "ligand" thus includes agonists, antagonists, partial agonists/antagonists, inverse agonists and other ligands which cause an effect at the receptor which is similar to the effect of agonists, antagonists, partial agonists/antagonists or inverse agonist.

For the purpose of the present invention, the term "G-protein coupled receptor (GPCR) antagonist" or "antagonist of GPCRs" refers to compounds of the invention that bind to G-protein coupled receptors (GPCR) as described and defined herein but do not elicit a proper activation of the receptors as assessed by recording an increase of intracellular calcium which is characteristic for activation of G-protein coupled receptors (GPCRs).

The ability to properly activate the receptors is assessed for any compound of the invention by comparing the degree of activation (increase of intracellular calcium) of a GPCR, for instance GPR103, by the compound to be tested (at 10⁻⁶ M concentration) to the degree of activation (increase of intracellular calcium) of the GPCR, for instance GPR103, by 10⁻⁶ M of its cognate ligand (100%), for instance QRFP(26)[26RFa] as ligand of GPR103, and to the basal level (0%). Such assessment can be readily performed by the skilled artisan due to his expert knowledge. The output is a percentage value for each compound to be tested.

Any compound of the invention that does not show a degree of activation (increase of intracellular calcium) of a respective GPCR as described and defined herein of at least 20 % as assessed in accordance with above specification is regarded as not eliciting a proper activation and therefore as GPCR receptor antagonist. Preferably such compounds do show an antagonizing effect (counteraction/decrease) on the intracellular calcium increase stimulated by the cognate ligand of the respective GPCR, for instance QRFP(26)[26RFa] as ligand of GPR103, and/or other activating ligand, prevent such stimulation or even act as inverse agonists (an inverse agonists is an ligand which binds to the same receptor binding-site as an agonist or antagonist but causes an inhibition of the basal/constitutive activity of the receptor, in principle an agonists with a negative intrinsic activity). Such compounds may furthermore exhibit an inhibitory activity on other physiological or pathophysiological conditions or effects, such as food intake or lipogenesis. Their effects may be dissociated. Thus, they may have no inihibitory impact at all on intracellular calcium increase while inhibiting other physiological effects.

For the purpose of the present invention, the term "G-protein coupled receptor (GPCR) agonist" or "agonist of GPCR" refers to compounds of the invention that bind to G-protein coupled receptors (GPCR) as described and defined herein and elicit a proper activation of the receptor as assessed by recording an increase of intracellular calcium which is characteristic for activation of G-protein coupled receptors.

Any compound of the invention that shows a degree of activation (increase of intracellular calcium) of a respective GPCR as described and defined herein of at least 20 % as assessed in accordance with above specification is regarded as eliciting a proper activation and therefore as a GPCR receptor agonist. Such compounds may mimic the effects of the cognate GPCR receptor ligangs, such as QRFP(26)[26RFa] as ligand of GPR103, and other stimulating ligands on intracellular calcium increase and for instance food intake or lipogenesis. Like for antagonists, the effects of agonist compounds may be dissociated from the intracellular calcium increase effect.

As illustrated above the following receptors are excluded from the genus of G-protein coupled receptors (GPCR) in the course of the present invention. These receptors are herein referred to as "other receptor(s)" and consist of "GHS receptors, GHS type 1 receptor, GHS-R 1a, GHS-R 1b, motilin receptor, motilin receptor 1a, neurotensin receptor, TRH receptor, GPR38 (FM1), GPR39 (FM2), FM3, GHS-R subtype, GHS binding site, cardiac GHS-R, mammary GHS-R".

With regard to the "other receptor(s)" and the terms "ligand", "agonist", "antagonist", "partial agonist/antagonist", "inverse agonist" and "other ligands which cause an effect at the receptor which is similar to the effect of agonists, antagonists, partial agonists/antagonists or inverse agonists" the same above definitions apply as for the genus of G-protein coupled receptors (GPCR) of the present invention as described and defined herein.

The term "GHS receptor" or "GHS-R" is intended to comprise for the purposes of the present invention receptors that bind at least one known peptidyl and/or non-peptidyl GHS and/or ghrelin. The term "GHS receptor" or "GHS-R" is also intended to comprise different GHS binding sites in the various tissues and/or organs as illustrated herein, that bind at least one known peptidyl and/or non-peptidyl GHS and/or ghrelin and which are probably not yet characterized GHS-R subtypes.

Binding of a given known peptidyl and/or non-peptidyl GHS and/or ghrelin can be easily verified by the skilled artisan on the basis of his expert knowledge, e.g. by appropriate binding assays which represent only routine experimentation.

Such GHS receptors may be stimulated/activated by ghrelin (ghrelin responsive) or may not be stimulated/activated by ghrelin (ghrelin non-responsive) - with regard to both acylated and non-acylated ghrelin, respectively. Stimulation/activation of such receptors may cause but does not compulsorily have to elicit GH production and/or GH secretion and/or increase GH plasma levels.

As illustrated supra, the compounds of the invention are ligands of GPCR as described and defined herein. They can be administered to various mammalian species, including human, for the treatment or prophylaxis of physiological and/or pathophysiological condition in such mammals.

For the purpose of the present invention, all mammalian species are regarded as being comprised. Preferably, such mammals are selected from the group consisting of "human, domestic animals, cattle, livestock, pets, cow, sheep, pig, goat, horse, pony, donkey, hinny, mule, hare, rabbit, cat, dog, guinea pig, hamster, rat, mouse". More preferably, such mammals are human.

As illustrated supra, the compounds of the invention are ligands of at least one GPCR as described and defined herein. In a preferred embodiment, the compounds of the invention can be used for the manufacture of a medicament for the herein described treatments, where the at least one G-protein coupled receptor (GPCR) is selected from the group consisting of: "5-hydroxytryptamine (serotonin) receptor, 5-hydroxytryptamine (serotonin) receptor 1A [5-HT1A, synonyms: 5HT1a; ADRBRL1, ADRB2RL1, GenBank accession number: NM_000524 (human 5-HT1A)], 5-hydroxytryptamine (serotonin) receptor 1B [5-HT1B, synonyms: S12; HTR1D2; HTR1DB; 5-HT1DB, GenBank accession number: NM_000863 (human 5-HT1 B)], 5-hydroxytryptamine (serotonin) receptor 1D [5-HT1D, synonyms: HTRL; RDC4; HT1DA; HTR1DA, GenBank accession number: NP_000855 (human 5-HT1D)], 5-hydroxytryptamine (serotonin) receptor 1E [5-HT1E, GenBank accession number:
NM_000865 (human 5-HT1E)], 5-hydroxytryptamine (serotonin) receptor 1 F [5-HT1F, synonyms: MR77; HTR1EL, GenBank accession number: NM_000866 (human 5-HT1F)], 5-hydroxytryptamine (serotonin) receptor 2A [5-HT2A, synonyms: HTR2; GenBank accession number: NM_000621 (human 5-HT2A)], 5-hydroxytryptamine (serotonin) receptor 2B [5-HT2B, synonyms: 5-HT(2B), GenBank accession number:
NM_000867 (human 5-HT2B)], 5-hydroxytryptamine (serotonin) receptor 2C [5-HT2C, synonyms: HTR1C; GenBank accession number: NM_000868 (human 5-HT2C)], 5-hydroxytryptamine (serotonin) receptor 4 [5-HT4, synonyms: 5-HT4R, GenBank accession number: NM_000870 (human 5-HT4, isoform b)], 5-hydroxytryptamine (serotonin) receptor 5A [5-HT5A, synonyms: MGC138226, GenBank accession number:
NM_024012 (human 5-HT5A)], 5-hydroxytryptamine (serotonin) receptor 6 [5-HT6, GenBank accession number: NM_000871 (human 5-HT6)], 5-hydroxytryptamine (serotonin) receptor 7 [5-HT7, synonyms: GenBank accession number: NM_000872 (human 5-HT7, transcript variant a)], muscarinic cholinergic receptor, muscarinic cholinergic receptor 1, [M1, synonyms: HM1; MGC30125, GenBank accession number:
NM_000738 (human M1)], muscarinic cholinergic receptor 2, [M2, synonyms: HM2; FLJ43243; MGC120006; MGC120007, GenBank accession number: NM_001006630 (human M2, transcript variant 1)], muscarinic cholinergic receptor 3, [M3, synonyms:
HM3, GenBank accession number: NM_000740 (human M3)], muscarinic cholinergic receptor 4, [M4, synonyms: HM4, GenBank accession number: NM_000741 (human M4)], muscarinic cholinergic receptor 5, [M5, synonyms: HM5; MGC41838, GenBank accession number: NM_012125 (human M5)], Alpha-adrenergic receptor, Alpha-1-adrenergic receptor, Alpha-1 A-adrenergic receptor, [alpha-1A, synonyms: ADRA1A, ADRA1C; ADRA1L1; ALPHA1AAR, GenBank accession number: NM_000680 (human alpha-1A transcript variant 1)], Alpha-1B-adrenergic receptor, [alpha-1B, synonyms: ADRA1; ALPHA1BAR, GenBank accession number: NM_000679 (human alpha-1B)], Alpha-1D-adrenergic receptor, [alpha-1D, synonyms: ADRA1D, DAR; ADRA1; ADRA1A; ADRA1R; ALPHA1; dJ779E11.2, GenBank accession number: NM_000678 (human alpha-1 D)], Alpha-2-adrenergic receptor, Alpha-2A-adrenergic receptor, [alpha-2A, synonyms: ADRA2A, ADRA2; ADRAR; ZNF32; ADRA2R; ALPHA2AAR, GenBank accession number: NM_000681 (human alpha-2A)], Alpha-2B-adrenergic receptor, [alpha-2B, synonyms: ADRA2B, ADRA2L1; ADRARL1; ADRA2RL1; ALPHA2BAR, GenBank accession number: NM_000682 (human alpha-2B)], Alpha-2C-adrenergic receptor, [alpha-2C, synonyms: ADRA2C, ADRA2L2; ADRARL2; ADRA2RL2; ALPHA2CAR, GenBank accession number: NM 000683 (human alpha-2C)], Beta-adrenergic receptor, Beta-1-adrenergic receptor, [beta-1, synonyms: ADRB1, RHR; B1AR; ADRB1 R; BETA1AR, GenBank accession number: NM 000684 (human beta-1)], Beta-2-adrenergic receptor, [beta-2, synonyms: ADRB2, BAR; B2AR; ADRBR; ADRB2R; BETA2AR, GenBank accession number: NM_000024 (human beta-2)], Beta-3-adrenergic receptor, [beta-3, synonyms: ADRB3, BETA3AR, GenBank accession number: NM_000025 (human beta-3)], angiotensin II receptor, angiotensin II receptor type 1, [AT1, synonyms: AGTR1, AG2S; AT1B; AT2R1; HAT1R; AGTR1A; AGTR1B; AT2R1A; AT2R1 B, GenBank accession number: NM_031850 (human AT1, transcript variant 4)], angiotensin II receptor type 2, [AT2, synonyms: AGTR2, ATGR2; MRX88, GenBank accession number: NM_000686 (human AT2)], neuromedin B receptor, [NMBR, synonyms: BB1, GenBank accession number: NM_002511 (human NMBR)], gastrin-releasing peptide receptor, [GRPR, synonyms: BB2, GenBank accession number: NM_005314 (human GRPR)], bombesin-like receptor 3, [BRS3, synonyms: BB3, GenBank accession number: NM_001727 (human BRS3)], calcitonin receptor, [CALCR, synonyms: CT, CRT; CTR; CTR1, GenBank accession number: NM_001742 (human CALCR)], calcitonin receptor-like, [CALCRL, synonyms: CRLR; CGRPR, GenBank accession number: NM_005795 (human CALCRL)], cannabinoid receptor, cannabinoid receptor 1, [CB1, synonyms: CNR1, CNR; CB-R; CB1A; CANN6; CB1K5, GenBank accession number: NM_016083 (human CB1, transcript variant 1)], cannabinoid receptor 2, [CB2, synonyms: CNR2, CX5, GenBank accession number:
NM_001841 (human CB2)], cholecystokinin receptor, cholecystokinin A receptor, [CCKAR, synonyms: CCK-A; CCKRA; CCK1-R, GenBank accession number: NM_000730 (human CCKAR)], cholecystokinin B receptor, [CCKBR, synonyms: GASR; CCK-B, GenBank accession number: NM_176875 (human CCKBR)], corticotropin releasing hormone receptor, corticotropin releasing hormone receptor 1, [CRF1, synonyms: CRHR1, CRHR; CRF-R; CRFR1; CRHRIf; CRH-R1h, GenBank accession number: NM_004382 (human CRF1)], corticotropin releasing hormone receptor 2, [CRF2, synonyms: CRHR2, GenBank accession number: NM_001883 (human CRF2)], dopamine receptor, dopamine receptor D1, [D1, synonyms: DRD1, DADR; DRD1A, GenBank accession number: NM_000794 (human D1)], dopamine receptor D2, [D2, synonyms: DRD2, D2R; D2DR, GenBank accession number: NM 000795 (human D2, transcript variant 1)], dopamine receptor D3, [D3, synonyms: DRD3, D3DR; ETM1; FET1; MGC149204; MGC149205, GenBank accession number: NM 000796 (human D3, transcript variant a)], dopamine receptor D4, [D4, synonyms: DRD4, D4DR, GenBank accession number: NM_000797 (human D4)], dopamine receptor D5, [D5, synonyms: DRD5, DBDR; DRD1B; DRD1L2; MGC10601, GenBank accession number:
NM_000798 (human D5)], glucagon-like peptide receptor, glucagon-like peptide 1 receptor, [GLP-1, synonyms: GLP1R, MGC138331, GenBank accession number:
NM_002062 (human GLP-1)], glucagon-like peptide 2 receptor, [GLP-2, GenBank accession number: NM 004246 (human GLP-2)], follicle stimulating hormone receptor, [FSHR, synonyms: LGR1; ODG1; FSHRO; MGC141667; MGC141668, GenBank accession number: NM_004246 (human FSHR)], histamine receptor, histamine receptor H1, [H1, synonyms: HRH1, H1-R; hisH1, GenBank accession number: NM_000861 (human H1)], histamine receptor H2, [H2, synonyms: HRH2, H2R, GenBank accession number: NM_022304 (human H2)], histamine receptor H3, [H3, synonyms: HRH3, HH3R; GPCR97, GenBank accession number: NM_007232 (human H3)], histamine receptor H4, [H4, synonyms: HRH4, H4; H4R; BG26; HH4R; AXOR35; GPRv53; GPCR105; MGC133027, GenBank accession number: NM_021624 (human H4)], endothelial differentiation lysophosphatidic acid G-protein-coupled receptor, endothelial differentiation lysophosphatidic acid G-protein-coupled receptor 2, [EDG2, synonyms: LPA1, LPAR1; edg-2; vzg-1; Gpcr26; Mrec1.3; rec.1.3, GenBank accession number:
NM_001401 (human EDG2, transcript variant 1)], endothelial differentiation lysophosphatidic acid G-protein-coupled receptor 4, [EDG4, synonyms: LPA2, EDG-4; LPAR2, GenBank accession number: NM_004720 (human EDG4)], endothelial differentiation lysophosphatidic acid G-protein-coupled receptor 7, [EDG7, synonyms: LPA3, GPCR; Edg-7; LP-A3; LPAR3; HOFNH30; RP4-67813, GenBank accession number:
NM_012152 (human EDG7)], melanin-concentrating hormone receptor, melanin-concentrating hormone receptor 1, [MCH1, synonyms: MCHR1, SLC1; GPR24; MCH1R; MGC32129, GenBank accession number: NM_005297 (human MCH1)], melanin-concentrating hormone receptor 2, [MCH2, synonyms: MCHR2, SLT; MCH2R; GPR145, GenBank accession number: NM_001 040179 (human MCH2, transcript variant 1)], melanocortin receptor, melanocortin 1 receptor (alpha melanocyte stimulating hormone receptor), [MC1, synonyms: MCHR1, SLC1; GPR24; MCH1R; MGC32129, GenBank accession number: NM_005297 (human MC1)], melanocortin 2 receptor (adrenocorticotropic hormone receptor), [MC2, synonyms: MCHR2, SLT; MCH2; MCH2R; GPR145, GenBank accession number: NM_001 040179 (human MC2, transcript variant 1)], melanocortin 3 receptor, [MC3, synonyms: MC3R, GenBank accession number: NM_019888 (human MC3)], melanocortin 4 receptor, [MC4, synonyms: MC4R, MGC126851; MGC138197, GenBank accession number: NM_005912 (human MC4)], melanocortin 5 receptor, [MC5, synonyms: MC5R, GenBank accession number:
NM_005913 (human MC5)], neuropeptide FF receptor, neuropeptide FF receptor 1, [NPFFR1, synonyms: NPFF1; GPR147; NPFF1R1; OT7TO22; FLJ10751, GenBank accession number: NM_022146 (human NPFFR1)], neuropeptide FF receptor 2, [NPFFR2, synonyms: GPR74; NPFF2; NPGPR, GenBank accession number:
NM_004885 (human NPFFR2)], neuropeptides B/W receptor, neuropeptides B/W receptor 1, [NPBWR1, synonyms: GPR7; MGC129755, GenBank accession number:
NM_005285 (human NPBWR1)], neuropeptides B/W receptor 2, [NPBWR2, synonyms: GPR8, GenBank accession number: NM_005286 (human NPBWR2)], neuropeptide Y receptor, neuropeptide Y receptor Y1, [NPY1R, synonyms: NPYR, GenBank accession number: NM_000909 (human NPY1 R)], neuropeptide Y receptor Y2, [NPY2R, GenBank accession number: NM_000910 (human NPY2R)], neuropeptide Y receptor Y4 (pancreatic polypeptide receptor 1), [NPY4R, synonyms: PPYR1, Y4; PP1; NPY4-R; MGC116897, GenBank accession number: NM_005972 (human NPY4R)], neuropeptide Y receptor Y5, [NPY5R, GenBank accession number: NM_006174 (human NPY5R)], opioid receptor, opioid receptor delta 1, [OPRD1, synonyms: OPRD, GenBank accession number: NM_000911 (human OPRD1)], opioid receptor kappa 1, [OPRK1, synonyms: KOR; OPRK, GenBank accession number: NM 000912 (human OPRK1)], opioid receptor mu 1, [OPRM1, synonyms: MOR1; OPRM; KIAA0403, GenBank accession number: NM_000914 (human OPRM1, transcript variant MOR-1)], opiate receptor-like 1, [OPRL1, synonyms: OOR; ORL1; KOR-3; NOCIR; MGC34578, GenBank accession number: NM_182647 (human OPRL1, transcript variant 1)], hypocretin (orexin) receptor, hypocretin (orexin) receptor 1, [HCRTR1, synonyms: OX1R, GenBank accession number: NM_001525 (human HCRTR1)], hypocretin (orexin) receptor 2, [HCRTR2, synonyms: OX2R, GenBank accession number: NM_001526 (human HCRTR2)], G protein-coupled receptor 103, [GPR103, synonyms: AQ27; SP9155; MGC149217, GenBank accession number: NM_198179 (human GPR103)], G protein-coupled receptor 103A, [GPR103A, GenBank accession number: NM_198192 (mouse GPR103A)], G protein-coupled receptor 103B, [GPR103B, GenBank accession number: AX665940 (mouse GPR103B)], prolactin releasing hormone receptor, [PRLHR, synonyms: GR3; GPR10; PrRPR; MGC126539; MGC126541, GenBank accession number: NM_004248 (human PRLHR)], somatostatin receptor, somatostatin receptor 1, [SSTR1, synonyms: SRIF-2, GenBank accession number: NM_001049 (human SSTR1)], somatostatin receptor 2, [SSTR2, GenBank accession number: NM_001050 (human SSTR2)], somatostatin receptor 3, [SSTR3, GenBank accession number:
NM_001051 (human SSTR3)], somatostatin receptor 4, [SSTR4, synonyms: SRIF-2, GenBank accession number: NM_001052 (human SSTR4)], somatostatin receptor 5, [SSTR5, GenBank accession number: NM_001053 (human SSTR5)], galanin receptor, galanin receptor 1, [GALR1, synonyms: GALNR; GALNR1, GenBank accession number: NM_001480 (human GALR1)], galanin receptor 2, [GALR2, synonyms: GALNR2; MGC125983; MGC125984, GenBank accession number: NM_003857 (human GALR2)], galanin receptor 3, [GALR3, GenBank accession number: NM_003614 (human GALR3)], cortistatin receptor, cortistatin receptor MAS-related GPR member X1, [MRGX1, synonyms: MRGPRX1, GPCR; SNSR4, GenBank accession number:
NM_147199 (human MRGX1)], cortistatin receptor MAS-related GPR member X2, [MRGX2, synonyms: MRGPRX2, GenBank accession number: NM_054030 (human MRGX2)], cortistatin receptor MAS-related GPR member X3, [MRGX3, synonyms: MRGPRX3, GPCR; SNSR1, GenBank accession number: NM_054031 (human MRGX3)], and/or cortistatin receptor MAS-related GPR member X4, [MRGX4, synonyms: MRGPRX4, GPCR; SNSR6; MGC129753; MGC129754, GenBank accession number: NM_054032 (human MRGX4)]".

Preferably, the at least one G-protein coupled receptor (GPCR) is selected from the group consisting of: "5-hydroxytryptamine (serotonin) receptor 2C [5-HT2C, synonyms: HTR1C; GenBank accession number: NM_000868 (human 5-HT2C)], muscarinic cholinergic receptor 3, [M3, synonyms: HM3, GenBank accession number: NM_000740 (human M3)], Beta-3-adrenergic receptor, [beta-3, synonyms: ADRB3, BETA3AR, GenBank accession number: NM_000025 (human beta-3)], angiotensin II receptor type 1, [AT1, synonyms: AGTR1, AG2S; AT1B; AT2R1; HAT1R; AGTR1A; AGTR1B; AT2R1A; AT2R1 B, GenBank accession number: NM_031850 (human AT1, transcript variant 4)], angiotensin II receptor type 2, [AT2, synonyms: AGTR2, ATGR2; MRX88, GenBank accession number: NM_000686 (human AT2)], gastrin-releasing peptide receptor, [GRPR, synonyms: BB2, GenBank accession number: NM_005314 (human GRPR)], bombesin-like receptor 3, [BRS3, synonyms: BB3, GenBank accession number: NM_001727 (human BRS3)], calcitonin receptor, [CALCR, synonyms: CT, CRT; CTR; CTR1, GenBank accession number: NM_001742 (human CALCR)], calcitonin receptor-like, [CALCRL, synonyms: CRLR; CGRPR, GenBank accession number:
NM_005795 (human CALCRL)], cannabinoid receptor 1, [CB1, synonyms: CNR1, CNR; CB-R; CB1A; CANN6; CB1K5, GenBank accession number: NM_016083 (human CB1, transcript variant 1)], cannabinoid receptor 2, [CB2, synonyms: CNR2, CX5, GenBank accession number: NM_001841 (human CB2)], cholecystokinin A receptor, [CCKAR, synonyms: CCK-A; CCKRA; CCK1-R, GenBank accession number:
NM_000730 (human CCKAR)], cholecystokinin B receptor, [CCKBR, synonyms: GASR; CCK-B, GenBank accession number: NM_176875 (human CCKBR)], corticotropin releasing hormone receptor 1, [CRF1, synonyms: CRHR1, CRHR; CRF-R; CRFR1; CRHRIf; CRH-R1 h, GenBank accession number: NM_004382 (human CRF1)], corticotropin releasing hormone receptor 2, [CRF2, synonyms: CRHR2, GenBank accession number: NM_001883 (human CRF2)], dopamine receptor D1, [D1, synonyms: DRD1, DADR; DRD1A, GenBank accession number: NM_000794 (human D1)], dopamine receptor D2, [D2, synonyms: DRD2, D2R; D2DR, GenBank accession number: NM_000795 (human D2, transcript variant 1)], dopamine receptor D3, [D3, synonyms: DRD3, D3DR; ETM1; FET1; MGC149204; MGC149205, GenBank accession number: NM_000796 (human D3, transcript variant a)], dopamine receptor D4, [D4, synonyms: DRD4, D4DR, GenBank accession number: NM_000797 (human D4)], dopamine receptor D5, [D5, synonyms: DRD5, DBDR; DRD1B; DRD1L2; MGC10601, GenBank accession number: NM_000798 (human D5)], glucagon-like peptide 1 receptor, [GLP-1, synonyms: GLP1R, MGC138331, GenBank accession number:
NM_002062 (human GLP-1)], follicle stimulating hormone receptor, [FSHR, synonyms: LGR1; ODG1; FSHRO; MGC141667; MGC141668, GenBank accession number:
NM_004246 (human FSHR)], histamine receptor H1, [H1, synonyms: HRH1, H1-R; hisH1, GenBank accession number: NM_000861 (human H1)], histamine receptor H3, [H3, synonyms: HRH3, HH3R; GPCR97, GenBank accession number: NM_007232 (human H3)], endothelial differentiation lysophosphatidic acid G-protein-coupled receptor 2, [EDG2, synonyms: LPA1, LPAR1; edg-2; vzg-1; Gpcr26; Mrec1.3; rec.1.3, GenBank accession number: NM_001401 (human EDG2, transcript variant 1)], melanin-concentrating hormone receptor 1, [MCH1, synonyms: MCHR1, SLC1; GPR24; MCH1R; MGC32129, GenBank accession number: NM_005297 (human MCH1)], melanocortin 1 receptor (alpha melanocyte stimulating hormone receptor), [MC1, synonyms: MCHR1, SLC1; GPR24; MCH1R; MGC32129, GenBank accession number: NM_005297 (human MC1)], melanocortin 3 receptor, [MC3, synonyms: MC3R, GenBank accession number: NM_019888 (human MC3)], melanocortin 4 receptor, [MC4, synonyms: MC4R, MGC126851; MGC138197, GenBank accession number:
NM 005912 (human MC4)], neuropeptide FF receptor 1, [NPFFR1, synonyms: NPFF1; GPR147; NPFF1R1; OT7T022; FLJ10751, GenBank accession number: NM_022146 (human NPFFR1)], neuropeptide FF receptor 2, [NPFFR2, synonyms: GPR74; NPFF2; NPGPR, GenBank accession number: NM_004885 (human NPFFR2)], neuropeptides B/W receptor 1, [NPBWR1, synonyms: GPR7; MGC129755, GenBank accession number: NM_005285 (human NPBWR1)], neuropeptides B/W receptor 2, [NPBWR2, synonyms: GPR8, GenBank accession number: NM 005286 (human NPBWR2)], neuropeptide Y receptor Y1, [NPY1R, synonyms: NPYR, GenBank accession number:
NM_000909 (human NPY1 R)], neuropeptide Y receptor Y2, [NPY2R, GenBank accession number: NM_000910 (human NPY2R)], neuropeptide Y receptor Y5, [NPY5R, GenBank accession number: NM_006174 (human NPY5R)], opioid receptor kappa 1, [OPRK1, synonyms: KOR; OPRK, GenBank accession number: NM_000912 (human OPRK1)], hypocretin (orexin) receptor 1, [HCRTR1, synonyms: OX1R, GenBank accession number: NM_001525 (human HCRTR1)], hypocretin (orexin) receptor 2, [HCRTR2, synonyms: OX2R, GenBank accession number: NM_001526 (human HCRTR2)], G protein-coupled receptor 103, [GPR103, synonyms: AQ27; SP9155; MGC149217, GenBank accession number: NM_198179 (human GPR103)], G protein-coupled receptor 103A, [GPR103A, GenBank accession number: NM_198192 (mouse GPR103A)], G protein-coupled receptor 103B, [GPR103B, GenBank accession number: AX665940 (mouse GPR103B)], prolactin releasing hormone receptor, [PRLHR, synonyms: GR3; GPR10; PrRPR; MGC126539; MGC126541, GenBank accession number: NM_004248 (human PRLHR)], galanin receptor 1, [GALR1, synonyms: GALNR; GALNR1, GenBank accession number: NM_001480 (human GALR1)] and/or cortistatin receptor MAS-related GPR member X2, [MRGX2, synonyms: MRGPRX2, GenBank accession number: NM_054030 (human MRGX2)]".

Most preferably, the at least one G-protein coupled receptor (GPCR) is selected from the group consisting of: "cholecystokinin A receptor, [CCKAR, synonyms: CCK-A; CCKRA; CCK1-R, GenBank accession number: NM_000730 (human CCKAR)], cholecystokinin B receptor, [CCKBR, synonyms: GASR; CCK-B, GenBank accession number: NM_176875 (human CCKBR)], neuropeptide FF receptor 1, [NPFFR1, synonyms: NPFF1; GPR147; NPFF1 R1; OT7T022; FLJ10751, GenBank accession number:
NM_022146 (human NPFFR1)], neuropeptide FF receptor 2, [NPFFR2, synonyms: GPR74; NPFF2; NPGPR, GenBank accession number: NM_004885 (human NPFFR2)], neuropeptide Y receptor Y1, [NPY1R, synonyms: NPYR, GenBank accession number: NM_000909 (human NPY1 R)], neuropeptide Y receptor Y2, [NPY2R, GenBank accession number: NM_000910 (human NPY2R)], neuropeptide Y receptor Y5, [NPY5R, GenBank accession number: NM_006174 (human NPY5R)], hypocretin (orexin) receptor 1, [HCRTR1, synonyms:OXIR, GenBank accession number:
NM_001525 (human HCRTR1)], hypocretin (orexin) receptor 2, [HCRTR2, synonyms: OX2R, GenBank accession number: NM_001526 (human HCRTR2)], G protein-coupled receptor 103, [GPR103, synonyms: AQ27; SP9155; MGC149217, GenBank accession number: NM_198179 (human GPR103)], G protein-coupled receptor 103A, [GPR103A, GenBank accession number: NM_198192 (mouse GPR103A)], G protein-coupled receptor 103B, [GPR103B, GenBank accession number: AX665940 (mouse GPR103B)], galanin receptor 1, [GALR1, synonyms: GALNR; GALNR1, GenBank accession number: NM_001480 (human GALR1)] and/or cortistatin receptor MAS-related GPR member X2, [MRGX2, synonyms: MRGPRX2, GenBank accession number: NM_054030 (human MRGX2)]".

Preferably, the compounds of the invention are antagonists and/or agonists of one or more of the above mentioned preferred, more preferred and/or most preferred GPCR, most preferably of G protein-coupled receptor 103, [GPR103, synonyms: AQ27; SP9155; MGC149217, GenBank accession number: NM_198179 (human GPR103)], G protein-coupled receptor 103A, [GPR103A, GenBank accession number: NM_198192 (mouse GPR103A)], G protein-coupled receptor 103B, [GPR103B, GenBank accession number: AX665940 (mouse GPR103B)].

The compounds of the invention being non-peptidic ligands of GPCR as described and defined herein are surprisingly characterized by a strong binding affinity to such receptors. Such compounds for instance may preferably exhibit an IC₅₀ value of less than 1000 nM for binding to a GPCR, for instance GPR103. More preferably, such compounds may exhibit an IC₅₀ value of less than 500 nM, even more preferably of less than 300 nM and most preferably of less than 100 nM for binding to a GPCR, such as GPR103.

Due to their surprisingly strong receptor binding, the compounds of the invention can be advantageously administered at lower doses compared to other less potent binders while still achieving equivalent or even superior desired biological effects. In addition, such a dose reduction may advantageously lead to less or even no medicinal adverse effects. Further, the high binding specificity of the compounds of the invention may translate into a decrease of undesired side effects on its own regardless of the dose applied.

Furthermore, the compounds of the invention, being of non-peptidic nature, are resistant to degradation by enzymes of the gastro-intestinal tract. Hence, they offer the advantage to be given by oral route. They surprisingly display an improved metabolic stability and/or an improved bioavailability. Hence, again an advantageous dose reduction may be achievable which may cause less or even no side effects.

In another aspect, the object of the invention has been surprisingly solved by providing the compounds of the invention according to the herein disclosed uses and treatments, where the physiological and/or pathophysiological conditions are mediated by at least one G-protein coupled receptor (GPCR) as described and defined herein and at least one other receptor (as defined herein) selected from the group consisting of: "GHS receptors, GHS type 1 receptor, GHS-R 1a, GHS-R 1b, motilin receptor, motilin receptor 1a, neurotensin receptor, TRH receptor, GPR38 (FM1), GPR39 (FM2), FM3, GHS-R subtype, GHS binding site, cardiac GHS-R, mammary GHS-R".

In a preferred embodiment, the treatment is achieved by modulation of one or more of the G-protein coupled receptors (GPCR) as described and defined herein and/or one or more of the other receptors (as defined herein).

In another preferred embodiment, the at least one G-protein coupled receptor (GPCR) is selected from the group consisting of: "cholecystokinin A receptor, [CCKAR, synonyms: CCK-A; CCKRA; CCK1-R, GenBank accession number: NM 000730 (human CCKAR)], cholecystokinin B receptor, [CCKBR, synonyms: GASR; CCK-B, GenBank accession number: NM_176875 (human CCKBR)], neuropeptide FF receptor 1, [NPFFR1, synonyms: NPFF1; GPR147; NPFF1R1; OT7T022; FLJ10751, GenBank accession number: NM 022146 (human NPFFR1)], neuropeptide FF receptor 2, [NPFFR2, synonyms: GPR74; NPFF2; NPGPR, GenBank accession number:
NM 004885 (human NPFFR2)], neuropeptide Y receptor Y1, [NPY1R, synonyms: NPYR, GenBank accession number: NM 000909 (human NPY1 R)], neuropeptide Y receptor Y2, [NPY2R, GenBank accession number: NM)_000910 (human NPY2R)], neuropeptide Y receptor Y5, [NPY5R, GenBank accession number: NM 006174 (human NPY5R)], hypocretin (orexin) receptor 1, [HCRTR1, synonyms: OX1R, GenBank accession number: NM 001525 (human HCRTR1)], hypocretin (orexin) receptor 2, [HCRTR2, synonyms: OX2R, GenBank accession number: NM 001526 (human HCRTR2)], G protein-coupled receptor 103, [GPR103, synonyms: AQ27; SP9155; MGC149217, GenBank accession number: NM_198179 (human GPR103)], G protein-coupled receptor 103A, [GPR103A, GenBank accession number: NM_198192 (mouse GPR103A)], G protein-coupled receptor 103B, [GPR103B, GenBank accession number: AX665940 (mouse GPR103B)], and/or galanin receptor 1, [GALR1, synonyms: GALNR; GALNR1, GenBank accession number: NM 001480 (human GALR1)]" and the at least one other receptor is selected from the group consisting of: "GHS type 1 receptor, GHS-R 1 a [GenBank accession number: NM_198407 (human GHS-R 1 a)], GHS-R 1 b [GenBank accession number: NM_004122 (human GHS-R 1 b)]". More preferably, the at least one G-protein coupled receptor (GPCR) is selected from the group consisting of: "G protein-coupled receptor 103, [GPR103, synonyms: AQ27; SP9155; MGC149217, GenBank accession number: NM_198179 (human GPR103)], G protein-coupled receptor 103A, [GPR103A, GenBank accession number: NM_198192 (mouse GPR103A)] and/or G protein-coupled receptor 103B, [GPR103B, GenBank accession number: AX665940 (mouse GPR103B)]" and the at least one other receptor is selected from the group consisting of: "GHS type 1 receptor, GHS-R 1 a [GenBank accession number: NM_198407 (human GHS-R 1a)], GHS-R 1b [GenBank accession number: NM_004122 (human GHS-R 1b)]".

In yet a further preferred embodiment, the compound of the invention is an antagonist of one or more of the G-protein coupled receptors (GPCR) as described and defined herein and/or one or more of the other receptors (as defined herein).

In yet a further preferred embodiment, the compound of the invention is an agonist of one or more of the G-protein coupled receptors (GPCR) as described and defined herein and/or one or more of the other receptors (as defined herein).

In yet a further preferred embodiment, the compound of the invention is an antagonist of one or more of the G-protein coupled receptors (GPCR) as described and defined herein and an agonist of one or more of the other receptors (as defined herein).

In yet a further preferred embodiment, the compound of the invention is an agonist of one or more of the G-protein coupled receptors (GPCR) as described and defined herein and an antagonist of one or more of the other receptors (as defined herein).

The terms "at least one GPCR", "one or more GPCRs", "at least one other receptor" and "one or more other receptors" in the course of the present invention mean that the treatment or prophylaxis as disclosed herein is achieved by modulation of one receptor or simultaneous modulation of two or more receptors as defined herein by at least one compound of the invention.

The compounds of the invention can either be antagonists or agonists of GPCR as described and defined herein and, optionally, in addition antagonists or agonists of the other receptors as described and defined herein.

Thus, the compounds of the invention are suitable for the treatment or prophylaxis of various physiological and/or pathophysiological conditions.

For the purpose of the present invention, all physiological and/or pathophysiological conditions are intended to be comprised that are known to be mediated by the GPCR as described and defined herein and, optionally, in addition the other receptors as described and defined herein.

Preferably, these physiological and/or pathophysiological conditions are selected from the group consisting of: "metabolic disorders, lipid metabolism, disorders of lipid metabolism, adipogenesis, adiposity, anorexia, body weight gain, body weight reduction, bulimia, cachexia, diabetes, diabetes type I, diabetes type II, energy balance, energy homeostasis, food intake, hyperlipidemia, hyperphagia, insulin resistance in the heart, insulin resistance in type 2 diabetic patients, insulin resistance including NIDDM, metabolic homeostasis maintenance, obesity, overweight, short-, medium- and/or long-term regulation of energy balance, short-, medium- and/or long-term regulation (stimulation and/or inhibition) of food intake, as well as diseases for which above mentioned physiological and/or pathophysiological conditions are risk factors and which are selected from the group consisting of: "premature death, diabetes, type 2 diabetes, heart disease, heart attack, coronary heart disease, congestive heart failure, stroke, hypertension (high blood pressure), gallbladder disease, osteoarthritis, sleep apnea, asthma, breathing problems, endometrial cancer, colon cancer, kidney cancer, gallbladder cancer, postmenopausal breast cancer, hypercholesterolemia (high blood cholesterol), insulin resistance, complications of pregnancy, menstrual irregularities, hirsutism, stress incontinence, increased surgical risk, psychological disorders such as depression (for the risk factors diseases, please refer to publication: "Statistics related to Overweight and Obesity, US Department of Health and Human Services, NIDDK 2006, http://win.niddk.nih.gov/publications/PDFs/stat904z.pdf).

More preferably, these physiological and/or pathophysiological conditions are selected from the group consisting of: "adipogenesis, adiposity, cachexia, diabetes, diabetes type I, diabetes type II, energy balance, energy homeostasis, food intake, hyperlipidemia, hyperphagia, obesity, short-, medium- and/or long-term regulation of energy balance, short-, medium- and/or long-term regulation (stimulation and/or inhibition) of food intake".

In a further aspect of the present invention, the compounds of the invention may be used in combination with at least one additional pharmacologically active substance.

Such additional pharmacologically active substance may be other compounds of the invention and/or other "suitable therapeutic agents" useful in the treatment or prophylaxis of the aforementioned physiological and/or pathophysiological conditions.

The additional pharmacologically active substance may be an antagonist or an agonist of GPCR as described and defined herein and, optionally, in addition an antagonist or an agonist of other receptors as described and defined herein, depending on the purpose of the combined use. Selection and combination of the additional pharmacologically active substance(s) can be easily performed by the skilled artisan on the basis of his expert knowledge and depending on the purpose of the combined use and physiological and/or pathophysiological conditions targeted.

In a preferred embodiment, the compounds of the invention are used for the treatment or prophylaxis of the aforementioned physiological and/or pathophysiological conditions in the form of a medicament, where such medicament comprises at least one additional pharmacologically active substance.

In another preferred embodiment, the compounds of the invention are used for the treatment or prophylaxis of the aforementioned physiological and/or pathophysiological conditions in the form of a medicament, where the medicament is applied before and/or during and/or after treatment with at least one additional pharmacologically active substance.

The above mentioned "suitable therapeutic agents" include: "GHS, anti-diabetic agents; anti-obesity agents; anti-hypertensive agents; cholesterol/lipid lowering agents; anabolic agents; testosterone and preferably, a "suitable therapeutic agents" is selected of the group consisting of this agents.

Examples of suitable GHS for use in combination with the compounds of the present invention include GHRP-6, GHRP-1 as described in U.S. Patent No. 4,411,890; and publications WO 89/07110 and WO 89/07111 and B-HT920 or growth hormone releasing factor and its analogs or growth hormone and its analogs or somatomedins including IGF-1 and IGF-2 as well as GHS described in WO 01/96300.

Examples of suitable anti-diabetic agents for use in combination with the compounds of the present invention include biguanides (e.g. metformin), glucosidase inhibitors (e.g. acarbose), insulins (including insulin secretagogues or insulin sensitizers), meglitinides (e.g. repaglinide), sulfonylureas (e.g., glimepiride, glyburide and glipizide), biguanide/glyburide combinations (e.g., glucovance), thiozolidinediones (e.g. troglitazone, rosiglitazone and pioglitazone), PPAR-alpha agonists, PPAR-gamma agonists, PPAR alpha/gamma dual agonists, SGLT2 inhibitors, inhibitors of fatty acid binding protein (aP2) such as those disclosed in US patent 6,548,529, glucagon-like peptide-1 (GLP-1), and dipeptidyl peptidase IV (DP4) inhibitors.

Examples of suitable anti-obesity agents for use in combination with the compounds of the present invention include endocannabinoid receptor antagonists, e.g. CB1 receptor antagonists such as rimonabant (1H-Pyrazole-3-carboxamide, 5-(4-chlorophenyl)-1-(2,4-dichlorophenyl)-4-methyl-N-1-piperidinyl-, monohydrochloride; CAS Registry Number: 158681-13-1; SR-141716A; US patent 5,624,941), aP2 inhibitors such as those disclosed in US patent 6,548,529, PPAR gamma antagonists, PPAR delta agonists, orlistat and neurotransmitter reuptake inhibitors, such as sibutramine [1-(4-chlorophenyl)-N,N-dimethyl-alpha-(2-methylpropyl)-cyclobutane-methanamine; CAS Registry Number: 106650-56-0] and sibutramide hydrochloride monohydrate, which block the reuptake of the neurotransmitters dopamine, norepinephrine and serotonin.

Examples of suitable anti-hypertensive agents for use in combination with the compounds of the present invention include beta adrenergic blockers, calcium channel blockers (L-type and T-type; e.g. diltiazem, verapamil, nifedipine, amlodipine and mybefradii), diruetics (e.g., chlorothiazide, hydrochlorothiazide, flumethiazide, hydroflumethiazide, bendroflumethiazide, methylchlorothiazide, trichloromethiazide, polythiazide, benzthiazide, ethacrynic acid tricrynafen, chlorthalidone, furosemide, musolimine, bumetanide, triamtrenene, amiloride, spironolactone), renin inhibitors, ACE inhibitors (e.g., captopril, zofenopril, fosinopril, enalapril, ceranopril, cilazopril, delapril, pentopril, quinapril, ramipril, lisinopril), AT-1 receptor antagonists (e.g., losartan, irbesartan, valsartan), ET receptor antagonists (e.g., sitaxsentan, atrsentan and compounds disclosed in U.S. Patent Nos. 5,612,359 and 6,043,265, Dual ET/All antagonist (e.g., compounds disclosed in WO 00/01389), neutral endopeptidase (NEP) inhibitors, vasopepsidase inhibitors (dual NEP-ACE inhibitors) (e.g., omapatrilat and gemopatrilat), and nitrates.

Examples of suitable cholesterol/lipid lowering agents for use in combination with the compounds of the present invention include HMG-CoA reductase inhibitors [e.g., pravastatin lovastatin, atorvastatin, simvastatin, NK-104 (a.k.a. itavastatin, or nisvastatin or nisbastatin] and ZD-4522 (a.k.a. rosuvastatin, or atavastatin or visastatin)), squalene synthetase inhibitors, fibrates, bile acid sequestrants, ACAT inhibitors, MTP inhibitors, lipooxygenase inhibitors, choesterol absorption inhibitors, and cholesterol ester transfer protein inhibitors (e.g., CP-529414).

Examples of suitable anabolic agents for use in combination with the compounds of the present invention include testosterone and SARMs.

The above other therapeutic agents, when employed in combination with the compounds of the present invention, may be used, for example, in those amounts indicated in the Physicians' Desk Reference (PDR) or as otherwise determined by one of ordinary skill in the art.

In a preferred embodiment, the compounds of the invention are used for the treatment or prophylaxis of the aforementioned physiological and/or pathophysiological conditions in the form of a medicament, where such medicament comprises at least one additional pharmacologically active substance selected from the group consisting of: "GHS receptor agonist and/or antagonist, GHS type 1 receptor agonist and/or antagonist, GHS-R 1 a agonist and/or antagonist, GHS-R 1 b agonist and/or antagonist, motilin receptor agonist and/or antagonist, motilin receptor 1 a agonist and/or antagonist, neurotensin receptor agonist and/or antagonist, TRH receptor agonist and/or antagonist, GPR38 (FM1) agonist and/or antagonist, GPR39 (FM2) agonist and/or antagonist, FM3 agonist and/or antagonist, GHS-R subtype agonist and/or antagonist, GHS binding site agonist and/or antagonist, cardiac GHS-R agonist and/or antagonist, mammary GHS-R agonist and/or antagonist, endocannabinoid receptor agonist and/or antagonist, CB1 receptor agonist and/or antagonist, rimonabant [1H-Pyrazole-3-carboxamide, 5-(4-chlorophenyl)-l-(2,4-dichlorophenyl)-4-methyl-N-1-piperidinyl-, monohydrochloride], sibutramine [1-(4-chlorophenyl)-N,N-dimethyl-alpha-(2-methylpropyl)-cyclobutane-methanamine; CAS Registry Number: 106650-56-0], sibutramide hydrochloride monohydrate".

In another preferred embodiment, the compounds of the invention are used for the treatment or prophylaxis of the aforementioned physiological and/or pathophysiological conditions in the form of a medicament, where the medicament is applied before and/or during and/or after treatment with at least one additional pharmacologically active substance selected from the group consisting of: "GHS receptor agonist and/or antagonist, GHS type 1 receptor agonist and/or antagonist, GHS-R 1 a agonist and/or antagonist, GHS-R 1 b agonist and/or antagonist, motilin receptor agonist and/or antagonist, motilin receptor 1 a agonist and/or antagonist, neurotensin receptor agonist and/or antagonist, TRH receptor agonist and/or antagonist, GPR38 (FM1) agonist and/or antagonist, GPR39 (FM2) agonist and/or antagonist, FM3 agonist and/or antagonist, GHS-R subtype agonist and/or antagonist, GHS binding site agonist and/or antagonist, cardiac GHS-R agonist and/or antagonist, mammary GHS-R agonist and/or antagonist, endocannabinoid receptor agonist and/or antagonist, CB1 receptor agonist and/or antagonist, rimonabant [1 H-Pyrazole-3-carboxamide, 5-(4-chlorophenyl)-1-(2,4-dichlorophenyl)-4-methyl-N-1-piperidinyl-, monohydrochloride], sibutramine [1-(4-chlorophenyl)-N,N-dimethyl-alpha-(2-methylpropyl)-cyclobutane-methanamine; CAS Registry Number: 106650-56-0], sibutramide hydrochloride monohydrate".

The compounds of the present invention and/or, where appropriate, the additional pharmacologically active substances can be administered in a known manner. The route of administration may thereby be any route which effectively transports the active compound to the appropriate or desired site of action, for example orally or non-orally, in particular topically, transdermally, pulmonary, rectally, intravaginally, nasally or parenteral or by implantation. Oral administration is preferred.

The compounds of the invention and/or where appropriate the additional pharmacologically active substances are converted into a form which can be administered and are mixed where appropriate with pharmaceutically acceptable carriers or diluents. Suitable excipients and carriers are described for example in Zanowiak P, Ullmann's Encyclopedia of Industrial Chemistry 2005, Pharmaceutical Dosage Forms, 1-33; Spiegel AJ et al., Journal of Pharmaceutical Sciences 1963, 52: 917-927; Czetsch-Lindenwald H, Pharm. Ind. 1961, 2: 72-74; Fiedler HP, Lexikon der Hilfsstoffe für Pharmazie, Kosmetik and angrenzende Gebiete 2002, Editio Cantor Verlag, p65-68.

Oral administration can take place for example in solid form as tablet, capsule, gel capsule, coated tablet, granulation or powder, but also in the form of a drinkable solution. The compounds of the invention can for oral administration be combined with known and ordinarily used, physiologically tolerated excipients and carriers such as, for example, gum arabic, talc, starch, sugars such as, for example, mannitol, methylcellulose, lactose, gelatin, surface-active agents, magnesium stearate, cyclodextrins, aqueous or nonaqueous carriers, diluents, dispersants, emulsifiers, lubricants, preservatives and flavorings (e.g. essential oils). The compounds of the invention can also be dispersed in a microparticulate, e.g. nanoparticulate, composition.

Non-oral administration can take place for example by intravenous, subcutaneous, intramuscular injection of sterile aqueous or oily solutions, suspensions or emulsions, by means of implants or by ointments, creams or suppositories. Administration as sustained release form is also possible where appropriate. Implants may comprise inert materials, e.g. biodegradable polymers or synthetic silicones such as, for example, silicone rubber. Intravaginal administration is possible for example by means of vaginal rings. Intrauterine administration is possible for example by means of diaphragms or other suitable intrauterine devices. Transdermal administration is additionally provided, in particular by means of a formulation suitable for this purpose and/or suitable means such as, for example, patches.

The dosage may vary within a wide range depending on type and/or severity of the physiological and/or pathophysiological condition, the mode of administration, the age, gender, bodyweight and sensitivity of the subject to be treated. It is within the ability of a skilled worker to determine a "pharmacologically effective amount" of a compound of the invention and/or additional pharmacologically active substance. Administration can take place in a single dose or a plurality of separate dosages.

A suitable unit dose is, for example, from 0.001 mg to 100 mg of the active ingredient, i.e. at least one compound of the invention and, where appropriate, at least one additional pharmacologically active substance, per kg of a patient's bodyweight.

### General syntheses schemes

The compounds of the present invention may be prepared according to the following general synthetic schemes, as well as relevant published literature procedures that are known to the one skilled in the art (e.g. WO 00/54729 and cited references therein).

Exemplary reagents and procedures for these reactions appear hereinafter and in the working examples. Unless otherwise specified, the various substituents (radicals) of the compounds have the meanings as defined for formula (I) herein.

Amide bond formation (peptide coupling) is conducted under standard peptide coupling procedures known in the prior art. Optimally, the reaction is conducted in a solvent such as dichloromethane (DCM) at room temperature using benzotriazol-1-yl-oxytris(dimethylamino)phosphonium-hexafluoorophosphate (BOP) (Castro B et al., Tetrahedron Lett. 1975, 14:1219-1222) and a base, for example N-methyl-morpholine or diisopropylethylamine.

Thionation of the formed amide was performed using Lawesson's reagent (Pons JF et al., Tetrahedron Lett. 2000, 41: 4965-4968).

Cyclisation: the obtained thioamide was then submitted to the conditions reported by Hitosuyanagi et al. (Hitotsuyanagi Y. et al., J. Org. Chem. 2002, 67: 3266-3271) which were slightly modified (5 eq. of hydrazide and 1.1 eq. of mercury (II) acetate in acetonitrile). Cyclisation into triazoles was generally achieved within three hours. When the hydrazide was not commercially available, it was prepared by known methods from its acid or methyl ester precursors.

Deprotection of the tert-butyloxycarbonyl group (Boc) was performed at room temperature in acidic medium as usually described.

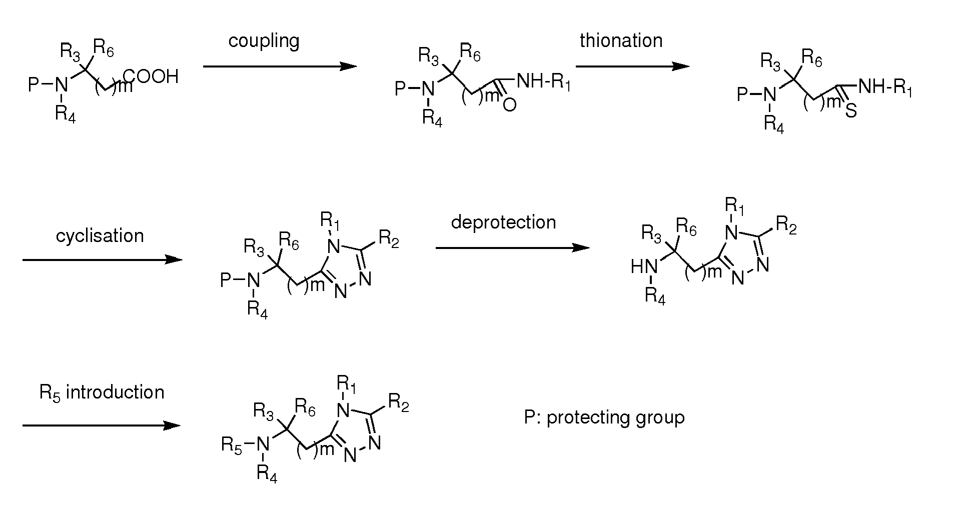

For R5 = -CO-alkyl, -CO-cycloalkyl, -CO-cycloalkylalkyl, -CO-aryl, -CO-arylalkyl, -CO-heteroaryl, -CO-heteroarylalkyl, -CO-heterocyclyl, -CO-heterocyclylalkyl, -CO-C*(R9R10)-NH₂, -CO-CH₂-C*(R9R10)-NH₂, -CO-C*(R9R10)-CH₂-NH₂ (R):

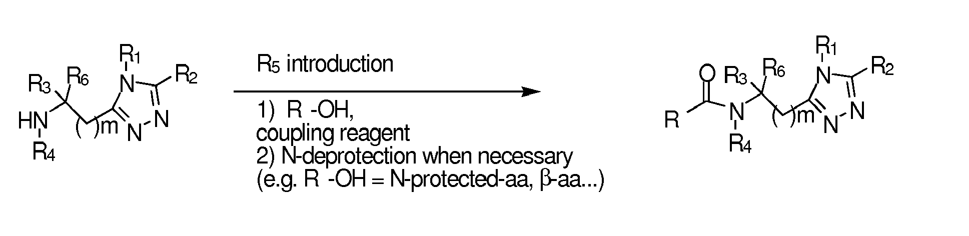

For R5 = alkyl, cycloalkyl, cycloalkylalkyl, aryl, heteroaryl, arylalkyl, heteroarylalkyl, heterocyclyl, heterocyclylalkyl (R):

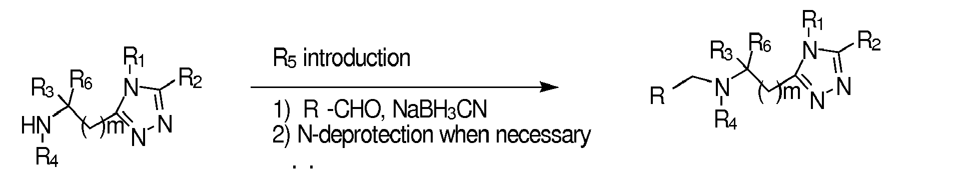

For R5 = alkyl-SO₂-, aryl-SO₂-, arylalkyl-SO₂- (R = alkyl, aryl, arylalkyl):

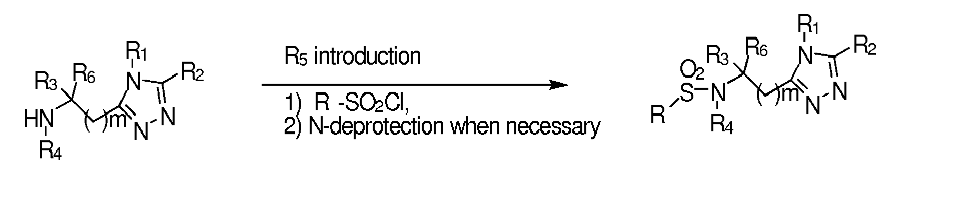

The compounds of the invention, especially compounds 1 to 190 were named from the drawn formula using the Chem Draw Ultra 8 software (CambridgeSoft Corporation, Cambridge, USA).

### Brief description of the drawings

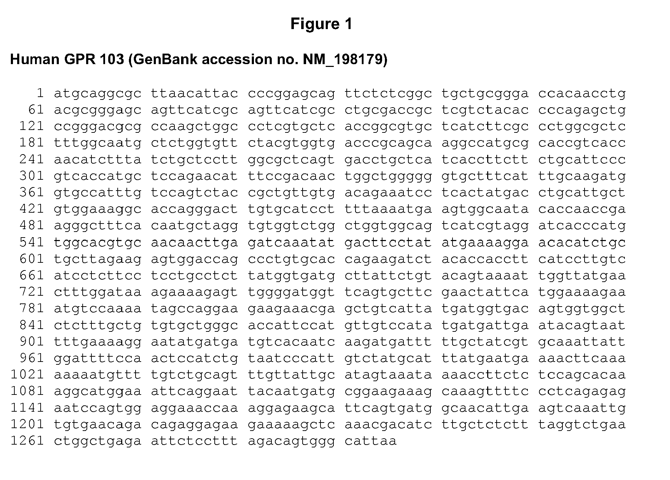

**Figure 1** depicts the nucleotide sequence (5'→3') of human GPR103 (GenBank accession no. NM_198179).

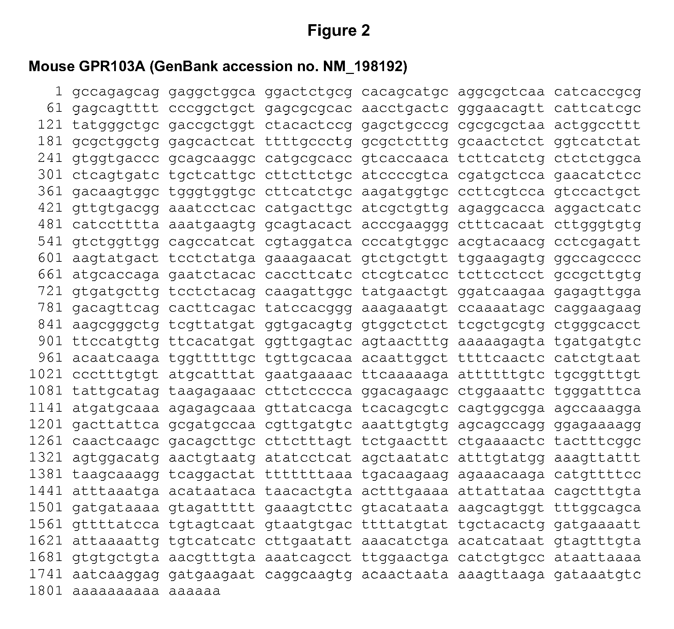

**Figure 2** depicts the nucleotide sequence (5'→3') of mouse GPR103A (GenBank accession no. NM_198192).

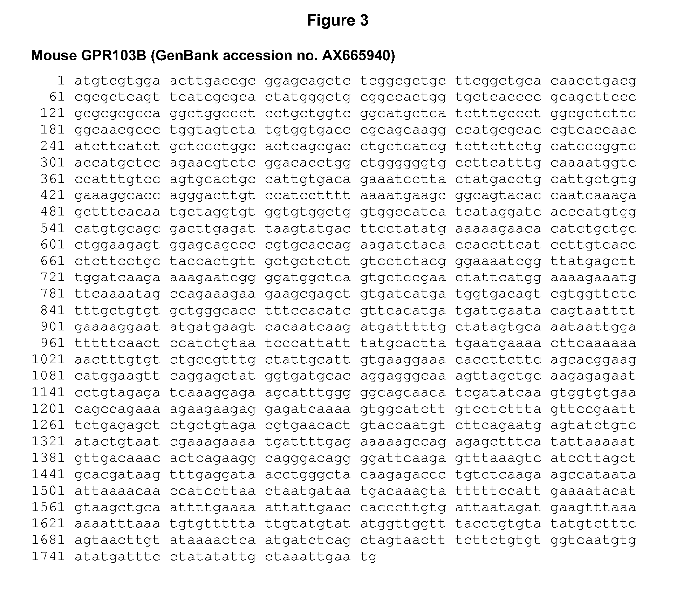

**Figure 3** depicts the nucleotide sequence (5'→3') of mouse GPR103B (GenBank accession no. AX665940).

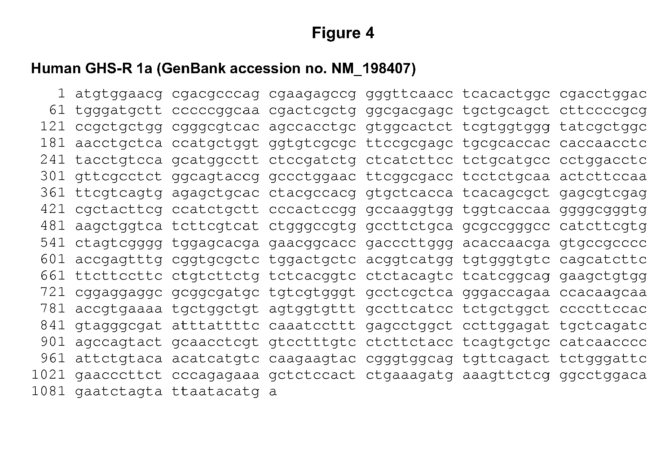

**Figure 4** depicts the nucleotide sequence (5'→3') of human GHS-R 1 a (GenBank accession no. NM_198407).

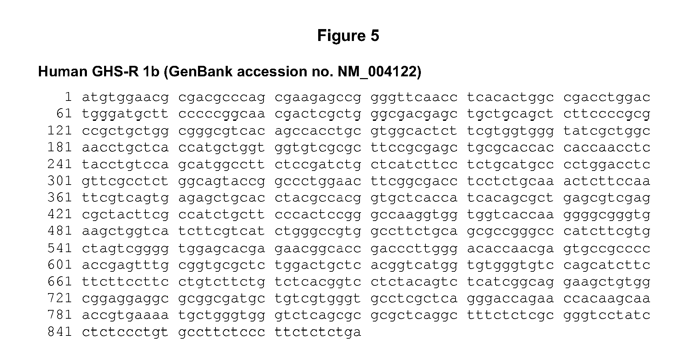

**Figure 5** depicts the nucleotide sequence (5'→3') of human GHS-R 1 b (GenBank accession no. NM_004122).
**Figure 6** shows the analysis of the mRNA expression of GPR103 receptors and QRFP peptide in mice adipocytes.
**Figure 7** shows the inhibitory effect of QRFP-43 and QRFP-26 on isoproterenol-induced lipolysis.
**Figure 8** shows the inhibitory effects of **compound 79** on the inhibitory effect of QRFP-26 on isoproterenol-induced lipolysis.
**Figure 9** shows the inhibitory effects of **compound 39** on the inhibitory effect of QRFP-26 on isoproterenol-induced lipolysis.

The contents of all cited references and patents are hereby incorporated by reference in their entirety. The invention is explained in more detail by means of the following examples without, however, being restricted thereto.

### Examples

### I) Synthesis of compounds of the invention

### Example 1:

### (R)-N-(1-(5-(2-(1H-indol-3-yl)ethyl)-4-(2,4-dimethoxybenzyl)-4H-1,2,4-triazol-3-yl)-2-(1H-indol-3-yl)ethyl)-2-amino-2-methylpropanamide (Compound 1)

Compound 1 was obtained from Boc-(D)-Trp (10 mmoles), (2,4-dimethoxyphenyl)-methanamine, 3-(1H-indol-3-yl)propane hydrazide and Boc-2-amino-2-methylpropanoic acid according to the general synthetic schemes with a total yield after purification by HPLC of 35%.
¹H NMR (400 MHz, 300°K, DMSO-d⁶):
δ 1.32 (3H, s, CH₃ Aib), 1.36 (3H, s, CH₃ Aib), 2.93 (2H, m, CH₂-CH₂-indole), 2.97 (2H, m, CH₂-CH₂-indole), 3.31 (1H, dd, J=14.5, J=6.1, 1 H CH₂ βTrp), 3.38 (1H, dd, J=14.5, J=9.1, 1 H CH₂ βTrp), 3.66 (3H, s, *o*-OCH₃), 3.72 (3H, s, *p*-OCH₃), 4.93 (1H, d, J=16.9, 1H CH₂ *o*,*p*-dimethoxybenzyl), 5.10 (1H, d, J=16.9, 1 H CH₂ *o*,*p*-dimethoxybenzyl), 5.23 (1H, m, CαH Trp), 6.31 (1H, dd, J=8.5, J=1.7, H₅ *o,p*-dimethoxybenzyl), 6.45 (1H, d, J=8.5, H₆ *o,p*-dimethoxybenzyl), 6.59 (1H, d, J=1.7, H₃ *o,p*-dimethoxybenzyl), 6.88 (1H, t, J=7.5, H₅ Trp), 6.94 (1H, t, J=7.5, H₅ indole), 7.04 (1H, t, H₆ Trp), 7.06 (1H, t, H₆ indole), 7.08 (1H, s, H₂ indole), 7.11 (1H, s, H₂ Trp), 7.18 (1H, d, J=7.9, H₄ Trp), 7.33 (3H, H₄, H₇ indole, H₇ Trp), 8.05 (2H, s, NH₂ Aib), 8.95 (1H, d, J=7.9, NH Trp), 10.80 (1H, s, NH indole), 10.82 (1H, s, NH indole Trp).
¹³C NMR (400 MHz, DMSO-d⁶):
δ 22.4 (CH₂-CH₂ indole), 23.2 (CH₃ Aib), 23.3 (CH₃ Aib), 25.4 (CH₂-CH₂ indole), 28.7 (Cβ Trp), 41.3 (CH₂- *o,p*-dimethoxybenzyl), 45.3 (Cα Trp), 55.2 (*p*-OCH₃), 55.4 (*o-*OCH₃), 56.3 (Cq Aib), 98.6 (C₃ *o,p*-dimethoxybenzyl), 104.7 (C₅ *o,p*-dimethoxybenzyl), 109.5 (C₃ Trp), 111.3 (C₇ Trp, C₇ indole), 112.9 (C₃ indole), 115.2 (C₁ *o,p-*dimethoxybenzyl), 117.8 (C₄ indole), 117.9 (C₄ Trp), 118.2 (C₅ Trp, C₅ indole), 120.9 (C₆ Trp, C₆ indole), 122.4 (C₂ indole), 124.3 (C₂ Trp), 126.8 (C₉ Indole), 126.9 (C₉ Trp), 127.5 (C₆ *o,p*-dimethoxybenzyl), 136.0 (C₈ Trp), 136.2 (C₈ indole), 154.6 (2Cq triazole), 157.3 (C₂ *o,p*-dimethoxybenzyl), 160.4 (C₄ *o,p*-dimethoxybenzyl), 171.3 (CO Aib).
ESI-MS: found: m/z 606.3 [M+H]⁺/ calculated: 604.3 g/mol

### Example 2:

### (R)-N-(1-(5-benzyl-4-(naphthalen-1-ylmethyl)-4H-1,2,4-triazol-3-yl)-2-(1H-indol-3-yl)ethyl)-2-amino-2-methylpropanamide (Compound 4)

Compound 4 was obtained from Boc-(D)-Trp (10 mmoles), naphthalen-1-yl-methanamine, 2-phenylacetohydrazide and Boc-2-amino-2-methylpropanoic acid according to the general synthetic schemes with a total yield after purification by HPLC of 42%.
¹H NMR (300 MHz, DMSO-d⁶, 300°K):
δ (ppm) 1.18 (3H, s, CH₃ Aib), 1.24 (3H, s, CH₃ Aib), 3.17 (1H, dd, J= 14 Hz and 5 Hz, CH₂ βTrp), 3.36 (1H, dd, J= 14 and 9 Hz, CH₂ βTrp), 4.05 (2H, m, CH₂-benzyl), 4.90 (1H, m, CH αTrp), 5.65 (1H, d, J= 18 Hz, CH₂-naphtyl), 5.81 (1H, d, J= 18 Hz, CH₂-naphtyl), 6.12 (1H, d, Jₒ= 7 Hz, H₂ naphtyl), 6.38 (1H, t, Jₒ= 7 Hz, H₅ Trp), 6.47 (1H, d, Jₒ= 8 Hz, H₄ Trp), 6.85 (1H, t, Jₒ= 8 Hz, H₆ Trp), 7.03 (1H, d, Jₘ= 2 Hz, H₂ Trp), 7.05-7.12 (5H, m, CHar benzyl), 7.15 (1H, d, Jₒ= 8 Hz, H₇ Trp), 7.19 (1H, d, Jₒ= 8 Hz, H₃ naphtyl), 7.58 (2H, m, H₆ and H₇ naphtyl), 7.81 (1H, d, Jₒ= 8 Hz, H₄ naphtyl), 7.89-8.01 (5H, m, NH₂ Aib, H₅ and H₈ naphtyl), 8.92 (1H, d, J= 8 Hz, NH amide), 10.73 (1H, s, NH indole Trp).
¹³C NMR (75 MHz, DMSO-d⁶, 300°K):
δ (ppm) 23.5 (CH₃ Aib), 23.6 (CH₃ Aib), 29.2 (CH₂ βTrp), 30.5 (CH₂-benzyl), 44.0 (CH₂-naphtyl), 45.6 (CH αTrp), 56.6 (Cq Aib), 109.7 (C₃ Trp), 111.7 (C₇ Trp), 117.9 (C₄ Trp), 118.4 (C₅ Trp), 121.1 (C₆ Trp), 122.1 (C₂ naphtyl), 122.8 (C₈ naphtyl), 124.9 (C₂ Trp), 125.7 (C₃ naphtyl), 126.7 (C₆ naphtyl), 126.9 (C₉ Trp), 127.0 (C₇ naphtyl), 128.2 (C₄ benzyl), 128.7-129.1 (C₂, C₃, C₅ and C₆ benzyl, C₄ and C₅ naphtyl), 129.9 (C₉ naphtyl), 131.5 (C₁ naphtyl), 133.5 (C₁₀ naphtyl), 136.2 (C₁ benzyl), 136.4 (C₈ Trp), 154.2 (Cq triazole), 155.7 (Cq triazole), 171.9 (CO Aib).
ESI-MS: found: m/z 543.4 [M+H]⁺/ calculated: 542.2 g/mol

### Example 3:

### (R)-N-(1-(5-(2-(1H-indl-3-yl)ethyl)-4-(naphthalen-1-ylmethyl)-4H-1,2,4-triazol-3-yl)-2-(1H-indol-3-yl)ethyl)-2-amino-2-methylpropanamide (Compound 5)

Compound 5 was obtained from Boc-(D)-Trp (10 mmoles), naphthalen-1-yl-methanamine, 3-(1H-indol-3-yl)propane hydrazide and Boc-2-amino-2-methylpropanoic acid according to the general synthetic schemes with a total yield after purification by HPLC of 33%.
¹H NMR (400 MHz, DMSO-d⁶, 300°K):
δ (ppm) 1.25 (3H, s, CH₃ Aib), 1.28 (3H, s, CH₃ Aib), 2.93 (2H, m, CH₂ -CH₂-indole), 3.01 (2H, m, CH₂-CH₂ -indole), 3.30 (1H, dd, ³J= 14.3 and 5.8 Hz, CH₂ βTrp), 3.40 (1H, dd, ³J= 14.3 and 8.8 Hz, CH₂ βTrp), 5.03 (1H, m, CH αTrp), 5.62 (1H, d, J= 18.0 Hz, CH₂-naphtyl), 5.76 (1H, J= 18.0 Hz, CH₂-naphtyl), 3.36 (1H, d, Jₒ= 7.2 Hz, H₂ naphtyl), 6.51 (1H, t, Jₒ= 7.4 Hz, H₅ Trp), 6.72 (1H, d, Jₒ= 7.9 Hz, H₄ Trp), 6.76 (1H, t, Jₒ= 7.5 Hz, H₅ indole), 6.92 (1H, t, Jₒ= 7.5 Hz, H₆ Trp), 7.0 (1H, t, Jₒ= 7.5 Hz, H₆ indole), 7.02 (1H, d, J= 2.0 Hz, H₂ indole), 7.09 (1H, d, J= 2.0 Hz, H₂ Trp), 7.13 (1H, d, Jₒ= 7.9 Hz, H₄ indole), 7.26 (1H, Jₒ= 7.9 Hz, H₇ Trp), 7.27 (1H, t, Jₒ= 8.2 Hz, H₃ naphtyl), 7.29 (1H, d, H₇ indole), 7.58-7.64 (2, m, H₆ and H₇ naphtyl), 7.88 (1H, d, Jₒ= 8.2 Hz, H₄ naphtyl), 7.93 (1H, d, Jₒ= 7.9 Hz, H₈ naphtyl), 7.98 (3H, brs, NH₂ Aib), 8.03 (1H, d, Jₒ= 8.2 Hz, H₅ naphtyl), 8.96 (1H, d, Jₒ= 7.9 Hz, NH Trp), 10.75 (1H, brs, NH indole), 10.77 (1H, brs, NH indole Trp).
¹³C NMR (100 MHz, DMSO-d⁶, 300°K):
δ (ppm) 22.6 (CH₂-CH₂-indole), 23.1 (CH₃ Aib), 23.2 (CH₃ Aib), 25.3 (CH₂-CH₂-indole), 28.8 (CH₂ βTrp), 43.3 (CH₂-naphtyl), 45.3 (CH αTrp), 56.2 (Cq Aib), 109.4 (C₃ Trp), 111.2 (C₇ indole and C₇ Trp), 112.9 (C₃ indole), 117.5 (C₄ Trp), 117.8 (C₄ indole), 118.0 (C₅ Trp), 118.1 (C₅ indole), 120.7 (C₆ Trp), 120.8 (C₆ indole), 121.6 (C₂ naphtyl), 122.5 (C₂ indole and C₈ naphtyl), 124.4 (C₂ Trp), 125.4 (C₃ naphtyl), 126.3 (C₆ naphtyl), 126.6 (C₉ indole, C₉ Trp and C₇ naphtyl), 127.9 (C₄ naphtyl), 128.6 (C₅ naphtyl), 129.5 (C₉ naphtyl), 131.4 (C₁ naphtyl), 133.1 (C₁₀ naphtyl), 135.9 (C₈ Trp), 136.1 (C₈ indole), 154.7 (2 Cq triazole), 171.4 (CO Aib).
ESI-MS: found: m/z 596.4 [M+H]⁺/ calculated: 595.3 g/mol

### Example 4:

### (R)-N-(1-(5-(2-(1H-indol-3-yl)ethyl)-4-(3-methoxybenzyl)-4H-1,2,4-triazol-3-yl)-2-(1H-indol-3-yl)ethyl)-2-amino-2-methylpropanamide (Compound 6)

Compound 6 was obtained from Boc-(D)-Trp (10 mmoles), (3-methoxyphenyl)-methanamine, 3-(1H-indol-3-yl)propane hydrazide and Boc-2-amino-2-methylpropanoic acid according to the general synthetic schemes with a total yield after purification by HPLC of 25%.
¹H NMR (400 MHz, DMSO-d⁶):
δ (ppm) 1.28 (3H, s, CH₃ Aib), 1.30 (3H, s, CH₃ Aib), 2.92 (2H, m, CH₂-CH₂-indole), 2.98 (2H, m, CH₂-CH₂-indole), 3.33 (1H, dd, J=14.5, J= 6.2, 1 H CH₂ βTrp), 3.40 (1H, dd, J=14.5, J= 8.8, 1 H CH₂ βTrp), 3.66 (3H, s, OCH₃), 5.09 (2H, m, CH₂ *m-*methoxybenzyl), 5.22 (1H, m, CαH Trp), 6.38 (1H, d, J=7.5, H₆ *m*-methoxybenzyl), 6.59 (1H, s, H₂ *m*-methoxybenzyl), 6.86 (1H, t, H₅ Trp), 6.87 (1H, d, H₄ *m*-methoxybenzyl), 6.92 (1H, t, J=7.5, H₅ indole), 7.03 (1H, t, J=7.9, H₆ Trp), 7.05 (1H, t, H₆ indole), 7.07 (1H, s, H₂ indole), 7.11 (1H, s, H₂ Trp), 7.18 (1H, t, H₅ *m*-methoxybenzyl), 7.19 (1H, d, H₄ Trp), 7.31 (1H, H₄ indole), 7.32 (2H, H₇ Trp, H₇ indole), 8.00 (2H, s, NH₂ Aib), 8.96 (1H, d, J=8.1, NH Trp), 10.78 (1H, s, NH indole), 10.80 (1H, s, NH indole Trp).
¹³C NMR (400 MHz, DMSO-d⁶):
δ (ppm) 22.4 (CH₂-CH₂ indole), 23.1 (CH₃ Aib), 23.3 (CH₃ Aib), 25.4 (CH₂-CH₂ indole), 28.7 (Cβ Trp), 45.3 (CH₂ *m*-methoxybenzyl), 45.4 (Cα Trp), 55.0 (OCH₃), 56.3 (Cq Aib), 109.5 (C₃ Trp), 111.3 (C₇ Trp, C₇ indole), 112.0 (C₂ *m*-methoxybenzyl), 113.0 (C₄ m-methoxybenzyl, C₃ indole), 117.8 (C₄ Trp, C₆ *m*-methoxybenzyl), 118.0 (C₄ indole), 118.2 (C₅ indole), 118.3 (C₅ Trp), 120.8 (C₆ indole), 120.9 (C₆ Trp), 122.4 (C₂ indole), 124.3 (C₂ Trp), 126.7 (C₉ indole), 126.9 (C₉ Trp), 130.0 (C₅ *m*-methoxybenzyl), 136.0 (C₈ indole), 136.1 (C₈ Trp), 137.2 (C₁ m-methoxybenzyl), 154.3 (2Cq triazole), 159.6 (C₃ m-methoxybenzyl), 171.4 (CO Aib).
ESI-MS: found: m/z 576.6 [M+H]⁺/ calculated: 575.3 g/mol

### Example 5:

### (R)-N-(1-(4-(3-methoxybenzyl)-5-benzyl-4H-1,2,4-triazol-3-yl)-2-(1H-indol-3-yl)ethyl)-2-amino-2-methylpropanamide (Compound 7)

Compound 7 was obtained from Boc-(D)-Trp (10 mmoles), (3-methoxyphenyl)-methanamine, 2-phenylacetohydrazide and Boc-2-amino-2-methylpropanoic acid according to the general synthetic schemes with a total yield after purification by HPLC of 30%.
¹H NMR (400 MHz, DMSO-d⁶):
δ (ppm) 1.25 (3H, s, CH₃ Aib), 1.29 (3H, s, CH₃ Aib), 3.24 (1H, dd, J=14.3, J=5.8, 1 H CH₂ βTrp), 3.38 (1H, dd, J=14.3, J=9.1, 1 H CH₂ βTrp), 3.61 (3H, s, *m*-OCH₃), 4.04 (2H, m, CH₂ benzyl), 5.07 (1H, d, J= 17.4, 1 H CH₂ *m*-methoxybenzyl), 5.13 (1H, d, J= 17.4, 1H CH₂ *m*-methoxybenzyl), 5.14 (1H, m, CαH Trp), 6.32 (1H, d, J=7.8, H₆ *m-*methoxybenzyl), 6.40 (1H, m, H₂ *m*-methoxybenzyl), 6.82 (1H, t, H₅ Trp), 6.83 (1H, d, J=7.8, H₄ *m*-methoxybenzyl), 7.01 (1H, t, J=8.2, H₆ Trp), 7.04 (1H, d, J=8.2, H₄ Trp), 7.06 (1H, d, J=2.0, H₂ Trp), 7.12 (2H, m, H₂, H₆ Benzyl), 7.13 (1H, t, J=7.9, H₅ *m-*methoxybenzyl), 7.20 (1H, m, H₄ benzyl), 7.24 (2H, m, H₃, H₅ benzyl), 7.29 (1H, d, J=8.2, H₇ Trp), 7.99 (2H, s, NH₂ Aib), 8.92 (1H, d, J=8.2, NH Trp), 10.77 (1H, s, NH indole Trp).
¹³C NMR (400 MHz, DMSO-d⁶):
δ (ppm) 23.0 (CH₃ Aib), 23.3 (CH₃ Aib), 28.6 (Cβ Trp), 30.1 (CH₂ benzyl), 45.2 (Cα Trp), 45.6 (CH₂- *m*-methoxybenzyl), 54.9 (*m-*OCH₃), 56.2 (Cq Aib), 109.4 (C₃ Trp), 111.2 (C₇ Trp), 111.7 (C₂ *m*-methoxybenzyl), 113.1 (C₄ *m*-methoxybenzyl), 117.9 (C₄ Trp, C₆ *m-*methoxybenzyl), 118.2 (C₅ Trp), 120.8 (C₆ Trp), 124.3 (C₂ Trp), 126.6 (C₄ benzyl), 126.8 (C₉ Trp), 128.3 (C₃, C₅ benzyl), 128.4 (C₂, C₆ benzyl), 129.9 (C₅ *m-*methoxybenzyl), 135.9 (C₁ benzyl, C₈ Trp), 137.0 (C₁ *m*-methoxybenzyl), 153.5 (Cq triazole), 154.8 (Cq triazole), 159.5 (C₃ *m*-methoxybenzyl), 171.3 (CO Aib).
ESI-MS: found: m/z 523,3 [M+H]⁺/ calculated: 522.3 g/mol

### Example 6:

### (R)-N-(1-(5-(2-(1H-indol-3-yl)ethyl)-4-benzyl-4H-1,2,4-triazol-3-yl)-2-(1H-indol-3-yl)ethyl)-2-amino-2-methylpropanamide (Compound 8)

Compound 8 was obtained from Boc-(D)-Trp (10 mmoles), phenylmethanamine, 3-(1H-indol-3-yl)propane hydrazide and Boc-2-amino-2-methylpropanoic acid according to the general synthetic schemes with a total yield after purification by HPLC of 45%.
¹H NMR (400 MHz, DMSO-d⁶):
δ (ppm) 1.29 (3H, s, CH₃ Aib), 1.30 (3H, s, CH₃ Aib), 2.88 (2H, m, CH₂-CH₂-indole), 2.97 (2H, m, CH₂-CH₂-indole), 3.37 (2H, m, CH₂ pTrp), 5.11 (2H, s, CH₂ benzyl), 5.21 (1H, m, CαH Trp), 6.86 (1H, t, J=7.4, H₅ Trp), 6.88 (2H, H₂, H₆ benzyl), 6.92 (1H, t, J=7.6, H₅ indole), 7.03 (1H, t, J=7.6, H₆ Trp), 7.05 (2H, H₆ indole, H₂ indole), 7.09 (1H, d, J=1.8, H₂ Trp), 7.17 (1H, d, J=7.9, H₄ Trp), 7.26 (2H, H₃, H₅ benzyl), 7.27 (1H, H₄ benzyl), 7.30 (1H, H₄ indole), 7.32 (2H, H₇ Trp, H₇ indole), 8.03 (2H, brs, NH₂ Aib), 8.95 (1H, d, J=8.1, NH Trp), 10.77 (1H, s, NH indole), 10.81 (1H, s, NH indole Trp).
¹³C NMR (400 MHz, DMSO-d⁶):
δ (ppm) 22.4 (CH₂-CH₂ indole), 23.1 (CH₃ Aib), 23.3 (CH₃ Aib), 25.4 (CH₂-CH₂ indole), 28.7 (Cβ Trp), 45.3 (Cα Trp, CH₂-benzyl), 56.3 (Cq Aib), 109.5 (C₃ Trp), 111.3 (C₇ Trp, C₇ indole), 113.0 (C₃ indole), 117.8 (C₄ Trp), 118.0 (C₄ indole), 118.2 (C₅ indole), 118.3 (C₅ Trp), 120.9 (C₆ Trp, C₆ indole), 122.4 (C₂ indole), 124.3 (C₂ Trp), 125.9 (C₂, C₆ benzyl), 126.7 (C₉ Indole), 126.9 (C₉ Trp), 127.6 (C₄ benzyl), 128.8 (C₃, C₅ benzyl), 135.7 (C₁ benzyl), 136.0 (C₈ Trp), 136.1 (C₈ indole), 154.3 (Cq triazole), 154.5 (Cq triazole), 171.4 (CO Aib).
ESI-MS: found: m/z 546.3 [M+H]⁺/ calculated: 545.3 g/mol

### Example 7:

### (R)-N-(1-(5-(3-(1H-indol-3-yl)propyl)-4-benzyl-4H-1,2,4-triazol-3-yl)-2-(1H-indol-3-yl)ethyl)-2-amino-2-methylpropanamide (Compound 9)

Compound 9 was obtained from Boc-(D)-Trp (10 mmoles), phenylmethanamine, 4-(1*H-*indol-3-yl)butanehydrazide and Boc-2-amino-2-methylpropanoic acid according to the general synthetic schemes with a total yield after purification by HPLC of 38%.
¹H NMR (400 MHz, DMSO-d⁶):
δ (ppm) 1.29 (3H, s, CH₃ Aib), 1.31 (3H, s, CH₃ Aib), 1.90 (2H, m, CH₂-CH₂-CH₂-indole), 2.61 (2H, m, CH₂-CH₂-CH₂-indole), 2.69 (2H, m, CH₂-CH₂-CH₂-indole), 3.37 (2H, m, CH₂ βTrp), 5.09 (2H, s, CH₂ benzyl), 5.20 (1H, m, CαH Trp), 6.85 (3H, m, H₂, H₆ benzyl, H₅ Trp), 6.94 (1H, t, J=7.5, H₅ indole), 7.01 (1H, s, H₂ indole), 7.02 (1H, t, J=7.8, H₆ Trp), 7.05 (1H, t, J=8, H₆ indole), 7.08 (1H, d, J=2.0, H₂ Trp), 7.14 (1H, d, J=8.0, H₄ Trp), 7.25 (3H, m, H₃, H₄, H₅ benzyl), 7.31 (1H, d, J=8.0, H₇ Trp), 7.32 (1H, d, J=8.0, H₇ indole), 7.42 (1H, d, J=7.8, H₄ indole), 8.03 (2H, s, NH₂ Aib), 8.95 (1H, d, J=8.1, NH Trp), 10.73 (1H, s, NH indole), 10.80 (1H, d, J=2.0, NH indole Trp).
¹³C NMR (400 MHz, DMSO-d⁶):
δ (ppm) 23.1 (CH₃ Aib), 23.3 (CH₃ Aib), 23.8 (CH₂-CH₂-CH₂-indole), 24.1 (CH₂-CH₂-CH₂-indole), 27.2 (CH₂-CH₂-CH₂-indole), 28.7 (Cβ Trp), 45.4 (Cα Trp), 45.5 (CH₂ benzyl), 56.3 (Cq Aib), 109.4 (C₃ Trp), 111.3 (C₇ Trp, C₇ indole), 113.6 (C₃ indole), 117.8 (C₄ Trp), 118.0 (C₅ indole), 118.2 (C₄ indole), 118.3 (C₅ Trp), 120.8 (C₆ indole, C₆ Trp), 122.2 (C₂ indole), 124.3 (C₂ Trp), 125.9 (C₂, C₆ benzyl), 126.8 (C₉ Trp), 127.0 (C₉ indole), 127.7 (C₄ benzyl), 128.7 (C₃, C₅ benzyl), 135.5 (C₁ benzyl), 136.0 (C₈ Trp), 136.2 (C₈ indole), 154.3 (Cq triazole), 154.7 (Cq triazole), 171.4 (CO Aib).
ESI-MS: found: m/z 560.4 [M+H]⁺/ calculated: 559.3 g/mol

### Example 8:

### (R)-N-(1-(5-(3-(1H-indol-3-yl)propyl)-4-(3-methoxybenzyl)-4H-1,2,4-triazol-3-yl)-2-(1H-indol-3-yl)ethyl)-2-amino-2-methylpropanamide (Compound 10)

Compound 10 was obtained from Boc-(D)-Trp (10 mmoles), (3-methoxyphenyl)-methanamine, 4-(1*H*-indol-3-yl)butanehydrazide and Boc-2-amino-2-methylpropanoic acid according to the general synthetic schemes with a total yield after purification by HPLC of 25%.
¹H NMR (400 MHz, DMSO-d⁶):
δ (ppm) 1.27 (3H, s, CH₃ Aib), 1.30 (3H, s, CH₃ Aib), 1.92 (2H, m, CH₂-CH₂-CH₂-indole), 2.62 (2H, m, CH₂-CH₂-CH₂-indole), 2.68 (2H, m, CH₂-CH₂-CH₂-indole), 3.24 (1H, dd, J=14.5, J=5.8, 1 H CH₂ βTrp), 3.39 (1H, dd, J=14.5, J=9.0, 1 H CH₂ βTrp), 3.66 (3H, s, *m-*OCH₃)*,* 5.07 (2H, s, CH₂ *m*-methoxybenzyl), 5.18 (1H, m, CαH Trp), 6.35 (1H, d, J=7.5, H₆ *m*-methoxybenzyl), 6.54 (1H, bs, H₂ *m*-methoxybenzyl), 6.84 (1H, t, J=7.5, H₅ Trp), 6.87 (1H, dd, J=8.0, J=2.1, H₄ *m*-methoxybenzyl), 6.94 (1H, t, J=7.3, H₅ indole), 7.02 (1H, t, H₆ Trp), 7.02 (1H, s, H₂ indole), 7.05 (1H, t, J=7.8, H₆ indole), 7.08 (1H, d, J=2.1, H₂ Trp), 7.13 (1H, d, J=8.1, H₄ Trp), 7.17 (1H, t, J=8.1, H₅ *m-*methoxybenzyl), 7.30 (1H, d, H₇ Trp), 7.32 (1H, d, J=8, H₇ indole), 7.42 (1H, d, J=7.6, H₄ indole), 7.98 (2H, s, NH₂ Aib), 8.93 (1H, d, J=8.2, NH Trp), 10.71 (1H, s, NH indole), 10.77 (1H, s, NH indole Trp).
¹³C NMR (400 MHz, DMSO-d⁶):
δ (ppm) 23.1 (CH₃ Aib), 23.3 (CH₃ Aib), 23.9 (CH₂-CH₂CH₂-indole), 24.3 (CH₂-CH₂-CH₂-indole), 27.4 (CH₂-CH₂-CH₂ indole), 28.8 (Cβ Trp), 45.2 (CH₂- *m*-methoxybenzyl), 45.4 (Cα Trp), 55.1 (*m*-OCH₃), 56.3 (Cq Aib), 109.5 (C₃ Trp), 111.3 (C₇ Trp, C₇ indole), 111.8 (C₂ *m*-methoxybenzyl), 113.0 (C₄ *m*-methoxybenzyl), 113.8 (C₃ indole), 117.8 (C₆ *m*-methoxybenzyl), 117.9 (C₄ Trp), 118.1 (C₅ indole), 118.2 (C₅ Trp, C₄ indole), 120.8 (C₆ Trp), 120.9 (C₆ indole), 122.2 (C₂ indole), 124.3 (C₂ Trp), 126.8 (C₉ Trp), 127.0 (C₉ Indole), 130.0 (C₅ *m*-methoxybenzyl), 136.0 (C₈ Trp), 136.2 (C₈ indole), 137.4 (C₁ *m-*methoxybenzyl), 154.3 (Cq triazole), 154.6 (Cq triazole), 159.7 (C₃ *m*-methoxybenzyl), 171.4 (CO Aib).
ESI-MS: found: m/z 590.3 [M+H]⁺/ calculated: 589.3 g/mol

### Example 9:

### (R)-N-(1-(5-(3-(1H-indol-3-yl)propyl)-4-(naphthalen-1-ylmethyl)-4H-1,2,4-triazol-3-yl)-2-(1H-indol-3-yl)ethyl)-2-amino-2-methylpropanamide (Compound 11)

Compound 11 was obtained from Boc-(D)-Trp (10 mmoles), naphthalen-1-ylmethanamine, 4-(1*H*-indol-3-yl)butanehydrazide and Boc-2-amino-2-methylpropanoic acid according to the general synthetic schemes with a total yield after purification by HPLC of 22%.
¹H NMR (300 MHz, DMSO-d⁶, 300°K):
δ (ppm) 1.20 (3H, s, CH₃ Aib), 1.25 (3H, s, CH₃ Aib), 1.93 (2H, m, CH₂-CH₂-CH₂-indole), 2.66 (4H, m, CH₂-CH₂-CH₂-indole), 3.25 (1H, dd, J= 14 Hz and 5 Hz, CH₂ βTrp), 3.40 (1H, dd, J= 14 Hz and 9 Hz, CH₂ βTrp), 4.95 (1H, m, CH αTrp), 5.66 (1H, d, J= 18 Hz, CH₂-naphtyl), 5.81 (1H, d, J= 18 Hz, CH₂-naphtyl), 6.37 (1H, d, Jₒ= 7 Hz, H₂ naphtyl), 6.43 (1H, t, Jₒ= 7 Hz, H₅ Trp), 6.59 (1H, d, Jₒ= 8 Hz, H₄ Trp), 6.86 (3H, m, H₅ and H₆ indole, H₆ Trp), 6.95 (1H, d, J= 2 Hz, H₂ indole), 7.00 (1H, d, Jₒ= 8 Hz, H₄ indole), 7.06 (1H, d, J= 2 Hz, H₂ Trp), 7.20-7.33 (4H, m, H₄ and H₇ indole, H₇ Trp, H₃ naphtyl), 7.60 (2H, m, H₆ and H₇ naphtyl), 7.87 (1H, d, Jₒ= 8 Hz, H₄ naphtyl), 7.99 (5H, m, NH₂ Aib, H₅ and H₈ naphtyl), 8.95 (1H, d, J= 8 Hz, NH amide), 10.70 (1H, s, NH indole), 10.77 (1H, s, NH indole Trp).
¹³C NMR (75 MHz, DMSO-d⁶, 300°K):
δ (ppm) 23.5 (CH₃ Aib), 23.6 (CH₃ Aib), 24.1 (CH₂-CH₂-CH₂-indole), 24.5 (CH₂-CH₂-CH₂-indole), 27.6 (CH₂-CH₂-CH₂-indole), 29.1 (CH₂ βTrp), 44.1 (CH₂-naphtyl), 45.7 (CH α Trp), 56.7 (Cq Aib), 109.7 (C₃ Trp), 111.7 (C₇ indole and C₇ Trp), 113.9 (C₃ indole), 117.9 (C₄ Trp), 118.5 (C₄ indole, C₅ Trp), 118.6 (C₅ indole), 121.1 (C₆ Trp), 121.2 (C₆ indole), 122.1 (C₂ naphtyl), 122.7 (C₂ indole), 122.9 (C₈ naphtyl), 125.0 (C₂ Trp), 125.9 (C₃ naphtyl), 126.8 (C₆ naphtyl), 127.0 (C₉ indole), 127.1 (C₇ naphtyl), 127.4 (C₉ Trp), 128.5 (C₄ naphtyl), 129.2 (C₅ naphtyl), 129.9 (C₉ naphtyl), 131.6 (C₁ naphtyl), 133.6 (C₁₀ naphtyl), 136.4 (C₈ Trp), 136.7 (C₈ indole), 155.4 (Cqs triazole), 171.9 (CO Aib).
ESI-MS: found: m/z 610.3 [M+H]⁺/ calculated: 609.3 g/mol

### Example 10:

### (R)-N-(1-(5-(2-(1H-indol-3-yl)ethyl)-4-(4-methoxybenzyl)-4H-1,2,4-triazol-3-yl)-2-(1H indol-3-yl)ethyl)-2-amino-2-methylpropanamide (Compound 12)

Compound 12 was obtained from Boc-(D)-Trp (10 mmoles), (4-methoxyphenyl)-methanamine, 3-(1 H-indol-3-yl)propane hydrazide and Boc-2-amino-2-methylpropanoic acid according to the general synthetic schemes with a total yield after purification by HPLC of 28%.
¹H NMR (400 MHz, DMSO-d⁶):
δ (ppm) 1.30 (3H, s, CH₃ Aib), 1.33 (3H, s, CH₃ Aib), 2.91 (2H, m, CH₂-CH₂-indole), 2.97 (2H, m, CH₂-CH₂-indole), 3.37 (2H, d, CH₂ βTrp), 3.71 (3H, s, OCH₃), 5.02 (2H, s, CH₂ *p*-methoxybenzyl), 5.23 (1H, m, CαH Trp), 6.78 (4H, m, CHar *p*-methoxybenzyl), 6.87 (1H, t, J=7.5, H₅ Trp), 6.93 (1H, t, J=7.5, H₅ indole), 7.03 (1H, t, H₆ Trp), 7.05 (1H, t, H₆ indole), 7.07 (1H, s, H₂ indole), 7.09 (1H, s, H₂ Trp), 7.21 (1H, d, J=8, H₄ Trp), 7.32 (3H, H₄ indole, H₇ Trp, H₇ indole), 8.02 (2H, s, NH₂ Aib), 8.97 (1H, d, J=8.1, NH Trp), 10.77 (1H, s, NH indole), 10.80 (1H, s, NH indole Trp).
¹³C NMR (400 MHz,DMSO-d⁶):
δ (ppm) 22.4 (CH₂-CH₂ indole), 23.1 (CH₃ Aib), 23.4 (CH₃ Aib), 25.5 (CH₂-CH₂ indole), 28.9 (Cβ Trp), 44.9 (CH₂ *p*-methoxybenzyl), 45.3 (Cα Trp), 55.0 (OCH₃), 56.3 (Cq Aib), 109.5 (C₃ Trp), 111.3 (C₇ Trp, C₇ indole), 113.0 (C₃ indole), 114.1 (C₃, C₅ *p-*methoxybenzyl), 117.9 (C₄ Trp), 118.0 (C₄ indole), 118.2 (C₅ indole), 118.3 (C₅ Trp), 120.9 (C₆ indole, C₆ Trp), 122.0 (C₂ indole), 124.4 (C₂ Trp), 126.7 (C₉ indole), 126.9 (C₉ Trp), 127.3 (C₂, C₆ *p*-methoxybenzyl), 127.4 (C₁ *p*-methoxybenzyl), 135.9 (C₈ Trp), 136.1 (C₈ indole), 154.2 (Cq triazole), 154.5 (Cq triazole), 158.4 C₄ *p*-methoxybenzyl), 171.4 (CO Aib).
ESI-MS: found: m/z 576.3 [M+H]⁺/ calculated: 575.3 g/mol

### Example 11:

### (R)-N-(1-(4-(4-methoxybenzyl)-5-benzyl-4H-1,2,4-triazol-3-yl)-2-(1H-indol-3-yl)ethyl)-2-amino-2-methylpropanamide (Compound 13)

Compound 13 was obtained from Boc-(D)-Trp (10 mmoles), (4-methoxyphenyl)-methanamine, 2-phenylacetohydrazide and Boc-2-amino-2-methylpropanoic acid according to the general synthetic schemes with a total yield after purification by HPLC of 37%.
¹H NMR (300 MHz, DMSO-d⁶, 300°K):
δ (ppm) 1.24 (3H, s, CH₃ Aib), 1.28 (3H, s, CH₃ Aib), 3.26 (1H, dd, ³J= 14 Hz and 6 Hz, CH₂ βTrp), 3.31 (1H, dd, ³J= 14 Hz and 9 Hz, CH₂ βTrp), 3.67 (3H, s, OCH₃), 3.99 (2H, s, CH₂-benzyl), 4.99 (2H, s, CH₂-*p*-methoxybenzyl), 5.12 (1 H, m, CH αTrp), 6.67 (4H, m, CHar *p*-methoxybenzyl), 6.80 (1 H, t, Jₒ= 8 Hz, H₅ Trp), 6.98 (1H, t, Jₒ= 8 Hz, H₆ Trp), 7.02-7.06 (4H, m, H₂ and H₆ benzyl, H₂ and H₄ Trp), 7.12-7.25 (3H, m, H₃, H₄ and H₅ benzyl), 7.26 (1H, d, Jₒ= 8 Hz, H₇ Trp), 8.01 (3H, brs, NH₂ Aib), 8.92 (1H, d, J= 8 Hz, NH Trp), 10.77 (1H, s, NH indole Trp).
¹³C NMR (75 MHz, DMSO-d⁶, 300°K):
δ (ppm) 23.5 (CH₃ Aib), 23.7 (CH₃ Aib), 29.1 (CH₂ pTrp), 30.6 (CH₂-benzyl), 45.7 (CH₂-*p*-methoxybenzyl), 45.7 (CH αTrp), 55.5 (OCH₃), 56.7 (Cq Aib), 109.8 (C₃ Trp), 111.7 (C₇ Trp), 114.5 (C₃ and C₅ *p*-methoxybenzyl), 118.3 (C₄ Trp), 118.7 (C₅ Trp), 121.3 (C₆ Trp), 124.8 (C₂ Trp), 127.1 (C₂ and C₆ benzyl), 127.3 (C₉ Trp), 127.6 (C₁ *p-*methoxybenzyl), 127.8 (C₂ and C₆ *p*-methoxybenzyl), 128.8 (C₃, C₄ and C₅ *p-*methoxybenzyl), 136.3 (C₁ benzyl), 136.4 (C₈ Trp), 153.8 (Cq triazole), 155.2 (Cq triazole), 159.1 (C₄ p-methoxybenzyl), 171.9 (CO Aib).
ESI-MS: found: m/z 524.1 [M+H]⁺/ calculated: 522.3 g/mol

### Example 12:

### (R)-N-(1-(5-(2-(1H-indol-3-yl)ethyl)-4-hexyl-4H-1,2,4-triazol-3-yl)-2-(1H-indol-3-yl)ethyl)-2-amino-2-methylpropanamide (Compound 15)

Compound 15 was obtained from Boc-(D)-Trp (10 mmoles), hexan-1-amine, 3-(1H-indol-3-yl)propane hydrazide and Boc-2-amino-2-methylpropanoic acid according to the general synthetic schemes with a total yield after purification by HPLC of 28%.
¹H NMR (400 MHz, DMSO-d⁶):
δ (ppm) 0.77 (3H, t, J=7.2 (CH₂)₅-CH₃), 1.01 (4H, m, 2CH₂), 1.11 (2H, m, CH2-CH₃), 1.14 (1H, m, 1 H N-CH₂-CH₂),1.33 (1 H, m, 1 H N-CH₂-CH₂.),1.40 (3H, s, CH₃ Aib), 1.42 (3H, s, CH₃ Aib), 3.05 (2H, m, CH₂-CH₂-indole), 3.10 (2H, m, CH₂-CH₂-indole), 3.37 (1 H, dd, J=14.2, J= 7.6, 1 H CH₂ βTrp), 3.44 (1 H, dd, J=14.2, J= 7.6, 1 H CH₂ βTrp), 3.58 (1 H, m, 1 H N-CH₂), 3.71 (1 H, m, 1 H N-CH₂), 5.21 (1 H, m, CαH Trp), 6.96 (1 H, H₅ Trp), 6.97 (1 H, H₅ indole), 7.06 (2H, H₆ Trp, H₆ indole), 7.09 (1 H, s, H₂ Trp), 7.13 (1 H, s, H₂ indole), 7.34 (2H, H₇ Trp, H₇ indole),7.48 (1 H, d, H₄ indole), 7.50 (1 H, H₄ Trp), 8.14 (2H, s, NH₂ Aib), 9.08 (1 H, d, J=7.8, NH Trp), 10.84 (1 H, s, NH indole), 10.88 (1 H, s, NH indole Trp).
¹³C NMR (400 MHz, DMSO-d⁶):
δ (ppm) 13.7 (CH₂)₅-CH₃), 21.7 (CH₂-CH₃), 22.4 (CH₂-CH_{□}indole), 23.1 (CH₃ Aib), 23.3 (CH₃ Aib), 25.1 (CH2.-CHLJindole), 25.5 (CH₃-CH₂-CH₂-CH₂), 29.1 (Cβ Trp), 29.3 (N-CH₂-CH₂), 30.4 (CH₃-CH₂-CH₂), 42.6 (N-CH₂-CH₂),45.6 (Cα Trp), 56.3 (Cq Aib), 109.2 (C₃ Trp), 111.4-111.5 (C₇ Trp, C₇ indole), 112.8 (C₃ indole), 117.7 (C₄ Trp), 118.0 (C₅ indole), 118.2 (C₄ indole), 118.4 (C₅ Trp), 120.9 (C₆ indole, C₆ Trp), 122.6 (C₂ indole), 124.3 (C₂ Trp), 126.8 (C₉ Trp), 126.9 (C₉ indole), 136.0 (C₈ Trp), 136.2 (C₈ indole), 154.0 (Cq triazole), 154.1 (Cq triazole), 171.4 (CO Aib).ESI-MS: found: m/z 540.3 [M+H]⁺/ calculated: 539.3 g/mol

### Example 13:

### (S)-N-(1-(5-(2-(1H-indol-3-yl)ethyl)-4-(2,4-dimethoxybenzyl)- 4H-1,2,4-triazol-3-yl)-2-(1H-indol-3-yl)ethyl)-2-amino-2-methylpropanamide (Compound 18)

Compound 18 was obtained from Boc-(L)-Trp (10 mmoles), (2,4-dimethoxyphenyl)-methanamine, 3-(1H-indol-3-yl)propane hydrazide and Boc-2-amino-2-methylpropanoic acid according to the general synthetic schemes with a total yield after purification by HPLC of 30%.
¹H NMR (300 MHz, DMSO-d⁶, 300°K):
δ (ppm) 1.27 (3H, s, CH₃ Aib), 1.31 (3H, s, CH₃ Aib), 2.89 (2H, m, CH₂CH₂-indole), 2.93 (2H, m, CH₂CH₂-indole), 3.27 (2H, m, CH₂ βTrp), 3.62 (3H, s, *o*-OCH₃), 3.68 (3H, s, *p-*OCH₃), 4.89 (1 H, d, ³J= 17Hz, CH₂-*o,p*-dimethoxybenzyl), 5.06 (1H, d, ³J= 17Hz, CH₂-*o,p*-dimethoxybenzyl), 5.18 (1H, m, CH αTrp), 6.27 (1H, dd, Jₒ= 8Hz and Jp= 2Hz, H₅ *o,p*-dimethoxybenzyl), 6.40 (1H, d, 8Hz, H₆ *o,p*-dimethoxybenzyl), 6.56 (1 H, d, Jp= 2Hz, H₃ *o,p*-dimethoxybenzyl), 6.83 (1H, t, Jₒ= 7Hz, H₅ Trp), 6.90 (1H, t, Jₒ= 7 Hz, H₅ indole), 7.02 (1H, t, H₆ Trp), 7.04 (1H, t, H₆ indole), 7.07 (1H s, H₂ indole), 7.08 (1 H, s, H₂ Trp), 7.12 (1H, d, Jₒ= 8 Hz, H₄ Trp), 7.29 (3H, H₄ and H₇ indole, H₇ Trp), 8.00 (3H, brs, NH₂ Aib), 8.93 (1 H, d, J= 8 Hz, NH amide), 10.76 (1H, s, NH indole), 10.79 (1H, s, NH indole Trp).
¹³C NMR (75 MHz, DMSO-d⁶, 300°K):
δ (ppm) 22.9 (CH₂CH₂-indole), 23.6 (CH₃ Aib), 23.7 (CH₃ Aib), 25.8 (CH₂CH₂-indole), 29.2 (CH₂ βTrp), 41.4 (CH₂-*o,p*-dimethoxybenzyl), 45.7 (CαTrp), 55.7 (*p*-OCH₃), 55.9 (*o*-OCH₃), 56.7 (Cq Aib), 99.0 (C₃ *o,p*-dimethoxybenzyl), 105.1 (C₅ *o,p-*dimethoxybenzyl), 109.9 (C₃ Trp), 111.8 (C₇ Trp, C₇ indole), 113.4 (C₃ indole), 115.6 (C₁ *o,p*-dimethoxybenzyl), 118.3 (C₄ indole), 118.4 (C₄ Trp), 118.6 (C₅ Trp, C₅ indole), 121.3 (C₆ Trp, C₆ indole), 122.6 (C₂ indole), 124.4 (C₂ Trp), 127.2 (C₉ indole), 127.3 (C₉ Trp), 128.0 (C₆ *o,p*-dimethoxybenzyl), 136.4 (Cₛ Trp), 136.6 (Cₛ indole), 155.0 (2 Cq triazole), 157.7 (C₂ *o,p*-dimethoxybenzyl), 160.9 (C₄ *o,p*-dimethoxybenzyl), 171.6 (CO Aib).
ESI-MS: found: m/z 606.2 [M+H]⁺/ calculated: 605.3 g/mol

### Example 14:

### (R)-N-(1-(5-(3-(1H-indol-3-yl)propyl)-4-(4-methoxybenzyl)-4H-1,2,4-triazol-3-yl)-2-(1H-indol-3-yl)ethyl)-2-amino-2-methylpropanamide (Compound 23)

Compound 23 was obtained from Boc-(D)-Trp (10 mmoles), (4-methoxyphenyl)-methanamine, 4-(1 H-indol-3-yl)butanehydrazide and Boc-2-amino-2-methylpropanoic acid according to the general synthetic schemes with a total yield after purification by HPLC of 25%.
¹H NMR (300 MHz, DMSO-d⁶, 300°K):
δ (ppm) 1.27 (3H, s, CH₃ Aib), 1.30 (3H, s, CH₃ Aib), 1.84 (2H, m, CH₂CH₂CH₂-indole), 2.58 (2H, m, CH₂CH₂CH₂-indole), 2.65 (2H, m, CH₂CH₂CH₂-indole), 3.34 (2H, d, ³J₌ 7 Hz, CH₂ βTrp), 3.67 (3H, s, OCH₃), 4.96 (2H, s, CH₂-*p*-methoxybenzyl), 5.19 (1H, m, CH αTrp), 6.71 (4H, s, CH ar *p*-methoxybenzyl), 6.89 (1H, t, Jₒ=7 Hz, H₅ Trp), 6.92 (1H, t, Jₒ=7 Hz, H₅ indole), 7.02 (1H, s, H₂ indole), 7.05 (1 H, s, H₂ Trp), 7.14 (1 H, d, Jₒ= 8 Hz, H₄ Trp), 7.33 (3H, H₄ indole, H₇ Trp, H₇ indole), 8.02 (3H, brs, NH₂ Aib), 7.90 (1 H, d, J= 8 Hz, NH amide), 10.73 (1 H, s, NH indole), 10.79 (1 H, s, NH indole Trp).
¹³C NMR (75 MHz, DMSO-d⁶, 300°K):
δ (ppm) 23.6 (CH₃ Aib), 23.8 (CH₃ Aib), 24.3 (CH₂CH₂CH₂-indole), 24.8 (CH₂CH₂CH₂-indole), 27.7 (CH₂CH₂CH₂-indole), 29.1 (Cβ Trp), 45.5 (N-CH₂-*p*-methoxybenzyl), 45.8 (Cα Trp), 55.5 (OCH₃), 56.8 (Cq Aib), 109.8 (C₃ Trp), 111.7 (C₇ Trp, C₇ indole), 114.0 (C₃ indole), 114.5 (C₃, C₅ *p*-methoxybenzyl), 118.3 (C₄ indole, C₄ Trp), 118.5 (C₅ indole), 118.8 (C₅ Trp), 121.3 (C₆ indole, C₆ Trp), 127.3 (C₉ indole), 127.4 (C₉ Trp), 127.6 (C₁ *p*-methoxybenzyl), 127.9 (C₂, C₆ *p*-methoxybenzyl, C₂ Trp, C₂ indole), 136.1 (C₈ indole), 136.4 (C₈ Trp), 154.7 (Cq triazole), 155.1 (Cq triazole), 159.2 (C₄ *p-*methoxybenzyl), 171.9 (CO Aib).
ESI-MS: found: m/z 590.0 [M+H]⁺/ calculated: 589.3 g/mol

### Example 15:

### (R)-N-(1-(5-(2-(1H-indol-3-yl)ethyl)-4-(2-methoxy)benzyl)-4H-1,2,4-triazol-3-yl)-2-(1H-indol-3-yl)ethyl)-2-amino-2-methylpropanamide (Compound 25)

Compound 25 was obtained from Boc-(D)-Trp (10 mmoles), (2-methoxyphenyl)-methanamine, 3-(1 H-indol-3-yl)propane hydrazide and Boc-2-amino-2-methylpropanoic acid according to the general synthetic schemes with a total yield after purification by HPLC of 28%.
¹H NMR (300 MHz, DMSO-d⁶, 300°K):
6(ppm) 1.27 (3H, s, CH₃ Aib), 1.29 (3H, s, CH₃ Aib), 2.90 (2H, m, CH₂-CH₂-indole), 2.96 (2H, m, CH₂-CH₂-indole), 3.29 (2H, m, CH₂ βTrp), 3.65 (3H, s, OCH₃), 5.09 (3H, m, CH₂-o- methoxybenzyl and CH αTrp), 6.49 (1 H, d, Jₒ= 8 Hz, H₃ *o*-methoxybenzyl), 6.76 (1 H, t, Jₒ= 8 Hz, H₅ Trp), 6.81 (1 H, t, Jₒ= 8 Hz, H₅ indole), 6.89 (1 H, t, Jₒ= 7 Hz, H₆ Trp), 6.96 (1 H, t, Jₒ= 8 Hz, H₆ indole), 6.98 (1 H, s, H₂ indole), 7.02 (3H, m, H₄, H₅ and H₆ *o-*methoxybenzyl), 7.07 (1 H, d, Jₒ= 6 Hz, H₄ Trp), 7.18 (1 H, m, H₄ indole), 7.29 (2H, m, H₇ indole and H₇ Trp), 8.07 (3H, brs, NH₂ Aib), 8.97 (1 H, d, J= 8 Hz, NH amide), 10.80 (1 H, s, NH indole), 10.82 (1 H, s, NH indole Trp).
¹³C NMR (75 MHz, DMSO-d⁶, 300°K):
δ (ppm) 22.8 (CH₂-CH₂-indole), 23.6 (CH₃ Aib), 23.7 (CH₃ Aib), 25.8 (CH₂-CH₂-indole), 29.1 (CH₂ βTrp), 42.3 (CH2-*o*-methoxybenzyl), 45.7 (CH α Trp), 55.8 (OCH₃), 56.7 (Cq Aib), 109.8 (C₃ Trp), 111.5 (C₃ *o*-methoxybenzyl), 111.8 (C₇ indole and C₇ Trp), 113.2 (C₃ indole), 118.2 (C₄ Trp), 118.4 (C₄ indole), 118.7 (C₅ indole and C₅ Trp), 121.0 (C₆ indole), 121.3 (C₆ Trp), 121.4 (C₅ o- methoxybenzyl), 123.0 (C₂ indole and C₂ Trp), 123.3 (C₁ o- methoxybenzyl), 127.0 (C₄ o- methoxybenzyl), 127.1 (C₉ indole), 127.3 (C₉ Trp), 129.8 (C₆ o- methoxybenzyl), 136.4 (C₈ indole), 136.6 (C₈ Trp), 155.2 (Cq triazole), 171.9 (CO Aib).
ESI-MS: found: m/z 576.1 [M+H]⁺/ calculated: 575.3 g/mol

Data on further exemplary embodiments that were synthesized according to the general sysnthesis schemes are compiled below (please refer also to **Table 1):**

### (R)-N-(1-(5-(3-(1H-indol-3-yl)propyl)-4-phenethyl-4H-1,2,4-triazol-3-yl)-2-(1H-indol-3-yl)ethyl)-2-amino-2-methylpropanamide (Compound 3):

¹H NMR (300 MHz, DMSO-d⁶, 300°K):
δ (ppm) 1.32 (s, 3H, CH₃ Aib), 1.37 (s, 3H, CH₃ Aib), 1.86 (2H, m, CH₂-CH₂-CH₂-indole), 2.38 (2H, m, CH₂-CH₂-CH₂-indole), 2.65 (4H, m, CH₂-CH₂-CH₂-indole and CH₂-CH₂-phenyl), 3.38 (2H, m, CH₂ -CH₂-phenyl), 3.74 (1 H, m, CH₂ βTrp), 3.92 (1 H, m, CH₂ βTrp), 5.23 (1 H, m, CH αTrp), 6.78 (2H, m, H₅ indole and H₅ Trp), 6.93 (1 H, t, Jₒ= 8 Hz, H₆ Trp), 7.01 (3H, m, H₆ indole, H₂ and H₆ phenyl), 7.05 (1 H, d, J= 2 Hz, H₂ Trp), 7.08 (1 H, d, J= 2 Hz, H₂ indole), 7.15 (3H, m, H₃, H₄ and H₅ phenyl), 7.29 (1 H, d, Jₒ= 8 Hz, H₄ Trp), 7.31 (1 H, d, Jo= 8 Hz, H₇ Trp), 7.44 (1 H, d, Jₒ= 8 Hz, H₇ indole), 7.46 (1 H, d, Jₒ= 8 Hz, H₄ indole), 8.06 (3H, brs, NH₂ Aib), 9.05 (1 H, d, 8 Hz, NH amide), 10.76 (1 H, s, NH indole), 10.85 (1 H, d, J= 2 Hz, NH indole Trp).
¹³C NMR (75 MHz, DMSO-d⁶, 300°K):
δ (ppm) 23.5 (CH₃ Aib), 23.6 (CH₂-CH₂-CH₂-indole), 23.9 (CH₃ Aib), 24.5 (CH₂-CH₂-CH₂-indole), 27.3 (CH₂-CH₂-CH₂-indole), 29.4 (CH₂ βTrp), 35.7 (CH₂-CH₂-phenyl), 44.5 (CH₂-CH₂-phenyl), 46.1 (CH αTrp), 56.8 (Cq Aib), 109.6 (C₃ Trp), 111.8 (C₇ indole), 111.9 (C₇ indole), 113.9 (C₃ indole), 118.3 (C₄ Trp), 118.6 (C₅ indole), 118.7 (C₄ indole), 118.9 (C₅ Trp), 121.3 (C₆ Trp), 121.4 (C₆ indole), 122.8 (C₂ indole and C₂ Trp), 127.1 (C₄ phenyl), 127.3 (C₉ Trp), 127.5 (C₉ indole), 128.8 (C₂ and C₆ phenyl), 129.1 (C₃ and C₅ phenyl), 136.5 (C₁ phenyl), 136.8 (C₈ Trp), 137.2 (C₈ indole), 154.7 (Cq triazole), 172.0 (CO Aib).

### (R)-N-(1-(5-(3-(1H-indol-3-yl)propyl)-4-hexyl-4H-1,2,4-triazol-3-yl)-2-(1H-indol-3-yl)ethyl)-2-amino-2-methylpropanamide (Compound 16):

¹H NMR (300 MHz, DMSO-d⁶, 300°K):
δ (ppm) 0.74 (3H, t, J= 6 Hz, CH₃-CH₂-CH₂-CH₂-CH₂-CH₂), 0.95 (4H, brs, CH₃-CH₂-CH₂-CH₂-CH₂-CH₂), 1.06 (3H, m, CH₃-CH₂-CH₂-CH₂-CH₂-CH₂ and 1 H N-CH₂-CH₂), 1.38 (7H, s, CH₃ Aib and 1 H N-CH₂-CH₂), 1.97 (2H, m, CH₂-CH₂-CH₂-indole), 2.71 (4H, m, CH₂-CH₂-CH₂-indole), 3.37 (2H, m, CH₂ βTrp), 3.56 (2H, m, N-CH₂), 5.15 (1 H, m, CH αTrp), 6.91 (2H, m, H₅ indole and H₅ Trp), 7.00 (2H, m, H₆ indole and H₆ Trp), 7.07 (2H, s, H₂ indole and H₂ Trp), 7.29 (2H, d, Jₒ= 8 Hz, H₇ indole and H₇ Trp), 7.45 (2H, d, Jo= 7 Hz, H₄ indole and H₄ Trp), 8.15 (3H, brs, NH₂ Aib), 9.10 (1 H, d, J= 6 Hz, NH amide), 10.77 (1 H, s, NH indole), 10.85 (1 H, s, NH indole Trp).
¹³C NMR (75 MHz, DMSO-d⁶, 300°K):
δ (ppm) 14.2 (CH₃-CH₂-CH₂-CH₂-CH₂-CH₂), 22.2 (CH₃-CH₂-CH₂-CH₂-CH₂-CH₂ and CH₂-CH₂-CH₂-indole), 23.6 (CH₃ Aib), 23.7 (CH₃ Aib), 24.5 (CH₂-CH₂-CH₂-indole), 25.9 (CH₃-CH₂-CH₂-CH₂-CH₂-CH₂), 27.5 (CH₂ βTrp and CH₂-CH₂-CH₂-indole), 29.7 (N-CH₂-CH₂), 30.8 (CH₃-CH₂-CH₂-CH₂-CH₂-CH₂), 43.2 (N-CH₂), 46.1 (CH αTrp), 56.8 (Cq Aib), 109.5 (C₃ Trp), 111.8 (C₇ Trp), 111.9 (C₇ indole), 113.9 (C₃ indole), 118.1 (C₄ Trp), 118.5 (C₅ indole), 118.6 (C₄ indole), 118.9 (C₅ Trp), 121.3 (C₆ indole and C₆ Trp), 122.8 (C₂ indole and C₂ Trp), 127.3 (C₉ Trp), 127.4 (C₉ indole), 136.5 (C₈ Trp), 136.8 (C₈ indole), 154.7 (Cq triazole), 172.0 (CO Aib).

### (R)-N-(1-(4,5-bis(2-(1H-indol-3-yl)ethyl)-4H-1,2,4-triazol-3-yl)-2-(1H-indol-3-yl)ethyl)-2-amino-2-methylpropanamide (Compound 17):

¹H NMR (300 MHz, DMSO-d⁶, 300°K):
δ (ppm) 1.30 (3H, s, CH₃ Aib), 1.37 (3H, s, CH₃ Aib), 2.50 (2H, m, N-CH₂-CH₂-indole), 2.68 (2H, t, Jₒ= 8 Hz, C-CH₂-CH₂-indole), 2.91 (2H, t, Jₒ= 8 Hz, C-CH₂-CH₂-indole), 3.34 (2H, m, N-CH₂-CH₂-indole), 3.93 (2H, m, CH₂ βTrp), 5.25 (1 H, m, CH αTrp), 6.72-6.94 (4H, m, H₅ and H₆ Trp, H₅ indole from C-CH₂-CH₂-indole and H₅ indole from N-CH₂-CH₂-indole), 6.98-7.04 (4H, m, H₂ Trp, H₆ indole from C-CH₂-CH₂-indole, H₂ and H₆ indole from N-CH₂-CH₂-indole), 7.11 (1 H, s, H₂ indole from C-CH₂-CH₂-indole), 7.19 (1 H, d, Jₒ= 8 Hz, H₄ indole from N-CH₂-CH₂-indole), 7.28 (3H, m, H₄ and H₇ Trp, H₇ indole from N-CH₂-CH₂-indole), 7.40 (1 H, d, Jₒ= 8 Hz, H₇ indole from C-CH₂-CH₂-indole), 7.44 (1 H, d, Jₒ= 8 Hz, H₄ indole from C-CH₂-CH₂-indole), 8.04 (3H, brs, NH₂ Aib), 9.69 (1 H, d, J= 8 Hz, NH amide), 10.73 (1 H, s, NH indole from C-CH₂-CH₂-indole), 10.82 (1 H, d, J₌ 2 Hz, NH indole Trp), 10.84 (1 H, s, NH indole from N-CH₂-CH₂-indole).
¹³C NMR (75 MHz, DMSO-d⁶, 300°K):
δ (ppm) 22.7 (C-CH₂-CH₂-indole), 23.6 (CH₃ Aib), 23.8 (CH₃ Aib), 25.4 (C-CH₂-CH₂-indole), 26.0 (N-CH₂-CH₂-indole), 29.6 (CH₂ βTrp), 43.9 (N-CH₂-CH₂-indole), 46.0 (CH αTrp), 56.8 (Cq Aib), 109.5 (C₃ indole from N-CH₂-CH₂-indole), 109.9 (C₃ Trp), 111.7 (C₇ Trp), 111.9 (C₇ indole from N-CH₂-CH₂-indole and C₇ indole from C-CH₂-CH₂-indole), 113.5 (C₃ indole from C-CH₂-CH₂-indole), 118.3 (C₄ indole from N-CH₂-CH₂-indole), 118.4 (C₄ Trp), 118.5 (C₅ indole from C-CH₂-CH₂-indole), 118.7 (C₄ indole from C-CH₂-CH₂-indole), 118.9 (C₅ Trp), 119.0 (C₅ indole from N-CH₂-CH₂-indole), 121.3 (C₆ Trp), 121.5 (C₆ indole from C-CH₂-CH₂-indole and C₆ indole from N-CH₂-CH₂-indole), 122.8 (C₂ Trp, C₂ indole from C-CH₂-CH₂-indole and indole from N-CH₂-CH₂-indole), 127.1 (C₉ Trp), 127.2 (C₉ indole from C-CH₂-CH₂-indole), 127.4 (C₉ indole from N-CH₂-CH₂-indole), 136.5 (C₈ Trp and C₈ indole from C-CH₂-CH₂-indole), 136.6 (C₈ indole from N-CH₂-CH₂-indole), 154.5 (Cq triazole), 154.8 (Cq triazole), 171.8 (CO amide).

### (R)-N-(1-(4-(3-methoxybenzyl)-5-phenethyl-4H-1,2,4-triazol-3-yl)-2-(1H-indol-3-yl)ethyl)-2-amino-2-methylpropanamide (Compound 19):

¹H NMR (300 MHz, DMSO-d⁶, 300°K):
δ (ppm) 1.24 (3H, s, CH₃ Aib), 1.27 (3H, s, CH₃ Aib), 2.82 (4H, m, CH₂-CH₂-phenyl), 3.32 (2H, m, CH₂ βTrp), 3.63 (3H, s, OCH₃), 5.08 (2H, m, CH₂-m-methoxybenzyl), 5.18 (1 H, m, CH αTrp), 6.35 (1 H, d, Jₒ= 8 Hz, H₆ m-methoxybenzyl), 6.57 (1 H, s, H₂ m-methoxybenzyl), 6.82 (1 H, t, Jₒ= 8 Hz, H₅ Trp), 6.84 (1 H, d, Jₒ= 8 Hz, H₄ m-methoxybenzyl), 6.99 (1 H, t, Jₒ= 8 Hz, H₆ Trp), 7.08 (1 H, m, H₄ phenyl), 7.11-7,16 (5H, m, H₂ and H₄ Trp, H₂ and H₆ phenyl, H₅ m-methoxybenzyl), 7.20 (2H, m, H₃ and H₅ phenyl), 7.27 (1 H, d, Jₒ= 8 Hz, H₇ Trp), 8.01 (3H, brs, NH₂ Aib), 8.96 (1 H, d, J= 8 Hz, NH amide), 10.81 (1 H, d, J₌ 2 Hz, NH indole).
¹³C NMR (75 MHz, DMSO-d⁶, 300°K):
δ (ppm) 23.5 (CH₃ Aib), 23.8 (CH₃ Aib), 26.4 (CH₂-CH₂-phenyl), 29.1 (CH₂ βTrp), 32.7 (CH₂-CH₂-phenyl), 45.7 (CH αTrp), 45.8 (CH₂-*m*-methoxybenzyl), 55.5 (OCH₃), 56.7 (Cq Aib), 109.8 (C₃ Trp), 111.8 (C₇ Trp), 112.5 (C₂ m-methoxybenzyl), 113.5 (C₄ m-methoxybenzyl), 118.2 (C₄ Trp), 118.4 (C₆ m-methoxybenzyl), 118.7 (C₅ Trp), 121.3 (C₆ Trp), 124.8 (C₂ Trp), 126.5 (C₄ phenyl), 127.3 (C₉ Trp), 128.7 (C₂, C₃, C₅ and C₆ phenyl), 130.5 (C₅ m-methoxybenzyl), 136.4 (C₈ Trp), 137.7 (C₁ m-methoxybenzyl), 170.9 (C₁ phenyl), 154.6 (Cq triazole), 154.9 (Cq triazole), 160.1 (C₃ m-methoxybenzyl), 171.9 (CO amide).

### (R)-N-(1-(4-(4-methoxybenzyl)-5-phenethyl-4H-1,2,4-triazol-3-yl)-2-(1H-indol-3-yl)ethyl)-2-amino-2-methylpropanamide (Compound 20):

¹H NMR (300 MHz, DMSO-d⁶, 300°K):
δ (ppm) 1.28 (3H, s, CH₃ Aib), 1.32 (3H, s, CH₃ Aib), 2.46 (2H, m, CH₂-CH₂-phenyl), 2.82 (2H, m, CH₂-CH₂-phenyl), 3.35 (2H, d, J= 7 Hz, CH₂ βTrp), 3.68 (3H, s, OCH₃), 5.02 (2H, s, CH₂-p-methoxybenzyl), 5.22 (1 H, m, CH αTrp), 6.73-6.81 (4H, m, CHar p-methoxybenzyl), 6.84 (1 H, t, Jₒ= 7 Hz, H₅ Trp), 7.00 (1 H, t, Jₒ= 7 Hz, H₆ Trp), 7.05-7.11 (4H, m, H₂ and H₆ phenyl, H₂ and H₄ Trp), 7.14-7.22 (3H, m, H₃, H₄ and H₅ phenyl), 7.29 (1 H, d, Jₒ= 8 Hz, H₇ Trp), 8.09 (3H, brs, NH₂ Aib), 8.99 (1 H, d, J= 8 Hz, NH amide), 10.83 (1 H, s, NH indole Trp).
¹³C NMR (75 MHz, DMSO-d⁶, 300°K):
δ (ppm) 23.5 (CH₃ Aib), 23.8 (CH₃ Aib), 26.5 (CH₂-CH₂-phenyl), 29.1 (CH₂ βTrp), 32.6 (CH₂-CH₂-phenyl), 45.5 (CH₂-p-methoxybenzyl), 45.7 (CH αTrp), 55.5 (OCH₃), 56.8 (Cq Aib), 109.7 (C₃ Trp), 111.8 (C₇ Trp), 114.6 (C₃ and C₅ *p*-methoxybenzyl), 118.3 (C₄ Trp), 118.7 (C₅ Trp), 121.3 (C₆ Trp), 124.9 (C₂ Trp), 126.6 (C₂ and C₆ phenyl), 127.3 (C₉ Trp), 127.6 (C₁ p-methoxybenzyl), 128.0 (C₂ and C₆ p-methoxybenzyl), 128.7 (C₃, C₄ and C₅ phenyl), 136.4 (C₈ Trp), 140.8 (C₁ phenyl), 154.5 (Cq triazole), 154.8 (Cq triazole), 159.2 (C₄ p-methoxybenzyl), 172.0 (CO Aib).

### (R)-N-(1-(4-(4-methoxybenzyl)-5-(3-phenylpropyl)-4H-1,2,4-triazol-3-yl)-2-(1H-indol-3-yl)ethyl)-2-amino-2-methylpropanamide (Compound 22):

¹H NMR (300 MHz, DMSO-d⁶, 300°K):
δ (ppm) 1.27 (3H, s, CH₃ Aib), 1.31 (3H, s, CH₃ Aib), 1.73 (2H, m, CH₂-CH₂-CH₂-phenyl), 2.47 (2H, m, CH₂-CH₂-CH₂-phenyl), 2.52 (2H, t, ³J= 7 Hz, CH₂-CH₂-CH₂-phenyl), 3.35 (2H, d, J= 7 Hz, CH₂ βTrp), 3.68 (3H, s, OCH₃), 4.98 (2H, s, CH₂-p-methoxybenzyl), 5.20 (1 H, m, CH αTrp), 6.75 (4H, m, CHar p-methoxybenzyl), 6.82 (1 H, t, Jₒ= 7 Hz, H₅ Trp), 6.99 (1 H, t, Jₒ= 7 Hz, H₆ Trp), 7.04-7.07 (4H, m, H₂ and H₆ phenyl, H₂ and H₄ Trp), 7.13-7.24 (3H, m, H₃, H₄ and H₅ phenyl), 7.29 (1 H, d, Jₒ= 8 Hz, H₇ Trp), 8.03 (3H, brs, NH₂ Aib), 8.96 (1 H, d, J= 8 Hz, NH amide), 10.80 (1 H, d, J= 2 Hz, NH indole Trp).
¹³C NMR (75 MHz, DMSO-d⁶, 300°K):
δ (ppm) 23.6 (CH₃ Aib), 23.6 (CH₃ Aib), 24.06 (CH₂-CH₂-CH₂-phenyl), 28.5 (CH₂-CH₂-CH₂-phenyl),29.2 (CH₂ βTrp), 34.7 (CH₂-CH₂-CH₂-phenyl), 45.5 (CH₂-p-methoxybenzyl), 45.8 (CH αTrp), 55.5 (OCH₃), 56.8 (Cq Aib), 109.8 (C₃ Trp), 111.8 (C₇ Trp), 114.6 (C₃ and C₅ *p*-methoxybenzyl), 118.3 (C₄ Trp), 118.7 (C₅ Trp), 121.3 (C₆ Trp), 124.9 (C₂ Trp), 126.2 (C₂ and C₆ phenyl), 127.3 (C₉ Trp), 127.8 (C₁ p-methoxybenzyl), 127.9 (C₂ and C₆ p-methoxybenzyl), 128.7 (C₃, C₄ and C₅ phenyl), 136.4 (Cg Trp), 141.7 (C₁ phenyl), 154.8 (Cq triazole), 159.2 (C₄ *p*-methoxybenzyl), 171.9 (CO Aib).

### (R)-N-(1-(4-(2-(1H-indol-3-yl)ethyl)-5-(3-(1H indol-3-yl)propyl)-4H 1,2,4-triazol-3-yl)-2-(1H-indol-3-yl)ethyl)-2-amino-2-methylpropanamide (Compound 24):

¹H NMR (300 MHz, DMSO-d⁶, 300°K):
δ (ppm) 1.29 (3H, s, CH₃ Aib), 1.35 (3H, s, CH₃ Aib), 1.78 (2H, m, CH₂-CH₂-CH₂-indole), 2.34 (2H, m, CH₂-CH₂-CH₂-indole), 2.48 (2H, m, N-CH₂-CH₂-indole), 2.80 (2H, m, CH₂-CH₂-CH₂-indole), 3.34 (2H, m, N-CH₂-CH₂-indole), 3.94 (2H, m, CH₂ βTrp), 5.27 (1 H, m, CH αTrp), 6.73-6.94 (4H, m, H₅ and H₆ Trp, H₅ indole from N-CH₂-CH₂-indole and H₅ indole from CH₂-CH₂-CH₂-indole), 6.99-7.04 (5H, m, H₂ Trp, H₂ and H₆ indole from N-CH₂-CH₂-indole, H₂ and H₆ indole from CH₂-CH₂-CH₂-indole), 7.20 (1 H, d, Jₒ= 8 Hz, H₄ indole from N-CH₂-CH₂-indole), 7.29 (3H, m, H₄ and H₇ Trp, H₇ indole from N-CH₂-CH₂-indole), 7.40 (1 H, d, Jₒ= 8 Hz, H₇ indole from CH₂-CH₂-CH₂-indole), 7.44 (1 H, d, Jₒ= 8 Hz, H₄ indole from CH₂-CH₂-CH₂-indole), 8.05 (3H, brs, NH₂ Aib), 9.07 (1 H, d, J= 8 Hz, NH amide), 10.75 (1 H, s, NH indole from CH₂-CH₂-CH₂-indole), 10.86 (1 H, s, NH indole Trp), 10.90 (1 H, s, NH indole from N-CH₂-CH₂-indole).
¹³C NMR (75 MHz, DMSO-d⁶, 300°K):
δ (ppm) 23.6 (CH₃ Aib), 23.8 (CH₃ Aib), 24.5 (CH₂-CH₂-CH₂-indole), 25.8 (CH₂-CH₂-CH₂-indole), 27.2 (CH₂-CH₂-CH₂-indole), 29.4 (CH₂ βTrp), 44.1 (N-CH₂-CH₂-indole), 46.0 (CH αTrp), 52.9 (N-CH₂-CH₂-indole), 56.8 (Cq Aib), 109.7 (C₃ Trp and C₃ indole from N-CH₂-CH₂-indole), 111.8 (C₇ Trp), 111.9 (C₇ indole from N-CH₂-CH₂-indole and C₇ indole from CH₂-CH₂-CH₂-indole), 114.0 (C₃ indole from CH₂-CH₂-CH₂-indole), 118.2 (C₄ indole from N-CH₂-CH₂-indole), 118.3 (C₄ Trp), 118.5 (C₅ indole from CH₂-CH₂-CH₂-indole), 118.6 (C₄ indole from CH₂-CH₂-CH₂-indole), 118.9 (C₅ Trp), 119.0 (C₅ indole from N-CH₂-CH₂-indole), 121.3 (C₆ Trp), 121.4 (C₆ indole from CH₂-CH₂-CH₂-indole), 121.6 (C₆ indole from N-CH₂-CH₂-indole), 122.7 (C₂ Trp, C₂ indole from N-CH₂-CH₂-indole and C₂ indole from CH₂-CH₂-CH₂-indole), 127.1 (C₉ Trp), 127.4 (C₉ indole from N-CH₂-CH₂-indole and C₉ indole from CH₂-CH₂-CH₂-indole), 136.4 (C₈ Trp), 136.5 (C₈ indole from CH₂-CH₂-CH₂-indole), 136.7 (C₈ indole from N-CH₂-CH₂-indole), 154.7 (2 Cq triazole), 171.9 (CO amide).

### (R)-N-(1-(4-(2-methoxybenzyl)-5-phenethyl-4H-1,2,4-triazol-3-yl)-2-(1H-indol-3-yl)ethyl)-2-amino-2-methylpropanamide (Compound 26):

¹H NMR (300 MHz, DMSO-d⁶, 300°K):
δ (ppm) 1.26 (3H, s, CH₃ Aib), 1.29 (3H, s, CH₃ Aib), 2.78-2.92 (4H, m, CH₂-CH₂-phenyl), 3.29 (2H, m, CH₂ βTrp), 3.65 (3H, s, OCH₃), 4.97-5.21 (3H, m, CH αTrp and CH₂-o-methoxybenzyl), 6.52 (1 H, d, Jₒ= 7 Hz, H₃ o-methoxybenzyl), 6.78 (1 H, t, Jₒ= 7 Hz, H₅ Trp), 6.82 (1 H, t, Jₒ= 8 Hz, H₆ Trp), 6.84-7.04 (3H, m, H₄, H₅ and H₆ *o-*methoxybenzyl), 7.15 (1 H, d, Jₒ= 7 Hz, H₄ Trp), 7.19-7.29 (4H, m, H₃, H₄ and H₅ phenyl, H₇ Trp), 8.03 (3H, brs, NH₂ Aib), 8.94 (1 H, d, J= 8 Hz, NH amide), 10.82 (1 H, s, NH indole Trp).
¹³C NMR (75 MHz, DMSO-d⁶, 300°K):
δ (ppm) 23.6 (CH₃ Aib), 23.7 (CH₃ Aib), 26.3 (CH₂-CH₂-phenyl), 29.0 (CH₂ βTrp), 32.5 (CH₂-CH₂-phenyl), 42.3 (CH₂-o-methoxybenzyl), 45.7 (CH αTrp), 55.8 (OCH₃), 56.7 (Cq Aib), 109.7 (C₃ Trp), 111.5 (C₇ Trp), 111.8 (C₃ o-methoxybenzyl), 118.2 (C₄ Trp), 118.7 (C₅ Trp), 121.0 (C₆ Trp), 121.3 (C₅ o-methoxybenzyl), 123.2 (C₁ o-methoxybenzyl), 124.9 (C₂ Trp), 126.6 (C₂ and C₆ phenyl), 127.2 (C₉ Trp and C₄ *o-*methoxybenzyl), 128.7 (C₃, C₄ and C₅ phenyl), 129.9 (C₆ o-methoxybenzyl), 136.4 (C₈ Trp), 140.6 (C₁ phenyl), 154.8 (Cq triazole), 155.2 (Cq triazole), 156.7 (C₂ *o-*methoxybenzyl), 171.9 (CO Aib).

### (R)-N-(2-(1H-indol-3-yl)-1-(4-(naphthalen-1-ylmethyl)-5-phenethyl-4H-1,2,4-triazol-3-yl)ethyl)-2-amino-2-methylpropanamide (Compound 27):

¹H NMR (300 MHz, DMSO-d⁶, 300°K):
δ (ppm) 1.21 (3H, s, CH₃ Aib), 1.25 (3H, s, CH₃ Aib), 2.46 (2H, m, CH₂-CH₂-phenyl), 2.88 (2H, m, CH₂-CH₂-phenyl), 3.26 (2H, dd, ³J= 14 Hz and 6 Hz, CH₂ βTrp), 3.36 (2H, dd, ³J= 14 Hz and 9 Hz, CH₂ βTrp), 4.99 (1 H, m, CH αTrp), 5.65 (1 H, d, ³J= 18 Hz, CH₂-naphtyl), 5.78 (1 H, d, ³J= 18 Hz, CH₂-naphtyl), 6.29 (1 H, d, Jₒ= 7 Hz, H₂ naphtyl), 6.45 (1 H, t, Jₒ= 7 Hz, H₅ Trp), 6.62 (1 H, d, Jₒ= 8 Hz, H₄ Trp), 6.88 (1 H, t, Jₒ= 8 Hz, H₆ Trp), 7.04-7.06 (4H, m, H₂ and H₇ Trp, H₂ and H₆ phenyl), 7.07-7.25 (H₃ naphtyl, H₃, H₄ and H₅ phenyl), 7.57-7.60 (2H, m, H₆ and H₇ naphtyl), 7.86 (1 H, d, Jₒ= 8 Hz, H₄ naphtyl), 7.98-8.00 (4H, m, H₅ and H₈ naphtyl, NH₂ Aib), 8.96 (1 H, d, J= 8 Hz, NH amide), 10.77 (1 H, s, NH indole Trp).
¹³C NMR (75 MHz, DMSO-d⁶, 300°K):
δ (ppm) 23.5 (CH₃ Aib), 23.6 (CH₃ Aib), 26.3 (CH₂CH₂-phenyl), 29.2 (CH₂ βTrp), 32.6 (CH₂-CH₂-phenyl), 43.8 (CH₂-naphtyl), 45.6 (CH αTrp), 56.7 (Cq Aib), 109.7 (C₃ Trp), 111.7 (C₇ Trp), 117.9 (C₄ Trp), 118.4 (C₅ Trp), 121.1 (C₆ Trp), 122.1 (C₂ naphtyl), 123.0 (C₈ naphtyl), 124.9 (C₂ Trp), 125.9 (C₃ naphtyl), 126.5 (C₆ naphtyl), 126.9 (C₂ and C₆ phenyl), 127.0 (C₉ Trp and C₇ naphtyl), 127.1 (C₄ naphtyl), 128.4 (C₅ naphtyl), 128.7 (C₃, C₄ and C₅ phenyl), 130.0 (C₉ naphtyl), 131.7 (C₁ naphtyl), 133.6 (C₁₀ naphtyl), 136.4 (C₈ Trp), 140.8 (C₁ phenyl), 154.8 (Cq triazole), 155.3 (Cq triazole), 171.9 (CO Aib).

### (R)-N-(1-(5-(2-(1H-indol-3-yl)ethyl)-4-(3,4-dichlorobenzyl)-4H-1,2,4-triazol-3-yl)-2-(1H-indol-3-yl)ethyl)-2-amino-2-methylpropanamide (Compound 28):

¹H NMR (300 MHz, DMSO-d⁶, 300°K):
δ (ppm) 1.26 (6H, s, CH₃ Aib), 2.87 (2H, m, CH₂-CH₂-indole), 2.96 (2H, m, CH₂-CH₂-indole), 3.32 (2H, m, CH₂ βTrp), 5.13 (3H, m, CH αTrp and CH₂-*m,p-*dichlorobenzyl), 6.58 (1 H, d, Jₒ= 8 Hz, H₆ *m,p*-dichlorobenzyl), 6.85 (1 H, t, Jₒ= 7 Hz, H₅ Trp), 6.96 (1 H, t, Jₒ= 7 Hz, H₅ indole), 7.01 (2H, m, H₆ indole and H₆ Trp), 7.04 (1 H, s, H₂ Trp), 7.08 (1 H, s, H₂ indole), 7.13 (1 H, d, Jₒ= 8 Hz, H₅ *m,p*-dichlorobenzyl), 7.20-7.30 (4H, m, H₄ and H₇ indole, H₇ Trp and H₂ *m,p-*dichlorobenzyl), 7.36 (1 H, d, Jₒ= 8 Hz, H₄ Trp), 8.08 (3H, brs, NH₂ Aib), 8.98 (1 H, d, J= 8 Hz, NH amide), 10.80 (1 H, s, NH indole), 10.82 (1 H, s, NH indole Trp).
¹³C NMR (75 MHz, DMSO-d⁶, 300°K):
δ (ppm) 22.8 (CH₂-CH₂-indole), 23.4 (CH₃ Aib), 23.8 (CH₃ Aib), 25.8 (CH₂-CH₂-indole), 29.0 (CH₂ βTrp), 44.8 (CH₂-*m,p-*dichlorobenzyl), 45.6 (CH αTrp), 56.8 (Cq Aib), 109.7 (C₃ indole), 111.8 (C₇ indole and C₇ Trp), 118.1 (C₄ Trp), 118.4 (C₅ indole), 118.6 (C₄ indole and C₅ Trp), 121.3 (C₆ indole and C₆ Trp), 123.0 (C₂ indole and C₂ Trp), 126.4 (C₆ *m,p-*dichlorobenzyl), 127.1 (C₉ Trp), 127.3 (C₉ indole), 128.6 (C₂ m,p-dichlorobenzyl), 130.9 (C₄ *m,p*-dichlorobenzyl),131.3 (C₅ *m,p*-dichlorobenzyl), 132.0 (C₃ *m,p*-dichlorobenzyl), 136.4 (C₈ Trp), 136.6 (C₈ indole), 137.2 (C₁ *m,p-*dichlorobenzyl), 154.7 (Cq triazole), 155.1 (Cq triazole), 172.0 (CO Aib).

### (R)-N-(1-(4-(4-fluorobenzyl)-5-benzyl-4H-1,2,4-triazol-3-yl)-2-(1H-indol-3-yl)ethyl)-2-amino-2-methylpropanamide (Compound 30):

¹H NMR (300 MHz, DMSO-d⁶, 300°K):
δ (ppm) 1.27 (3H, s, CH₃ Aib), 1.29 (3H, s, CH₃ Aib), 3.33 (2H, m, CH₂ βTrp), 4.02 (2H, s, CH₂-benzyl), 5.10 (3H, m, CH₂-*p*-fluorobenzyl and CH αTrp), 6.71 (2H, m, H₃ and H₅ *p*-fluorobenzyl), 6.80 (1 H, t, Jₒ= 8 Hz, H₅ Trp), 6.90 (2H, d, Jₒ= 8 Hz, H₂ and H₆ p-fluorobenzyl), 6.94 (1 H, t, Jₒ= 8 Hz, H₆ Trp), 6.99-7.10 (4H, m, H₂ and H₄ Trp, H₂ and H₆ benzyl), 7.20 (3H, m, H₃, H₄ and H₅ benzyl), 7.27 (1 H, d, Jₒ= 8 Hz, H₇ Trp), 8.09 (3H, brs, NH₂ Aib), 8.97 (1 H, d, J= 8 Hz, NH amide), 10.79 (1 H, s, NH indole Trp). ¹³C NMR (75 MHz, DMSO-d⁶, 300°K):
6(ppm) 23.5 (CH₃ Aib), 23.8 (CH₃ Aib), 29.0 (CH₂ βTrp), 31.1 (CH₂-benzyl), 45.7 (CH₂-p-fluorobenzyl), 45.8 (CH αTrp), 56.8 (Cq Aib), 109.6 (C₃ Trp), 111.8 (C₇ Trp), 115.6 and 115.9 (C₃ and C₅ p-fluorobenzyl), 118.2 (C₄ Trp), 118.7 (C₅ Trp), 121.3 (C₆ Trp), 124.8 (C₂ Trp), 127.1 (C₄ benzyl), 127.2 (C₉ Trp), 128.8 and 128.9 (C₂ and C₆ *p-*fluorobenzyl), 129.4 (C₂, C₃, C₅ and C₆ p-fluorobenzyl), 131.6 (C₁ *p*-fluorobenzyl), 135.9 (C₁ benzyl), 136.4 (C₈ Trp), 154.0 (C₄ p-fluorobenzyl), 155.3 (Cq triazole), 172.0 (CO amide).

### (R)-N-(1-(4-(4-methylbenzyl)-5-(3-phenylpropyl)-4H-1,2,4-triazol-3-yl)-2-(1H-indol-3-yl)ethyl)-2-amino-2-methylpropanamide (Compound 33):

¹H NMR (300 MHz, DMSO-d⁶, 300°K):
δ (ppm) 1.25 (3H, s, CH₃ Aib), 1.28 (3H, s, CH₃ Aib), 1.73 (2H, m, CH₂-CH₂-CH₂-phenyl), 2.23 (3H, s, CH₃ *p*-methylbenzyl), 2.49-2.54 (4H, m, CH₂-CH₂-CH₂-phenyl), 3.33 (2H, m, CH₂ βTrp), 5.04 (2H, s, CH₂-*p*-methylbenzyl), 5.16 (1 H, m, CH αTrp), 6.74 (2H, d, Jₒ= 8 Hz, H₃ and H₅ *p-*methylbenzyl), 6.80 (1 H, t, Jₒ=7 Hz, H₅ Trp), 6.98 (1 H, t, Jₒ= 7 Hz, H₆ Trp), 7.03 (1 H, d, J= 2 Hz, H₂ Trp), 7.06 (5H, m, CHar phenyl), 7.14 (1 H, d, Jₒ= 7 Hz, H₄ Trp), 7.20 (2H, d, Jₒ= 7 Hz, H₂ and H₆ *p-*methylbenzyl), 7.27 (1 H, d, Jₒ= 8 Hz, H₇ Trp), 8.01 (3H, brs, NH₂ Aib), 8.95 (1 H, d, J= 8 Hz, NH amide), 10.80 (1 H, d, J= 2 Hz, NH indole Trp).
¹³C NMR (75 MHz, DMSO-d⁶, 300°K):
δ (ppm) 21.0 (CH₃ p-methylbenzyl), 23.5 (CH₃ Aib), 23.8 (CH₃ Aib), 24.0 (CH₂-CH₂-CH₂-phenyl), 28.5 (CH₂-CH₂-CH₂-phenyl), 29.1 (CH₂ βTrp), 34.7 (CH₂-CH₂-CH₂-phenyl), 45.7 (CH αTrp), 45.8 (CH₂-*p*-methylbenzyl), 56.8 (Cq Aib), 109.8 (C₃ Trp), 111.8 (C₇ Trp), 118.3 (C₄ Trp), 118.7 (C₅ Trp), 121.3 (C₆ Trp), 124.9 (C₂ Trp), 126.2 (C₄ phenyl), 126.4 (C₃ and C₅ *p-*methylbenzyl), 127.3 (C₉ Trp), 128.7 (C₂, C₃, C₅ and C₆ phenyl), 129.8 (C₂ and C₆ *p-*methylbenzyl), 133.0 (C₁ *p-*methylbenzyl), 136.4 (C₈ Trp), 137.5 (C₄ *p*-methylbenzyl), 141.7 (C₁ phenyl), 154.8 (Cq triazole), 171.9 (CO Aib).

### (R)-N-(1-(5-(2-(1H-indol-3-yl)ethyl)-4-(4-methylbenzyl)-4H-1 1,2,4-triazol-3-yl)-2-(1H-indol-3-yl)ethyl)-2-amino-2-methylpropanamide (Compound 34):

¹H NMR (300 MHz, DMSO-d⁶, 300°K):
δ (ppm) 1.25 (3H, s, CH₃ Aib), 1.28 (3H, s, CH₃ Aib), 2.23 (3H, s, CH₃-*p*-methylbenzyl), 2.84-2.97 (4H, m, CH₂-CH₂-indole), 3.32 (2H, m, CH₂ βTrp), 5.04 (2H, s, CH₂- *p-*methylbenzyl), 5.16 (1H, m, CH αTrp), 6.79-6.86 (4H, m, CH ar *p*-methylbenzyl), 6.99-7.05 (4H, m, H₅ and H₆ indole, H₅ and H₆ Trp), 7.08 (3H, m, H₂ indole, H₂ and H₄ Trp), 7.25-7.30 (3H, m, H₄ and H₇ indole, H₇ Trp), 8.00 (3H, brs, NH₂ Aib), 8.94 (1H, d, J= 8 Hz, NH amide), 10.76 (1H, s, NH indole), 10.78 (1H, s, NH indole Trp).
¹³C NMR (75 MHz, DMSO-d⁶, 300°K):
δ (ppm) 21.0 (CH₃- p-methylbenzyl), 22.8 (CH₂-CH₂-indole), 23.8 (CH₃ Aib), 23.9 (CH₃ Aib), 25.9 (CH₂-CH₂-indole), 28.5 (CH₂ βTrp), 45.7 (CH₂-p-methylbenzyl and CH αTrp), 56.7 (Cq Aib), 109.9 (C₃ Trp), 111.8 (C₇ indole and C₇ Trp), 113.4 (C₃ indole), 118.1 (C₄ Trp), 118.3 (C₄ indole), 118.5 (C₅ indole), 118.7 (C₅ Trp), 120.9 (C₆ indole and C₆ Trp), 121.3 (C₂ indole and C₂ Trp), 126.3 (C₃ and C₅ p-methylbenzyl), 127.2 (C₉ indole), 127.3 (C₉ Trp), 129.8 (C₂ and C₆ *p*-methylbenzyl), 133.1 (C₁ p-methylbenzyl), 135.8 (C₈ indole, C₈ Trp), 136.4 (C₄ p-methylbenzyl), 154.8 (Cq triazole), 155.0 (Cq triazole), 171.9 (CO Aib).

### (R)-N-(1-(4-(4-methylbenzyl)-5-phenethyl-4H-1,2,4-triazol-3-yl)-2-(1H-indol-3-yl)ethyl)-2-amino-2-methylpropanamide (Compound 37):

¹H NMR (300 MHz, DMSO-d⁶, 300°K):
δ (ppm) 1.25 (3H, s, CH₃ Aib), 1.28 (3H, s, CH₃ Aib), 2.23 (3H, s, CH₃ p-methylbenzyl), 2.83 (4H, m, CH₂-CH₂-phenyl), 3.32 (2H, m, CH₂ βTrp), 5.05 (2H, s, CH₂-p-methylbenzyl), 5.18 (1H, m, CH αTrp), 6.75 (2H, d, Jₒ= 8 Hz, H₃ and H₅ p-methylbenzyl), 6.82 (1H, t, Jₒ= 8 Hz, H₅ Trp), 6.99 (1H, t, Jₒ= 8 Hz, H₆ Trp), 7.02-7.11 (6H, m, H₂ Trp and CHar phenyl), 7.15 (1H, d, Jₒ= 7 Hz, H₄ Trp), 7.20 (2H, d, Jₒ= 7 Hz, H₂ and H₆ p-methylbenzyl), 7.28 (1H, d, Jₒ= 8 Hz, H₇ Trp), 8.01 (3H, brs, NH₂ Aib), 8.93 (1H, d, J= 8 Hz, NH amide), 10.77 (1H, s, NH indole Trp).
¹³C NMR (75 MHz, DMSO-d⁶, 300°K):
δ (ppm) 21.0 (CH₃ *p*-methylbenzyl), 23.6 (CH₃ Aib), 23.8 (CH₃ Aib), 26.5 (CH₂-CH₂-phenyl), 29.1 (CH₂ βTrp), 32.7 (CH₂-CH₂-phenyl), 45.7 (CH αTrp and CH₂-p-methylbenzyl), 56.8 (Cq Aib), 109.8 (C₃ Trp), 111.8 (C₇ Trp), 118.3 (C₄ Trp), 118.7 (C₅ Trp), 121.3 (C₆ Trp), 124.8 (C₂ Trp), 126.4 (C₃ and C₅ *p*-methylbenzyl), 126.6 (C₄ phenyl), 127.3 (C₉ Trp), 128.7 (C₂, C₃, C₅ and C₆ phenyl), 129.8 (C₂ and C₆ p-methylbenzyl), 133.0 (C₁ p-methylbenzyl), 136.4 (C₈ Trp), 137.5 (C₄ p-methylbenzyl), 140.9 (C₁ phenyl), 154.5 (Cq triazole), 154.9 (Cq triazole), 171.9 (CO amide).

### (R)-N-(1-(5-benzyl-4-(pyridin-2-ylmethyl)-4H-1,2,4-triazol-3-yl)-2-(1H-indol-3-yl)ethyl)-2-amino-2-methylpropanamide (Compound 39):

¹H NMR (300 MHz, DMSO-d⁶, 300°K):
δ (ppm) 1.23 (3H, s, CH₃ Aib), 1.27 (3H, s, CH₃ Aib), 3.34 (1H, dd, J= 14 Hz and 6 Hz, CH₂ βTrp), 3.43 (1H, dd, J= 14 Hz and 9 Hz, CH₂ βTrp), 4.13 (2H, s, CH₂-benzyl), 5.22 (1H, s, CH αTrp), 5.35 (2H, s, CH₂-*o*-pyridyl), 6.80 (1H, t, Jₒ= 8 Hz, H₅ Trp), 6.92 (1H, t, Jₒ= 8 Hz, H₅ pyridyl), 6.97 (1H, t, Jₒ= 8 Hz, H₆ Trp), 7.04 (1H, d, Jₒ= 8 Hz, H₄ Trp), 7.07 (1H, d, J= 2 Hz, H₂ Trp), 7.10-7.16 (5H, m, CHar benzyl), 7.19 (1H, s, H₃ o-pyridyl), 7.26 (1H, d, Jₒ= 8 Hz, H₇ Trp), 7.57 (1H, t, Jₒ= 9 Hz, H₄ o-pyridyl), 8.16 (3H, brs, NH₂ Aib), 8.36 (1H, d, J_{αβ}= 5 Hz, H₆ o-pyridyl), 9.01 (1H, d, J= 8 Hz, NH amide), 10.85 (1H, s, NH indole Trp).
¹³C NMR (75 MHz, DMSO-d⁶, 300°K):
δ (ppm) 23.4 (CH₃ Aib), 23.7 (CH₃ Aib), 28.6 (CH₂ βTrp), 30.4 (CH₂-benzyl), 45.7 (CH αTrp), 47.7 (CH₂- o-pyridyl), 56.7 (Cq Aib), 109.8 (C₃ Trp), 111.8 (C₇ Trp), 118.3 (C₄ Trp), 118.6 (C₅ Trp), 121.2 (C₆ Trp), 121.7 (C₃ o-pyridyl), 123.3 (C₅ o-pyridyl), 124.8 (C₂ Trp), 127.1 (C₄ benzyl), 127.3 (C₉ Trp), 128.8 (C₂ and C₆ benzyl), 129.0 (C₃ and C₅ benzyl), 135.6 (C₁ benzyl), 136.4 (C₈ Trp), 137.5 (C₄ o-pyridyl), 149.5 (C₆ o-pyridyl), 154.1 (Cq triazole), 154.2 (Cq triazole), 155.7 (C₂ o-pyridyl), 172.0 (CO amide).

### (R)-N-(1-(4-(4-ethylbenzyl)-5-phenethyl-4H-1,2,4-triazol-3-yl)-2-(1H-indol-3-yl)ethyl)-2-amino-2-methylpropanamide (Compound 43):

¹H NMR (300 MHz, DMSO-d⁶, 300°K):
δ (ppm) 1.10 (3H, t, J= 8 Hz, CH₃-CH₂ *p*-ethylbenzyl), 1.25 (3H, s, CH₃ Aib), 1.28 (3H, s, CH₃ Aib), 2.53 (2H, q, J= 8 Hz, CH₃-CH₂ p-ethylbenzyl), 2.83 (4H, m, CH₂-CH₂-phenyl), 3.34 (2H, m, CH₂ βTrp), 5.07 (2H, s, CH₂-*p*-ethylbenzyl), 5.19 (1H, m, CH αTrp), 6.77 (2H, d, Jₒ= 8 Hz, H₃ and H₅ p-ethylbenzyl), 6.81 (1H, t, Jₒ= 7 Hz, H₅ Trp), 6.99 (1H, t, Jₒ= 8 Hz, H₆ Trp), 7.05-7.10 (7H, m, CHar phenyl, H₂ and H₆ *p*-ethylbenzyl), 7.13 (1H, d, J= 2 Hz, H₂ Trp), 7.20 (1H, d, Jₒ= 7 Hz, H₄ Trp), 7.28 (1H, d, Jₒ= 8 Hz, H₇ Trp), 8.03 (3H, brs, NH₂ Aib), 8.94 (1H, d, J= 8 Hz, NH amide), 10.79 (1H, s NH indole Trp).
¹³C NMR (75 MHz, DMSO-d⁶, 300°K):
δ (ppm) 15.9 (CH₃-CH₂ *p*-ethylbenzyl), 23.5 (CH₃ Aib), 23.8 (CH₃ Aib), 26.5 (CH₂-CH₂-phenyl), 28.1 (CH₃-CH₂ *p*-ethylbenzyl), 29.1 (CH₂ βTrp), 32.7 (CH₂-CH₂-phenyl), 45.7 (CH αTrp), 45.8 (CH₂-*p*-ethylbenzyl), 56.8 (Cq Aib), 109.8 (C₃ Trp), 111.8 (C₇ Trp), 118.3 (C₄ Trp), 118.7 (C₅ Trp), 121.3 (C₆ Trp), 124.9 (C₂ Trp), 126.5 (C₃ and C₅ p-ethylbenzyl), 126.6 (C₄ phenyl), 127.3 (C₉ Trp), 128.6 (C₂ and C₆ p-ethylbenzyl, C₂, C₃, C₅ and C₆ phenyl), 133.1 (C₁ p-ethylbenzyl), 136.5 (C₈ Trp), 140.8 (C₁ phenyl), 143.8 (C₄ p-ethylbenzyl), 154.6 (Cq triazole), 154.9 (Cq triazole), 171.9 (CO amide).

### (R)-N-(1-(4-(4-methoxybenzyl)-5-phenethyl-4H-1,2,4-triazol-3-yl)-2-(1H-indol-3-yl)ethyl)piperidine-4-carboxamide (Compound 44):

¹H NMR (300 MHz, DMSO d⁶, 300°K):
δ (ppm) 1.42 (m, 2H, H₃ and H₅ piperidyl), 1.55 (m, 1H, H₅ piperidyl), 2.23 (m, 1H, H₄ piperidyl), 2.75 (m, 5H, H₂ piperidyl and CH₂-CH₂-phenyl), 3.04 (m, 1H, H₆ piperidyl), 3.13 (m, 1H, H₂ piperidyl), 3.32 (m, 2H, CH₂ βTrp), 3.66 (s, 3H, OCH₃), 4.97 (m, 2H, CH₂-*p*-methoxybenzyl), 5.23 (m, 1H, CH αTrp), 6.70 (s, 4H, CHar *p*-methoxybenzyl), 6.87 (t, 1H, Jₒ= 8 Hz, H₅ Trp), 7.00 (m, 2H, H₂ and H₆ Trp), 7.07 (d, 2H, Jₒ= 8 Hz, H₂ and H₆ phenyl), 7.14 (d, 1H, Jₒ= 7 Hz, H₄ Trp), 7.18-7.30 (m, 4H, H₇ Trp, H₃, H₄ and H₅ phenyl), 8.16 and 8.46 (2 m, 2H, NH piperidyl TFA salt), 8.66 (d, 1H, J= 8 Hz, NH amide), 10.75 (1H, s, NH indole Trp).
¹³C NMR (75 MHz, DMSO d⁶, 300°K):
δ (ppm) 24.9 (C₃ piperidyl), 25.4 (C₅ piperidyl), 26.5 (CH₂-CH₂-phenyl), 29.2 (CH₂ βTrp), 32.7 (CH₂-CH₂-phenyl), 38.7 (C₄ piperidyl), 42.7 (C₂ and C₆ piperidyl), 44.7 (CH Trp), 45.3 (CH₂-p-methoxybenzyl), 55.5 (OCH₃), 110.2 (C₃ Trp), 111.7 (C₇ Trp), 114.4 (C₃ and C₅ *p*-methoxybenzyl), 118.5 (C₄ Trp), 118.7 (C₅ Trp), 121.3 (C₆ Trp), 124.4 (C₂ Trp), 126.5 (C₂ and C₆ phenyl), 127.5 (C₉ Trp), 127.8 (C₁, C₂ and C₆ *p*-methoxybenzyl), 128.7 (C₃, C₄ and C₅ phenyl), 136.4 (C₈ Trp), 140.8 (C₁ phenyl), 155.3 (Cq triazole), 155.4 (Cq triazole), 159.1 (C₄ p-methoxybenzyl), 173.1 (CO amide).

### (R)-N-(1-(5-(2-(1H-indol-3-yl)ethyl)-4-(4-methoxybenzyl)-4H-1,2,4-triazol-3-yl)-2-H-indol-3-yl)ethyl)piperidine-4-carboxamide (Compound 45):

¹H NMR (300 MHz, DMSO d⁶, 300°K):
δ (ppm) 1.41 (m, 2H, H₃ and H₅ piperidyl), 1.54 (dd, 1H, J= 13 Hz and 2 Hz, H₅ piperidyl), 2.23 (m, 1H, H₄ piperidyl), 2.72 (m, 2H, H₂ and H₆ piperidyl), 2.77-2.93 (m, 4H, CH₂-CH₂-indole), 3.06 (m, 2H, H₂ and H₆ piperidyl), 3.32 (m, 2H, CH₂ βTrp), 3.65 (s, 3H, OCH₃), 4.94 (s, 2H, CH₂-p-methoxybenzyl), 5.22 (m, 1H, CH αTrp), 6.68 (s, 4H, CHar p-methoxybenzyl), 6.87 (m, 3H, H₅ and H₆ Trp, H₅ indole), 6.98 (m, 4H, H₂ and H₆ indole, H₂ and H₄ Trp), 7.20-7.33 (m, 3H, H₄ and H₇ indole, H₇ Trp), 8.15 and 8.46 (2 m, 2H, NH piperidyl TFA salt), 8.64 (d, 1H, J= 8 Hz, NH amide), 10.74 (s, 2H, NH indole and NH indole Trp).
¹³C NMR (75 MHz, DMSO d⁶, 300°K):
δ (ppm) 22.9 (CH₂-CH₂-indole), 24.9 (C₃ piperidyl), 25.4 (C₅ piperidyl), 26.0 (CH₂-CH₂-indole), 29.3 (CH₂ βTrp), 39.1 (C₄ piperidyl), 42.7 (C₂ and C₆ piperidyl), 44.7 (CH αTrp), 45.3 (CH₂-p-methoxybenzyl), 55.5 (OCH₃), 109.5 (C₃ Trp), 111.7 (C₇ indole and C₇ Trp), 113.5 (C₃ indole), 114.4 (C₃ and C₅ p-methoxybenzyl), 118.5 (C₄ indole and C₄ Trp), 118.6 (C₅ indole and C₅ Trp), 121.2 (C₆ indole), 121.3 (C₆ Trp), 122.9 (C₂ indole and C₂ Trp), 127.2 (C₉ indole), 127.6 (C₉ Trp, C₂ and C₆ p-methoxybenzyl), 127.9 (C₁ p-methoxybenzyl), 136.4 (C₈ Trp), 136.6 (C₈ indole), 154.9 (Cq triazole), 155.2 (Cq triazole), 159.0 (C₄ p-methoxybenzyl), 173.0 (CO amide).

### (R)-N-(1-(4-(4-methoxybenzyl)-5-phenethyl-4H-1,2,4-triazol-3-yl)-2-(1H-indol-3-yl)ethyl)-2-aminoacetamide (Compound 50):

¹H NMR (300 MHz, DMSO d⁶, 300°K):
δ (ppm) 2.78 (m, 4H, CH₂-CH₂-phenyl), 3.26 (1H, dd, J= 14 Hz and 7 Hz, CH₂ βTrp), 3.39 (m, 3H, CH₂ βTrp and CH₂-NH₂), 3.65 (s, 3H, OCH₃), 4.95 (m, 2H, CH₂-p-methoxybenzyl), 5.20 (m, 1H, CH αTrp), 6.63 (s, 4H, CHar *p*-methoxybenzyl), 6.86 (t, 1H, Jₒ= 7 Hz, H₅ Trp), 6.99 (s, 1H, H₂ Trp), 7.02 (t, 1H, Jₒ= 7 Hz, H₆ Trp), 7.10 (m, 2H, H₂ and H₆ phenyl), 7.15 (d, 1H, Jₒ= 7 Hz, H₄ Trp), 7.23 (m, 3H, H₃, H₄ and H₅ Trp), 7.31 (d, 1H, Jₒ= 8 Hz, H₇ Trp), 7.95 (brs, 3H, NH₂ Gly, TFA salt), 9.20 (d, 1H, J= 8 Hz, NH amide), 10.82 (s, 1H, NH indole Trp).
¹³C NMR (75 MHz, DMSO d⁶, 300°K):
δ (ppm) 26.5 (CH₂-CH₂-phenyl), 29.8 (CH₂ βTrp), 32.7 (CH₂-CH₂-phenyl), 39.0 (CH₂-NH₂), 45.3 (CH₂- p-methoxybenzyl), 45.4 (CH αTrp), 55.4 (OCH₃), 109.7 (C₃ Trp), 111.8 (C₇ Trp), 114.5 (C₃ and C₅ p-methoxybenzyl), 118.3 (C₄ Trp), 118.9 (C₅ Trp), 121.4 (C₆ Trp), 124.6 (C₂ Trp), 126.5 (C₂ and C₆ phenyl), 127.3 (C₉ Trp), 127.7 (C₁ p-methoxybenzyl), 127.8 (C₂ and C₆ *p*-methoxybenzyl), 128.7 (C₃, C₄ and C₅ phenyl), 136.4 (C₈ Trp), 140.9 (C₁ phenyl), 154.3 (Cq triazole), 154.8 (Cq triazole), 159.0 (C₄ *p-*methoxybenzyl), 166.1 (CO amide).

### (R)-N-(1-(4-(4-methoxybenzyl)-5-phenethyl-4H-1,2,4-triazol-3-yl)-2-(1H-indol-3-yl)ethyl)-2-(pyridin-2-yl)acetamide (Compound 51):

¹H NMR (300 MHz, DMSO d⁶, 300°K):
δ (ppm) 2.77-2,88 (m, 4H, CH₂-CH₂-phenyl), 3.37 (m, 2H, CH₂ βTrp), 3.64 (s, 3H, OCH₃), 3.74 (m, 2H, CH₂-*o*-pyridyl), 5.03 (m, 2H, CH₂-*p*-methoxybenzyl), 5.24 (m, 1H, CH αTrp), 6.65 (s, 4H, CHar p-methoxybenzyl), 6.85 (t, 1H, Jₒ= 7 Hz, H₅ Trp), 7.01 (m, 2H, H₂ and H₆ Trp), 7.08 (d, 2H, Jₒ= 7 Hz, H₂ and H₆ phenyl), 7.15 (d, 1H, Jₒ= 7 Hz, H₄ Trp), 7.21 (m, 3H, H₃, H₄ and H₅ phenyl), 7.27-7.36 (m, 2H, H₇ Trp and H₃ o-pyridyl), 7.58 (t, 1H, J= 6 Hz, H₅ o-pyridyl), 8.04 (t, 1H, Jₒ= 8 Hz, H₄ o-pyridyl), 8.62 (d, 1H, J_{αβ}= 5 Hz, H₆ o-pyridyl), 9.17 (d, 1H, J= 8 hz, NH amide), 10.81 (s, 1H, NH indole Trp).
¹³C NMR (75 MHz, DMSO d⁶, 300°K):
δ (ppm) 26.4 (CH₂-CH₂-phenyl), 29.2 (CH₂ βTrp), 32.4 (CH₂-CH₂-phenyl), 41.7 (CH₂-*o-*pyridyl), 45.3 (CH αTrp), 45.7 (CH₂-*p*-methoxybenzyl), 55.5 (OCH₃), 109.7 (C₃ Trp), 111.8 (C₇ Trp), 114.4 (C₃ and C₅ p-methoxybenzyl), 118.4 (C₄ Trp), 118.8 (C₅ Trp), 121.4 (C₆ Trp), 124.1 (C₃ o-pyridyl), 124.6 (C₂ Trp), 126.4 (C₅ o-pyridyl), 126.6 (C₂ and C₆ phenyl), 127.2 (C₉ Trp), 127.4 (C₁ *p*-methoxybenzyl), 127.9 (C₂ and C₆ p-methoxybenzyl), 128.7 (C₃, C₄ and C₅ phenyl), 136.4 (C₈ Trp), 140.5 (C₁ phenyl), 142.1 (C₄ o-pyridyl), 145.3 (C₆ o-pyridyl), 153.0 (C₂ o-pyridyl), 154.5 (Cq triazole), 155.3 (Cq triazole), 159.1 (C₄ p-methoxybenzyl), 167.9 (CO amide).

### (R)-N-(1-(4-(2,4-dimethoxybenzyl)-5-phenethyl-4H-1,2,4-triazol-3-yl)-2-(1H-indol-3-yl)ethyl)picolinamide (Compound 64):

¹H NMR (300 MHz, DMSO d⁶, 300°K):
δ (ppm) 2.83 (m, 2H, CH₂-CH₂-phenyl), 2.90 (m, 2H, CH₂-CH₂-phenyl), 3.48 (m, 2H, CH₂ βTrp), 3.57 (s, 3H, OCH₃), 3.61 (s, 3H, OCH₃), 4.97 (d, 1H, J= 17 Hz, CH₂-*o,p-*dimethoxybenzyl), 5.09 (d, 1H, J= 17 Hz, CH₂-o,p-dimethoxybenzyl), 5.56 (m, 1H, CH αTrp), 6.18 (dd, 1H, Jₒ= 8 Hz and Jₘ= 2 Hz, H₅ o,p-dimethoxybenzyl), 6.41 (d, 1H, Jₘ= 2 Hz, H₃ o,p-dimethoxybenzyl), 6.55 (d, 1H, Jₒ= 8 Hz, H₆ o,p-dimethoxybenzyl), 6.87 (t, 1H, Jₒ= 8 Hz, H₅ Trp), 7.01 (t, 1H, Jₒ= 8 Hz, H₆ Trp), 7.08 (m, 3H, H₂ Trp, H₂ and H₆ phenyl), 7.14 (d, 1H, Jₒ= 7 Hz, H₄ Trp), 7.19-7.31 (m, 4H, H₇ Trp, H₃, H₄ and H₅ phenyl), 7.56 (t, 1H, J= 8 Hz, NH amide), 7.91 (m, 2H, H₄ and H₅ o-pyridyl), 8.57 (d, 1H, J_{αβ}= 5 Hz, H₆ *o*-pyridyl), 9.16 (d, 1H, Jₒ= 8 Hz, H₃ *o*-pyridyl), 10.80 (s, 1H, NH indole Trp).
¹³C NMR (75 MHz, DMSO d⁶, 300°K):
δ (ppm) 26.2 (CH₂-CH₂-phenyl), 28.8 (CH₂ βTrp), 32.1 (CH₂-CH₂-phenyl), 43.0 (CH₂-*o,p*-dimethoxybenzyl), 45.5 (CH αTrp), 55.6 (OCH₃), 55.8 (OCH₃), 98.9 (C₃ *o,p-*dimethoxybenzyl), 105.0 (C₅ o,p-dimethoxybenzyl), 109.5 (C₃ Trp), 111.8 (C₇ Trp), 114.4 (C₁ o,p-dimethoxybenzyl), 118.4 (C₄ Trp), 118.8 (C₅ Trp), 121.4 (C₆ Trp), 122.4 (C₃ o-pyridyle), 124.4 (C₂ Trp), 126.7 (C₆ *o,p*-dimethoxybenzyl), 127.2 (C₅ o-pyridyle), 127.5 (C₉ Trp), 128.6-128.8 (C₂, C₃, C₄, C₅ and C₆ phenyl), 136.4 (C₈ Trp), 138.1 (C₄ *o-*pyridyle), 140.2 (C₁ phenyl), 148.8 (C₆ *o*-pyridyle), 149.3 (C₂ *o*-pyridyle), 155.2 (Cq triazole), 155.4 (Cq triazole), 157.9 (C₂ *o,p*-dimethoxybenzyl), 161.0 (C₄ o,p-dimethoxybenzyl), 163.9 (CO amide).

### (R)-N-(1-(4-(2,4-dimethoxybenzyl)-5-phenethyl-4H-1,2,4-triazol-3-yl)-2-(1H-indol-3-yl)ethyl)pyrazine-2-carboxamide (Compound 66):

¹H NMR (300 MHz, DMSO d⁶, 300°K):
δ (ppm) 2.87 (m, 4H, CH₂-CH₂-phenyl), 3.51 (m, 2H, CH₂ βTrp), 3.58 (s, 3H, OCH₃), 3.59 (s, 3H, OCH₃), 4.97 (d, 1 H, J= 17 Hz, CH₂-*o,p*-dimethoxybenzyl), 5.08 (d, 1 H, J= 17 Hz, CH₂-*o,p*-dimethoxybenzyl), 5.56 (s, 1H, CH αTrp), 6.10 (dd, 1H, Jₒ= 8 Hz and Jₘ= 2 Hz, H₅ *o,p*-dimethoxybenzyl), 6.36 (d, 1 H, Jm= 2 Hz, H₃ *o,p*-dimethoxybenzyl), 6.40 (d, 1 H, Jₒ= 8 Hz, H₆ *o,p*-dimethoxybenzyl), 6.87 (t, 1 H, Jₒ= 8 Hz, H₅ Trp), 7.00 (t, 1 H, Jₒ= 7 Hz, H₆ Trp), 7.09 (m, 3H, H₂ Trp, H₂ and H₆ phenyl), 7.15 (d, 1 H, Jₒ= 7 Hz, H₄ Trp), 7.19-7.28 (m, 3H, H₃, H₄ and H₅ phenyl), 7.36 (d, 1 H, Jₒ= 8 Hz, H₇ Trp), 8.61 (t, 1 H, J= 2 Hz, H₃ o-pyrazinyl), 8.78 (d, 1 H, J= 2 Hz, H₅ o-pyrazinyl), 8.94 (d, 1 H, J= 1 Hz, H₆ o-pyrazinyl), 9.26 (d, 1 H, J= 8 Hz, NH amide), 10.78 (s, 1 H, NH indole Trp).
¹³C NMR (75 MHz, DMSO d⁶, 300°K):
δ (ppm) 26.1 (CH₂-CH₂-phenyl), 28.4 (CH₂ βTrp), 32.1 (CH₂-CH₂-phenyl), 42.9 (CH₂-*o*,*p*-dimethoxybenzyl), 45.5 (CH αTrp), 55.5 (OCH₃), 55.8 (OCH₃), 98.7 (C₃ o,p-dimethoxybenzyl), 104.9 (C₅ *o*,*p*-dimethoxybenzyl), 109.7 (C₃ *o*,*p*-dimethoxybenzyl), 111.8 (C₇ Trp), 114.5 (C₁ o,p-dimethoxybenzyl), 118.5 (C₄ Trp), 118.8 (C₅ Trp), 121.4 (C₆ Trp), 124.4 (C₂ Trp), 126.7 (C₆ *o*,*p*-dimethoxybenzyl), 127.5 (C₉ Trp), 128.1 (C₄ phenyl), 128.7-128.8 (C₂, C₃, C₅ and C₆ phenyl), 136.4 (C₈ Trp), 140.3 (C₁ phenyl), 141.2 (C₆ o,p-dimethoxybenzyl), 144.2 (C₂ o-pyrazinyl), 144.3 (C₃ o-pyrazinyl), 146.8 (C₅ o-pyrazinyl), 155.2 (Cqs triazole), 157.7 (C₂ *o*,*p*-dimethoxybenzyl), 160.7 (CO amide), 162.9 (C₄ o,p-dimethoxybenzyl).

### (S)-N-((R)-1-(5-(2-(1H-indol-3-yl)ethyl)-4-(2,4-dimethoxybenzyl)-4H-1,2,4-triazol-3-yl)-2-(1H-indol-3-yl)ethyl)pyrrolidine-2-carboxamide (Compound 70):

¹H NMR (300 MHz, DMSO d⁶, 300°K):
δ (ppm) 1.40 (m, 1 H, H₃ Pro ), 1.53 (m, 1 H, H₄ Pro ), 1.71 (m, 1 H, H₄ Pro ), 2.09 (m, 1 H, H₃ Pro ), 2.90 (m, 4H, CH₂-CH₂-indole), 3.06 (t, 2H, J= 6 Hz, H₅ Pro ), 3.28 (m, 2H, CH₂ βTrp), 3.40(s, 3H, OCH₃), 3.80(s, 3H, OCH₃), 3.80(m, 1 H, CH αPro ), 4.80(d, 1 H, J= 17 Hz, CH₂-*o,p*-dimethoxybenzyl), 5.4 (d, 1H, J= 17 Hz, CH₂-*o,p-*dimethoxybenzyl), 5.1 (m, 1 H, CH αTrp), 6.26 (dd, 1 H, Jₒ= 8 Hz and Jₘ= 2 Hz, H₅ *o,p*-dimethoxybenzyl), 6.38 (d, 1 H, Jₒ= 8 Hz, H₆ *o,p*-dimethoxybenzyl), 6.53 (d, 1 H, Jₘ= 2 Hz, H₃ *o,p-*dimethoxybenzyl), 6.84 (t, 1 H, H₅ indole), 6.91 (t, 1 H, Jₒ= 8 Hz, H₅ Trp), 6.93-7.07 (m, 4H, H₂ and H₆ indole, H₂ and H₆ Trp), 7.16 (d, 1 H, Jₒ= 8 Hz, H₄ Trp), 7.29 (m, 3H, H₄ and H₇ indole, H₇ Trp), 8.39 and 9.10 (2 m, 2H, NH Pro TFA salt), 9.22 (d, 1 H, J= 8 Hz, NH amide), 10.76 (s, 1 H, NH indole), 10.80 (s, 1 H, NH indole Trp).
¹³C NMR (75 MHz, DMSO d⁶, 300°K):
δ (ppm) 22.9 (CH₂-CH₂-indole), 23.5 (C₄ Pro), 25.8 (CH₂-CH₂-indole), 29.6 (CH₂ βTrp), 29.8 (C₃ Pro), 41.9 (CH₂-*o,p*-dimethoxybenzyl), 45.2 (CH αTrp), 46.0 (C₅ Pro ), 55.7 (OCH₃), 55.9 (OCH₃), 59.3 (CH αPro), 99.0 (C₃ *o,p*-dimethoxybenzyl), 105.1 (C₅ *o,p-*dimethoxybenzyl), 109.7 (C₃ Trp), 111.8 (C₇ indole and C₇ Trp), 113.4 (C₃ indole), 115.6 (C₁ o,p-dimethoxybenzyl), 118.4 (C₄ indole and C₄ Trp), 118.7 (C₅ indole and C₅ Trp), 121.4 (C₆ indole and C₆ Trp), 123.0 (C₂ indole and C₂ Trp), 127.2 (C₉ indole), 127.3 (C₉ Trp), 128.1 (C₆ *o,p*-dimethoxybenzyl), 136.5 (C₈ Trp), 136.6 (C₈ indole), 155.0 (Cq triazole), 157.8 (C₂ *o,p-*dimethoxybenzyl), 160.9 (C₄ *o,p*-dimethoxybenzyl), 168.1 (CO amide).

### (R)-N-(1-(5-(2-(1H-indol-3-yl)ethyl)-4-(2,4-dimethoxybenzyl)-4H-1,2,4-triazol-3-yl)-2-(1H-indol-3-yl)ethyl)pyrazine-2-carboxamide (Compound 71):

¹H NMR (300 MHz, DMSO d⁶, 300°K):
δ (ppm) 3.01 (m, 2H, CH₂-CH₂-indole), 3.10 (m, 2H, CH₂-CH₂-indole), 3.51 (m, 2H, CH₂ βTrp), 3.55 (s, 3H, OCH₃), 3.57 (s, 3H, OCH₃), 5.15 (d, 2H, J= 7 Hz, CH₂-*o,p-*dimethoxybenzyl), 5.63 (m, 1 H, CH αTrp), 6.08 (dd, 1 H, Jₒ= 8 Hz and Jₘ= 2 Hz, H₅ *o,p-*dimethoxybenzyl), 6.35 (d, 1 H, Jₘ= 2 Hz, H₃ *o,p*-dimethoxybenzyl), 6.53 (d, 1 H, Jₒ= 8 Hz, H₆ *o,p*-dimethoxybenzyl), 6.89 (m, 2H, H₅ indole and H₅ Trp), 6.99 (m, 2H, H₆ indole and H₆ Trp), 7.08 (m, 2H, H₂ indole and H₂ Trp), 7.29 (m, 3H, H₄ Trp, H₄ and H₇ indole), 7.41 (d, 1 H, Jₒ= 8 Hz, H₇ Trp), 8.61 (t, 1 H, J= 2 Hz, H₃ o-pyrazine), 8.79 (d, 1 H, J= 2 Hz, H₅ o-pyrazine), 8.94 (d, 1 H, J= 1 Hz, H₆ o-pyrazine), 9.43 (d, 1 H, J= 8 Hz, NH amide), 10.84 (s, 2H, NH indole and NH indole Trp).
¹³C NMR (75 MHz, DMSO d⁶, 300°K):
δ (ppm) 22.0 (CH₂-CH₂-indole), 25.3 (CH₂-CH₂-indole), 27.9 (CH₂ βTrp), 44.1 (CH₂-o,p-dimethoxybenzyl), 45.5 (CH αTrp), 55.5 (OCH₃), 55.8 (OCH₃), 98.8 (C₃ o,p-dimethoxybenzyl), 104.9 (C₅ o,p-dimethoxybenzyl), 109.3 (C₃ Trp), 111.8 (C₇ indole and C₇ Trp), 112.1 (C₃ indole), 113.4 (C₁ o,p-dimethoxybenzyl), 118.4 (C₄ indole), 118.5 (C₄ Trp), 118.8 (C₅ indole and C₅ Trp), 121.5 (C₆ indole and C₆ Trp), 127.0 (C₉ indole), 127.4 (C₉ Trp), 136.4 (C₈ Trp), 136.6 (C₈ indole), 141.2 (C₆ o-pyrazine), 144.1 (C₂ o-pyrazine), 144.3 (C₃ o-pyrazine), 146.8 (C₅ o-pyrazine), 155.4 (Cq triazole), 156.0 (Cq triazole), 157.8 (C₂ o,p-dimethoxybenzyl), 161.0 (CO amide), 163.2 (C₄ o,p-dimethoxybenzyl).

### (R)-N-(1-(5-(2-(1H-indol-3-yl)ethyl)-4-(2,4-dimethoxybenzyl)-4H-1,2,4-triazol-3-yl)-2-(1H-indol-3-yl)ethyl)picolinamide (Compound 73):

¹H NMR (300 MHz, DMSO d⁶, 300°K):
δ (ppm) 2.94 (m, 4H, CH₂-CH₂-indole), 3.47 (m, 2H, CH₂ βTrp), 3.57 (s, 3H, OCH₃), 3.60 (s, 3H, OCH₃), 5.05 (m, 2H, CH₂-o,p-dimethoxybenzyl), 5.56 (m, 1H, CH αTrp), 6.14 (dd, 1 H, Jₒ= 8 Hz and Jₘ= 2 Hz, H₅ o,p-dimethoxybenzyl), 6.41 (d, 1 H, Jₘ= 2 Hz, H₃ o,p-dimethoxybenzyl), 6.52 (d, 1 H, Jₒ= 8 Hz, H₆ o,p-dimethoxybenzyl), 6.88 (t, 2H, Jₒ= 7 Hz, H₅ indole and H₅ Trp), 6.99 (t, 1 H, Jₒ= 8 Hz, H₆ Trp), 7.01 (t, 1 H, Jₒ= 8 Hz, H₆ indole), 7.04 (d, 1 H, J= 2 Hz, H₂ Trp), 7.07 (d, 1 H, J= 2 Hz, H₂ indole), 7.27-7.33 (m, 4H, H₄ and H₇ Trp, H₄ and H₇ indole), 7.55 (m, 1 H, NH amide), 7.90 (m, 2H, H₄ and H₅ o-pyridyl), 8.57 (d, 1H, J_{αβ}= 4 Hz, H₆ o-pyridyl), 9.15 (d, 1H, J= 8 Hz, H₃ o-pyridyl), 10.78 (brs, 2H NH indole and NH indole Trp).
¹³C NMR (75 MHz, DMSO d⁶, 300°K):
δ (ppm) 22.3 (CH₂-CH₂-indole), 25.6 (CH₂-CH₂-indole), 28.9 (CH₂ βTrp), 43.1 (CH₂-o,p-dimethoxybenzyl), 45.5 (CH αTrp), 55.5 (OCH₃), 55.8 (OCH₃), 98.9 (C₃ *o,p-*dimethoxybenzyl), 105.0 (C₅ *o,p*-dimethoxybenzyl), 109.5 (C₃ Trp), 111.8 (C₇ indole and C₇ Trp), 112.8 (C₃ indole), 114.4 (C₁ o,p-dimethoxybenzyl), 118.4 (C₄ indole and C₄ Trp), 118.7 (C₅ indole), 118.8 (C₅ Trp), 121.4 (C₆ indole and C₆ Trp), 122.5 (C₂ indole and C₃ o-pyridyl), 123.1 (C₂ Trp), 127.1 (C₅ o-pyridyl), 127.2 (C₉ indole), 127.5 (C₉ Trp), 128.6 (C₆ *o,p*-dimethoxybenzyl), 136.4 (C₈ Trp), 136.6 (C₈ indole), 139.1 (C₄ o-pyridyl), 146.6 (C₆ *o*-pyridyl), 150.6 (C₂ o-pyridyl), 155.5 (Cq triazole), 155.6 (Cq triazole), 157.8 (C₂ *o,p*-dimethoxybenzyl), 161.0 (C₄ *o,p*-dimethoxybenzyl), 163.9 (CO amide).

### (R)-1-(5-(2-(1H-indol-3-yl)ethyl)-4-(2,4-dimethoxybenzyl)-4H-1,2,4-triazol-3-yl)-2-(1H-indol-3-yl)ethanamine (Compound 74):

¹H NMR (300 MHz, DMSO d⁶, 300°K):
δ (ppm) 2.79 (m, 2H, CH₂-CH₂-indole), 2.86 (m, 2H, CH₂-CH₂-indole), 3.30 (dd, 1 H, ³J= 14 Hz and 5 Hz, CH₂ βTrp), 3.38 (dd, 1H, ³J= 14 Hz and 6 Hz, CH₂ βTrp), 3.62 (s, 3H, OCH₃), 3.63 (s, 3H, OCH₃), 4.47 (d, 1H, ³J= 17 Hz, CH₂-o,p-dimethoxybenzyl), 4.59 (d, 1 H, ³J= 17 Hz, CH₂-o,p-dimethoxybenzyl), 6.11 (dd, 1 H, Jₒ= 8 Hz and Jₘ= 2 Hz, H₅ *o,p-*dimethoxybenzyl), 6.20 (d, 1H, Jₒ= 8 Hz, H₆ o,p-dimethoxybenzyl), 6.45 (d, 1 H, Jₘ= 2 Hz, H₃ o,p-dimethoxybenzyl), 6.87 (t, 1 H, Jₒ= 8 Hz, H₅ Trp), 6.91 (t, 1 H, Jₒ= 8 Hz, H₅ indole), 7.00-7.04 (m, 2H, H₆ indole and H₆ Trp), 7.07 (s, 1 H, H₂ indole), 7.09 (s, 1 H, H₂ Trp), 7.17-7.35 (m, 4H, H₄ and H₇ indole, H₄ and H₇ Trp), 8.75 (brs, 3H, NH₂ TFA salt), 10.78 (s, 1 H, NH indole), 11.00 (s, 1 H, NH indole Trp).
¹³C NMR (75 MHz, DMSO d⁶, 300°K):
δ (ppm) 22.9 (CH₂-CH₂-indole), 25.7 (CH₂-CH₂-indole), 29.9 (CH₂ βTrp), 41.5 (CH₂-*o*,*p-*dimethoxybenzyl), 46.5 (CH αTrp), 55.6 (OCH₃), 55.9 (OCH₃), 98.8 (C₃ *o,p-*dimethoxybenzyl), 105.0 (C₅ *o*,*p*-dimethoxybenzyl), 107.4 (C₃ Trp), 111.8 (C₇ indole and C₇ Trp), 113.4 (C₃ indole), 115.1 (C₁ o,p-dimethoxybenzyl), 118.0 (C₄ indole), 118.4 (C₄ Trp), 118.6 (C₅ Trp), 119.0 (C₅ indole), 121.3 (C₆ Trp), 121.6 (C₆ indole), 122.9 (C₂ indole), 125.4 (C₂ Trp), 127.1 (C₉ indole and C₉ Trp), 128.5 (C₆ o,p-dimethoxybenzyl), 136.5 (C₈ Trp), 136.6 (C₈ indole), 152.3 (Cq triazole), 155.6 (Cq triazole), 157.6 (C₂ o,p-dimethoxybenzyl), 160.8 (C₄ *o*,*p*-dimethoxybenzyl).

### (R)-N-(1-(4-(4-methoxybenzyl)-5-phenethyl-4H-1,2,4-triazol-3-yl)-2-(1H-indol-3-yl)ethyl)picolinamide (Compound 79):

¹H NMR (300 MHz, DMSO d⁶, 300°K):
δ (ppm) 2.85 (m, 4H, CH₂-CH₂ -phenyl), 3.51 (m, 2H, CH₂ βTrp), 3.59 (s, 3H, OCH₃), 5.11 (d, 1 H, J= 17 Hz, CH₂-*p*-methoxybenzyl), 5.23 (d, 1 H, J= 17 Hz, CH₂- *p-*methoxybenzyl), 5.51 (m, 1 H, CH αTrp), 6.59 (d, 2H, Jₒ= 8 Hz, H₃ and H₅ *p-*methoxybenzyl), 6.73 (d, 2H, Jₒ= 8 Hz, H₂ and H₆ *p*-methoxybenzyl), 6.87 (t, 1 H, Jₒ= 8 Hz, H₅ Trp), 7.01 (t, 1 H, Jₒ= 8 Hz, H₆ Trp), 7.06 (m, 2H, H₂ and H₆ phenyl), 7.10 (d, 1 H, J= 2 Hz, H₂ Trp), 7.14 (d, 1 H, Jₒ= 7 Hz, H₄ Trp), 7.24 (m, 3H, H₃, H₄ and H₅ phenyl), 7.34 (d, 1 H, Jₒ= 8 Hz, H₇ Trp), 7.55 (m, 1 H, NH amide), 7.88 (m, 2H, H₄ and H₅ o-pyridyl), 8.56 (d, 1 H, J_{αβ}= 4 Hz, H6 o-pyridyl), 9.20 (d, 1 H, Jₒ= 8 Hz, H₃ *o*-pyridyl), 10.80 (s, 1 H, NH indole Trp).
¹³C NMR (75 MHz, DMSO d⁶, 300°K):
δ (ppm) 26.2 (CH₂-CH₂-phenyl), 28.6 (CH₂ βTrp), 32.1 (CH₂-CH₂-phenyl), 45.5 (CH αTrp), 46.2 (CH₂-*p*-methoxybenzyl), 55.4 (OCH₃), 109.6 (C₃ Trp), 111.8 (C₇ Trp), 114.3 (C₃ and C₅ *p*-methoxybenzyl), 118.5 (C₄ Trp), 118.8 (C₅ Trp), 121.4 (C₆ Trp), 122.5 (C₃ o-pyridyl), 124.5 (C₂ Trp), 127.2 (C₂ and C₈ phenyl), 127.4 (Cg Trp and C₁ p-methoxybenzyl), 127.8 (C₅ o-pyridyl), 128.7 (C₂ and C₆ p-methoxybenzyl), 128.8 (C₃, C₄ and C₅ phenyl), 136.4 (C₈ Trp), 138.1 (C₄ o-pyridyl), 140.3 (C₁ phenyl), 148.7 (C₆ *o-*pyridyl), 149.3 (C₂ o-pyridyl), 155.0 (Cq triazole), 155.3 (Cq triazole), 159.0 (C₄ p-methoxybenzyl), 164.1 (CO amide).

### (R)-N-(1-(5-(2-(1H-indol-3-yl)ethyl)-4-(4-methoxybenzyl)-4H-1,2,4-triazol-3-yl)-2-(1H-indol-3-yl)ethyl)picolinamide (Compound 80):

¹H NMR (300 MHz, DMSO d⁶, 300°K):
δ (ppm) 2.95 (m, 4H, CH₂-CH₂-indole), 3.48 (m, 2H, CH₂ βTrp), 3.58 (s, 3H, OCH₃), 5.16 (m, 2H, CH₂-*p*-methoxybenzyl), 5.50 (m, 1 H, CH αTrp), 6.57 (d, 2H, Jₒ= 8 Hz, H₃ and H₅ *p*-methoxybenzyl), 6.72 (d, 2H, Jₒ= 8 Hz, H₂ and H₆ *p*-methoxybenzyl), 6.87 (t, 2H, Jₒ= 8 Hz, H₅ Trp and H₅ indole), 6.96-7.07 (m, 5H, H₂ and H₆ indole, H₂, H₄ and H₆ Trp), 7.27-7.34 (m, 3H, H₄ and H₇ indole, H₇ Trp), 7.55 (m, 1 H, NH amide), 7.88 (m, 2H, H₄ and H₅ o-pyridyl), 8.56 (d, 1 H, J_{αβ}= 4 Hz, H₆ o-pyridyl), 9.18 (d, 1 H, Jₒ= 8 Hz, H₃ o-pyridyl), 10.77 (brs, 2H, NH indole Trp and NH indole).
¹³C NMR (75 MHz, DMSO d⁶, 300°K):
δ (ppm) 22.4 (CH₂-CH₂-indole), 25.7 (CH₂-CH₂-indole), 28.9 (CH₂ βTrp), 45.5 (CH αTrp), 46.2 (CH₂-p-methoxybenzyl), 55.4 (OCH₃), 109.7 (C₃ Trp), 111.8 (C₇ Trp and C₇ indole), 112.6 (C₃ indole), 114.3 (C₃ and C₅ p-methoxybenzyl), 118.4 (C₄ Trp and C₄ indole), 118.7 (C₅ indole), 118.8 (C₅ Trp), 121.4 (C₆ Trp and C₆ indole), 122.4 (C₃ *o-*pyridyl and C₂ indole), 124.5 (C₂ Trp), 126.7 (C₉ indole), 127.1 (C₉ Trp), 127.2 (C₅ *o-*pyridyl), 127.5 (C₁ p-methoxybenzyl), 127.7 (C₂ and C₆ p-methoxybenzyl), 136.4 (C₈ Trp), 136.6 (C₈ indole), 138.1 (C₄ o-pyridyl), 148.7 (C₆ o-pyridyl), 149.3 (C₂ o-pyridyl), 155.3 (Cq triazole), 159.0 (C₄ p-methoxybenzyl), 164.0 (CO amide).

### (R)-N-1-(5-(2-(1H-indol-3-yl)ethyl)-4-(4-methoxybenzyl)-4H-1,2,4-triazol-3-yl)-2-(1H-indol-3-yl)ethyl)piperazine-2-carboxamide (Compound 81):

¹H NMR (300 MHz, DMSO d⁶, 300 °K):
δ (ppm) 2.18 (m, 2H, NH piperazine), 2.96 (m, 6H, H₂, H₅ and H₆ piperazine), 3.34 (d, 2H, J= 7 Hz, CH₂ βTrp), 3.57 (m, 4H, CH₂-CH -indole), 3.61 (s, 3H, OMe), 3.64 (m, 1 H, H₃ piperazine), 4.82 (m, 2H, CH₂-p-methoxybenzyl), 5.40 (m, 1 H, CH αTrp), 6.45 (d, 2H, Jₒ= 8 Hz, H₃ and H₅ p-methoxybenzyl), 6.51 (d, 2H, Jₒ= 8 Hz, H₂ and H₆ p-methoxybenzyl), 6.65-7.47 (m, 10H, CHar, indole and indole Trp), 8.95 (m, 1 H, NH amide), 10.88 (d, 1 H, J= 2 Hz, NH indole), 10.91 (s, 1 H, NH indole Trp).
¹³C NMR (75 MHz, DMSO d⁶, 300°K):
δ (ppm) 22.5 (CH₂-CH₂-indole), 25.6 (CH₂-CH₂-indole), 31.3 (CH₂ βTrp), 41.9 (CH₂-*p-*methoxybenzyl), 47.7 (CH αTrp, C₅ and C₆ piperazine), 55.5 (OCH₃ and C₂ piperazine), 61.1 (C₃ piperazine), 109.3 (C₃ Trp), 111.7 (C₇ indole and C₇ Trp), 114.0 (C₃ indole), 114.3 (C₃ and C₅ *p*-methoxybenzyl), 118.6 (C₄ indole), 118.7 (C₄ Trp), 118.9 (C₅ indole and C₅ Trp), 121.4 (C₆ indole), 121.5 (C₆ Trp), 123.9 (C₂ indole and C₂ Trp), 127.0 (C₉ indole), 127.2 (C₉ Trp), 127.7 (C₁ *p*-methoxybenzyl), 128.1 (C₂ and C₆ p-methoxybenzyl), 136.3 (C₈ Trp), 136.5 (C₈ indole), 155.5 (Cq triazole), 162.2 (C₄ *p-*methoxybenzyl), 171.1 (CO amide).

### (S)-N-((R)-1-(5-(2-(1H-indol-3-yl)ethyl)-4-(4-methoxybenzyl)-4H-1,2,4-triazol-3-yl)-2-(1H-indol-3-yl)ethyl)pyrrolidine-2-carboxamide (Compound 89):

¹H NMR (300 MHz, DMSO d⁶, 300°K):
δ (ppm) 1.42 (m, 1 H, H₃ Pro ), 1.52 (m, 1 H, H₄ Pro), 1.72 (m, 1 H, H₄ Pro), 2.07 (m, 1 H, H₃ Pro), 2.94 (m, 4H, CH₂-CH₂-indole), 3.05 (t, 2H, J= 6 Hz, H₅ Pro), 3.30 (m, 2H, CH₂ βTrp), 3.67 (s, 3H, OCH₃), 3.96 (m, 1H, CH αPro ), 5.02 (s, 2H, CH₂-p-methoxybenzyl), 5.19 (m, 1H, CH αTrp), 6.73 (s, 4H, CHar p-methoxybenzyl), 6.84 (t, 1 H, Jₒ= 8 Hz, H₅ indole), 6.90 (t, 1 H, Jₒ= 8 Hz, H₅ Trp), 6.93-7.06 (m, 4H, H₂ and H₆ indole, H₂ and H₆ Trp), 7.17 (d, 1 H, Jₒ= 8 Hz, H₄ Trp), 7.29 (d, 3H, Jₒ= 8 Hz, H₄ and H₇ indole, H₇ Trp), 8.39 and 9.10 (2 m, 2H, NH Pro TFA salt), 9.25 (d, 1 H, J₌ 8 Hz, NH amide), 10.76 (s, 1 H, NH indole), 10.80 (d, 1 H, J= 2 Hz, NH indole Trp).
¹³C NMR (75 MHz, DMSO d⁶, 300°K):
δ (ppm) 22.8 (CH₂-CH₂-indole), 23.5 (C₄ Pro), 25.8 (CH₂-CH₂-indole), 29.4 (CH₂ βTrp), 29.8 (C₃ Pro), 45.2 (CH αTrp), 45.5 (CH₂-*p*-methoxybenzyl), 46.0 (C₅ Pro), 55.5 (OCH₃), 59.3 (CH αPro), 109.6 (C₃ Trp), 111.8 (C₇ indole and C₇ Trp), 113.4 (C₃ indole), 114.6 (C₃ and C₅ p-methoxybenzyl), 118.4 (C₄ indole), 118.5 (C₄ Trp), 118.7 (C₅ indole and C₅ Trp), 121.4 (C₆ indole and C₆ Trp), 123.0 (C₂ Trp), 124.7 (C₂ indole), 127.2 (C₉ indole), 127.3 (C₉ Trp), 127.7 (C₁ p-methoxybenzyl), 127.8 (C₂ and C₆ p-methoxybenzyl), 126.5 (C₈ Trp), 136.6 (C₈ indole), 154.8 (Cq triazole), 154.9 (Cq triazole), 159.2 (C₄ p-methoxybenzyl), 168.2 (CO amide).

### (R)-N-((R)-1-(5-(2-(1H-indol-3-yl)ethyl)-4-(4-methoxybenzyl)-4H-1,2,4-triazol-3-yl)-2-(1H-indol-3-yl)ethyl)pyrrolidine-2-carboxamide (Compound 90):

¹H NMR (300 MHz, DMSO d⁶, 300°K):
δ (ppm) 1.23 (m,1 H, H₃ Pro), 1.52 (m, 1 H, H₄ Pro ), 1.74 (m, 1 H, H₄ Pro ), 2.08 (m, 1 H, H₃ Pro ), 2.81 (m, 2H, H₅ Pro ), 2.90-3,14 (m, 4H, CH₂-CH₂-indole), 3.31 (dd, 1H, J= 14 Hz and 7 Hz, CH₂ βTrp), 3.41 (dd, 1H, J= 14 Hz and 8 Hz, CH₂ βTrp), 3.63 (s, 3H, OCH₃), 4.05 (m, 1 H, CH αPro ), 4.86 (s, 2H, CH₂-*p*-methoxybenzyl), 5.21 (m, 1 H, CH αTrp), 6.63 (s, 4H, CHar *p*-methoxybenzyl), 6.88 (t, 2H, Jₒ= 7 Hz, H₅ indole and H₅ Trp), 7.02 (m, 4H, H₂ and H₆ indole, H₂ and H₆ Trp), 7.26-7.34 (m, 4H, H₄ and H₇ indole, H₄ and H₇ Trp), 8.51 and 9.18 (2 m, 2H, NH Pro, TFA salt), 9.27 (d, 1 H, J= 8 Hz, NH amide), 10.73 (s, 1 H, NH indole), 10.80 (s, 1 H, NH indole Trp).
¹³C NMR (75 MHz, DMSO d⁶, 300°K):
δ (ppm) 22.8 (CH₂-CH₂-indole), 23.8 (C₄ Pro ), 25.9 (CH₂-CH₂-indole), 29.7 (CH₂ f3Trp and C₃ Pro ), 45.4 (CH₂-p-methoxybenzyl), 45.8 (CH αTrp), 46.1 (C₅ Pro ), 55.5 (OCH₃), 59.2 (CH αPro), 109.7 (C₃ Trp), 111.8 (C₇ Trp), 111.9 (C₇ indole), 113.4 (C₃ indole), 114.4 (C₃ and C₅ p-methoxybenzyl), 118.3 (C₄ indole), 118.5 (C₄ Trp), 118.6 (C₅ indole), 118.9 (C₅ Trp), 121.4 (C₆ indole and C₆ Trp), 122.9 (C₂ indole and C₂ Trp), 127.2 (C₉ indole), 127.4 (C₉ Trp), 127.6 (C₁, C₂ and C₆ p-methoxybenzyl), 136.5 (C₈ Trp), 136.6 (C₈ indole), 154.4 (Cq triazole), 155.0 (Cq triazole), 159.1 (C₄ *p-*methoxybenzyl), 168.3 (CO amide).

### (R)-N-(1-(5-(2-(1H-indol-3-yl)ethyl)-4-(4-bromobenzyl)-4H-1,2,4-triazol-3-yl)-2-(1H-indol-3-yl)ethyl)-2-amino-2-methylpropanamide (Compound 92):

¹H NMR (400 MHz, DMSO-d⁶):
δ (ppm) 1.28 (3H, s, CH₃ Aib), 1.30 (3H, s, CH₃ Aib), 2.90 (2H, m, CH₂-CH₂-indole), 3.00 (2H, m, CH₂-CH₂-indole), 3.37 (2H, m, CH₂ βTrp), 5.10 (2H, s, CH₂ 4-bromobenzyl), 5.13 (1 H, m, CαH Trp), 6.75 (2H, d, J=8.1, H₂, H₆ 4-bromobenzyl), 6.88 (1 H, t, J=7.3, H₅ Trp), 6.93 (1 H, t, J=7.5, H₅ indole), 7.03 (1 H, t, J=7.0, H₆ Trp), 7.05 (1 H, H₆ indole), 7.07 (1 H, d, J=1.7, H₂ indole), 7.09 (1 H, d, J=1.8, H₂ Trp), 7.12 (1 H, d, J=8.2, H₄ Trp), 7.28 (1 H, d, J=7.9, H₄ indole), 7.32 (2H, d, J=8.2, H₇ Trp, H₇ indole), 7.41 (2H, d, J=8.1, H₃, H₅ 4-bromobenzyl), 8.01 (2H, s, NH₂ Aib), 8.95 (1 H, d, J=7.9, NH Trp), 10.77 (1 H, brs, NH indole), 10.80 (1 H, brs, NH indole Trp).
¹³C NMR (400 MHz,DMSO-d⁶):
6(pprq) 22.4 (CH₂-CH_{□}indole), 23.1 (CH₃ Aib), 23.4 (CH₃ Aib), 25.4 (CH₂-CH_{□}indole), 28.7 (Cβ Trp), 44.8 (CH₂ 4-bromobenzyl), 45.2 (Cα Trp,), 56.3 (Cq Aib), 109.4 (C₃ Trp), 111.3 (C₇ Trp, C₇ indole), 113.0 (C₃ indole), 117.8 (C₄ Trp), 118.0 (C₄ indole), 118.2 (C₅ indole), 118.3 (C₅ Trp), 120.8 (C₄ 4-bromobenzyl), 120.9 (C₆ Trp, C₆ indole), 122.5 (C₂ indole), 124.4 (C₂ Trp), 126.7 (C₉ Indole), 126.8 (C₉ Trp), 128.0 (C₂, C₆ 4-bromobenzyl), 131.6 (C₃, C₅ 4-bromobenzyl), 135.1 (C₁ 4-bromobenzyl), 136.1 (C₈Trp, C₈ indole), 154.2 (Cq triazole), 154.5 (Cq triazole), 171.4 (CO Aib).

### (R)-N-(1-(5-(2-(1H-indol-3-yl)ethyl)-4-(4-methoxybenzyl)-4H-1,2,4-triazol-3-yl)-2-phenylethyl)-2-amino-2-methylpropanamide (Compound 93):

¹H NMR (300 MHz, DMSO-d⁶, 300°K):
δ (ppm) 1.18 (3H, s, CH₃ Aib), 1.27 (3H, s, CH₃ Aib), 2.91 (4H, m, CH₂-CH₂-indole), 3.19 (2H, m, CH₂ βPhe), 3.69 (3H, s, OCH₃), 5.05 (2H, m, CH₂-*p*-methoxybenzyl), 5.20 (1 H, m, CH αPhe), 6.82 (2H, d, Jₒ= 8 Hz, H₃ and H₅ *p*-methoxybenzyl), 6.88 (2H, d, Jₒ= 8 Hz, H₂ and H₆ p-methoxybenzyl), 6.92 (1 H, t, Jₒ= 8 Hz, H₅ indole), 7.02 (1 H, t, Jₒ= 7 Hz, H₆ indole), 7.03 (1 H, d, J= 2 Hz, H₂ indole), 7.11-7.20 (5H, m, CHar Phe), 7.29 (2H, d, Jₒ= 8 Hz, H₄ and H₇ indole), 7.99 (3H, brs, NH₂ Aib), 8.93 (1 H, d, J= 8 Hz, NH amide), 10.77 (1 H, s, NH indole).
¹³C NMR (75 MHz, DMSO-d⁶, 300°K):
δ (ppm) 22.8 (CH₂-CH₂-indole), 23.5 (CH₃ Aib), 23.9 (CH₃ Aib), 25.9 (CH₂-CH₂-indole), 38.7 (CH₂ βPhe), 45.7 (CH₂-p-methoxybenzyl), 46.4 (CH αPhe), 55.6 (OCH₃), 56.8 (Cq Aib), 111.8 (C₇ indole), 113.4 (C₃ indole), 114.7 (C₃ and C₅ p-methoxybenzyl), 118.5 (C₄ Trp), 118.6 (C₅ Trp), 121.4 (C₆ Trp), 123.0 (C₂ Trp), 127.0 (C₄ phenyl), 127.2 (C₉ indole), 127.9 (C₁ *p-*methoxybenzyl), 128.1 (C₂ and C₆ phenyl), 128.5 (C₃ and C₅ phenyl), 129.8 (C₂ and C₆ *p*-methoxybenzyl), 136.6 (C₈ indole), 137.7 (C₁ phenyl), 155.2 (Cq triazole), 154.4 (Cq triazole), 159.3 (C₄ *p-* methoxybenzyl), 171.7 (CO Aib).

### (R)-N-(1-(4-(2-(1H-indol-3-yl)ethyl)-4H-1,2,4-triazol-3-yl)-2-(1H-indol-3-yl)ethyl)-2-amino-2-methylpropanamide (Compound 95):

¹H NMR (400 MHz, DMSO-d⁶):
δ (ppm) 1.29 (3H, s, CH₃ Aib), 1.35 (3H, s, CH₃ Aib), 2.85 (1 H, m, 1 H N-CH₂-CH₂-In), 2.89 (1 H, m, 1 H, -N-CH₂-C*H*₂-ln), 3.28 (1 H, dd, J=14.2, J=6.8, 1 H CH₂β Trp), 3.40 (1 H, dd, J=14.2, J=8.4, 1 H CH₂β Trp), 4.10 (2H, m,-N-CH₂-CH₂-In), 5.25 (1 H, m, CHα Trp), 6.85 (1 H, d, J=2.0, H₂ indole), 6.90-6.98 (2H, m, H₅ indole), 7.01 (1 H, d, J=2.0, H₂ indole), 7.02-7.12 (2H, m, H₆ indole), 7.30 (1 H, d, J=8.2, H₇ indole), 7.33 (1 H, d, J=8.3, H₇ indole), 7.40 (1 H, d, J=7.9, H₄ indole), 7.47 (1 H, d, J=7.8, H₄ indole), 8.04 (2H, brs, NH₂ Aib), 8.42 (1 H, s, H triazole), 9.01 (1 H, d, J=8.0, NH Trp), 10.81 (1 H, s, NH indole), 10.90 (1 H, s, NH indole).

### (R)-N-(1-(5-((1H-indol-3-yl)methyl)-4-methyl-4H-1,2,4-triazol-3-yl)-2-(1H-indol-3-yl)ethyl)-2-amino-2-methylpropanamide (Compound 96):

¹H NMR (400 MHz, DMSO-d⁶):
δ (ppm) 1.20 (3H, s, CH₃ Aib), 1.28 (3H, s, CH₃ Aib), 3.32 (3H, d, N-CH₃), 3.30-3.45 (2H, m, CH₂β Trp), 4.22 (2H, s, -CH₂-In), 5.30 (1 H, m, CHα Trp), 6.91 (1 H, t, J=7.5, H₅ indole), 6.94 (1 H, d, J=7.5, H₅ indole), 7.02 (1 H, t, J=7.9, H₆ indole), 7.05 (1 H, t, J=7.9, H₆ indole), 7.08 (1 H, d, J=1.9, H₂ indole), 7.12 (1 H, d, J=1.9, H₂ indole), 7.29 (1 H, d, J=8.1, H₇ indole), 7.33 (1 H, d, J=8.2, H₇ indole), 7.48 (1 H, d, J=7.9, H₄ indole), 7.57 (1 H, d, J=7.9 H₄ indole), 8.00 (2H, brs, NH₂ Aib), 8.85 (1 H, d, J=8.2, NH Trp), 10.82 (1 H, s, NH indole), 10.98 (1 H, s, NH indole).

### (R)-N-(1-(5-(2-(1H-indol-3-yl)ethyl)-4-methyl-4H-1,2,4-triazol-3-yl)-2-(1H-indol-3-yl)ethyl)-2-amino-2-methylpropanamide (Compound 97):

¹H NMR (400 MHz, DMSO-d⁶):
δ (ppm) 1.30 (3H, s, CH₃ Aib), 1.40 (3H, s, CH₃ Aib), 3.00-3.20 (4H, m, -CH₂-CH₂-ln), 3.36 (3H, s, N-CH₃), 3.45-3.50 (2H, m, CH₂β Trp), 5.30 (1 H, m, CHα Trp), 6.95-7.04 (2H, t, H₅ indole), 7.06-7.13 (2H, m, H₆ indole), 7.18 (2H, brs, H₂ indole), 7.34 (1 H, d, J=8.0, H₇ indole), 7.36 (1 H, d, J=8.0, H₇ indole), 7.48 (1 H, d, J=7.8, H₄ indole), 7.58 (1 H, d, J=7.8, H₄ indole), 8.10 (2H, brs, NH₂ Aib), 8.95 (1 H, d, J=8.1, NH Trp), 10.95 (1 H, s, NH indole), 10.96 (1 H, s, NH indole).
¹³C NMR (100 MHz,DMSO-d⁶):
δ (ppm) 22.9 - 25.9 (-CH₂-CH₂-indole), 24.1 (CH₃ Aib), 24.3 (CH₃ Aib), 28.8 (CH₂β Trp), 30.7 (-NCH₃), 46.2 (CHα Trp), 57.2 (Cq Aib), 110.2 (C₃ indole), 112.3 (2C₇ indole), 113.5 (C₃ indole), 118.9-119.2 (2C₅, 2C₄ indole), 121.9 (2C₆ indole), 123.6 (C₂ indole), 125.3 (C₂ indole), 127.7 (C₉ indole), 128.0 (C₉ indole), 136.9 (C₈ indole), 137.1 (C₈ indole), 155.3 (Cq triazole), 155.8 (Cq triazole), 172.2 (CO Aib).

### (R)-N-(1-(5-((1H-indol-3-yl)methyl)-4H-1,2,4-triazol-3-yl)-2-(1H-indol-3-yl)ethyl)-2-amino-2-methylpropanamide (Compound 98):

¹H NMR (400 MHz, DMSO-d⁶):
δ (ppm) 1.31 (3H, s, CH₃ Aib), 1.42 (3H, s, CH₃ Aib), 3.19 (1 H, dd, J=14.5, J= 9.6, 1 H CH₂ βTrp), 3.35 (1 H, dd, J=14.5, J= 5.3, 1 H CH₂ βTrp), 4.15 (2H, s, CH₂ indole), 5.26 (1 H, m, CαH Trp), 6.95 (1 H, t, H₅ Trp), 6.96 (1 H, t, H₅ indole), 7.05 (1 H, t, H₆ Trp), 7.06 (1 H, s, H₂ Trp), 7.07 (1 H, t, H₆ indole), 7.21 (1 H, s, H₂ indole), 7.32 (1 H, d, H₇ Trp), 7.37 (1 H, d, H₇ indole), 7.51 (1 H, d, J=7.8, H₄ indole), 7.58 (1 H, d, J=7.8, H₄ Trp), 8.00 (2H, s, NH₂ Aib), 8.64 (1 H, d, J=8.7, NH Trp), 10.77 (1 H, s, NH indole Trp), 10.92 (1 H, s, NH indole).
¹³C NMR (400 MHz,DMSO-d⁶):
δ (ppm) 22.9 (CH₂ indole), 23.2 (CH₃ Aib), 23.3 (CH₃ Aib), 29.4 (Cβ Trp), 48.3 (Cα Trp), 56.3 (Cq Aib), 109.7 (C₃ indole), 110.3 (C₃ Trp), 111.2 (C₇ Trp), 111.3 (C₇ indole), 118.1 (C₄ Trp, C₅ Trp), 118.3 (C₄ indole), 118.4 (C₅ indole), 120.7 (C₆ Trp), 121.0 (C₆ indole), 123.4 (C₂ indole), 123.6 (C₂ Trp), 126.8 (C₉ indole), 127.1 (C₉ Trp), 136.0 (C₈ Trp), 136.2 (C₈ indole), 157.5 (Cq triazole), 161.7 (Cq triazole), 170.8 (CO Aib).

### (R)-N-(1-(5-((1H-indol-3-yl)methyl)-4-(2,4-dimethoxybenzyl)-4H-1,2,4-triazol-3-yl)-2-(1H-indol-3-yl)ethyl)-2-amino-2-methylpropanamide (Compound 99):

¹H NMR (400 MHz, DMSO-d⁶):
δ (ppm) 1.27 (3H, s, CH₃ Aib), 1.29 (3H, s, CH₃ Aib), 3.25 (1 H, dd, J=14.3, J= 5.6, 1H CH₂ βTrp), 3.38 (1 H, dd, J=14.3, J= 9.1, 1 H CH₂ βTrp), 3.68 (3H, s, OCH₃), 3.72 (3H, s, OCH₃), 4.10 (1 H, d, J=16.5, 1 H CH₂ indole), 4.16 (1 H, d, J=16.5, 1 H CH₂ indole), 4.96 (1 H, d, J=16.8, 1 H CH₂ o,p-dimethoxybenzyl), 5.12 (1 H, d, J=16.8, 1 H CH₂ *o,p-*dimethoxybenzyl), 5.16 (1 H, m, CαH Trp), 6.21 (1 H, dd, J=8.5, J=2.1, H₅ o,p-dimethoxybenzyl), 6.27 (1 H, d, J=8.5, H₆ o,p-dimethoxybenzyl), 6.57 (1 H, d, J=2.1, H₃ o,p-dimethoxybenzyl), 6.83 (1 H, t, H₅ Trp), 6.94 (1 H, t, H₅ indole), 7.02 (1 H, t, H₆ Trp), 7.05 (1 H, t, H₂ indole),7.06 (1 H, t, H₆ indole), 7.07 (1 H, s, H₂ Trp), 7.07 (1 H, t, H₄ Trp), 7.31 (1 H, d, H₇ Trp), 7.33 (1 H, d, H₇ indole), 7.36 (1 H, d, J=7.8, H₄ indole), 8.00 (2H, br s, NH₂ Aib), 8.92 (1 H, d, J=8.2, NH Trp), 10.79 (1 H, s, NH indole Trp), 10.89 (1 H, s, NH indole).
¹³C NMR (400 MHz,DMSO-d⁶):
δ (ppm) 21.2 (CH₂indole), 23.1 (CH₃ Aib), 23.2 (CH₃ Aib), 28.6 (Cβ Trp), 41.4 (N-CH₂ o,p-dimethoxybenzyl), 45.1 (Cα Trp), 55.2 (OCH₃), 55.4 (OCH₃), 56.2 (Cq Aib), 98.5 (C₃ o,p-dimethoxybenzyl), 104.6 (C₅ o,p-dimethoxybenzyl), 107.9 (C₃ indole), 109.5 (C₃ Trp), 111.2 (C₇ Trp), 111.3 (C₇ indole), 115.1 (C₁ o,p-dimethoxybenzyl), 117.8 (C₄ Trp), 118.1 (C₅ Trp), 118.3 (C₄ indole), 118.4 (C₅ indole), 120.8 (C₆ Trp), 121.1 (C₆ indole), 123.5 (C₂ indole), 124.3 (C₂ Trp), 126.6 (C₉ indole), 126.8 (C₉ Trp), 127.2 (C₆ o,p-dimethoxybenzyl), 136.0 (C₈ Trp), 136.2 (C₈ indole), 157.5 (Cq triazole), 154.9 (Cq triazole), 157.2 (C₂ o,p-dimethoxybenzyl), 160.3 (C₄ o,p-dimethoxybenzyl), 171.2 (CO Aib).

### (R)-N(1-(5-((1H-indol-3-yl)methyl)-4-(4-methoxybenzyl)-4H-1,2,4-triazol-3-yl)-2-(1H-indol-3-yl)ethyl)-2-amino-2-methylpropanamide (Compound 101):

¹H NMR (300 MHz, DMSO-d⁶, 300°K):
δ (ppm) 1.22 (3H, s, CH₃ Aib), 1.25 (3H, s, CH₃ Aib), 3.22 (1 H, dd, J= 14 Hz and 6 Hz, CH₂ βTrp), 3.34 (1 H, dd, J= 14 Hz and 9 Hz, CH₂ βTrp), 3.68 (3H, s, OCH₃), 4.11 (2H, m, CH₂-indole), 5.09 (3H, m, CH αTrp and CH₂-*p*-methoxybenzyl), 6.70 (4H, s, CHar p-methoxybenzyl), 6.78 (2H, m, H₅ indole and H₅ Trp), 6.93 (2H, m, H₆ indole and H₆ Trp), 7.01-7,06 (3H, m, H₂ indole, H₂ and H₄ Trp), 7.31 (3H, m, H₄ and H₇ indole, H₇ Trp), 7.98 (3H, brs, NH₂ Aib), 8.92 (1 H, d, J= 8 Hz, NH amide), 10.77 (1 H, s, NH indole), 10.89 (1 H, s, NH indole Trp).
¹³C NMR (75 MHz, DMSO-d⁶, 300°K):
δ (ppm) 21.7 (CH₂-indole), 23.5 (CH₃ Aib), 23.7 (CH₃ Aib), 28.9 (CH₂ βTrp), 45.6 (CH αTrp), 45.8 (CH₂- p-methoxybenzyl), 55.5 (OCH₃), 56.7 (Cq Aib), 108.1 (C₃ indole), 109.7 (C₃ Trp), 111.7 (C₇ Trp), 111.9 (C₇ indole), 114.5 (C₃ and C₅ p-methoxybenzyl), 118.3 (C₄ Trp), 118.7 (C₄ indole), 118.8 (C₅ indole), 118.9 (C₅ Trp), 121.3 (C₆ indole), 121.6 (C₆ Trp), 124.2 (C₂ indole), 125.3 (C₂ Trp), 127.1 (C₉ indole), 127.2 (C₉ Trp), 127.6 (C₁ p-methoxybenzyl), 127.8 (C₂ and C₆ p-methoxybenzyl), 136.4 (C₈ Trp), 136.7 (C₈ indole), 154.2 (Cq triazole), 155.2 (Cq triazole), 159.2 (C₄ p-methoxybenzyl), 171.9 (CO Aib).

### (R)-N-(1-(4-(2,4-dimethoxybenzyl)-5-benzyl-4H-1,2,4-triazol-3-yl)-2-(1H-indol-3-yl)ethyl)-2-amino-2-methylpropanamide (Compound 102):

¹H NMR (400 MHz, DMSO-d⁶):
δ (ppm) 1.30 (3H, s, CH₃ Aib), 1.33 (3H, s, CH₃ Aib), 3.26 (1 H, dd, J=14.2, J=5.9, 1 H CH₂ βTrp), 3.38 (1 H, dd, J=14.2, J=8.7, 1 H CH₂ βTrp), 3.69 (3H, s, OCH₃), 3.72 (3H, s, OCH₃), 4.02 (2H, s, CH₂ benzyl), 4.87 (1 H, d, J=16.7, 1 H CH₂ *o,p*-dimethoxybenzyl), 5.08 (1 H, d, J=16.7, 1 H CH₂ *o,p*-dimethoxybenzyl), 5.17 (1 H, m, CαH Trp), 6.24 (1 H, dd, J=8.4, J=1.7, H₅ *o,p*-dimethoxybenzyl), 6.28 (1 H, d, J=8.4, H₆ o,p-dimethoxybenzyl), 6.56 (1 H, d, J=1.7, H₃ *o,p*-dimethoxybenzyl), 6.85 (1 H, t, J=7.5, H₅ Trp), 7.02 (1 H, t, H₆ Trp), 7.07 (2H, m, H₂, H₆ benzyl), 7.08 (1 H, s, H₂ Trp), 7.09 (1 H, d, H₄ Trp), 7.16-7.29 (3H, m, H₃, H₄, H₅ benzyl), 7.31 (1 H, d, J=8.2, H₇ Trp), 8.01 (2H, s, NH₂ Aib), 8.92 (1 H, d, J=7.9, NH Trp), 11.79 (1 H, s, NH indole Trp).
¹³C NMR (400 MHz,DMSO-d⁶):
δ (ppm) 23.2 (2CH₃ Aib), 28.7 (Cβ Trp), 30.2 (CH₂-benzyl), 41.3 (CH₂- *o,p-*dimethoxybenzyl), 45.2 (Cα Trp), 55.2 (OCH₃), 55.4 (OCH₃), 56.2 (Cq Aib), 98.5 (C₃ *o,p*-dimethoxybenzyl), 104.7 (C₅ *o,p*-dimethoxybenzyl), 109.5 (C₃ Trp), 111.3 (C₇ Trp), 115.1 (C₁ o,p-dimethoxybenzyl), 117.8 (C₄ Trp), 118.2 (C₅ Trp), 120.8 (C₆ Trp), 124.3 (C₂ Trp), 126.5 (C₂, C₆ benzyl), 126.8 (C₉ Trp), 127.3 (C₆ *o,p*-dimethoxybenzyl), 128.3 (C₃, C₄, C₅ Benzyl), 135.8 (C₁ benzyl), 136.0 (C₈ Trp), 153.4 (Cq triazole), 155.0 (Cq triazole), 157.2 (C₂ *o,p*-dimethoxybenzyl), 160.3 (C₄ *o,p*-dimethoxybenzyl), 171.3 (CO Aib).

### (R)-N-(1-(5-((1H-indol-3-yl)methyl)-4-phenethyl-4H-1,2,4-triazol-3-yl)-2-(1H-indol-3-yl)ethyl)-2-amino-2-methylpropanamide (Compound 104):

¹H NMR (300 MHz, DMSO-d⁶, 300°K):
δ (ppm) 1.27 (3H, s, CH₃ Aib), 1.29 (3H, s, CH₃ Aib), 2.39-2.53 (4H, m, CH₂-CH₂-phenyl), 3.74 (1 H, m, CH₂ βTrp), 3.92 (1 H, m, CH₂ βTrp), 3.99 (2H, s, CH₂-indole), 5.21 (1 H, m, CH αTrp), 6.74 (2H, m, H₅ indole and H₅ Trp), 6.90 (1 H, t, Jₒ= 8 Hz, H₆ Trp), 6.92 (1 H, t, Jₒ= 8 Hz, H₆ indole), 7.01-7.06 (4H, m, H₂ and H₆ phenyl, H₂ indole and H₂ Trp), 7.16 (3H, m, H₃, H₄ and H₅ phenyl), 7.27 (1 H, d, Jₒ= 8 Hz, H₄ Trp), 7.32 (1 H, d, Jₒ= 8 Hz, H₇ Trp), 7.36 (1 H, d, Jₒ= 8 Hz, H₇ indole), 7.50 (1 H, d, Jₒ= 8 Hz, H₄ indole), 7.99 (3H, brs, NH₂ Aib), 9.02 (1 H, s, J= 8 Hz, NH amide), 10.79 (1 H, s, NH indole Trp), 10.94 (1 H, s, NH indole).
¹³C NMR (75 MHz, DMSO-d⁶, 300°K):
δ (ppm) 21.4 (CH₂-indole), 23.5 (CH₃ Aib), 23.8 (CH₃ Aib), 29.5 (CH₂ βTrp), 35.8 (CH₂-CH₂-phenyl), 44.5 (CH₂-CH₂-phenyl), 45.8 (CH αTrp), 56.7 (Cq Aib), 108.5 (C₃ indole), 109.9 (C₃ Trp), 114.0 (C₇ indole and C₇ Trp), 118.4 (C₄ Trp), 118.8 (C₄ indole and C₅ Trp), 119.0 (C₅ indole), 121.4 (C₆ Trp), 121.7 (C₆ indole), 124.0 (C₂ indole and C₂ Trp), 127.1 (C₄ phenyl), 127.7 (C₉ indole and C₉ Trp), 128.8 (C₂ and C₆ phenyl), 129.1 (C₃ and C₅ phenyl), 136.5 (C₈ Trp), 136.6 (C₈ indole), 137.5 (C₁ phenyl), 153.6 (Cq triazole), 155.0 (Cq triazole), 171.8 (CO Aib).

### (R)-N-(1-(5-benzyl-4-(2,2-diphenylethyl)-4H-1,2,4-triazol-3-yl)-2-(1H-indol-3-yl)ethyl)-2-amino-2-methylpropanamide (Compound 106):

¹H NMR (300 MHz, DMSO-d⁶, 300°K):
δ (ppm) 1.29 (3H, s, CH₃ Aib), 1.34 (3H, s, CH₃ Aib), 3.37 (4H, m, CH₂ βTrp and CH₂-benzyl), 3.74 (1 H, t, J= 7 Hz, CH₂-CH(Phe)₂), 4.21 (1 H, dd, J= 14 Hz and 8 Hz, CH₂-CH(Phe)₂), 4.51 (1 H, dd, J= 14 Hz and 8 Hz, CH₂-CH(Phe)₂), 5.08 (1 H, m, CH αTrp), 6.72 (2H, m, H₂ and H₆ benzyl), 6.86-6.93 (5H, m, H₃, H₄ and H₅ benzyl, H₅ and H₆ Trp), 7.03 (1 H, s, H₂ Trp), 7.06-7.25 (CHar phenyl from CH(Phe)₂), 7.33 (1 H, d, Jₒ= 8 Hz, H₄ Trp), 7.47 (1 H, d, Jₒ= 8 Hz, H₇ indole), 8.10 (3H, brs, NH₂ Aib), 8.98 (1 H, d, J= 8 Hz, NH amide), 10.94 (1 H, s, NH indole Trp).
¹³C NMR (75 MHz, DMSO-d⁶, 300°K):
δ (ppm) 23.5 (CH₃ Aib), 23.7 (CH₃ Aib), 29.4 (CH₂ βTrp), 30.1 (CH₂-benzyl), 46.0 (CH αTrp), 47.7 (CH₂-CH(Phe)₂), 51.3 (CH(Phe)₂), 56.8 (Cq Aib), 109.8 (C₃ Trp), 112.0 (C₇ Trp), 118.5 (C₄ Trp), 119.0 (C₅ Trp), 121.5 (C₆ Trp), 124.8 (C₂ Trp), 127.1 (C₄ phenyl from CH(Phe)₂), 127.4 (C₉ Trp, C₂ and C₆ benzyl), 128.3 (C₂ and C₆ phenyl from CH(Phe)₂), 128.8-129.1 (C₃ and C₅ phenyl from CH(Phe)₂, C₃, C₄ and C₅ benzyl), 136.2 (C₁ benzyl), 136.5 **(C₈** Trp), 141.0 (C₁ phenyl from CH(Phe)₂), 153.5 (Cq triazole), 155.1 (Cq triazole), 172.0 (CO Aib)

### (R)-N-(1-(5-(2-(1H-indol-3-yl)ethyl)-4-(2,2-diphenylethyl)-4H-1,2,4-triazol-3-yl)-2-(1H-indol-3-yl)ethyl)-2-amino-2-methylpropanamide (Compound 107):

¹H NMR (300 MHz, DMSO-d⁶, 300°K):
δ (ppm) 1.34 (3H, s, CH₃ Aib), 1.38 (3H, s, CH₃ Aib), 2.06 (1 H, m, CH₂-CH₂-indole), 2.30 (1 H, m, CH₂-CH₂-indole), 2.78 (2H, m, CH₂-CH₂-indole), 3.35 (1 H, dd, J= 14 Hz and 7 Hz, CH₂ βTrp), 3.46 (1 H, dd, J= 14 Hz and 9 Hz, CH₂ βTrp), 3.58 (1 H, t, J= 7 Hz, CH₂-CH(Phe)₂), 4.14 (1 H, dd, J= 14 Hz and 8 Hz, CH₂-CH(Phe)₂), 4.39 (1 H, dd, J= 14 Hz and 7 Hz, CH₂-CH(Phe)₂), 5.12 (1 H, m, CH αTrp), 6.50 (2H, m, H₅ indole and H₅ Trp), 6.76 (2H, m, H₆ indole and H₆ Trp), 6.87 (2H, m, H₂ indole and H₂ Trp), 6.89-6.96 (2H, m, H₄ phenyl), 7.03-7,15 (8H, m, H₂, H₃, H₅ and H₆ phenyl), 7.33 (3H, m, H₄ indole, H₄ and H₇ Trp), 7.47 (1 H, d, J= 8 Hz, H₇ indole), 8.11 (3H, brs, NH₂ Aib), 9.04 (1 H, d, J= 8 Hz, NH amide), 10.76 (1 H, s, NH indole), 10.96 (1 H, s, NH indole Trp).
¹³C NMR (75 MHz, DMSO-d⁶, 300°K):
δ (ppm) 22.4 (CH₂-CH₂-indole), 23.6 (CH₃ Aib), 23.8 (CH₃ Aib), 24.9 (CH₂-CH₂-indole), 29.6 (CH₂ βTrp), 46.1 (CH αTrp), 47.5 (CH₂-CH(Phe)₂), 51.5 (CH₂-CH(Phe)₂), 56.8 (Cq Aib), 109.8 (C₃ Trp), 111.8 (C₇ Trp), 112.1 (C₇ indole), 113.5 (C₃ indole), 118.4 (C₄ Trp), 118.7 (C₄ and C₅ indole), 119.0 (C₅ Trp), 121.4 (C₆ indole and C₆ Trp), 122.8 (C₂ indole), 125.0 (C₂ Trp), 127.2 (C₉ indole and C₉ Trp), 127.3 (C₄ phenyl), 128.2 (C₂ and C₆ phenyl), 128.7 (C₃ and C₅ phenyl), 136.6 (C₈ indole and C₈ Trp), 141.0 (C₁ phenyl), 154.6 (2 Cq triazole), 172.0 (CO Aib).

### (R)-N-(1-(4,5-dibenzyl-4H-1,2,4-triazol-3-yl)-2-(1H-indol-3-yl)ethyl)-2-amino-2-methylpropanamide (Compound 109):

¹H NMR (300 MHz, DMSO-d⁶, 300°K):
δ (ppm) 1.23 (3H, s, CH₃ Aib), 1.26 (3H, s, CH₃ Aib), 3.23 (1 H, dd, J= 14 Hz and 6 Hz, CH₂ βTrp), 3.35 (1 H, dd, J= 14 Hz and 9 Hz, CH₂ βTrp), 3.99 (2H, s, C-CH₂-phenyl), 5.10 (3H, m, N-CH₂-phenyl and CH αTrp), 6.77 (3H, m, H₅ Trp, H₂ and H₆ phenyl from N-CH₂-phenyl), 6.99 (2H, m, H₂ and H₆ Trp), 7.01-7.07 (3H, m, H₄ Trp, H₂ and H₆ from C-CH₂-phenyl), 7.15-7.23 (6H, m, H₃, H₄ and H₅ phenyl from N-CH₂-phenyl and from C-CH₂-phenyl), 7.25 (1 H, d, J= 8 Hz, H₇ Trp), 8.01 (3H, brs, NH₂ Aib), 8,91 (1 H, d, J= 8 Hz, NH amide), 10.78 (1 H, s, NH indole Trp).
¹³C NMR (75 MHz, DMSO-d⁶, 300°K):
δ (ppm) 23.5 (CH₃ Aib), 23.7 (CH₃ Aib), 29.0 (CH₂ βTrp), 30.6 (C-CH₂-phenyl), 45.7 (CH αTrp), 46.1 (N-CH₂-phenyl), 56.7 (Cq Aib), 109.8 (C₃ Trp), 111.7 (C₇ Trp), 118.3 (C₄ Trp), 118.7 (C₅ Trp), 121.2 (C₆ Trp), 124.8 (C₂ Trp), 126.3 (C₂ and C₆ phenyl from N-CH₂-phenyl), 127.0 (C₂ and C₆ phenyl from C-CH₂-phenyl), 127.2 (Cg Trp), 128.0 (C₄ phenyl from N-CH₂-phenyl), 128.8 (C₃, C₄ and C₅ phenyl from C-CH₂-phenyl), 128.9 (C₃ and C₅ phenyl from N-CH₂-phenyl), 135.8 (C₁ phenyl from N-CH₂-phenyl), 136.2 (C₁ phenyl from C-CH₂-phenyl), 136.4 (C₈ Trp), 153.9 (Cq triazole), 155.3 (Cq triazole), 171.9 (CO Aib).

### (R)-N-(1-(5-benzyl-4-hexyl-4H-1,2,4-triazol-3-yl)-2-(1H-indol-3-yl)ethyl)-2-amino-2-methylpropanamide (Compound 110):

¹H NMR (300 MHz, DMSO-d⁶, 300°K):
δ (ppm) 0.71 (3H, t, ³J= 7 Hz, (CH₂)₅-CH₃), 0.87 (4H, m, 2 CH₂), 0.95 (2H, m, CH₂-CH₃), 1.00 (2H, m, N-CH₂-CH₂), 1.36 (6H, s, CH₃ Aib), 3.36 (1 H, dd, ³J= 14 Hz and 7 Hz, CH₂ βTrp), 3.41 (1 H, dd, ³J= 14 Hz and 7 Hz, CH₂ βTrp), 3.50 (1 H, m, N-CH₂), 3.65 (1 H, m, N-CH₂), 4.11 (2H, s, CH₂-benzyl), 5.14 (1 H, m, CH αTrp), 6.90 (1 H, t, Jₒ= 7 Hz, H₅ Trp), 7.01 (1 H, t, Jₒ= 7 Hz, H₆ Trp), 7.04 (1 H, s, H₂ Trp), 7.09 (2H, m, H₂ and H₆ benzyl), 7.17-7.29 (4H, m, H₄ Trp, H₃, H₄ and H₅ benzyl), 7.47 (1 H, d, Jₒ= 8 Hz, H₇ Trp), 8.10 (3H, brs, NH₂ Aib), 9.05 (1 H, d, J= 7 Hz, NH amide), 10.84 (1 H, s, NH indole Trp).
¹³C NMR (75 MHz, DMSO-d⁶, 300°K):
δ (ppm) 14.1 ((CH₂)₅-CH₃), 22.1 (CH₂-CH₃), 23.8 (CH₃ Aib), 23.5 (CH₃ Aib), 25.8 (CH₃-CH₂-CH₂-CH₂), 29.5 (CH₂ βTrp), 30.3 (N-CH₂-CH₂), 30.8 (CH₂-benzyl and CH₃-CH₂-CH₂), 43.3 (N-CH₂-CH₂), 46.1 (CH αTrp), 56.8 (Cq Aib), 109.6 (C₃ Trp), 111.9 (C₇ Trp), 118.2 (C₄ Trp), 118.8 (C₅ Trp), 121.4 (C₆ Trp), 124.7 (C₂ Trp), 127.2 (C₂ and C₆ benzyl), 127.3 (C₉ Trp), 128.8 (C₃, C₄ and C₅ benzyl), 136.2 (C₁ benzyl), 136.5 (C₈ Trp), 153.1 (Cq triazole), 155.1 (Cq triazole), 171.9 (CO Aib).

### (R)-N-(1-(4-(2-(1H-indol-3-yl)ethyl)-5-benzyl-4H-1,2,4-triazol-3-yl)-2-(1H-indol-3-yl)ethyl)-2-amino-2-methylpropanamide (Compound 111):

¹H NMR (400 MHz, DMSO-d⁶, 300°K):
δ (ppm) 1.31 (3H, s, CH₃ Aib), 1.35 (3H, s, CH₃ Aib), 2.51 (2H, m, CH₂-CH₂-indole), 3.37 (2H, m, CH₂ βTrp), 3.76-3.90 (4H, m, CH₂-benzyl and CH₂-CH₂-indole), 5.25 (1 H, m, CH αTrp), 6.88 (2H, t, Jₒ= 7 hz, H₅ indole and H₅ Trp), 6.95 (2H, t, Jₒ= 7 Hz, H₆ indole and H₆ Trp), 7.03 (4H, m, H₂ Trp, H₂ indole, H₂ and H₆ benzyl), 7.16 (2H, d, Jₒ= 8 Hz, H₄ indole and H₄ Trp), 7.20-7,30 (4H, 3, H₇ indole, H₃, H₄ and H₅ benzyl), 7.47 (1 H, d, Jₒ= 8 Hz, H₇ Trp), 8.05 (3H, brs, NH₂ Aib), 9.05 (1 H, d, J= 8 Hz, NH amide), 10.83 (1 H, s, NH indole), 10.88 (1 H, s, NH indole Trp).
¹³C NMR (100 MHz, DMSO-d⁶, 300°K):
6(ppm) 23.5 (CH₃ Aib), 23.7 (CH₃ Aib), 29.6 (CH₂ βTrp), 30.3 (CH₂-benzyl and CH₂-CH₂-indole), 44.1 (CH₂-CH₂-indole), 46.1 (CH αTrp), 56.8 (Cq Aib), 109.8 (C₃ Trp), 109.9 (C₃ indole), 111.9 (C₇ indole and C, Trp), 118.3 (C₄ Trp), 118.4 (C₄ indole), 118.9 (C₅ indole and C₅ Trp), 121.3 (C₆ Trp), 121.5 (C₆ indole), 123.7 (C₂ indole), 124.7 (C₂ Trp), 127.0 (C₉ indole), 127.2 (C₂ and C₆ benzyl), 127.4 (C₉ Trp), 128.8 (C₃ and C₅ benzyl), 129.0 (C₄ benzyl), 136.3 (C₁ benzyl), 136.4 (C₈ indole and C₈ Trp), 153.1 (Cq triazole), 155.3 (Cq triazole), 171.8 (CO Aib).

### (S)-N-(1-(4-(2,4-dimethoxybenzyl)-5-benzyl-4H-1,2,4-triazol-3-yl)-2-(1H-indol-3-yl)ethyl)-2-amino-2-methylpropanamide (Compound 112):

¹H NMR (300 MHz, DMSO-d⁶, 300°K):
δ (ppm) 1.26 (3H, s, CH₃ Aib), 1.29 (3H, s, CH₃ Aib), 3.24 (1 H, dd, J= 14 Hz and 6 Hz, CH₂ βTrp), 3.33 (1 H, dd, J= 14 Hz and 9 Hz, CH₂ βTrp), 3.64 (3H, s, OCH₃), 3.68 (3H, s, OCH₃), 3.99 (2H, s, CH₂ phenyl), 4.84 (1 H, d, J= 17 Hz, CH₂-o,p-dimethoxybenzyl), 5.05 (1 H, d, J= 17 Hz, CH₂-o,p-dimethoxybenzyl), 5.13 (1 H, m, CH αTrp), 6.24 (2H, m, H₅ and H₆ o,p-dimethoxybenzyl), 6.52 (1 H, d, Jₘ= 2 Hz, H₃ o,p-dimethoxybenzyl), 6.83 (1 H, t, Jₒ= 7 Hz, H₅ Trp), 7.01 (1 H, t, Jₒ= 8 Hz, H₆ Trp), 7.04 (1 H, s, H₂ Trp), 7.05-7.23 (6H, m, H4 Trp and CHar phenyl), 7.27 (1 H, d, Jₒ= 8 Hz, H₇ Trp), 8.01 (3H, brs, NH₂ Aib), 8.90 (1 H, d, J= 8 Hz, NH amide), 10.77 (1 H, d, J= 2 Hz, NH indole Trp).
¹³C NMR (75 MHz, DMSO-d⁶, 300°K):
δ (ppm) 23.6 (CH₃ Aib), 29.1 (CH₂ βTrp), 30.6 (CH₂-phenyl), 41.9 (CH₂-*o,p-*dimethoxybenzyl), 45.7 (CH αTrp), 55.7 (OCH₃), 55.9 (OCH₃), 56.7 (Cq Aib), 99.9 (C₃ *o,p*-dimethoxybenzyl), 105.1 (C₅ *o,p*-dimethoxybenzyl), 109.9 (C₃ Trp), 111.7 (C₇ Trp), 115.5 (C₁ *o,p*-dimethoxybenzyl), 118.3 (C₄ Trp), 118.6 (C₅ Trp), 121.3 (C₆ Trp), 124.2 (C₂ Trp), 127.0 (C₆ *o,p*-dimethoxybenzyl), 127.3 (C₉ Trp), 127.8 (C₄ phenyl), 128.8 (C₂, C₃, C₅ and C₆ phenyl), 136.2 (C₈ Trp), 136.4 (C₁ phenyl), 153.9 (Cq triazole), 155.5 (Cq triazole), 157.6 (C₂ *o,p*-dimethoxybenzyl), 160.8 (C₄ *o,p*-dimethoxybenzyl), 171.8 (CO amide).

### (R)-N-(1-(4-(3,5-dimethoxybenzyl)-5-phenethyl-4H-1,2,4-triazol-3-yl)-2-(1H-indol-3-yl)ethyl)-2-amino-2-methylpropanamide (Compound 113):

¹H NMR (300 MHz, DMSO-d⁶, 300°K):
δ (ppm) 1.24 (3H, s, CH₃ Aib), 1.27 (3H, s, CH₃ Aib), 2.83 (4H, s, CH₂-CH₂-phenyl), 3.32 (2H, m, CH₂ βTrp), 3.61 (6H, s, OCH₃), 5.02 (2H, m, CH₂-*m*-dimethoxybenzyl), 5.18 (1 H, m, CH αTrp), 6.07 (2H, d, Jₘ= 2 Hz, H₂ and H₆ *m*-dimethoxybenzyl), 6.42 (1 H, brs, H₄ *m*-dimethoxybenzyl), 6.83 (1 H, t, Jₒ= 7 Hz, H₅ Trp), 6.99 (1 H, t, Jₒ= 8 Hz, H₆ Trp), 7.08 (1 H, d, J= 2 Hz, H₂ Trp), 7.13 (3H, t, Jₒ= 8 Hz, H₃, H₄ and H₅ phenyl), 7.20 (3H, d, Jₒ= 7 Hz, H₂ and H₆ phenyl, H₄ Trp), 7.28 (1 H, d, Jₒ= 8 Hz, H₇ Trp), 7.99 (3H, brs, NH₂ Aib), 8.92 (1 H, d, J= 8 Hz, NH amide), 10.77 (1 H, s, NH indole).
¹³C NMR (75 MHz, DMSO-d⁶, 300°K):
δ (ppm) 23.5 (CH₃ Aib), 23.8 (CH₃ Aib), 26.5 (CH₂-CH₂-phenyl), 29.2 (CH₂ pTrp), 32.7 (CH₂-CH₂-phenyl), 45.6 (CH αTrp), 45.8 (CH₂- m-dimethoxybenzyl), 55.6 (OCH₃), 56.8 (Cq Aib), 99.6 (C₄ m-dimethoxybenzyl), 104.6 (C₂ and C₆ m-dimethoxybenzyl), 109.9 (C₃ Trp), 111.8 (C₇ Trp), 118.2 (C₄ Trp), 118.7 (C₅ Trp), 121.3 (C₆ Trp), 124.8 (C₂ Trp), 126.5 (C₄ phenyl), 127.3 (C₉ Trp), 128.7 (C₂, C₃, C₅ and C₆ phenyl), 136.4 (C₈ Trp), 138.6 (C₄ m-dimethoxybenzyl), 140.9 (C₁ phenyl), 154.6 (Cq triazole), 154.8 (Cq triazole), 161.4 (C₃ and C₅ m-dimethoxybenzyl), 171.8 (CO amide).

### (R)-N-(1-(4-(4-bromobenzyl)-5-benzyl-4H-1,2,4-triazol-3-yl)-2-(1H-indol-3-yl)ethyl)-2-amino-2-methylpropanamide (Compound 114):

¹H NMR (300 MHz, DMSO-d⁶, 300°K):
δ (ppm)1.23 (3H, s, CH₃ Aib), 1.25 (3H, s, CH₃ Aib), 3.26 (1 H, dd, ³J= 14 Hz and 6 Hz, CH₂ Trp), 3.34 (1 H, dd, ³J= 14 Hz and 9 Hz, CH₂ βTrp), 4.01 (2H, m, CH₂-benzyl), 5.01 (1 H, m, CH αTrp), 5.08 (2H, s, CH₂-*p*-bromobenzyl), 6.59 (2H, d, Jₒ= 8 Hz, H₂ and H₆ *p*-bromobenzyl), 6.81 (1 H, t, Jₒ= 7 Hz, H₅ Trp), 6.94 (1 H, s, H₂ Trp), 6.98 (1 H, t, Jₒ= 7 Hz, H₆ Trp), 7.06 (2H, m, H₂ and H₆ benzyl), 7.12 (1 H, d, Jₒ= 7 Hz, H₄ Trp), 7.16-7.20 (3H, m, H₃, H₄ and H₅ benzyl), 7.26 (1 H, d, Jₒ= 8 Hz, H₇ Trp), 7.29 (2H, d, Jₒ= 8 Hz, H₃ and H₅ benzyl), 8.00 (3H, brs, NH₂ Aib), 8.92 (1 H, d, J= 8 Hz, NH amide), 10.78 (1 H, s, NH indole Trp).
¹³C NMR (75 MHz, DMSO-d⁶, 300°K):
δ (ppm) 23,5 (CH₃ Aib), 23,8 (CH₃ Aib), 29,0 (CH₂ βTrp), 30,5 (CH₂-benzyl), 45,6 (CH₂-p-bromobenzyl), 45,7 (CH α Trp), 56,7 (Cq Aib), 109,7 (C₃ Trp), 111,8 (C₇ Trp), 118,2 (C₄ tryptophae), 118,7 (C₅ Trp), 121,2 (C₄ *p*-bromobenzyl), 121,3 (C₆ Trp), 124,9 (C₂ typtophane), 127,0 (C₂ and C₆ benzyl), 127,2 (C₉ Trp), 128,4 (C₂ and C₆ *p-*bromobenzyl), 128,9 (C₃, C₄ and C₅ benzyl), 131,9 (C₃ and C₅ *p*-bromobenzyl), 135,2 (C₁ *p*-bromobenzyl), 136,2 (C₈ Trp), 136,4 (C₁ benzyl), 153,9 (Cq triazole), 155,3 (Cq triazole), 171,9 (CO Aib).

### (R)-N-(1-(4-(2-methoxybenzyl)-5-benzyl-4H-1,2,4-triazol-3-yl)-2-(1H-indol-3-yl)ethyl)-2-amino-2-methylpropanamide (Compound 115):

¹H NMR (300 MHz, DMSO-d⁶, 300°K):
δ (ppm) 1.24 (3H, s, CH₃ Aib), 1.27 (3H, s, CH₃ Aib), 3.20 (1 H, dd, J= 14 Hz and 5 Hz, CH₂ βTrp), 3.33 (1 H, dd, J= 14 Hz and 9 Hz, CH₂ βTrp), 3.68 (3H, s, OCH₃), 4.00 (2H, s, CH₂-phenyl), 4.95 (1 H, d, J= 17 Hz, CH₂-o- methoxybenzyl), 5.07 (1 H, m, CH αTrp), 5.18 (1 H, d, J= 17 Hz, CH₂-o- methoxybenzyl), 6.27 (1 H, d, Jₒ= 8 Hz, H₃ o-methoxybenzyl), 6.67 (1 H, t, Jₒ= 7 Hz, H₅ Trp), 6.77 (1 H, t, Jₒ= 6 Hz, H₆ Trp), 6.92-7.05 (6H, m, H₂ Trp, H₂ and H₆ phenyl, H₄, H₅ and H₆ o- methoxybenzyl), 7.14-7.26 (5H, m, H₄ and H₇ Trp, H₃, H₄ and H₅ phenyl), 8.03 (3H, brs, NH₂ Aib), 8.91 (1 H, d, J= 8 Hz, NH amide), 10.78 (1 H, s, NH indole Trp).
¹³C NMR (75 MHz, DMSO-d⁶, 300°K):
δ (ppm) 23.5 (CH₃ Aib), 23.6 (CH₃ Aib), 29.1 (CH₂ β Trp), 30.5 (CH₂-phenyl), 42.1 (CH₂-o- methoxybenzyl), 45.7 (CH α Trp), 55.9 (OCH₃), 56.7 (Cq Aib), 109.8 (C₃ Trp), 111.3 (C₃ o-methoxybenzyl), 111.7 (C₇ Trp), 118.2 (C₄ Trp), 118.7 (C₅ Trp), 120.9 (C₅ o-methoxybenzyl), 121.2 (C₆ Trp), 123.3 (C₁ o- methoxybenzyl), 124.8 (C₂ Trp), 126.6 (C₂ and C₆ phenyl), 127.1 (C₄ 0- methoxybenzyl), 127.2 (C₉ Trp), 128.8 (C₃, C₄ and C₅ phenyl), 129.5 (C₆ 0- methoxybenzyl), 136.0 (C₁ phenyl), 136.4 (C₈ Trp), 154.0 (Cq triazole), 155.6 (Cq triazole), 156.5 (C₂ 0- methoxybenzyl), 171.8 (CO Aib).

### (S)-N-(1-(4-(2,4-dimethoxybenzyl)-5-phenethyl-4H-1,2,4-triazol-3-yl)-2-(1H-indol-3-yl)ethyl)-2-amino-2-methylpropanamide (Compound 116):

¹H NMR (300 MHz, DMSO-d⁶, 300°K):
δ (ppm) 1.28 (3H, s, CH₃ Aib), 1.32 (3H, s, CH₃ Aib), 2.81 (4H, m, CH₂-CH₂-phenyl), 3.30 (2H, t, CH₂ βTrp), 3.61 (3H, s, OCH₃), 3.69 (3H, s, OCH₃), 4.87 (1 H, d, J= 17 Hz, CH₂-o,p-dimethoxybenzyl), 5.03 (1 H, d, J= 17 Hz, CH₂-o,p-dimethoxybenzyl), 5.20 (1 H, m, CH αTrp), 6.29 (1 H, dd, Jₒ= 8 Hz and Jₘ= 2 Hz, H₅ o,p-dimethoxybenzyl), 6.43 (1 H, d, Jₒ= 8 Hz, H₆ o,p-dimethoxybenzyl), 6.55 (1 H, d, Jₘ= 2 Hz, H₃ o,p-dimethoxybenzyl), 6.83 (1 H, t, Jₒ= 8 Hz, H₅ Trp), 6.99 (1 H, t, Jₒ= 8 Hz, H₆ Trp), 7.06 (1 H, d, J= 2 Hz, H₂ Trp), 7.09-7.25 (6H, m, H₄ Trp and CHar phenyl), 7.28 (1 H, d, Jₒ= 8 Hz, H₇ Trp), 8.04 (3H, brs, NH₂ Aib), 8.92 (1 H, d, J= 8 Hz, NH amide), 10.79 (1 H, s, NH indole Trp).
¹³C NMR (75 MHz, DMSO-d⁶, 300°K):
δ (ppm) 23.6 (CH₃ Aib), 23.7 (CH₃ Aib), 26.5 (CH₂-CH₂-phenyl), 29.1 (CH₂ pTrp), 32.7 (CH₂-CH₂-phenyl), 41.8 (CH₂-*o,p*-dimethoxybenzyl), 45.7 (CH αTrp), 55.7 (OCH₃), 55.9 (OCH₃), 56.8 (Cq Aib), 99.1 (C₃ o,p-dimethoxybenzyl), 105.2 (C₅ o,p-dimethoxybenzyl), 109.9 (C₃ Trp), 111.8 (C₇ Trp), 115.6 (C₁ *o,p*-dimethoxybenzyl), 118.3 (C₄ Trp), 118.7 (C₅ Trp), 121.3 (C₆ Trp), 124.4 (C₂ Trp), 126.6 (C₄ phenyl), 127.3 (C₉ Trp), 128.2 (C₆ o,p-dimethoxybenzyl), 128.7 (C₂, C₃, C₅ and C₆ phenyl), 136.4 (C₈ Trp), 140.9 (C₁ phenyl), 154.6 (Cq triazole), 155.0 (Cq triazole), 157.8 (C₂ *o,p*-dimethoxybenzyl), 160.9 (C₄ *o,p*-dimethoxybenzyl), 171.8 (CO Aib).

### (R)-N-(1-(4,5-diphenethyl-4H-1,2,4-triazol-3-yl)-2-(1H-indol-3-yl)ethyl)-2-amino-2-methylpropanamide (Compound 117):

¹H NMR (300 MHz, DMSO-d⁶, 300°K):
δ (ppm) 1.32 (3H, s, CH₃ Aib), 1.37 (3H, s, CH₃ Aib), 2.59 (4H, m, C-CH₂-CH₂-phenyl), 2.83 (2H, t, J= 8 Hz, N-CH₂-CH₂-phenyl), 3.38 (2H, m, N-CH₂-CH₂-phenyl), 3.84 (1 H, m, CH₂ βTrp), 3.94 (1 H, m, CH₂ βTrp), 5.23 (1 H, m, CH αTrp), 6.84 (2H, m, H₄ phenyl from C-CH₂-CH₂-phenyl and H₄ phenyl from N-CH₂-CH₂-phenyl), 6.93 (1 H, t, Jₒ= 8 Hz, H₅ Trp), 7.00 (1 H, t, Jₒ= 8 Hz, H₆ Trp), 7.07 (1 H, d, J= 2 Hz, H₂ Trp), 7.11-7.27 (9H, m, H₂, H₃, H₅ and H₆ phenyl from C-CH₂-CH₂-phenyl, H₂, H₃, H₅ and H₆ phenyl from N-CH₂-CH₂-phenyl and H₄ Trp), 7.50 (1 H, d, Jₒ= 8 Hz, H₇ Trp), 8.07 (3H, brs, NH₂ Aib), 9.04 (1 H, d, J= 8 Hz, NH amide), 10.85 (1 H, s, NH indole Trp).
¹³C NMR (75 MHz, DMSO-d⁶, 300°K):
δ (ppm) 23.5 (CH₃ Aib), 23.8 (CH₃ Aib), 25.9 (C-CH₂-CH₂-phenyl), 29.5 (CH₂ βTrp), 32.4 (C-CH₂-CH₂-phenyl), 35.8 (N-CH₂-CH₂-phenyl), 44.2 (N-CH₂-CH₂-phenyl), 45.9 (CH αTrp), 56.8 (Cq Aib), 109.7 (C₃ Trp), 111.9 (C₇ Trp), 118.4 (C₄ Trp), 118.9 (C₅ Trp), 121.4 (C₆ Trp), 124.8 (C₂ Trp), 126.6 (C₄ phenyl from C-CH₂-CH₂-phenyl), 127.2 (C₄ phenyl from N-CH₂-CH₂-phenyl), 127.4 (C₉ Trp), 128.7 (C₂ and C₆ phenyl from C-CH₂-CH₂-phenyl, C₂ and C₆ phenyl from N-CH₂-CH₂-phenyl), 128.8 (C₃ and C₅ phenyl from C-CH₂-CH₂-phenyl, C₃ and C₅ phenyl from N-CH₂-CH₂-phenyl), 136.5 (C₁ phenyl from N-CH₂-CH₂-phenyl), 137.5 (C₁ phenyl from C-CH₂-CH₂-phenyl), 140.8 (C₈ Trp), 154.1 (Cq triazole), 154.7 (Cq triazole), 171.9 (CO Aib).

### (R)-N-(1-(4-(3,4-dichlorobenzyl)-5-benzyl-4H-1,2,4-triazol-3-yl)-2-(1H-indol-3-yl)ethyl)-2-amino-2-methylpropanamide (Compound 118):

¹H NMR (300 MHz, DMSO-d⁶, 300°K):
δ (ppm) 1.24 (3H, s, CH₃ Aib), 1.25 (3H, s, CH₃ Aib), 3.33 (2H, m, CH₂ βTrp), 4.04 (2H, s, CH₂-benzyl), 5.05 (1 H, m, CH αTrp), 5.12 (2H, s, CH₂-*m,p*-dichlorobenzyl), 6.49 (1 H, dd, Jₒ= 8 Hz and Jₘ= 2 Hz, H₆ *m,p*-dichlorobenzyl), 6.80 (1 H, t, Jₒ= 8 Hz, H₅ Trp), 6.87 (1 H, d, Jₘ= 2 Hz, H₂ Trp), 6.98 (1 H, t, Jₒ= 7 Hz, H₆ Trp), 7.02-7.10 (3H, m, H₂ and H₆ benzyl, H₅ *m,p*-dichlorobenzyl), 7.18 (3H, m, H₃, H₄ and H₅ benzyl), 7.26 (2H, m, H₄ and H₇ Trp), 8.04 (3H, brs, NH₂ Aib), 8.94 (1 H, d, J= 9 Hz, NH amide), 10.81 (1 H, s, NH indole Trp).
¹³C NMR (75 MHz, DMSO-d⁶, 300°K):
δ (ppm) 23.4 (CH₃ Aib), 23.8 (CH₃ Aib), 29.0 (CH₂ βTrp), 30.4 (CH₂-benzyl), 45.1 (CH₂-m,p-dichlorobenzyl), 45.6 (CH αTrp), 56.7 (Cq Aib), 109.6 (C₃ Trp), 111.8 (C₇ Trp), 118.1 (C₄ Trp), 118.6 (C₅ Trp), 121.3 (C₆ Trp), 124.9 (C₂ Trp), 126.4 (C₆ *m,p-*dichlorobenzyl), 127.0 (C₂ *m,p*-dichlorobenzyl), 127.2 (C₉ Trp), 128.4 (C₂ and C₆ benzyl), 130.7 (C₄ and C₅ *m,p*-dichlorobenzyl), 131.8 (C₃ *m,p*-dichlorobenzyl), 136.0 (C₁ benzyl), 136.4 (C₈ Trp), 136.7 (C₁ *m,p*-dichlorobenzyl), 154.1 (Cq triazole), 155.2 (Cq triazole), 172.0 (CO Aib).

### (R)-N-(1-(4-(4-methoxybenzyl)-5-benzyl-4H-1,2,4-triazol-3-yl)-2-phenylethyl)-2-amino-2-methylpropanamide (Compound 120):

¹H NMR (300 MHz, DMSO-d⁶, 300°K):
δ (ppm) 1.18 (3H, s, CH₃ Aib), 1.26 (3H, s, CH₃ Aib), 3.09 (1 H, dd, J= 14 Hz and 6 Hz, CH₂ βPhe), 3.18 (1 H, dd, J= 14 Hz and 9 Hz, CH₂ (3Phe), 3.99 (2H, d, J= 4 Hz, CH₂-phenyl), 4.95 (1 H, d, J= 16 Hz, CH₂-p-methoxybenzyl), 5.06 (1 H, d, J= 16 Hz, CH₂-p-methoxybenzyl), 5.13 (1 H, m, CH αPhe), 6.78 (4H, s, CHar p-methoxybenzyl), 7.02-7.08 (4H, m, H₂ and H₆ phenyl, H₂ and H₆ Phe), 7.12-7.25 (6H, m, H₃, H₄ and H₅ phenyl, H₃, H₄ and H₅ Phe), 7.99 (3H, brs, NH₂ Aib), 8.92 (1 H, d, J= 8 Hz, NH amide).
¹³C NMR (75 MHz, DMSO-d⁶, 300°K):
δ (ppm) 23.4 (CH₃ Aib), 23.8 (CH₃ Aib), 30.7 (CH₂-phenyl), 38.7 (CH₂ βPhe), 45.9 (CH₂-p-methoxybenzyl), 46.4 (CH αPhe), 55.6 (OCH₃), 56.7 (Cq Aib), 114.6 (C₃ and C₅ *p-*methoxybenzyl), 126.9 and 127.1 (C₄ phenyl and C₄ Phe), 127.7 (C₁ *p*-methoxybenzyl), 128.2 (C₂ and C₆ Phe), 128.5 (C₃ and C₅ Phe), 128.9 (C₂, C₃, C₅ and C₆ phenyl), 129.7 (C₂ and C₆ *p*- methoxybenzyl), 136.3 (C₁ phenyl), 137.6 (C₁ Phe), 154.0 (Cq triazole), 154.9 (Cq triazole), 159.2 (C₄ *p*-methoxybenzyl), 171.7 (CO amide).

### (R)-N-(1-(4-(4-fluorobenzyl)-5-phenethyl-4H-1,2,4-triazol-3-yl)-2-(1H-indol-3-yl)ethyl)-2-amino-2-methylpropanamide (Compound 121):

¹H NMR (300 MHz, DMSO-d⁶, 300°K):
δ (ppm) 1.27 (3H, s, CH₃ Aib), 1.28 (3H, s, CH₃ Aib), 2.82 (4H, m, CH₂-CH₂-phenyl), 3.34 (2H, m, CH₂ βTrp), 5.06 (2H, s, CH₂-*p*-fluorobenzyl), 5.16 (1 H, m, CH αTrp), 6.85 (3H, m, H₅ Trp, H₃ and H₅ p-fluorobenzyl), 6.98-7.04 (4H, m, H₂ and H₆ Trp, H₂ and H₆ phenyl), 7.09-7.11 (2H, m, H₂ and H₆ p-fluorobenzyl), 7.15 (1 H, d, Jₒ= 6 Hz, H₄ Trp), 7.19 (3H, t, Jₒ= 8 Hz, H₃, H₄ and H₅ phenyl), 7.29 (1 H, d, Jₒ= 8 Hz, H₇ Trp), 8.01 (3H, brs, NH₂ Aib), 8.94 (1 H, d, J= 8 Hz, NH amide), 10.78 (1 H, s, NH indole Trp).
¹³C NMR (75 MHz, DMSO-d⁶, 300°K):
δ (ppm) 23.5 (CH₃ Aib), 23.8 (CH₃ Aib), 26.5 (CH₂-CH₂-phenyl), 29.2 (CH₂ βTrp), 32.7 (CH₂-CH₂-phenyl), 45.4 (CH₂-*p*-fluorobenzyl), 45.7 (CH αTrp), 56.8 (Cq Aib), 109.8 (C₃ Trp), 111.8 (C₇ Trp), 115.9 and 116.2 (C₃ and C₅ *p*-fluorobenzyl), 118.3 (C₄ Trp), 118.7 (C₅ Trp), 121.3 (C₆ Trp), 124.8 (C₂ Trp), 126.5 (C₄ phenyl), 127.3 (C₉ Trp), 128.5 (C₂ and C₆ p-fluorobenzyl), 128.7 (C₂, C₃, C₅ and C₆ phenyl), 132.2 (C₁ *p*-fluorobenzyl), 136.4 (C₈ Trp), 140.9 (C₁ phenyl), 154.5 (Cq triazole), 154.7 (Cq triazole), 160.3 (C₄ p-fluorobenzyl), 171.9 (CO amide).
NMR ¹⁹F (282 MHz, DMSO-d⁶, 300°K):
δ (ppm) -114.9 (1 F, m, *p*-fluorobenzyl).

### (R)-N-(1-(4-(3,4-dichlorobenzyl)-5-phenethyl-4H-1,2,4-triazol-3-yl)-2-(1H-indol-3-yl)ethyl)-2-amino-2-methylpropanamide (Compound 122):

¹H NMR (300 MHz, DMSO-d⁶, 300°K):
6(ppm) 1.25 (3H, s, CH₃ Aib), 1.26 (3H, s, CH₃ Aib), 2.84 (4H, m, CH₂-CH₂-phenyl), 3.34 (2H, d, J= 7 Hz, CH₂ βTrp), 5.11 (3H, m, CH αTrp and CH₂-m,p-dichlorobenzyl), 6.63 (1 H, dd, Jₒ= 8 Hz and Jₘ= 2 Hz, H₆ m,p-dichlorobenzyl), 6.83 (1 H, t, Jₒ= 8 Hz, H₅ Trp), 6.99 (1 H, t, Jₒ= 8 Hz, H₆ Trp), 7.05 (1 H, d, J= 2 Hz, H₂ Trp), 7.12 (5H, m, CHar phenyl), 7.17 (1 H, d, Jₒ= 8 Hz, H₄ Trp), 7.21 (1 H, s, H₂ m,p-dichlorobenzyl), 7.27 (1 H, d, Jₒ= 8 Hz, H₅ m,p-dichlorobenzyl), 7.39 (1 H, d, Jₒ= 8 Hz, H₇ Trp), 8.02 (3H, brs, NH₂ Aib), 8.94 (1 H, d, J= 8 Hz, NH amide), 10.78 (1 H, s, NH indole Trp).
¹³C NMR (75 MHz, DMSO-d⁶, 300°K):
δ (ppm) 23.5 (CH₃ Aib), 23.8 (CH₃ Aib), 26.4 (CH₂-CH₂-phenyl), 29.1 (CH₂ βTrp), 32.6 (CH₂-CH₂-phenyl), 44.7 (CH₂-*m,p*-dichlorobenzyl), 45.6 (CH α Trp), 56.7 (Cq Aib), 109.7 (C₃ Trp), 111.8 (C₇ Trp), 118.1 (C₄ Trp), 118.7 (C₅ Trp), 121.3 (C₆ Trp), 124.9 (C₂ Trp), 126.5 (C₄ phenyl and C₆ m,p-dichlorobenzyl), 127.2 (C₉ Trp), 128.7 (C₂, C₃, C₅ and C₆ phenyl, C₂ m,p-dichlorobenzyl), 130.9 (C₄ m,p-dichlorobenzyl), 131.4 (C₅ m,p-dichlorobenzyl), 132.0 (C₃ m,p-dichlorobenzyl), 136.4 **(C₈** Trp), 137.2 (C₁ *m,p-*dichlorobenzyl), 140.8 (C₁ phenyl), 154.5 (Cq triazole), 154.7 (Cq triazole), 171.9 (CO amide).

### (R)-N-(1-(4-(4-methylbenzyl)-5-benzyl-4H 1,2,4-triazol-3-yl)-2-(1H-indol-3-yl)ethyl)-2-amino-2-methylpropanamide (Compound 124):

¹H NMR (300 MHz, DMSO-d⁶, 300°K):
δ (ppm) 1.22 (3H, s, CH₃ Aib), 1.27 (3H, s, CH₃ Aib), 2.22 (3H, s, CH₃ *p*-methylbenzyl), 3.22 (1 H, dd, J= 14 Hz and 6 Hz, CH₂ βTrp), 3.33 (1 H, dd, J= 14 Hz and 9 Hz, CH₂ βTrp), 3.99 (2H, s, CH₂-benzyl), 5.04 (2H, s, CH₂-*p*-methylbenzyl), 5.09 (1 H, m, CH αTrp), 6.64 (2H, d, Jₒ= 8 Hz, H₃ and H₅ *p*-methylbenzyl), 6.78 (1 H, t, Jₒ= 7 Hz, H₅ Trp), 6.98 (4H, t, Jₒ= 7 Hz, H₆ Trp, H₃, H₄ and H₅ benzyl), 7.01 (1 H, d, J= 2 Hz, H₂ Trp), 7.07 (2H, d, Jₒ= 7 Hz, H₂ and H₆ *p*-methylbenzyl), 7.20 (3H, m, H₄ Trp, H₂ and H₆ benzyl), 7.26 (1 H, d, Jₒ= 8 Hz, H₇ Trp), 7.98 (3H, brs, NH₂ Aib), 8.89 (1 H, d, J= 8 Hz, NH amide), 10.74 (1 H, s, NH indole Trp).
¹³C NMR (75 MHz, DMSO-d⁶, 300°K):
δ (ppm) 21.0 (CH₃ *p*-methylbenzyl), 23.5 (CH₃ Aib), 23.7 (CH₃ Aib), 29.1 (CH₂ βTrp), 30.6 (CH₂-benzyl), 45.7 (CH αTrp), 46.0 (CH₂-*p*-methylbenzyl), 56.7 (Cq Aib), 109.8 (C₃ Trp), 111.7 (C₇ Trp), 118.3 (C₄ Trp), 118.6 (C₅ Trp), 121.2 (C₆ Trp), 124.8 (C₂ Trp), 126.3 (C₃ and C₅ p-methylbenzyl), 127.0 (C₄ benzyl), 127.2 (C₉ Trp), 128.9 (C₂, C₃, C₅ and C₆ benzyl), 129.7 (C₂ and C₆ p-methylbenzyl), 132.9 (C₁ *p*-methylbenzyl), 136.3 (C₄ p-methylbenzyl), 136.4 (C₈ Trp), 137.4 (C₁ benzyl), 153.9 (Cq triazole), 155.3 (Cq triazole), 171.8 (CO amide).

### (S)-N-(1-(4-(4-methoxybenzyl)-5-(3-phenylpropyl)-4H-1,2,4-triazol-3-yl)-2-(1H-indol-3-yl)ethyl)-2-amino-2-methylpropanamide (Compound 125):

¹H NMR (300 MHz, DMSO-d⁶, 300°K):
δ (ppm) 1.27 (3H, s, CH₃ Aib), 1.31 (3H, s, CH₃ Aib), 1.74 (2H, m, (CH₂-CH₂-CH₂-phenyl), 2.52 (4H, t, J= 7 Hz, CH₂-CH₂-CH₂-phenyl), 3.35 (2H, d, J= 7 Hz, CH₂ βTrp), 3.68 (3H, s, OCH₃), 4.98 (2H, s, CH₂-p- methoxybenzyl), 5.20 (1 H, m, CH αTrp), 6.75 (4H, s, CHar p- methoxybenzyl), 6.83 (1 H, t, Jₒ= 8 Hz, H₅ Trp), 6.99 (1 H, t, Jₒ= 7 Hz, H₆ Trp), 7.06 (3H, m, H₂ and H₆ phenyl, H₂ Trp), 7.14 (1 H, d, Jₒ= 7 Hz, H₄ Trp), 7.19 (3H, t, Jₒ= 8 Hz, H₃, H₄ and H₅ phenyl), 7.29 (1 H, d, Jₒ= 8 Hz, H₇ Trp), 8.01 (3H, brs, NH₂ Aib), 8.94 (1 H, d, J= 8 Hz, NH amide), 10.79 (1 H, d, J= 2 Hz, NH indole Trp).
¹³C NMR (75 MHz, DMSO-d⁶, 300°K):
δ (ppm) 23.6 (CH₃ Aib), 23.8 (CH₃ Aib), 24.1 (CH₂-CH₂-CH₂-phenyl), 28.5 (CH₂-CH₂-CH₂-phenyl), 29.2 (CH₂ βTrp), 34.7 (CH₂-CH₂-CH₂-phenyl), 45.5 (CH₂-p-methoxybenzyl), 45.8 (CH αTrp), 55.5 (OCH₃), 56.8 (Cq Aib),109.8 (C₃ Trp), 111.8 (C₇ Trp), 114.6 (C₃ and C₅ p- methoxybenzyl), 118.3 (C₄ Trp), 118.7 (C₅ Trp), 121.3 (C₆ Trp), 124.9 (C₂ Trp), 126.2 (C₄ phenyl), 127.3 (C₉ Trp), 127.8 (C₁ p- methoxybenzyl), 127.9 (C₃, C₄ and C₅ phenyl), 128.7 (C₂ and C₆ p- methoxybenzyl), 136.4 (C₈ Trp), 141.7 (C₁ phenyl), 154.7 (Cq triazole), 154.8 (Cq triazole), 159.2 (C₁ p-methoxybenzyl), 171.9 (CO amide).

### (S)-N-(1-(4-(4-methoxybenzyl)-5-benzyl-4H-1,2,4-triazol-3-yl)-2-(1H-indol-3-yl)ethyl)-2-amino-2-methylpropanamide (Compound 126):

¹H NMR (300 MHz, DMSO-d⁶, 300°K):
δ (ppm) 1.25 (3H, s, CH₃ Aib), 1.28 (3H, s, CH₃ Aib), 3.26 (1 H, dd, J= 14 Hz and 6 Hz, CH₂ βTrp), 3.34 (1 H, dd, J= 14 Hz and 8 Hz, CH₂ βTrp), 3.99 (2H, s, CH₂-phenyl), 4.98 (2H, s, CH₂-p-methoxybenzyl), 5.13 (1 H, m, CH αTrp), 6.68 (4H, s, CHar p-methoxybenzyl), 6.80 (1 H, t, Jₒ= 8 Hz, H₅ Trp), 6.99 (1 H, t, Jₒ= 8 Hz, H₆ Trp), 7.02-7.06 (4H, m, H₂ and H₄ Trp, H₂ and H₆ phenyl), 7.20 (3H, m, H₃, H₄ and H₅ phenyl), 7.27 (1 H, d, Jₒ= 8 Hz, H₇ Trp), 8.00 (3H, s, NH₂ Aib), 8.91 (1 H, d, J= 8 Hz, NH amide), 10.76 (1 H, s, NH indole Trp).
¹³C NMR (75 MHz, DMSO-d⁶, 300°K):
δ (ppm) 23.5 (CH₃ Aib), 23.7 (CH₃ Aib), 29.1 (CH₂ βTrp), 30.6 (CH₂-phenyl), 45.7 (CH αTrp and CH₂-p-methoxybenzyl), 55.5 (OCH₃), 56.7 (Cq Aib), 109.8 (C₃ Trp), 111.7 (C₇ Trp), 114.5 (C₃ and C₅ *p-methoxybenzyl),* 118.3 (C₄ Trp), 118.7 (C₅ Trp), 121.3 (C₆ Trp), 124.8 (C₂ Trp), 127.1 (C₄ phenyl), 127.3 (C₉ Trp), 127.6 (C₁ *p*-methoxybenzyl), 127.8 (C₂ and C₆ *p-methoxybenzyl),* 128.8 (C₂ and C₆ phenyl), 128.9 (C₃ and C₅ phenyl), 136.3 (C₈ Trp), 136.4 (C₁ phenyl), 153.8 (Cq triazole), 155.2 (Cq triazole), 159.2 (C₄ p- methoxybenzyl), 171.9 (CO amide).

### N-((R)-1-(4-(4-nitrobenzyl)-5-phenethyl-4H-1,2,4-triazol-3-yl)-2-(1H-indol-3-yl)ethyl)-2-amino-2-methylpropanamide (Compound 128):

¹H NMR (300 MHz, DMSO-d⁶, 300°K):
δ (ppm) 1.28 (3H, s, CH₃ Aib), 1.29 (3H, s, CH₃ Aib), 2.77-2.94 (4H, m, CH₂-CH₂-phenyl), 3.28 (1 H, dd, ³J= 14 Hz and 8 Hz, CH₂ βTrp), 3.43 (1 H, dd, ³J= 14 Hz and 7 Hz, CH₂ βTrp), 5.05 (1 H, m, CH αTrp), 5.25 (2H, d, J= 7 Hz, CH₂-p-nitrobenzyl), 6.72 (1 H, t, Jₒ= 7 Hz, H₅ Trp), 6.89 (2H, d, Jₒ= 9 Hz, H₂ and H₆ p-nitrobenzyl), 6.92 (1 H, t, Jₒ= 7 Hz, H₆ Trp), 7.00 (1 H, d, Jₘ= 2 Hz, H₂ Trp), 7.08-7.15 (4H, m, H₄ and H₇ Trp, H₂ and H₆ phenyl), 7.17 (2H, Jₒ= 7 hz, H₃ and H₅ phenyl), 7.24 (1 H, t, Jₒ= 8 Hz, H₄ phenyl), 7.92 (2H, d, Jₒ= 9 Hz, H₃ and H₅ *p*-nitrobenzyl), 8.06 (3H, brs, NH₂ Aib), 8.98 (1 H, d, J= 8 Hz, NH amide), 10.79 (1 H, s, NH indole Trp).
¹³C NMR (75 MHz, DMSO-d⁶, 300°K):
δ (ppm) 23.5 (CH₃ Aib), 23.8 (CH₃ Aib), 26.4 (CH₂-CH₂-phenyl), 29.1 (CH₂ βTrp), 32.6 (CH₂-CH₂-phenyl), 45.3 (CH₂-p-nitrobenzyl), 45.7 (CH αTrp), 56.8 (Cq Aib), 109.6 (C₃ Trp), 111.8 (C₇ Trp), 118.1 (C₄ Trp), 118.5 (C₅ Trp), 121.2 (C₆ Trp), 124.1 (C₂ and C₅ p-nitrobenzyl), 124.8 (C₂ Trp), 126.5 (C₂ and C₅ phenyl), 127.2 (C₉ Trp, C₁ and C₆ *p-*nitrobenzyl), 128.7 (C₃, C₄ and C₅ phenyl), 136.4 (C₈ Trp), 140.8 (C₁ phenyl), 143.5 (C₁ *p*-nitrobenzyl), 154.5 (Cq triazole), 154.8 (Cq triazole), 172.0 (CO Aib).

### (S)-N-(1-(4-(4-methoxybenzyl)-5-phenethyl-4H-1,2,4-triazol-3-yl)-2-(1H-indol-3-yl)ethyl)-2-amino-2-methylpropanamide (Compound 129):

¹H NMR (300 MHz, DMSO-d⁶, 300°K):
δ (ppm) 1.27 (3H, s, CH₃ Aib), 1.30 (3H, s, CH₃ Aib), 2.81 (4H, m, CH₂-CH₂-pheny!), 3.34 (2H, d, J= 7 Hz, CH₂ βTrp), 3.68 (3H, s, OCH₃), 4.99 (2H, s, CH₂-p-methoxybenzyl), 5.20 (1 H, m, CH αTrp), 6.75 (4H, m, CHar *p*- methoxybenzyl), 6.83 (1 H, t, Jₒ= 7 Hz, H₅ Trp), 7.03 (1 H, t, Jₒ= 8 Hz, H₆ Trp), 7.04 (1 H, d, J= 2 Hz, H₂ Trp), 7.10 (2H, d, Jₒ= 7 Hz, H₂ and H₆ phenyl), 7.13 (1 H, d, Jₒ= 8 Hz, H₄ Trp), 7.19 (3H, t, Jₒ= 7 Hz, H₃, H₄ and H₅ phenyl), 7.29 (1 H, d, Jₒ= 8 Hz, H₇ Trp), 8.01 (3H, brs, NH₂ Aib), 8.94 (1 H, d, J= 8 Hz, NH amide), 10.78 (1 H, d, J= 2 Hz, NH indole Trp).
¹³C NMR (75 MHz, DMSO-d⁶, 300°K):
δ (ppm) 23.6 (CH₃ Aib), 23.8 (CH₃ Aib), 26.6 (CH₂-CH₂-phenyl), 29.2 (CH₂ βTrp), 32.7 (CH₂-CH₂-phenyl), 45.3 (CH₂-p- methoxybenzyl), 45.7 (CH αTrp), 55.5 (OCH₃), 56.8 (Cq Aib), 109.9 (C₃ Trp), 111.8 (C₇ Trp), 114.6 (C₃ and C₅ p- methoxybenzyl), 118.3 (C₄ Trp), 118.7 (C₅ Trp), 121.3 (C₆ Trp), 124.8 (C₂ Trp), 126.5 (C₄ phenyl), 127.3 (C₉ Trp and C₁ p- methoxybenzyl), 127.9 (C₂ and C₆ p- methoxybenzyl), 128.7 (C₂, C₃, C₅ and C₆ p- methoxybenzyl), 136.5 (C₈ Trp), 140.9 (C₁ phenyl), 154.4 (Cq triazole), 154.7 (Cq triazole), 159.2 (C₄ *p*- methoxybenzyl), 171.9 (CO amide).

### (R)-N-(1-(4-(4-methoxyphenethyl)-5-phenethyl-4H-1,2,4-triazol-3-yl)-2-(1H-indol-3-yl)ethyl)-2-amino-2-methylpropanamide (Compound 130):

¹H NMR (300 MHz, DMSO-d⁶, 300°K):
δ (ppm) 1.30 (3H, s, CH₃ Aib), 1.35 (3H, s, CH₃ Aib), 2.55 (4H, m, CH₂-CH₂-phenyl and CH₂-CH₂-p-methoxybenzyl), 2.83 (2H, t, J= 8 Hz, CH₂-CH₂-phenyl), 3.37 (2H, m, CH₂-*CH₂-p-methoxybenzyl),* 3.65 (3H, s, OCH₃), 3.77 (1 H, m, CH₂ βTrp), 3.89 (1 H, m, CH₂ βTrp), 5.20 (1 H, m, CH αTrp), 6.72 (4H, s, CHar p-methoxybenzyl), 6.94 (1 H, t, Jₒ= 7 Hz, H₅ Trp), 7.02 (1 H, t, Jₒ= 8 Hz, H₆ Trp), 7.05 (1 H, d, J= 2 Hz, H₂ Trp), 7.11 (2H, d, Jₒ= 7 Hz, H₂ and H₆ phenyl), 7.16 (1 H, d, Jₒ= 7 Hz, H₄ Trp), 7.25 (3H m, H₃, H₄, H₅ phenyl), 7.50 (1 H, d, Jₒ= 8 Hz, H₇ Trp), 8.05 (3H, brs, NH₂ Aib), 9.02 (1 H, d, J= 8 hz, NH amide), 10.83 (1 H, d, J= 2 Hz, NH indole Trp).
¹³C NMR (75 MHz, DMSO-d⁶, 300°K):
δ (ppm) 23.5 (CH₃ Aib), 23.8 (CH₃ Aib), 26.0 (CH₂-CH₂-phenyl), 29.5 (CH₂ βTrp), 32.5 (CH₂-CH₂-phenyl), 35.0 (CH₂-CH₂-p- methoxybenzyl), 44.4 (CH₂-CH₂-p-methoxybenzyl), 45.8 (CH αTrp), 55.4 (OCH₃), 56.8 (Cq Aib), 109.9 (C₃ Trp), 111.9 (C₇ Trp), 114.2 (C₃ and C₅ p- methoxybenzyl), 118.4 (C₄ Trp), 118.9 (C₅ Trp), 121.4 (C₆ Trp), 124.7 (C₂ Trp), 126.5 (C₄ phenyl), 127.4 (C₉ Trp), 128.7 (C₂, C₃, C₅ and C₆ phenyl), 129.4 (C₁ p- methoxybenzyl), 130.3 (C₂ and C₆ p- methoxybenzyl), 136.5 (C₈ Trp), 141.0 (C₁ phenyl), 154.0 (Cq triazole), 154.5 (Cq triazole), 158.6 (C₄ p-methoxybenzyl), 171.8 (CO amide).

### (R)-N-(2-(1H-indol-3-yl)-1-(5-phenethyl-4-(pyridin-2-ylmethyl)-4H-1,2,4-triazol-3-yl)ethyl)-2-amino-2-methylpropanamide (Compound 132):

¹H NMR (300 MHz, DMSO-d⁶, 300°K):
δ (ppm) 1.26 (3H, s, CH₃ Aib), 1.29 (3H, s, CH₃ Aib), 2.95 (4H, m, CH₂-CH₂-phenyl), 3.40 (2H, m, CH₂ βTrp), 5.26 (1 H, m, CH αTrp), 5.37 (2H, s, CH₂-*o*-pyridyl), 6.83 (1 H, t, Jₒ= 7 Hz, H₅ Trp), 6.98 (1 H, t, Jₒ= 8 Hz, H₆ Trp), 7.11-7.30 (10H, m, H₂, H₄ and H₇ Trp, CHar phenyl, H₃ and H₅ o-pyridyl), 7.71 (1 H, t, Jₒ= 7 Hz, H₄ o-pyridyl), 8.22 (3H, brs, NH₂ Aib), 8.42 (1 H, d, J_{αβ}= 4 Hz, H₆ o-pyridyl), 9.05 (1 H, d, J= 8 Hz, NH amide), 10.87 (1 H, s, NH indole Trp).
¹³C NMR (75 MHz, DMSO-d⁶, 300°K):
δ (ppm) 23.4 (CH₃ Aib), 23.7 (CH₃ Aib), 26.4 (CH₂-CH₂-phenyl), 28.6 (CH₂ βTrp), 32.5 (CH₂-CH₂-phenyl), 45.7 (CH αTrp), 47.6 (CH₂ o-pyridyl), 56.8 (Cq Aib), 109.8 (C₃ Trp), 111.8 (C₇ Trp), 118.3 (C₄ Trp), 118.6 (C₅ Trp), 121.2 (C₆ Trp), 122.0 (C₃ o-pyridyl), 123.6 (C₅ o-pyridyl), 126.3 (C₂ Trp), 126.6 (C₄ phenyl), 127.3 (Cg Trp), 128.7 (C₂, C₃, C₅ and C₆ phenyl), 136.4 (C₈ trypophane), 137.7 (C₄ o-pyridyl), 140.7 (C₁ phenyl), 150.1 (C₆ o-pyridyl), 154.9 (Cq triazole), 155.2 (Cq triazole), 158.7 (C₂ o-pyridyl), 172.0 (CO amide).

### N-((R)-1-(4-(2,4-dimethoxybenzyl)-5-phenethyl-4H-1,2,4-triazol-3-yl)-2-(1H-indol-3-yl)ethyl)-2-amino-2-methylpropanamide (Compound 179):

¹H NMR (300 MHz, DMSO d⁶, 300°K):
δ (ppm) 1.26 (s, 3H, CH₃ Aib), 1.30 (s, 3H, CH₃ Aib), 2.82 (m, 4H, CH₂-CH₂-phenyl), 3.29 (t, 2H, J= 8 Hz, CH₂ βTrp), 3.61 (s, 3H, OCH₃), 3.68 (s, 3H, OCH₃), 4.85 (d, 1 H, J= 17 Hz, CH₂-o,p-dimethoxybenzyl), 5.02 (d, 1H, J= 17 Hz, CH₂-o,p-dimethoxybenzyl), 5.18 (m, 1 H, CH αTrp), 6.29 (dd, 1 H, Jₒ= 8 Hz and Jₘ= 2 Hz, H₅ *o,p*-dimethoxybenzyl), 6.40 (d, 1 H, Jₒ= 8 Hz, H₆ *o,p-*dimethoxybenzyl), 6.55 (d, 1 H, Jₘ= 2 Hz, H₃ o,p-dimethoxybenzyl), 6.82 (t, 1 H, Jₒ= 8 Hz, H₅ Trp), 6.99 (t, 1 H, Jₒ= 8 Hz, H₆ Trp), 7.05 (s, 1 H, H₂ Trp), 7.09-7.24 (m, 6H, H₄ Trp and CHar phenyl), 7.27 (d, 1 H, Jₒ= 8 Hz, H₇ Trp), 7.99 (s, 3H, large, NH₂ Aib TFA salt), 8.89 (d, 1 H, J= 8 Hz, NH amide), 10.77 (s, 1 H, NH indole Trp).
¹³C NMR (75 MHz, DMSO d⁶, 300°K):
δ (ppm) 23.6 (CH₃ Aib), 23.7 (CH₃ Aib), 26.5 (CH₂-CH₂-phenyl), 29.2 (CH₂ βTrp), 32.7 (CH₂-CH₂-phenyl), 41.6 (CH₂-*o,p-*dimethoxybenzyl), 45.7 (CH αTrp), 55.7 (OCH₃), 55.9 (OCH₃), 56.7 (Cq Aib), 99.1 (C₃ *o,p*-dimethoxybenzyl), 105.2 (C₅ *o,p*-dimethoxybenzyl), 110.0 (C₃ Trp), 111.8 (C₇ Trp), 115.7 (C₁ o,p-dimethoxybenzyl), 118.3 (C₄ Trp), 118.7 (C₅ Trp), 121.3 (C₆ Trp), 126.5 (C₂ Trp and C₆ *o,p*-dimethoxybenzyl), 127.3 (C₉ Trp), 128.1 (C₄ phenyl), 128.7 (C₂, C₃, C₅ and C₆ phenyl), 136.4 (C₈ Trp), 140.9 (C₁ phenyl), 154.5 (Cq triazole), 155.0 (Cq triazole), 157.8 (C₂ *o,p*-dimethoxybenzyl), 160.9 (C₄ o,p-dimethoxybenzyl), 171.7 (CO amide).

### N-((R)-1-(4-(4-methoxybenzyl)-5-phenethyl-4H-1,2,4-triazol-3-yl)-2-(1H-indol-3-yl)ethyl)-tetrahydro-2H-pyran-4-carboxamide (Compound 184):

¹H NMR (300 MHz, DMSO d⁶, 300°K):
δ (ppm) 1.20 (m, 1H, H₅ tetrahydropyrane), 1.30 (m, 3H, H₃ and H₅ tetrahydropyrane), 2.17 (m, 1 H, H₄ tetrahydropyrane), 2.82 (m, 4H, CH₂-CH₂-phenyl), 3.16 (m, 2H, H₂ and H₆ tetrahydropyrane), 3.31 (dd, 1 H, J= 14 Hz and 8 Hz, CH₂ βTrp), 3.35 (dd, 1 H, J= 14 Hz and 7 Hz, CH₂ βTrp), 3.66 (s, 3H, OCH₃), 3.72 (m, 2H, H₂ and H₆ tetrahydropyrane), 5.08 (m, 2H, CH₂-p-methoxybenzyl), 5.26 (m, 1 H, CH αTrp), 6.73 (s, 4H, CHar p-methoxybenzyl), 6.87 (t, 1 H, Jₒ= 8 Hz, H₅ Trp), 7.02 (m, 2H, H₂ and H₆ Trp), 7.07 (d, 2H, Jₒ= 7 Hz, H₂ and H₆ phenyl), 7.18 (m, 3H, H₃, H₄ and H₅ phenyl), 7.30 (m, 2H, H₄ and H₇ Trp), 8.52 (d, 1 H, J= 8 Hz, NH amide), 10.77 (s, 1 H, NH indole Trp).
¹³C NMR (75 MHz, DMSO d⁶, 300°K):
δ (ppm) 26.3 (CH₂-CH₂-phenyl), 28.8 (C₃ and C₅ tetrahydropyrane), 29.2 (CH₂ βTrp), 32.2 (CH₂-CH₂-phenyl), 40.7 (C₄ tetrahydropyrane), 44.8 (CH αTrp), 45.9 (CH₂-*p-*methoxybenzyl), 55.5 (OCH₃), 66.6 (C₂ and C₆ tetrahydropyrane), 109.8 (C₃ Trp), 111.7 (C₇ Trp), 114.5 (C₃ and C₅ p-methoxybenzyl), 118.4 (C₄ Trp), 118.7 (C₅ Trp), 121.3 (C₆ Trp), 124.5 (C₂ Trp), 126.7 (C₄ phenyl), 127.2 (C₉ Trp), 127.5 (C₁ *p*-methoxybenzyl), 128.8 (C₂ and C₆ phenyl), 128.7 (C₃ and C₅ phenyl), 127.9 (C₂ and C₆ p-methoxybenzyl), 136.4 (C₈ Trp), 140.4 (C₁ phenyl), 154.7 (Cq triazole), 155.7 (Cq triazole), 159.2 (C₄ p-methoxybenzyl), 174.2 (CO amide).

### N-((R)-1-(5-((1H indol-3-yl)methyl)-4-(3-methoxybenzyl)-4H-1,2,4-triazol-3-yl)-2-(1H-indol-3-yl)ethyl)-2-amino-2-methylpropanamide (Compound 185):

¹H NMR (300 MHz, DMSO d⁶, 300 °K):
δ (ppm) 1.19 (s, 3H, CH₃ Aib), 1.23 (s, 3H, CH₃ Aib), 3.14 (dd, 1 H, J= 14 Hz and 5 Hz, CH₂ βTrp), 3.34 (dd, 1 H, J= 14 Hz and 9 Hz, CH₂ βTrp), 3.46 (s, 3H, OCH₃), 3.72 (m, 2H, CH₂-indole), 5.06 (m, 1H, CH αTrp), 5.14 (m, 2H, CH₂-m-methoxybenzyl), 6.31 (s, 1H, H₂ m-methoxybenzyl), 6.32 (d, 1H, Jₒ= 7 Hz, H₆ m-methoxybenzyl), 6.77 (m, 3H, H₅ indole, H₅ Trp and H₄ m-methoxybenzyl), 6.92 (m, 2H, H₆ indole and H₆ Trp), 7.02 (m, 2H, H₂ indole and H₂ Trp), 7.26-7.30 (m, 3H, H₅ *m*-methoxybenzyl, H₄ and H₇ indole, H₄ Trp), 7.41 (d, 1 H, Jₒ= 8 Hz, H₇ Trp), 7.94 (brs, 3H, NH₂ Aib TFA salt), 8.87 (d, 1 H, J₌ 8 Hz, NH amide), 10.73 (d, 1 H, J= 2 Hz, NH indole), 10.85 (s, 1 H, NH indole Trp).
¹³C NMR (75 MHz, DMSO d⁶, 300°K):
δ (ppm) 21.7 (CH₂-indole), 23.5 (CH₃ Aib), 23.7 (CH₃ Aib), 28.9 (CH₂ βTrp), 45.5 (CH αTrp), 46.2 (CH₂-*m*-methoxybenzyl), 55.2 (OCH₃), 56.7 (Cq Aib), 108.2 (C₃ indole), 109.8 (C₃ Trp), 111.7 (C₇ Trp), 111.9 (C₇ indole and C₂ m-methoxybenzyl), 113.7 (C₄ m-methoxybenzyl), 118.2 (C₆ m-methoxybenzyl), 118.4 (C₄ Trp), 118.6 (C₄ indole), 118.9 (C₅ indole and C₅ Trp), 121.2 (C₆ indole), 121.6 (C₆ Trp), 127.1 (C₉ indole), 127.2 (C₉ Trp), 136.4 (C₈ Trp), 136.7 (C₈ indole), 137.5 (C₁ m-methoxybenzyl), 154.2 (Cq triazole), 155.3 (Cq triazole), 160.0 (C₃ m-methoxybenzyl), 171.8 (CO amide).

**Table 1: Further exemplary embodiments with synthetic sequence and MS data (compounds no. 2, 3,14,16,17,19-22, 24, 26-35, 36-122, 124-126, 128-150, 152-190):**

| **No.** | **Chemical name (Chem Draw Ultra 8)** | **ESI-MS** (calculated) | **ESI-MS** [found (M+H)⁺] |
|---|---|---|---|
| **2** | (R)-N-(1-(5-(2-(1 H-indol-3-yl)ethyl)-4-phenethyl-4H-1,2,4-triazol-3-yl)-2-(1H-indol-3-yl)ethyl)-2-amino-2-methylpropanamide | 559.3 | 560.4 |
| **3** | (R)-N-(1-(5-(3-(1H-indol-3-yl)propyl)-4-phenethyl-4H-1,2,4-triazol-3-yl)-2-(1H-indol-3-yl)ethyl)-2-amino-2-methylpropanamide | 573.3 | 574.3 |
| **14** | (R)-N-(1-(5-(3-(1H-indol-3-yl)propyl)-4-(4-bromobenzyl)-4H-1,2,4-triazol-3-yl)-2-(1 H-indol-3-yl)ethyl)-2-amino-2-methylpropanamide | 637.2 | 638.1 |
| **16** | (R)-N-(1-(5-(3-(1H-indol-3-yl)propyl)-4-hexyl-4H-1,2,4-triazol-3-yl)-2-(1H-indol-3-yl)ethyl)-2-amino-2-methylpropanamide | 553.3 | 554.4 |
| **17** | (R)-N-(1-(4,5-bis(2-(1H-indol-3-yl)ethyl)-4H-1,2,4-triazol-3-yl)-2-(1H-indol-3-yl)ethyl)-2-amino-2-methylpropanamide | 598.3 | 599.2 |
| **19** | (R)-N-(1-(4-(3-methoxybenzyl)-5-phenethyl-4H-1,2,4-triazol-3-yl)-2-(1H-indol-3-yl)ethyl)-2-amino-2-methylpropanamide | 536.3 | 537.1 |
| **20** | (R)-N-(1-(4-(4-methoxybenzyl)-5-phenethyl-4H-1,2,4-triazol-3-yl)-2-(1H-indol-3-yl)ethyl)-2-amino-2-methylpropanamide | 536.3 | 537.3 |
| **21** | (R)-N-(1-(5-(2-(1H-indol-3-yl)ethyl)-4-(3,5-dimethoxybenzyl)-4H-1,2,4-triazol-3-yl)-2-(1H-indol-3-yl)ethyl)-2-amino-2-methylpropanamide | 605.3 | 606.4 |
| **22** | (R)-N-(1-(4-(4-methoxybenzyl)-5-(3-phenylpropyl)-4H-1,2,4-triazol-3-yl)-2-(1 H-indol-3-yl)ethyl)-2-amino-2-methylpropanamide | 550.3 | 551.3 |
| **24** | (R)-N-(1-(4-(2-(1H-indol-3-yl)ethyl)-5-(3-(1H-indol-3-yl)propyl)-4H-1,2,4-triazol-3-yl)-2-(1H-indol-3-yl)ethyl)-2-amino-2-methylpropanamide | 612.3 | 612.8 |
| **26** | (R)-N-(1-(4-(2-methoxybenzyl)-5-phenethyl-4H-1,2,4-triazol-3-yl)-2-(1H-indol-3-yl)ethyl)-2-amino-2-methylpropanamide | 536.3 | 537.1 |
| **27** | (R)-N-(2-(1H-indol-3-yl)-1-(4-(naphthalen-1-ylmethyl)-5-phenethyl-4H-1,2,4-triazol-3-yl)ethyl)-2-amino-2-methylpropanamide | 556.3 | 557.2 |
| **28** | (R)-N-(1-(5-(2-(1H-indol-3-yl)ethyl)-4-(3,4-dichlorobenzyl)-4H-1,2,4-triazol-3-yl)-2-(1H-indol-3-yl)ethyl)-2-amino-2-methylpropanamide | 613.2 | 614.2 |
| **29** | (R)-N-(1-(5-(2-(1H-indol-3-yl)ethyl)-4-(4-fluorobenzyl)-4H-1,2,4-triazol-3-yl)-2-(1H-indol-3-yl)ethyl)-2-amino-2-methylpropanamide | 563.3 | 564.1 |
| **30** | (R)-N-(1-(4-(4-fluorobenzyl)-5-benzyl-4H-1,2,4-triazol-3-yl)-2-(1H-indol-3-yl)ethyl)-2-amino-2-methylpropanamide | 510.3 | 511.0 |
| **31** | (R)-N-(1-(5-(2-(1H-indol-3-yl)ethyl)-4-(2,4-dimethoxybenzyl)-4H-1,2,4-triazol-3-yl)-2-(1H-indol-3-yl)ethyl)piperidine-4-carboxamide | 631.3 | 632.0 |
| **32** | (R)-N-(1-(5-(2-(1H-indol-3-yl)ethyl)-4-(2,4-dimethoxybenzyl)-4H-1,2,4-triazol-3-yl)-2-(1H-indol-3-yl)ethyl)piperidine-3-carboxamide | 631.3 | 631.8 |
| **33** | (R)-N-(1-(4-(4-methylbenzyl)-5-(3-phenylpropyl)-4H-1,2,4-triazol-3-yl)-2-(1H-indol-3-yl)ethyl)-2-amino-2-methylpropanamide | 534.3 | 535.4 |
| **34** | (R)-N-(1-(5-(2-(1H-indol-3-yl)ethyl)-4-(4-methylbenzyl)-4H-1,2,4-triazol-3-yl)-2-(1H-indol-3-yl)ethyl)-2-amino-2-methylpropanamide | 559.7 | 559.9 |
| **36** | (R)-N-(1-(5-(2-(1H-indol-3-yl)ethyl)-4-(2,4-dimethoxybenzyl)-4H-1,2,4-triazol-3-yl)-2-(1H-indol-3-yl)ethyl)piperidine-2-carboxamide | 631.3 | 631.8 |
| **37** | (R)-N-(1-(4-(4-methylbenzyl)-5-phenethyl-4H-1,2,4-triazol-3-yl)-2-(1 H-indol-3-yl)ethyl)-2-amino-2-methylpropanamide | 520.3 | 521.0 |
| **38** | (R)-N-(1-(5-(2-(1H-indol-3-yl)ethyl)-4-(2,4-dimethoxybenzyl)-4H-1,2,4-triazol-3-yl)-2-(1H-indol-3-yl)ethyl)-2-aminobenzamide | 639.3 | 639.8 |
| **39** | (R)-N-(1-(5-benzyl-4-(□yridine-2-ylmethyl)- 4H-1,2,4-triazol-3-yl)-2-(1H-indol-3-yl)ethyl)-2-amino-2-methylpropanamide | 493.3 | 493.9 |
| **40** | (2S,4R)-N-((R)-1-(4-(4-methoxybenzyl)-5-phenethyl-4H-1,2,4-triazol-3-yl)-2-(1H-indol-3-yl)ethyl)-4-hydroxypyrrolidine-2-carboxamide | 564.3 | 565.0 |
| **41** | (S)-N-((R)-1-(4-(4-methoxybenzyl)-5-phenethyl-4H-1,2,4-triazol-3-yl)-2-(1H-indol-3-yl)ethyl)piperidine-3-carboxamide | 562.3 | 563.0 |
| **42** | (R)-N-((R)-1-(4-(4-methoxybenzyl)-5-phenethyl-4H-1,2,4-triazol-3-yl)-2-(1H-indol-3-yl)ethyl)piperidine-3-carboxamide | 562.3 | 562.9 |
| **43** | (R)-N-(1-(4-(4-ethylbenzyl)-5-phenethyl-4H-1,2,4-triazol-3-yl)-2-(1H-indol-3-yl)ethyl)-2-amino-2-methylpropanamide | 534.3 | 535.0 |
| **44** | (R)-N-(1-(4-(4-methoxybenzyl)-5-phenethyl-4H-1,2,4-triazol-3-yl)-2-(1H-indol-3-yl)ethyl)piperidine-4-carboxamide | 562.3 | 563.0 |
| **45** | (R)-N-(1-(5-(2-(1H-indol-3-yl)ethyl)-4-(4-methoxybenzyl)-4H-1,2,4-triazol-3-yl)-2-(1H-indol-3-yl)ethyl)piperidine-4-carboxamide | 601.3 | 602.0 |
| **46** | (S)-N-((R)-1-(4-(4-methoxybenzyl)-5-phenethyl-4H-1,2,4-triazol-3-yl)-2-(1H-indol-3-yl)ethyl)pyrrolidine-2-carboxamide | 548.3 | 548.9 |
| **47** | (R)-N-((R)-1-(4-(4-methoxybenzyl)-5-phenethyl-4H-1,2,4-triazol-3-yl)-2-(1H-indol-3-yl)ethyl)pyrrolidine-2-carboxamide | 548.3 | 548.9 |
| **48** | (S)-N-((R)-1-(4-(4-methoxybenzyl)-5-phenethyl-4H-1,2,4-triazol-3-yl)-2-(1H-indol-3-yl)ethyl)piperidine-2-carboxamide | 562.3 | 563.0 |
| **49** | (R)-N-((R)-1-(4-(4-methoxybenzyl)-5-phenethyl-4H-1,2,4-triazol-3-yl)-2-(1H-indol-3-yl)ethyl)piperidine-2-carboxamide | 562.3 | 562.9 |
| **50** | (R)-N-(1-(4-(4-methoxybenzyl)-5-phenethyl-4H-1,2,4-triazol-3-yl)-2-(1H-indol-3-yl)ethyl)-2-aminoacetamide | 508.3 | 508.9 |
| **51** | (R)-N-(1-(4-(4-methoxybenzyl)-5-phenethyl-4H-1,2,4-triazol-3-yl)-2-(1H-indol-3-yl)ethyl)-2-(□yridine-2-yl)acetamide | 570.3 | 571.2 |
| **52** | N-((R)-1-(4-(4-methoxybenzyl)-5-phenethyl-4H-1,2,4-triazol-3-yl)-2-(1H-indol-3-yl)ethyl)-2-(□yridine-4-yl)acetamide | 570.3 | 570.9 |
| **53** | (R)-N-(1-(4-(4-methoxybenzyl)-5-phenethyl-4H-1,2,4-triazol-3-yl)-2-(1H-indol-3-yl)ethyl)cyclohexanecarboxamide | 561.3 | 562.4 |
| **54** | (R)-N-(1-(5-(2-(1H-indol-3-yl)ethyl)-4-benzyl-4H-1,2,4-triazol-3-yl)-2-(1H-indol-3-yl)ethyl)piperidine-4-carboxamide | 571.3 | 572.5 |
| **55** | (R)-N-(1-(5-(2-(1H-indol-3-yl)ethyl)-4-benzyl-4H-1,2,4-triazol-3-yl)-2-(1H-indol-3-yl)ethyl)piperidine-3-carboxamide | 571.3 | 572.4 |
| **56** | (R)-N-(1-(4-(4-methoxybenzyl)-5-phenethyl-4H-1,2,4-triazol-3-yl)-2-(1H-indol-3-yl)ethyl)-3-aminopropanamide | 522.3 | 523.3 |
| **57** | (S)-N-((R)-1-(4-(4-methoxybenzyl)-5-phenethyl-4H-1,2,4-triazol-3-yl)-2-(1H-indol-3-yl)ethyl)-2-aminopropanamide | 522.3 | 523.3 |
| **58** | (R)-N-(1-(4-(4-methoxybenzyl)-5-phenethyl-4H-1,2,4-triazol-3-yl)-2-(1H-indol-3-yl)ethyl)-2-(pyridin-3-yl)acetamide | 570.3 | 571.2 |
| **59** | (R)-N-(1-(4-(4-methoxybenzyl)-5-phenethyl-4H-1,2,4-triazol-3-yl)-2-(1H-indol-3-yl)ethyl)-3-(pyridin-3-yl)propanamide | 584.3 | 585.3 |
| **60** | (R)-N-(1-(5-(2-(1H-indol-3-yl)ethyl)-4-benzyl-4H-1,2,4-triazol-3-yl)-2-(1H-indol-3-yl)ethyl)-2-(pyridin-2-yl)acetamide | 579.3 | 580.2 |
| **61** | (R)-N-(1-(5-(2-(1H-indol-3-yl)ethyl)-4-(2,4-dimethoxybenzyl)-4H-1,2,4-triazol-3-yl)-2-(1 H-indol-3-yl)ethyl)-2-(pyridin-2-yl)acetamide | 639.3 | 640.5 |
| **62** | (R)-N-(1-(4-(2,4-dimethoxybenzyl)-5-phenethyl-4H-1,2,4-triazol-3-yl)-2-(1 H-indol-3-yl)ethyl)piperidine-4-carboxamide | 592.3 | 593.3 |
| **63** | (R)-N-((R)-1-(4-(2,4-dimethoxybenzyl)-5-phenethyl-4H-1,2,4-triazol-3-yl)-2-(1 H-indol-3-yl)ethyl)piperidine-2-carboxamide | 592.3 | 593.3 |
| **64** | (R)-N-(1-(4-(2,4-dimethoxybenzyl)-5-phenethyl-4H-1,2,4-triazol-3-yl)-2-(1H-indol-3-yl)ethyl)picolinamide | 586.3 | 587.2 |
| **65** | (R)-N-(1-(4-(2,4-dimethoxybenzyl)-5-phenethyl-4H-1,2,4-triazol-3-yl)-2-(1H-indol-3-yl)ethyl)isonicotinamide | 586.3 | 587.2 |
| **66** | (R)-N-(1-(4-(2,4-dimethoxybenzyl)-5-phenethyl-4H-1,2,4-triazol-3-yl)-2-(1H-indol-3-yl)ethyl)pyrazine-2-carboxamide | 587.3 | 588.2 |
| **67** | (R)-N-1-(4-(2,4-dimethoxybenzyl)-5-phenethyl-4H-1,2,4-triazol-3-yl)-2-(1H-indol-3-yl)ethyl)piperazine-2-carboxamide | 593.3 | 594.2 |
| **68** | (S)-N-((R)-1-(4-(2,4-dimethoxybenzyl)-5-phenethyl-4H-1,2,4-triazol-3-yl)-2-(1H-indol-3-yl)ethyl)pyrrolidine-2-carboxamide | 578.3 | 579.4 |
| **69** | (R)-N-(1-(5-(2-(1H-indol-3-yl)ethyl)-4-(2,4-dimethoxybenzyl)-4H-1,2,4-triazol-3-yl)-2-(1 H-indol-3-yl)ethyl)-2-aminoacetamide | 577.3 | 578.0 |
| **70** | (S)-N-((R)-1-(5-(2-(1H-indol-3-yl)ethyl)-4-(2,4-dimethoxybenzyl)-4H-1,2,4-triazol-3-yl)-2-(1H-indol-3-yl)ethyl)pyrrolidine-2-carboxamide | 617.3 | 618.0 |
| **71** | (R)-N-(1-(5-(2-(1 H-indol-3-yl)ethyl)-4-(2,4-dimethoxybenzyl)-4H-1,2,4-triazol-3-yl)-2-(1 H-indol-3-yl)ethyl)pyrazine-2-carboxamide | 626.3 | 627.2 |
| **72** | (R)-N-1-(5-(2-(1H-indol-3-yl)ethyl)-4-(2,4-dimethoxybenzyl)-4H-1,2,4-triazol-3-yl)-2-(1H-indol-3-yl)ethyl)piperazine-2-carboxamide | 632.3 | 633.2 |
| **73** | (R)-N-(1-(5-(2-(1H-indol-3-yl)ethyl)-4-(2,4-dimethoxybenzyl)-4H-1,2,4-triazol-3-yl)-2-(1H-indol-3-yl)ethyl)picolinamide | 625.3 | 626.3 |
| **74** | (R)-1-(5-(2-(1H-indol-3-yl)ethyl)-4-(2,4-dimethoxybenzyl)-4H-1,2,4-triazol-3-yl)-2-(1H-indol-3-yl)ethanamine | 520.3 | 520.8 |
| **75** | (R)-N-(1-(4-(2,4-dimethoxybenzyl)-5-phenethyl-4H-1,2,4-triazol-3-yl)-2-(1H-indol-3-yl)ethyl)-2-aminoacetamide | 538.3 | 539.3 |
| **76** | (R)-N-(1-(4-(4-methoxybenzyl)-5-phenethyl-4H-1,2,4-triazol-3-yl)-2-(1H-indol-3-yl)ethyl)pyrazine-2-carboxamide | 557.3 | 558.0 |
| **77** | (R)-N-(1-(4-(4-methoxybenzyl)-5-phenethyl-4H-1,2,4-triazol-3-yl)-2-(1H-indol-3-yl)ethyl)isonicotinamide | 556.3 | 556.9 |
| **78** | (R)-N-1-(4-(4-methoxybenzyl)-5-phenethyl-4H-1,2,4-triazol-3-yl)-2-(1H-indol-3-yl)ethyl)piperazine-2-carboxamide | 563.3 | 564.0 |
| **79** | (R)-N-(1-(4-(4-methoxybenzyl)-5-phenethyl-4H-1,2,4-triazol-3-yl)-2-(1H-indol-3-yl)ethyl)picolinamide | 556.3 | 557.3 |
| **80** | (R)-N-(1-(5-(2-(1H-indol-3-yl)ethyl)-4-(4-methoxybenzyl)-4H-1,2,4-triazol-3-yl)-2-(1H-indol-3-yl)ethyl)picolinamide | 595.3 | 596.2 |
| **81** | (R)-N-1-(5-(2-(1H-indol-3-yl)ethyl)-4-(4-methoxybenzyl)-4H-1,2,4-triazol-3-yl)-2-(1 H-indol-3-yl)ethyl)piperazine-2-carboxamide | 602.3 | 603.3 |
| **82** | (R)-N-(1-(4-(4-ethylbenzyl)-5-phenethyl-4H-1,2,4-triazol-3-yl)-2-(1H-indol-3-yl)ethyl)-2-(Dyridine-2-yl)acetamide | 568.3 | 569.3 |
| **83** | (R)-N-(1-(4-(4-ethylbenzyl)-5-phenethyl-4H-1,2,4-triazol-3-yl)-2-(1H-indol-3-yl)ethyl)piperidine-4-carboxamide | 560.3 | 561.3 |
| **84** | (R)-N-1 -(4-(4-ethylbenzyl)-5-phenethyl-4H-1,2,4-triazol-3-yl)-2-(1H-indol-3-yl)ethyl)piperazine-2-carboxamide | 561.3 | 562.2 |
| **85** | (R)-N-(1-(4-(4-ethylbenzyl)-5-phenethyl-4H-1,2,4-triazol-3-yl)-2-(1H-indol-3-yl)ethyl)pyrazine-2-carboxamide | 555.3 | 556.2 |
| **86** | (R)-N-(1-(5-(2-(1H-indol-3-yl)ethyl)-4-(2,4-dimethoxybenzyl)-4H-1,2,4-triazol-3-yl)-2-(1H-indol-3-yl)ethyl)-2-cis-aminocyclohexanecarboxamide | 645.3 | 646.2 |
| **87** | (S)-N-((R)-1-(5-(2-(1 H-indol-3-yl)ethyl)-4-(4-methoxybenzyl)-4H-1,2,4-triazol-3-yl)-2-(1 H-indol-3-yl)ethyl)piperidine-3-carboxamide | 601.3 | 601.9 |
| **88** | (R)-N-((R)-1-(5-(2-(1 H-indol-3-yl)ethyl)-4-(4-methoxybenzyl)-4H-1,2,4-triazol-3-yl)-2-(1 H-indol-3-yl)ethyl)piperidine-2-carboxamide | 601.3 | 602.2 |
| **89** | (S)-N-((R)-1 -(5-(2-(1H-indol-3-yl)ethyl)-4-(4-methoxybenzyl)-4H-1,2,4-triazol-3-yl)-2-(1H-indol-3-yl)ethyl)pyrrolidine-2-carboxamide | 587.3 | 588.2 |
| **90** | (R)-N-((R)-1 -(5-(2-(1H-indol-3-yl)ethyl)-4-(4-methoxybenzyl)-4H-1,2,4-triazol-3-yl)-2-(1H-indol-3-yl)ethyl)pyrrolidine-2-carboxamide | 587.3 | 588.0 |
| **91** | (R)-N-(1-(5-(2-(1H-indol-3-yl)ethyl)-4-(4-methoxybenzyl)-4H-1,2,4-triazol-3-yl)-2-(1H-indol-3-yl)ethyl)-2-(pyridin-2-yl)acetamide | 609.3 | 610.0 |
| **92** | (R)-N-(1-(5-(2-(1H-indol-3-yl)ethyl)-4-(4-bromobenzyl)-4H-1,2,4-triazol-3-yl)-2-(1H-indol-3-yl)ethyl)-2-amino-2-methylpropanamide | 623.2 | 624.1 |
| **93** | (R)-N-(1-(5-(2-(1H-indol-3-yl)ethyl)-4-(4-methoxybenzyl)-4H-1,2,4-triazol-3-yl)-2-phenylethyl)-2-amino-2-methylpropanamide | 536.3 | 536.9 |
| **94** | (R)-N-(2-(1 H-indol-3-yl)-1-(5-phenethyl-4-(thiophen-2-ylmethyl)-4H-1,2,4-triazol-3-yl)ethyl)piperidine-4-carboxamide | 538.3 | 538.8 |
| **95** | (R)-N-(1-(4-(2-(1H-indol-3-yl)ethyl)-4H-1,2,4-triazol-3-yl)-2-(1H-indol-3-yl)ethyl)-2-amino-2-methylpropanamide | 455.2 | 456.2 |
| **96** | (R)-N-(1-(5-((1H-indol-3-yl)methyl)-4-methyl-4H-1,2,4-triazol-3-yl)-2-(1H-indol-3-yl)ethyl)-2-amino-2-methylpropanamide | 455.2 | 456.4 |
| **97** | (R)-N-(1-(5-(2-(1H-indol-3-yl)ethyl)-4-methyl-4H-1,2,4-triazol-3-yl)-2-(1H-indol-3-yl)ethyl)-2-amino-2-methylpropanamide | 469.3 | 470.2 |
| **98** | (R)-N-(1-(5-((1H-indol-3-yl)methyl)-4H-1,2,4-triazol-3-yl)-2-(1H-indol-3-yl)ethyl)-2-amino-2-methylpropanamide | 441.3 | 442.1 |
| **99** | (R)-N-(1-(5-((1H-indol-3-yl)methyl)-4-(2,4-dimethoxybenzyl)-4H-1,2,4-triazol-3-yl)-2-(1H-indol-3-yl)ethyl)-2-amino-2-methylpropanamide | 591.3 | 592.1 |
| **100** | (R)-N-(1-(4-(2,4-dimethoxybenzyl)-5-methyl-4H-1,2,4-triazol-3-yl)-2-(1H-indol-3-yl)ethyl)-2-amino-2-methylpropanamide | 476.3 | 477.4 |
| **101** | (R)-N-(1-(5-((1H-indol-3-yl)methyl)-4-(4-methoxybenzyl)- 4H-1,2,4-triazol-3-yl)-2-(1H-indol-3-yl)ethyl)-2-amino-2-methylpropanamide | 561.3 | 562.3 |
| **102** | (R)-N-(1-(4-(2,4-dimethoxybenzyl)-5-benzyl-4H-1,2,4-triazol-3-yl)-2-(1 H-indol-3-yl)ethyl)-2-amino-2-methylpropanamide | 552.3 | 553.2 |
| **103** | (R)-N-(1-(5-(3-(1 H-indol-3-yl)propyl)-4-(2,4-dimethoxybenzyl)-4H-1,2,4-triazol-3-yl)-2-(1H-indol-3-yl)ethyl)-2-amino-2-methylpropanamide | 619.3 | 620.2 |
| **104** | (R)-N-(1-(5-((1 H-indol-3-yl)methyl)-4-phenethyl-4H-1,2,4-triazol-3-yl)-2-(1H-indol-3-yl)ethyl)-2-amino-2-methylpropanamide | 545.3 | 546.4 |
| **105** | (R)-N-(1-(5-benzyl-4-phenethyl-4H-1,2,4-triazol-3-yl)-2-(1H-indol-3-yl)ethyl)-2-amino-2-methylpropanamide | 506.3 | 507.4 |
| **106** | (R)-N-(1-(5-benzyl-4-(2,2-diphenylethyl)-4H-1 ,2,4-triazol-3-yl)-2-(1H-indol-3-yl)ethyl)-2-amino-2-methylpropanamide | 582.3 | 583.3 |
| **107** | (R)-N-(1-(5-(2-(1H-indol-3-yl)ethyl)-4-(2,2-diphenylethyl)-4H-1,2,4-triazol-3-yl)-2-(1H-indol-3-yl)ethyl)-2-amino-2-methylpropanamide | 635.3 | 636.4 |
| **108** | (R)-N-(1-(4-(3,5-dimethoxybenzyl)-5-benzyl-4H-1,2,4-triazol-3-yl)-2-(1H-indol-3-yl)ethyl)-2-amino-2-methylpropanamide | 552.3 | 553.3 |
| **109** | (R)-N-(1-(4,5-dibenzyl-4H-1,2,4-triazol-3-yl)-2-(1H-indol-3-yl)ethyl)-2-amino-2-methylpropanamide | 492.3 | 493.3 |
| **110** | (R)-N-(1 -(5-benzyl-4-hexyl-4H-1,2,4-triazol-3-yl)-2-(1 H-indol-3-yl)ethyl)-2-amino-2-methylpropanamide | 486.3 | 487.4 |
| **111** | (R)-N-(1-(4-(2-(1 H-indol-3-yl)ethyl)-5-benzyl-4H-1,2,4-triazol-3-yl)-2-(1 H-indol-3-yl)ethyl)-2-amino-2-methylpropanamide | 545.3 | 546.2 |
| **112** | (S)-N-(1-(4-(2,4-dimethoxybenzyl)-5-benzyl-4H-1,2,4-triazol-3-yl)-2-(1 H-indol-3-yl)ethyl)-2-amino-2-methylpropanamide | 552.3 | 553.1 |
| **113** | (R)-N-(1-(4-(3,5-dimethoxybenzyl)-5-phenethyl-4H-1,2,4-triazol-3-yl)-2-(1 H-indol-3-yl)ethyl)-2-amino-2-methylpropanamide | 566.3 | 567.3 |
| **114** | (R)-N-(1 1-( 4-( 4-bromobenzyl)-5-benzyl-4H-1 ,2,4-triazol-3-yl)-2-(1 H-indol-3-yl)ethyl)-2-amino-2-methylpropanamide | 570.2 | 571.0 |
| **115** | (R)-N-(1-(4-(2-methoxybenzyl)-5-benzyl-4H-1,2,4-triazol-3-yl)-2-(1H-indol-3-yl)ethyl)-2-amino-2-methylpropanamide | 522.3 | 523.0 |
| **116** | (S)-N-(1-(4-(2,4-dimethoxybenzyl)-5-phenethyl-4H-1,2,4-triazol-3-yl)-2-(1H-indol-3-yl)ethyl)-2-amino-2-methylpropanamide | 566.3 | 567.0 |
| **117** | (R)-N-(1-(4,5-diphenethyl-4H-1,2,4-triazol-3-yl)-2-(1 H-indol-3-yl)ethyl)-2-amino-2-methylpropanamide | 520.3 | 521.1 |
| **118** | (R)-N-(1-(4-(3,4-dichlorobenzyl)-5-benzyl-4H-1,2,4-triazol-3-yl)-2-(1 H-indol-3-yl)ethyl)-2-amino-2-methylpropanamide | 560.2 | 561.3 |
| **119** | (R)-1-(5-(2-(1H-indol-3-yl)ethyl)-4-(2,4-dimethoxybenzyl)-4H-1,2,4-triazol-3-yl)-2-(1 H-indol-3-yl)ethanamine | 451.3 | 452.1 |
| **120** | (R)-N-(1-(4-( 4-methoxybenzyl)-5-benzyl-4H-1 ,2,4-triazol-3-yl)-2-phenylethyl)-2-amino-2-methylpropanamide | 483.3 | 484.0 |
| **121** | (R)-N-(1-(4-(4-fluorobenzyl)-5-phenethyl-4H-1,2,4-triazol-3-yl)-2-(1H-indol-3-yl)ethyl)-2-amino-2-methylpropanamide | 524.3 | 524.9 |
| **122** | (R)-N-(1-(4-(3,4-dichlorobenzyl)-5-phenethyl-4H-1,2,4-triazol-3-yl)-2-(1H-indol-3-yl)ethyl)-2-amino-2-methylpropanamide | 574.2 | 574.9 |
| **124** | (R)-N-(1-(4-(4-methylbenzyl)-5-benzyl-4H-1,2,4-triazol-3-yl)-2-(1H-indol-3-yl)ethyl)-2-amino-2-methylpropanamide | 506.3 | 507.3 |
| **125** | (S)-N-(1-(4-(4-methoxybenzyl)-5-(3-phenylpropyl)-4H-1,2,4-triazol-3-yl)-2-(1H-indol-3-yl)ethyl)-2-amino-2-methylpropanamide | 550.3 | 551.3 |
| **126** | (S)-N-(1-(4-(4-methoxybenzyl)-5-benzyl-1,2,4-triazol-3-yl)-2-(1H-indol-3-yl)ethyl)-2-amino-2-methylpropanamide | 522.3 | 523.4 |
| **128** | (R)- N-(1-( 4-( 4-nitrobenzyl)-5-phenethyl-4H-1,2,4-triazol-3-yl)-2-(1H-indol-3-yl)ethyl)-2-amino-2-methylpropanamide | 551.3 | 551.9 |
| **129** | (S)-N-(1-(4-(4-methoxybenzyl)-5-phenethyl-4H-1,2,4-triazol-3-yl)-2-(1H-indol-3-yl)ethyl)-2-amino-2-methylpropanamide | 536.3 | 537.0 |
| **130** | (R)-N-(1-(4-(4-methoxyphenethyl)-5-phenethyl-4H-1,2,4-triazol-3-yl)-2-(1H-indol-3-yl)ethyl)-2-amino-2-methylpropanamide | 550.3 | 551.0 |
| **131** | (R)-N-(2-(1H-indol-3-yl)-1-(5-phenethyl-4-(thiophen-2-ylmethyl)-4H-1,2,4-triazol-3-yl)ethyl)-2-amino-2-methylpropanamide | 512.2 | 512.8 |
| **132** | (R)-N-(2-(1H-indol-3-yl)-1-(5-phenethyl-4-(pyridin-2-ylmethyl)-4H-1,2,4-triazol-3-yl)ethyl)-2-amino-2-methylpropanamide | 507.3 | 508.4 |
| **133** | (R)-N-(-2-(1H-indol-3-yl)-1-(5-phenethyl-4-(pyridin-2-ylmethyl)-4H-1,2,4-triazol-3-yl)ethyl)piperidine-3-carboxamide | 533.3 | 534.0 |
| **134** | (S)-N-((R)-1-(4-(4-ethylbenzyl)-5-phenethyl-4H-1,2,4-triazol-3-yl)-2-(1H-indol-3-yl)ethyl)pyrrolidine-2-carboxamide | 546.3 | 547.3 |
| **135** | N-((R)-1-( 4-( 4-ethylbenzyl)-5-phenethyl-4H-1 ,2,4-triazol-3-yl)-2-(1H-indol-3-yl)ethyl)-2-aminoacetamide | 506.3 | 507.3 |
| **136** | N-((R)-1-(5-(2-(1 H-indol-3-yl)ethyl)-4-(4-methoxybenzyl)-4H-1,2,4-triazol-3-yl)-2-(1H-indol-3-yl)ethyl)-2-(pyridin-4-yl)acetamide | 609.3 | 610.4 |
| **137** | (2R)-N-((R)-1-(4-(4-ethylbenzyl)-5-phenethyl-4H-1,2,4-triazol-3-yl)-2-(1H-indol-3-yl)ethyl)piperidine-2-carboxamide | 560.3 | 561.3 |
| **138** | N-((R)-1-(4-(4-ethylbenzyl)-5-phenethyl-4H-1 ,2,4-triazol-3-yl)-2-(1H-indol-3-yl)ethyl)picolinamide | 554.3 | 555.3 |
| **139** | N-((R)-1-(4-(4-ethylbenzyl)-5-phenethyl-4H-1,2,4-triazol-3-yl)-2-(1H-indol-3-yl)ethyl)-2-aminopyridine-3-carboxamide | 569.3 | 570.3 |
| **140** | (2S)-N-((R)-1-(4-(4-ethylbenzyl)-5-phenethyl-4H-1,2,4-triazol-3-yl)-2-(1H-indol-3-yl)ethyl)-2-aminopropanamide | 520.3 | 521.2 |
| **141** | N-((R)-1-(4-(4-ethylbenzyl)-5-phenethyl-4H-1 ,2,4-triazol-3-yl)-2-(1H-indol-3-yl)ethyl)isonicotinamide | 554.3 | 555.4 |
| **142** | N-((R)-2-(1H-indol-3-yl)-1-(5-phenethyl-4-phenyl-4H-1,2,4-triazol-3-yl)ethyl)piperidine-4-carboxamide | 518.3 | 519.3 |
| **143** | (2S)-N-((R)-2-(1 H-indol-3-yl)-1-(5-phenethyl-4-phenyl-4H-1 ,2,4-triazol-3-yl)ethyl)pyrrolidine-2-carboxamide | 504.3 | 505.1 |
| **144** | N-((R)-2-(1H-indol-3-yl)-1-(5-phenethyl-4-phenyl-4H-1,2,4-triazol-3-yl)ethyl)-2-aminoacetamide | 464.3 | 465.1 |
| **145** | N-((R)-2-(1H-indol-3-yl)-1-(5-phenethyl-4-phenyl-4H-1,2,4-triazol-3-yl)ethyl)-2-(pyridin-2-yl)acetamide | 526.3 | 527.2 |
| **146** | N-((R)-2-(1H -indol-3-yl)-1-(5-phenethyl-4-phenyl-4H-1,2,4-triazol-3-yl)ethyl)picolinamide | 512.3 | 513.4 |
| **147** | N-((R)-1-(5-(2-(1H-indol-3-yl)ethyl)-4-(4-ethylphenyl)-4H-1,2,4-triazol-3-yl)-2-(1H-indol-3-yl)ethyl)picolinamide | 579.3 | 580.1 |
| **148** | N-((R)-1-(5-(2-(1H-indol-3-yl)ethyl)-4-(4-ethylphenyl)-4H-1,2,4-triazol-3-yl)-2-(1H-indol-3-yl)ethyl)-2-(pyridin-2-yl)acetamide | 593.3 | 594.2 |
| **149** | N-((R)-1-(5-(2-(1H-indol-3-yl)ethyl)-4-(4-ethylphenyl)-4H-1,2,4-triazol-3-yl)-2-(1H-indol-3-yl)ethyl)-2-aminoacetamide | 531.3 | 532.2 |
| **150** | (2S)-N-((R)-1-(5-(2-(1H-indol-3-yl)ethyl)-4-(4-ethylphenyl)-4H-1,2,4-triazol-3-yl)-2-(1H-indol-3-yl)ethyl)pyrrolidine-2-carboxamide | 571.3 | 572.4 |
| **152** | N-((R)-1-(5-(2-(1H-indol-3-yl)ethyl)-4-(4-methoxybenzyl)-4H-1,2,4-triazol-3-yl)-2-(1H-indol-3-yl)ethyl)-2-aminoacetamide | 547.3 | 548.0 |
| **153** | N-((R)-1-(4-(4-methoxybenzyl)-5-phenethyl-4H-1,2,4-triazol-3-yl)-2-(1H-indol-3-yl)ethyl)-2-trans-aminocyclohexanecarboxamide | 576.3 | 577.2 |
| **154** | N-((R)-1-(4-(4-ethylbenzyl)-5-phenethyl-4H-1,2,4-triazol-3-yl)-2-(1H-indol-3-yl)ethyl)-2-(pyridin-3-yl)acetamide | 568.3 | 569.3 |
| **155** | (3S)-N-((R)-1-(4-(4-ethylbenzyl)-5-phenethyl-4H-1,2,4-triazol-3-yl)-2-(1H-indol-3-yl)ethyl)piperidine-3-carboxamide | 560.3 | 561.5 |
| **156** | N-((R)-1-(4-(4-ethylbenzyl)-5-phenethyl-4H-1,2,4-triazol-3-yl)-2-(1H-indol-3-yl)ethyl)-2-aminobenzamide | 568.3 | 569.1 |
| **157** | N-((R)-1-(5-(2-(1H-indol-3-yl)ethyl)-4-phenyl-4H-1,2,4-triazol-3-yl)-2-(1H-indol-3-yl)ethyl)picolinamide | 551.3 | 552.2 |
| **158** | N-((R)-1-(5-(2-(1H-indol-3-yl)ethyl)-4-phenyl-4H-1,2,4-triazol-3-yl)-2-(1H-indol-3-yl)ethyl)piperidine-4-carboxamide | 557.3 | 558.1 |
| **159** | N-((R)-2-(1H-indol-3-yl)-1-(4-(2,4-dimethoxyphenyl)-5-phenethyl-4H-1,2,4-triazol-3-yl)ethyl)picolinamide | 572.3 | 573.4 |
| **160** | N-((R)-2-(1H-indol-3-yl)-1-(4-(2,4-dimethoxyphenyl)-5-phenethyl-4H-1,2,4-triazol-3-yl)ethyl)-2-(pyridin-2-yl)acetamide | 586.3 | 587.3 |
| **161** | N-( (R)-2-(1H-indol-3-yl)-1-(4-(2,4-dimethoxyphenyl)-5-phenethyl-4H-1,2,4-triazol-3-yl)ethyl)pyrazine-2-carboxamide | 573.3 | 574.2 |
| **162** | N-((R)-2-(1 H-indol-3-yl)-1-(4-(2,4-dimethoxyphenyl)-5-phenethyl-4H-1,2,4-triazol-3-yl)ethyl)-2-aminoacetamide | 524.3 | 525.3 |
| **163** | N-((R)-2-(1 H-indol-3-yl)-1-(4-(2,4-dimethoxyphenyl)-5-phenethyl-4H-1,2,4-triazol-3-yl)ethyl)piperidine-4-carboxamide | 578.3 | 579.4 |
| **164** | N-( (R)-1-(5-benzyl-4-( (pyridin-2-yl)methyl)-4H-1,2,4-triazol-3-yl)-2-(1H-indol-3-yl)ethyl)picolinamide | 513.3 | 514.3 |
| **165** | N-((R)-1-(5-benzyl-4-((pyridin-2-yl)methyl)-4H-1,2,4-triazol-3-yl)-2-(1H-indol-3-yl)ethyl)-2-amino-acetamide | 465.3 | 466.4 |
| **166** | N-((R)-1-(5-benzyl-4-((pyridin-2-yl)methyl)-4H-1,2,4-triazol-3-yl)-2-(1H-indol-3-yl)ethyl)piperidine-4-carboxamide | 519.3 | 520.2 |
| **167** | N-((R)-1-(5-benzyl-4-((pyridin-4-yl)methyl)-4H-1,2,4-triazol-3-yl)-2-(1H-indol-3-yl)ethyl)-2-amino-2-methylpropanamide | 493.3 | 494.3 |
| **168** | N-((R)-1-(5-(4-methoxybenzyl)-4-phenethyl-4H-1,2,4-triazol-3-yl)-2-(1H-indol-3-yl)ethyl)-2-amino-2-methylpropanamide | 536.3 | 537.3 |
| **169** | N-((R)-1-(5-benzyl-4-((pyridin-4-yl)methyl)-4H-1,2,4-triazol-3-yl)-2-(1H-indol-3-yl)ethyl)picolinamide | 513.3 | 514.2 |
| **170** | N-((R)-1-(5-benzyl-4-((pyridin-4-yl)methyl)-4H-1,2,4-triazol-3-yl)-2-(1H-indol-3-yl)ethyl)2-amino-acetamide | 465.3 | 466.1 |
| **171** | (R)-benzyl-3-(2-aminoisobutyramido)-3-(5-(2-(1H-indol-3-yl)ethyl)-4-(4-methoxybenzyl)-4H-1,2,4-triazol-3-yl)-propanoate | 594.3 | 595.2 |
| **172** | N-((R)-1-(5-benzyl-4-((pyridin-3-yl)methyl)-4H-1,2,4-triazol-3-yl)-2-(1H-indol-3-yl)ethyl)-2-amino-2-methylpropanamide | 493.3 | 494.3 |
| **173** | N-((R)-1-(4-benzyl-5-phenethyl-4H-1,2,4-triazol-3-yl)-2-(1 H-indol-3-yl)ethyl)-2-amino-2-methylpropanamide | 506.3 | 507.3 |
| **174** | N-((R)-2-(1H-indol-3-yl)-1 -(4-methyl-5-phenethyl-4H-1,2,4-triazol-3-yl)ethyl)picolinamide | 450.3 | 451.3 |
| **175** | N-((R)-1-(5-(2-(1H-indol-3-yl)ethyl)-4-phenyl-4H-1,2,4-triazol-3-yl)-2-(1H-indol-3-yl)ethyl)-2-amino-2-methylpropanamide | 531.3 | 532.4 |
| **176** | N-((R)-1-(4-(4-methoxybenzyl)-5-phenethyl-4H-1,2,4-triazol-3-yl)-2-(1H-indol-3-yl)ethyl)benzamide | 555.3 | 556.2 |
| **177** | (R)-1-(4-(2,4-dimethoxybenzyl)-5-phenethyl-4H-1,2,4-triazol-3-yl)-2-(1H-indol-3-yl)-N-phenylmethanesulfonylamine | 635.3 | 637.0 |
| **178** | (R)-1-(4-(2,4-dimethoxybenzyl)-5-phenethyl-4H-1,2,4-triazol-3-yl)-2-(1H-indol-3-yl)-N-tosylethanamine | 635.3 | 636.3 |
| **179** | N-((R)-1-(4-(2,4-dimethoxybenzyl)-5-phenethyl-4H-1,2,4-triazol-3-yl)-2-(1H-indol-3-yl)ethyl)-2-amino-2-methylpropanamide | 566.3 | 567.2 |
| **180** | N-1-((R)-1-(4-(2,4-dimethoxybenzyl)-5-phenethyl-4H-1,2,4-triazol-3-yl)-2-(1H-indol-3-yl)ethyl)ethane-1,2-diamine | 524.3 | 525.2 |
| **181** | N-((R)-1-(4-((furan-2-yl)methyl)-5-phenethyl-4H-1,2,4-triazol-3-yl)-2-(1 H-indol-3-yl)ethyl)-2-amino-2-methylpropanamide | 496.3 | 497.1 |
| **182** | N-((R)-1-(4-((furan-2-yl)methyl)-5-phenethyl-4H-1 ,2,4-triazol-3-yl)-2-(1H-indol-3-yl)ethyl)picolinamide | 516.3 | 517.1 |
| **183** | N-((R)-1-(4-((furan-2-yl)methyl)-5-phenethyl-4H-1,2,4-triazol-3-yl)-2-(1 H-indol-3-yl)ethyl)piperidine-4-carboxamide | 522.3 | 523.2 |
| **184** | N-((R)-1-(4-(4-methoxybenzyl)-5-phenethyl-4H-1,2,4-triazol-3-yl)-2-(1 H-indol-3-yl)ethyl)-tetrahydro-2H-pyran-4-carboxamide | 563.3 | 564.2 |
| **185** | N-((R)-1-(5-((1 H-indol-3-yl)methyl)-4-(3-methoxybenzyl)-4H-1,2,4-triazol-3-yl)-2-(1 H-indol-3-yl)ethyl)-2-amino-2-methylpropanamide | 561.3 | 562.2 |
| **186** | (2S)-N-((R)-1-(4-(4-methoxybenzyl)-5-phenethyl-4H-1,2,4-triazol-3-yl)-2-(1 H-indol-3-yl)ethyl)-2-amino-3-phenylpropanamide | 598.3 | 599.1 |
| **187** | (R)-1-(5-(2-(1 H-indol-3-yl)ethyl)-4-(2,4-dimethoxybenzyl)-4H-1,2,4-triazol-3-yl)-2-(1 H-indol-3-yl)-N-tosylethanamine | 674.3 | 675.0 |
| **188** | N-((R)-1-(4-(2,4-dimethoxybenzyl)-5-phenethyl-4H-1,2,4-triazol-3-yl)-2-(1 H-indol-3-yl)ethyl)-4-azidobenzamide | 626.3 | 627.3 |
| **189** | N-benzyl-(R)-1-(4-(2,4-dimethoxybenzyl)-5-phenethyl-4H-1,2,4-triazol-3-yl)-2-(1 H-indol-3-yl)ethanamine | 571.3 | 572.3 |
| **190** | (2S)-N-((R)-1-(5-(2-(1 H-indol-3-yl)ethyl)-4-(4-methoxybenzyl)-4H-1,2,4-triazol-3-yl)-2-(1 H-indol-3-yl)ethyl)-2,5-dihydro-1H-pyrrole-2-carboxamide | 585.3 | 586.2 |

### II) RT-PCR analysis of total RNA extracts from primary adipocytes and differentiated 3T3-L1 cells

Total RNA was extracted from primary mouse adipocytes or differentiated 3T3-L1 cells (ATCC CL-173) with Trizol LS reagent (Invitrogen, cat. no. 15596-018) according to the manufacturer's instructions. Total RNA were treated with *DNase I A*mplification *Grade* (Invitrogen, cat. No. 18068-015) according to manufacturer's instructions. cDNA was synthesized from 1 µg of DNase treated total RNA with random primers (250 ng/µl, Invitrogen, cat. No. 48190-011) and 20 U M-MLV Reverse Transcriptase (Invitrogen, cat. No. 28025-013) at 37°C for 2 h in a total volume of 20 µl containing 50 mM Tris-HCl pH 8.3, 75 mM KCI, 3 mM MgCl₂, 10 mM DTT and 0.5 mM dNTP. After sample denaturation at 95°C for 5 min, cDNAs were amplified in a volume of 50 µL with 2 µl of reverse transcription reaction and 0.2 µM of each specific primer using 2.5 U of Taq DNA polymerase (Invitrogen, cat. No. 18038-042). Cycling conditions for GPR103A were 35 cycles at 94°C for 45 sec; 60°C for 45 sec and 72°C for 1 min; for GPR103B, 35 cycles at 94°C for 45 sec; 59.5°C for 45 sec and 72°C for 1 min; for QRFP, 35 cycles at 94°C for 45 sec; 60°C for 45 sec and 72°C for 1 min; and for RNA 18S, 26 cycles at 94°C for 45 sec; 62.5°C for 45 sec and 72°C for 1.5 min. PCR products were separated on 1.5% agarose gel, and the relative signal intensity was measured with an image analyzer (Typhoon, Amersham). The primer sequences and the expected product size were as follows: GPR103A, sense primer: 5'-TCT TTG GCA ACT CTC TGG TCA TC-3', GPR103A, antisense primer: 5'-CTT CGG GTA GTG TAC TGC CAC T-3' (304 bp); GPR103B, sense primer: 5'-CGA TAT CAA GTG GTG TGA ACA GCC-3', GPR103B, antisense primer: 5'-GGG TCT CTT GTA GCC CAG GT-3' (299 bp); QRFP, sense primer: 5'-TCT GCC GTC CTT ACC ATC TCA-3', QRFP, antisense primer: 5'-TCT CAG GAC TGT CCC AAA GGA G-3' (142 bp) and RNA 18S, sense primer 5'-GGA CCA GAG CGA AAG CAT TTG CC-3', RNA 18S, anti-sense primer (TCA ATC TCG GGT GGC TGA ACG C-3' (496 bp). All values were normalized against RNA 18S.

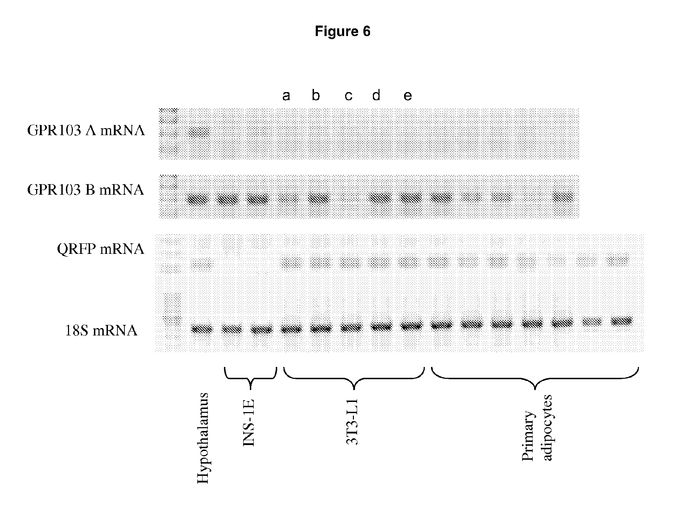

**Figure 6** shows the analysis of the mRNA expression of GPR103 receptors and QRFP peptide in differentiated 3T3-L1 cells and primary adipocytes from mice. From the analysis, it is clear that only GPR103B mRNA is present in differentiated 3T3-L1 cells and in primary adipocytes as follows: a) day 0, b) day 2, c) day 4, d) day 6 and e) day 8. Both primary adipocytes and differentiated 3T3-L1 cells also express QRFP mRNA. mRNA from hypothalamus was used as positive control as both GPR103A and GPR103B mRNA were found in this tissue ("Hypothalamus"). INS-1 E is a pancreatic cell line which was found to express GPR103B ("INS-1 E").

### III) Study of anti-inhibitory effects of isoproterenol-induced lipolysis in an adipocyte cell line model

The effectiveness of compounds of the invention to antagonize the QRFP-induced inhibition of isoproterenol-induced lipolysis was investigated using an adipocyte cell line model.

### Preparation of differentiated 3T3-L cells

Cell Culture. Prior to differentiation, 3T3-L1 cells (ATCC CL-173 ATCC) (passage 6) were seeded in 150 mm Polystyrene plates (Corning) and grown in Dulbecco's modified Eagle's medium (DMEM) supplemented with 10% calf serum and 100 units/mL of penicillin/streptomycin in a 10% CO₂ incubator at 37°C.

At 95% confluence, cells were passed and seeded in 12-wells Costar plate (Corning 3513) in DMEM supplemented with 10% calf serum.

Upon 95% confluence, differentiation was induced by culturing cells with DMEM supplemented with 10% fetal bovine serum, antibiotics, insulin (1 µg/mL), dexamethasone (1 µM) and isobutylmethylxanthine (0.5 mM) for 4 days. After the differentiation step, cells were then cultured in DMEM with 10 % FBS containing 167 nM insulin for 2 days. Cells were then were maintained in DMEM with 10% FBS for 2 days before lipolysis study.

### Lipolysis study:

All the cellular assays were performed in triplicate for each time point if not otherwise specified.

Differentiated 3T3-L1 cells were first washed with DMEM and incubated with DMEM containing 200 nM adenosine for 1 h at 37°C. The incubation medium was then removed. The lipolysis was induced by incubating the cells with DMEM supplemented with 2% bovine serum albumin fatty acid free and adenosine deaminase (1 U/mL) containing 100nM of isoproterenol for 90 min at 37°C. The incubation medium (600 µL) was collected for glycerol assay following triglyceride hydrolysis.

The inhibitory effect of QRFP on isoproterenol-induced lipolysis was documented by incubating the cells with DMEM supplemented with 2% bovine serum albumin and adenosine deaminase (1 U/mL) containing 100nM of isoproterenol in presence of 10⁻¹², 10⁻¹⁰, 10⁻⁹, 10⁻⁸, 10⁻⁷ and 10⁻⁶ M QRFP-26 for 90 min at 37°C. After the incubation period, the incubation medium (600 µL) was collected for free glycerol measurement.

To document the antagonistic effect of compounds of the invention on the anti-lipolytic effect of QRFP, the dose-response curve of QRFP was carried out as described above in presence of selected compounds of the invention at 10⁻⁹ to 10⁻⁷ M. To document the net effect of QRFP and selected compounds of the invention on lipolysis induced by isoproterenol as control, cells were incubated in absence of isoproterenol in presence of QRFP or selected compounds of the invention at 10⁻⁶ M.

### Glycerol assays:

Glycerol assays were performed using the free glycerol reagent (Sigma F 6428). The absorbance was read at 540 nm. Glycerol released was calculated as µmol/mg of total protein.

Lipolysis index was calculated as: (µmol of glycerol released/mg of protein of sample minus µmol of glycerol released/mg of protein in basal condition) / (µmol of glycerol released/mg of protein induced by 100 nM of isoproterenol minus µmol of glycerol released/mg of protein of basal condition).

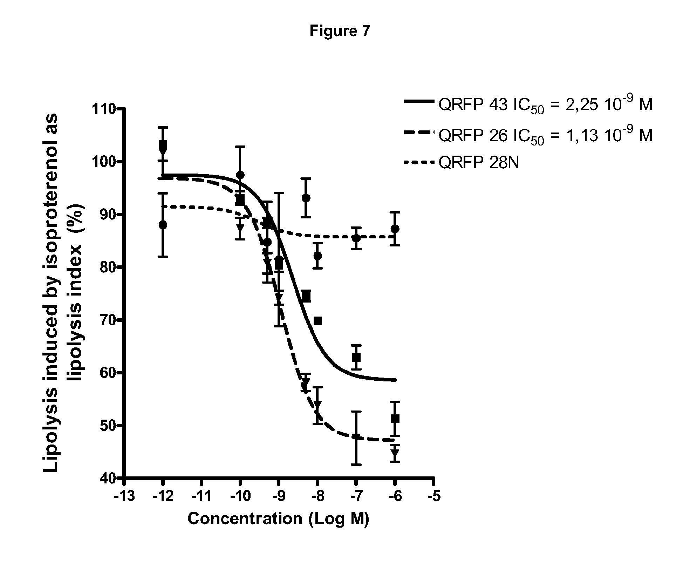

**Figure 7** shows the inhibitory effect of QRFP-43 and QRFP-26 on lipolysis induced by isoproterenol with IC₅₀ of 2.25 x 10⁻⁹ M and 1.13 x 10⁻⁹ M, respectively. QRFP-28N (the N-terminal fragment of QRFP-43), which displays no binding affinity to GRP103 is used as negative control.

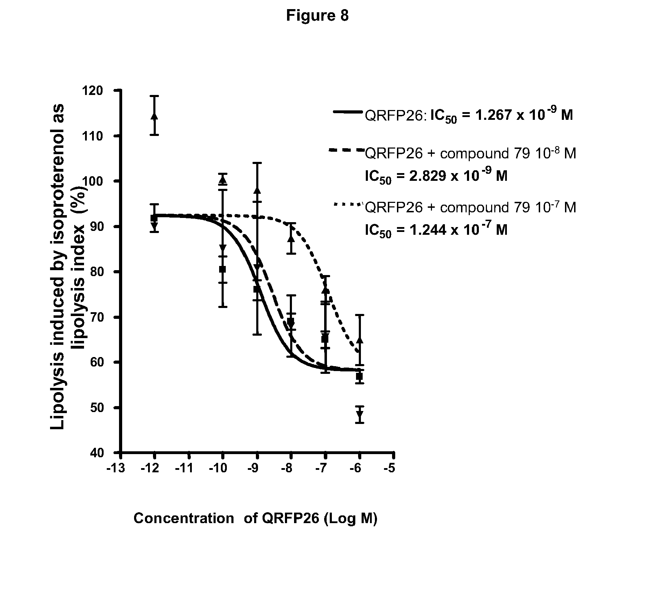

**Figure 8** shows that QRFP-26 induces an inhibitory effect on lipolysis induced by isoproterenol in a dose-dependant manner with IC₅₀ of 1.267 x 10-⁹ M. **Compound 79** at 10⁻⁹ M and 10⁻⁷ M displays an antagonist effect on the inhibitory effect of QRFP with a shift of IC⁵⁰ at 2.829 x 10⁻⁹ M and 1.244 x 10⁻⁷ M, respectively.

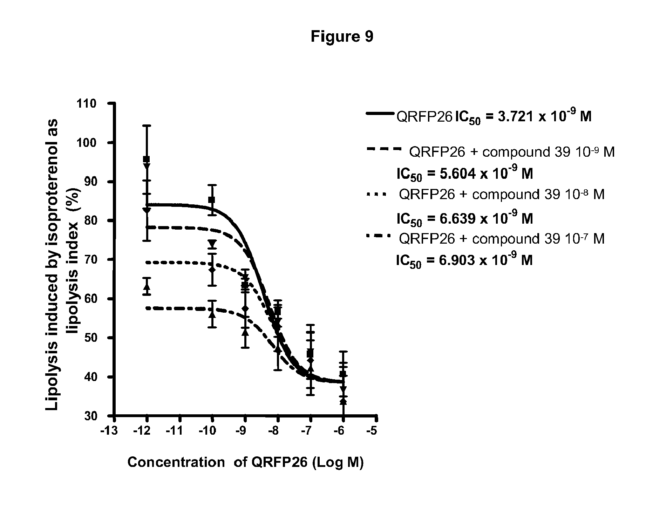

**Figure 9** shows that QRFP-26 induces an inhibitory effect on lipolysis induced by isoproterenol in a dose-dependant manner with IC₅₀ of 3.72 x 10-⁹ M. **Compound 39** at 10⁻⁹ M, 10⁻⁸ M and 10⁻⁷ M displays a non-competitive antagonist effect on the inhibitory effect of QRFP with a shift of IC₅₀ at 5.6 x 10⁻⁹ M, 6.6 x 10⁻⁹ M and 6.9 x 10⁻⁹ M, respectively.

### IV) Study of the effect of compounds of the invention on diet-induced obesity (DIO) in mice

The effectiveness of selected compounds of the invention, i.e. their subchronic effects, on the energy balance of mice rendered obese by a high-fat diet was investigated.

Adult C57BU6 male mice were fed ad libitum with either chow or a high-fat diet (60% of the calorie content from fat) for 5 weeks.

C57BL/6 mice fed with a high-fat diet readily develop obesity and represent a widely used model for investigating human obesity.

During the last two weeks under these regimen, mice were given daily subcutaneous injections of **compound 9** or **compound 50** (1,5 mg/kg per day, 6 mg/kg per day or 24 mg/kg per day). One-third of the dose was injected in the morning and two-third in the evening.

Body weight and food intake were monitored every day during the experiment. Body composition (lean and fat mass) was assessed using dual energy x-ray absorptiometry (DEXA) on day 0, 13 and 27. Blood was also sampled at sacrifice for the determination of plasma glucose, insulin and triglycerides.

Two weeks of treatment with 6 mg/kg per day and 24 mg/kg per day of compound 9 and of compound 50 significantly decreased body weight gain in mice. The reduced weight gain was accounted for by a reduction in the fat mass deposition. There was no effect of the drugs at any doses on the lean body mass. Compound 9 at 6 mg/kg per day and 24 mg/kg per day and compound 50 at 24 mg/kg per day also reduced the size of the fat depots. The reduced fat depositions were not accompanied by a reduced food intake but by a diminished food efficiency [(body weight gain / food ingested) x 100]. In addition, animals with reduced fat deposition also demonstrated diminished plasma insulin levels (for all data, refer to **table 2**).

The presented results provide clear evidence for the ability of selected compounds of the invention to reduce fat gain and improve insulin sensitivity in mice rendered obese by a high-fat diet. The effect on energy balance occurred through a reduced food efficiency demonstrating the ability of selected compounds of the invention to stimulate energy expenditure.

**Table 2: Values of different variables measured in high-fat diet fed mice injected subcutaneously with saline, compound 9 or compound 50 after a 2 week treatment period.**

| **Parameters** | **Chow control** | **Saline control** | **Compound 9** | | | **Compound 50** | | |
|---|---|---|---|---|---|---|---|---|
| **Dose (mg/kg per day)** | --- | --- | 1,5 | 6,0 | 24 | 1,5 | 6,0 | 24 |
| **Body weight gain (g)*** | 0.5 | 1,9 | 1,03 | 0,20 | -0,54 | 1,61 | 1,44 | -0,04 |
| **Cumulative food intake (kJ)*** | 959 | 819 | 793 | 809 | 790 | 852 | 813 | 801 |
| **Food efficiency (mg/kJ)** | 0.52 | 2.31 | 1.30 | 0.25 | -0,68 | 1.88 | 1.77 | -0,05 |
| **Fat mass (g)** | 4,18 | 8,3 | 7,9 | 5,8 | 57 | 7,6 | 8,2 | 6,3 |
| **Lean mass (g)** | 21.0 | 20,6 | 20,2 | 21,2 | 20,5 | 21,3 | 20,4 | 20,6 |
| **Σ F at depots (mg)** | 467 | 1184 | 1137 | 791 | 792 | 1101 | 1192 | 902 |
| **Insulin (pmol/l)** | 78,0 | 73,4 | 63,8 | 57,4 | 52,0 | 66,3 | 64,8 | 60,3 |
| **Glucose (mmol/l)** | 13,2 | 16,1 | 14,9 | 15,6 | 13,1 | 15,6 | 15,2 | 15,4 |

| | | | | | | | | |
|---|---|---|---|---|---|---|---|---|
| * Results correspond to the difference between the values at day 35 and day 21 (first day of administration). | | | | | | | | |

### V) Assessment of fasting-induced feeding behavior (food intake) of mice

The effectiveness of selected compounds of the invention on fasting-induced feeding behavior of mice was investigated.

The experiments were performed according to Asakawa A et al. (Asakawa A et al., Gut 2003, 52: 947-952) with the following technical details:

Preparation of compounds of the invention: dissolved in dimethylsulfoxide (DMSO) and then diluted in distilled water (i.e. 0.5, 1 and 10% DMSO for 0.5, 1 and 2 µmol/mouse, respectively);

Preparation of reference substance (positive control) sibutramine: dissolved in distilled water;

Control substance (negative vehicle control): 10% DMSO in distilled water (compounds of the invention); distilled water (sibutramine);
Route of administration: p.o.;
Pretreatment time: 30 min before presenting the food to the animals;
Volume of administration: 0.5 ml/mouse;
Animals: male C57BL/6J mice;
Body weight range: 18.3 - 21.4 g
Number per group: 8

The corresponding results are presented in **table 3.**

**Table 3**

| **Compound** | **Oral dose** (per mouse) | **change of food intake - compounds versus vehicle treated negative control** | | | |
|---|---|---|---|---|---|
| | | **at 20 min** | **at 1 h** | **at 2h** | **at 4h** |
| **Sibutramine** (positive control) | 0,6 µmol | -63% | -66% | -46% | -21% |
| **5** | 2 µmol | -56% | -58% | -53% | -51 % |
| **11** | 2 µmol | -56% | -65% | -55% | -55% |
| **27** | 2 µmol | -57% | -58% | -48% | -38% |
| **28** | 2 µmol | -17% | -28% | -21 % | -18% |
| **31** | 2 µmol | +50% | +20% | +16% | +9% |
| **37** | 2 µmol | -37% | -49% | -51 % | -34% |
| **45** | 2 µmol | -36% | -28% | -33% | -34% |
| **48** | 2 µmol | -22% | -30% | -27% | -21 % |
| **49** | 2 µmol | -59% | -48% | -39% | -23% |
| **50** | 2 µmol | -54% | -44% | -44% | -28% |
| **62** | 2 µmol | +56% | +68% | +50% | +26% |
| **66** | 2 µmol | -56% | -40% | -45% | -51 % |
| **117** | 2 µmol | -31 % | -24% | -11 % | -6% |
| **118** | 2 µmol | -31 % | -30% | -25% | -24% |
| **122** | 2 µmol | -52% | -42% | -28% | -16% |
| **142** | 2 µmol | -56% | -57% | -55% | -49% |
| **179** | 2 µmol | -25% | -39% | -40% | -38% |
| **190** | 2 µmol | -39% | -30% | -33% | -30% |

## Claims

1. Use of a compound according to formula (1) 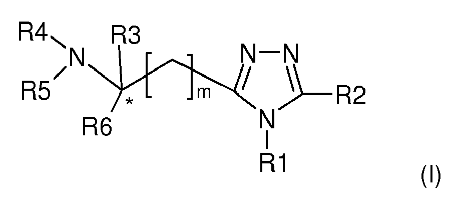 wherein:
R1 and R2 are independently of one another selected from the group consisting of "hydrogen atom, alkyl, alkenyl, alkynyl, cycloalkyl, cycloalkylalkyl, aryl, heteroaryl, arylalkyl, heteroarylalkyl, heterocyclyl, heterocyclylalkyl, alkylsulfonyl, arylsulfonyl, arylalkylsulfonyl" which are optionally substituted in the alkyl, cycloalkyl, cycloalkylalkyl, aryl, heteroaryl, arylalkyl, heteroarylalkyl, heterocyclyl and/or heterocyclylalkyl group by up to 3 substituents independently selected from the group consisting of "halogen, -F, -Cl, -Br, -1, -N₃, -CN, -NR7R8, -OH, -NO₂, alkyl, aryl, arylalkyl, -O-alkyl, -O-aryl, -O-arylalkyl"; and preferably are selected from the group consisting of "alkyl, aryl, heteroaryl, arylalkyl, heteroarylalkyl" optionally being substituted by up to 3 substituents independently selected from the group consisting of "halogen, -F, -Cl, -Br, -1, -N₃, -CN, -NR7R8, -OH, -NO₂, alkyl, aryl, arylalkyl, - O-alkyl, -O-aryl, -O-arylalkyl";
one of radicals R3 and R4 is a hydrogen atom, whereas the other radical is selected from the group consisting of "hydrogen atom, alkyl, aryl, heteroaryl, arylalkyl, heteroarylalkyl, heterocyclyl, heterocyclylalkyl, -alkyl-O-aryl, -alkyl-O-arylalkyl, - alkyl-O-heteroaryl, -alkyl-O-heteroarylalkyl, -alkyl-O-heterocyclyl, alkyl-O-heterocyclylalkyl, -alkyl-CO-aryl, -alkyl-CO-arylalkyl, -alkyl-CO-heteroaryl, - alkyl-CO-heteroarylalkyl, -alkyl-CO-heterocyclyl, -alkyl-CO-heterocyclylalkyl, - alkyl-C(O)O-aryl, -alkyl-C(O)O-arylalkyl, -alkyl-C(O)O-heteroaryl, -alkyl-C(O)O-heteroarylalkyl, -alkyl-C(O)O-heterocyclyl, -alkyl-C(O)O-heterocyclylalkyl, -alkyl-CO-NH₂, -alkyl-CO-OH, -alkyl-NH₂, -alkyl-NH-C(NH)-NH₂, alkylsulfonyl, arylsulfonyl, arylalkylsulfonyl, alkyl-S-alkyl, alkyl-S-H" which are optionally substituted in the aryl, heteroaryl, arylalkyl, heteroarylalkyl, heterocyclyl and/or heterocyclylalkyl group by up to 3 substituents independently selected from the group consisting of "halogen, -F, -Cl, -Br, -l, -N₃, -CN, -NR7R8, -OH, - NO₂, alkyl, aryl, arylalkyl, -O-alkyl, -O-aryl, -O-arylalkyl"; and preferably are selected from the group consisting of "arylalkyl, heteroarylalkyl, heterocyclylalkyl, - alkyl-O-aryl, -alkyl-O-arylalkyl, -alkyl-O-heteroaryl, -alkyl-O-heteroarylalkyl, - alkyl-O-heterocyclyl, alkyl-O-heterocyclylalkyl, -alkyl-CO-aryl, -alkyl-CO-arylalkyl, -alkyl-CO-heteroaryl, -alkyl-CO-heteroarylalkyl, -alkyl-CO-heterocyclyl, alkyl-CO-heterocyclylalkyl, -alkyl-C(O)O-aryl, -alkyl-C(O)O-arylalkyl, -alkyl-C(O)O-heteroaryl, -alkyl-C(O)O-heteroarylalkyl, -alkyl-C(O)O-heterocyclyl, -alkyl-C(O)O-heterocyclylalkyl, -alkyl-CO-NH₂, -alkyl-CO-OH, - alkyl-NH₂, -alkyl-NH-C(NH)-NH₂," optionally being substituted in the aryl, heteroaryl, arylalkyl, heteroarylalkyl, heterocyclyl and/or heterocyclylalkyl group by up to 3 substituents independently selected from the group consisting of "halogen, -F, -Cl, -Br, -I, -N₃, -CN, -NR7R8, -OH, -NO₂, alkyl, aryl, arylalkyl, -O-alkyl, -O-aryl, -O-arylalkyl";
R5 is selected from the group consisting of "hydrogen atom, alkyl, cycloalkyl, cycloalkylalkyl, aryl, heteroaryl, arylalkyl, heteroarylalkyl, heterocyclyl, heterocyclylalkyl, -CO-alkyl, -CO-cycloalkyl, -CO-cycloalkylalkyl, -CO-aryl, -CO-arylalkyl, -CO-heteroaryl, -CO-heteroarylalkyl, -CO-heterocyclyl, -CO-heterocyclylalkyl, - CO-C*(R9R10)-NH₂, -CO-CH₂-C*(R9R10)-NH₂, -CO-C*(R9R10)-CH₂-NH₂, alkylsulfonyl, arylsulfonyl, arylalkylsulfonyl" which are optionally substituted by up to 3 substituents independently selected from the group consisting of "halogen, -F, - Cl, -Br, -I, -N₃, -CN, -NR7R8, -OH, -NO₂, alkyl, aryl, arylalkyl, -O-alkyl, -O-aryl, -O-arylalkyl"; and preferably is selected from the group consisting of "hydrogen atom, -CO-alkyl, -CO-cycloalkyl, -CO-aryl, -CO-heteroaryl, -CO-arylalkyl, - CO-heteroarylalkyl, -CO-heterocyclyl, -CO-C*(R9R10)-NH₂, -CO-CH₂-C*(R9R10)-NH₂, -CO-C*(R9R10)-CH₂-NH₂, optionally being substituted by up to 3 substituents independently selected from the group consisting of "halogen, -F, - Cl, -Br, -I, -N₃, -CN, -NR7R8, -OH, -NO₂, alkyl, aryl, arylalkyl, -O-alkyl, -O-aryl, -O-arylalkyl";
R6 is selected from the group consisting of "hydrogen atom, alkyl, cycloalkyl, cycloalkylalkyl" and preferably is a hydrogen atom; R7 and R8 are independently of one another selected from the group consisting of "hydrogen atom, alkyl, cycloalkyl, cycloalkylalkyl" and preferably are a hydrogen atom;
R9 and R10 are independently of one another selected from the group consisting of "hydrogen atom, alkyl, natural alpha-amino acid side chain, unnatural alpha-amino acid side chain" and preferably are selected from the group consisting of "hydrogen atom, alkyl";
m is 0, 1 or 2 and preferably is 0; and
* means a carbon atom of R or S configuration when chiral;
for the manufacture of a medicament for the treatment or prophylaxis of physiological and/or pathophysiological conditions in mammals, where the physiological and/or pathophysiological conditions are mediated by at least one G-protein coupled receptor (GPCR);
with the proviso that the following G-protein coupled receptors (GPCR) are excluded: "GHS receptors, GHS type 1 receptor, GHS-R 1 a, GHS-R 1 b, motilin receptor, motilin receptor 1a, neurotensin receptor, TRH receptor, GPR38 (FM1), GPR39 (FM2), FM3, GHS-R subtype, GHS binding site, cardiac GHS-R, mammary GHS-R".

2. The use of a compound according to formula (I) as claimed in claim 1, where
R3 is selected from the group consisting of "-alkyl-CO-aryl, -alkyl-CO-arylalkyl, - alkyl-CO-heteroaryl, -alkyl-CO-heteroarylalkyl, -alkyl-CO-heterocyclyl, alkyl-CO-heterocyclylalkyl, -alkyl-C(O)O-aryl, -alkyl-C(O)O-arylalkyl, -alkyl-C(O)O-heteroaryl, -alkyl-C(O)O-heteroarylalkyl, -alkyl-C(O)O-heterocyclyl, -alkyl-C(O)O-heterocyclylalkyl, -alkyl-CO-NH₂, -alkyl-CO-OH, -alkyl-NH-C(NH)-NH₂, alkyl-S-alkyl, alkyl-S-H", and preferably is selected from the group consisting of "-alkyl-CO-arylalkyl, -alkyl-C(O)O-arylalkyl, -alkyl-CO-NH₂, -alkyl-CO-OH".

3. The use of a compound according to formula (I) as claimed in claims 1, where R4 is a hydrogen atom;
R5 is selected from the group consisting of "hydrogen atom, alkyl, cycloalkyl, cycloalkylalkyl, aryl, heteroaryl, arylalkyl, heteroarylalkyl, heterocyclyl, heterocyclylalkyl, alkylsulfonyl, arylsulfonyl, arylalkylsulfonyl, -CO-cycloalkyl, -CO-cycloalkylalkyl, -CO-aryl, -CO-arylalkyl, -CO-heteroaryl, -CO-heteroarylalkyl, -CO-heterocyclyl, -CO-heterocyclylalkyl";
with the proviso that if R5 is "-CO-heteroarylalkyl", "heteroaryl" is not imidazole; and
with the proviso that if R5 is "-CO-heterocyclyl" and "heterocyclyl" contains only nitrogen atoms as heteroatoms, that at least two nitrogen atoms are contained in "heterocyclyl"; and
with the proviso that if R5 is "-CO-heterocyclylalkyl" and "heterocyclyl" contains only nitrogen atoms as heteroatoms that in the case that one or two nitrogen atoms are contained in "heterocyclyl" no nitrogen atom is positioned at position 1 of "heterocyclyl" that is the atom directly linking "heterocyclyl" to the carbonyl group "-CO-";
where "alkyl, cycloalkyl, cycloalkylalkyl, aryl, heteroaryl, arylalkyl, heteroarylalkyl, heterocyclyl, heterocyclylalkyl, alkylsulfonyl, arylsulfonyl, arylalkylsulfonyl, -CO-cycloalkyl, -CO-cycloalkylalkyl, -CO-aryl, -CO-arylalkyl, -CO-heteroaryl, -CO-heteroarylalkyl, -CO-heterocyclyl, and/or -CO-heterocyclylalkyl" are optionally substituted by up to 3 substituents independently selected from the group consisting of "halogen, -F, -Cl, -Br, -I, -N₃, -CN, -NR7R8, -OH, -NO₂, alkyl, aryl, arylalkyl, -O-alkyl, -O-aryl, -O-arylalkyl";
with the proviso that if R5 is "-CO-cycloalkyl" or "-CO-cycloalkylalkyl", R5 is not substituted with NR7R8 at position 1 of "cycloalkyl", that is the C atom directly linking "cycloalkyl" to the carbonyl group "-CO-" in case of R5 = "-CO-cycloalkyl" or to the "alkyl" in case R5 = "-CO-cycloalkylalkyl"; and
with the proviso that if R5 is "-CO-aryl" or "-CO-arylalkyl" and "aryl" is phenyl/benzene and is only substituted with one substituent, this one substituent is not -NR7R8;
R6 is a hydrogen atom;
R7 and R8 are independently of one another selected from the group consisting of "hydrogen atom, alkyl, cycloalkyl, cycloalkylalkyl" and preferably are a hydrogen atom; and
m is 0, 1 or 2, and more preferably is 0.

4. The use of a compound according to formula (I) as claimed in claim 1, where
R1 is selected from the group consisting of "hydrogen, methyl, (2-methoxyphenyl)-methyl, (3-methoxyphenyl)-methyl, (4-methoxyphenyl)-methyl, (3-methoxyphenyl)-ethyl, (4-methoxyphenyl)-ethyl, phenyl, phenyl-methyl, phenyl-ethyl, (4-ethylphenyl)-methyl, (4-methylphenyl)-methyl, (4-fluorophenyl)-methyl, (4-bromophenyl)-methyl, (2,4-dimethoxyphenyl)-methyl, (3,5-dimethoxyphenyl)-methyl, 2,2-diphenyl-ethyl, naphthaline-1-yl-methyl, 1*H-indole-3-yl-methyl,* 2-(1*H* indole-3-yl)-ethyl, 3-(1*H-indole-3-yl)-propyl,* 4-methyl-phenyl, 4-ethyl-phenyl, n-hexyl, (3,4-dichlorophenyl)-methyl, (4-nitro-phenyl)-methyl, (pyridine-2-yl)-methyl, (pyridine-3-yl)-methyl, (pyridine-4-yl)-methyl, (thiophene-2-yl)-methyl, (thiophene-3-yl)-methyl, (furan-2-yl)-methyl, (furan-3-yl)-methyl";
R2 is selected from the group consisting of "methyl, 1*H*-indole-3-yl-methyl, 2-(1 H indole-3-yl)-ethyl, 3-(1*H*-indole-3-yl)-propyl, 2-phenyl-ethyl, 3-phenyl-propyl, 4-phenyl-butyl, 2-methoxy-phenylmethyl, 3-methoxy-phenylmethyl, 4-methoxy-phenylmethyl, 2-methoxy-phenylethyl, 3-methoxy-phenylethyl, 4-methoxy-phenylethyl";
R3 is selected from the group consisting of "hydrogen atom, methyl, propan-2-yl, 2-methyl-propan-1-yl, butan-2-yl, butan-1-yl, -CH₂-SH, -(CH₂)₂-S-CH₃, 1H-indole-3-yl-methyl, phenyl-methyl, 2-phenyl-ethyl, -CH₂-O-CH₂-phenyl, -CH₂-CO-CH₂-phenyl, -(CH₂)₂-CO-CH₂-phenyl, -CH₂-C(O)O-phenyl, -(CH₂)₂-C(O)O-phenyl, hydroxy-methyl, 1-hydroxy-ethan-1-yl, -CH₂-CO-NH₂, -(CH₂)₂-CO-NH₂, (1-hydroxy-benzene-4-yl)-methyl, -CH₂-CO-OH, -(CH₂)₂-CO-OH, -(CH₂)₄-NH₂, (1*H*-imidazol-5-yl)-methyl, -(CH₂)₃-NH-C(NH)-NH₂, -(CH₂)₃-NH₂, -(CH₂)₃-NH-CO-NH₂", and preferably is selected from the group consisting of "1*H*-indole-3-yl-methyl, -CH₂-CO-CH₂-phenyl, -(CH₂)₂-CO-CH₂-phenyl, -CH₂-C(O)O-phenyl, - (CH₂)₂-C(O)O-phenyl";
R4 is a hydrogen atom;
R5 is selected from the group consisting of "hydrogen atom, -CO-CH₂-NH₂ (Gly residue), -CO-CH₂-CH₂-NH₂ (beta-Ala residue), -CO-CHCH₃-NH₂ (D- and/or L-alpha-Ala residue), -CO-(pyrrolidine-2-yl) (D- and/or L-Pro residue), 2-amino-2-carbonyl-propane (2-amino-isobutyric acid/Aib residue), 4-carbonyl-1 *H*-piperidine, 3-carbonyl-1 *H*-piperidine, R-(3-carbonyl-1 *H*-piperidine), S-(3-carbonyl-1*H-*piperidine), 2-carbonyl-1 *H*-piperidine, R-(2-carbonyl-1 *H*-piperidine), S-(2-carbonyl-1*H*-piperidine), 1-amino-2-carbonyl-benzene, carbonyl-cyclohexane, 2-acetyl-pyridine, 3-acetyl-pyridine, 4-acetyl-pyridine, 2-propionyl-pyridine, 3-propionyl-pyridine, 4-propionyl-pyridine, (R-1-amino)-2-carbonyl-cyclohexane, (S-1-amino)-2-carbonyl-cyclohexane, 2-carbonyl-1 *H*-imidazole, 2-carbonyl-pyridine, 3-carbonyl-pyridine, 4-carbonyl-pyridine, 2-amino-3-carbonyl-pyridine, 2-carbonyl-pyrazine, 2-carbonyl-4-hydroxy-1 *H*-pyrrolidine*,* 4-carbonyl-1*H*,3*H*-diazacyclohexane, methylsulfonyl, phenylsulfonyl, 1-carbonyl-1-amino-2-phenylethane, phenylmethyl, 1-carbonyl-4-azide-benzene, 2-carbonyl-2,5-dihydro-1 H-pyrrole, 2-carbonyl-piperazine, 2-carbonyl-1 H-pyrrolidine, 2-aminoethane, carbonyl-benzene, 2-carbonyl-pyrazine, 3-carbonyl-pyrazine, 4-carbonyl-oxacyclohexane, 4-methylphenylsulfonyl, phenylmethyl-sulfonyl";
R6 is a hydrogen atom; and
m is 0;.

5. The use of a compound according to formula (I) as claimed in claim 4, where
R3 is selected from the group consisting of "-CH₂-CO-CH₂-phenyl, -(CH₂)₂-CO-CH₂-phenyl, -CH₂-CO-NH₂, -(CH₂)₂-CO-NH₂, -CH₂-CO-OH, -(CH₂)₂-CO-OH, -(CH₂)₃-NH-C(NH)-NH₂, -CH₂-SH, -(CH₂)₂-S-CH₃".

6. The use of a compound according to formula (I) as claimed in claim 4, where
R5 is selected from the group consisting of "hydrogen atom, methylsulfonyl, phenylsulfonyl, carbonyl-cyclohexane, (R-1-amino)-2-carbonyl-cyclohexane, (S-1-amino)-2-carbonyl-cyclohexane, 2-carbonyl-pyridine, 3-carbonyl-pyridine, 4-carbonyl-pyridine, 2-acetyl-pyridine, 3-acetyl-pyridine, 4-acetyl-pyridine, 2-propionyl-pyridine, 3-propionyl-pyridine, 4-propionyl-pyridine, 2-amino-3-carbonyl-pyridine, 2-carbonyl-1 H-imidazole, 2-carbonyl-pyrazine, 4-carbonyl-1 H,3H diazacyclohexane".

7. The use as claimed in any of claims 1 to 6, where the compound is selected from the group consisting of:
**compound 1** (*R*)-*N*-(1-(5-(2-(1*H*-indol-3-yl)ethyl)-4-(2,4-dimethoxybenzyl)-4*H-*1,2,4-triazol-3-yl)-2-(1*H*-indol-3-yl) ethyl)-2-amino-2-methylpropanamide,
**compound 2** *(R)-N-(*1-(5-(2-(1*H-*indol-3-yl)ethyl)-4-phenethyl-4*H*-1,2,4-triazol-3-yl)-2-(1*H*-indol-3-yl)ethyl)-2-amino-2-methylpropanamide,
**compound 3** *(R)-N-(*1-(5-(3-(1*H*-indol-3-yl)propyl)-4-phenethyl-4*H*-1,2,4-triazol-3-yl)-2-(1*H*-indol-3-yl)ethyl)-2-amino-2-methylpropanamide,
**compound 4** (*R*)-*N*-(1-(5-benzyl-4-(naphthalen-1-ylmethyl)-4*H*-1,2,4-triazol-3-yl)-2-(1*H*-indol-3-yl)ethyl)-2-amino-2-methylpropanamide,
**compound 5** *(R)-N-(*1-(5-(2-(1*H*-indol-3-yl)ethyl)-4-(naphthalen-1-ylmethyl)-4*H-*1,2,4-triazol-3-yl)-2-(1*H*-indol-3-yl)ethyl)-2-amino-2-methylpropanamide,
**compound 6** (*R*)-*N*-(1-(5-(2-(1*H*-indol-3-yl)ethyl)-4-(3-methoxybenzyl)-4*H*-1,2,4-triazol-3-yl)-2-(1*H*-indol-3-yl)ethyl)-2-amino-2-methylpropanamide,
**compound 7** (*R*)-*N*-(1-(4-(3-methoxybenzyl)-5-benzyl-4*H*-1,2,4-triazol-3-yl)-2-(1*H*-indol-3-yl)ethyl)-2-amino-2-methylpropanamide,
**compound 8** (*R*)-*N*-(1-(5-(2-(1*H*-indol-3-yl)ethyl)-4-benzyl-4*H*-1,2,4-triazol-3-yl)-2-(1*H*-indo-3-yl)ethyl)-2-amino-2-methylpropanamide,
**compound 9** (*R*)-*N*-(1-(5-(3-(1*H*-indol-3-yl)propyl)-4-benzyl-4*H*-1,2,4-triazol-3-yl)-2-(1*H*-indol-3-yl)ethyl)-2-amino-2-methylpropanamide,
**compound 10** (*R*)-*N*-(1-(5-(3-(1*H*-indol-3-yl)propyl)-4-(3-methoxybenzyl)-4*H-*1,2,4-triazol-3-yl)-2-(1*H*-indol-3-yl)ethyl)-2-amino-2-methylpropanamide,
**compound 11** *(R)-N-*(1-(5-(3-(1*H*-indol-3-yl)propyl)-4-(naphthalen-1-ylmethyl)-4*H-*1,2,4-triazol-3-yl)-2-(1*H*-indol-3-yl)ethyl)-2-amino-2-methylpropanamide,
**compound 12** *(R)-N-*(1-(5-(2-(1*H*-indol-3-yl)ethyl)-4-(4-methoxybenzyl)-4*H*-1,2,4-triazol-3-yl)-2-(1*H*-indol-3-yl)ethyl)-2-amino-2-methylpropanamide,
**compound 13** *(R)-N-*(1-(4-(4-methoxybenzyl)-5-benzyl-4*H*-1,2,4-triazol-3-yl)-2-(1*H*-indol-3-yl)ethyl)-2-amino-2-methylpropanamide,
**compound 14** (*R*)-*N*-(1-(5-(3-(1H-indol-3-yl)propyl)-4-(4-bromobenzyl)-4*H*-1,2,4-triazol-3-yl)-2-(1*H-*indol-3-yl)ethyl)-2-amino-2-methylpropanamide,
**compound 15** *(R)-N*-(1-(5-(2-(1*H*-indol-3-yl)ethyl)-4-hexyl-4*H*-1,2,4-triazol-3-yl)-2-(1*H*-indol-3-yl)ethyl)-2-amino-2-methylpropanamide,
**compound 16** (*R*)*-N-* (1-(5-(3-(1 *H* indol-3-yl)propyl)-4-hexyl-4*H* 1,2,4-triazol-3-yl)-2-(1*H*-indol-3-yl)ethyl)-2-amino-2-methyipropanamide,
**compound 17** (*R*)-*N*-(1-(4,5-bis(2-(1*H*-indol-3-yl)ethyl)-4*H*-1,2,4-triazol-3-yl)-2-(1*H*-indol-3-yl)ethyl)-2-amino-2-methylpropanamide,
**compound 18** (S)-*N*-(1-(5-(2-(1 *H*-indol-3-yl)ethyl)-4-(2,4-dimethoxybenzyl)- 4*H* 1 ,2 ,4-triazol-3-yl)-2-( 1 *H*-indol-3-yl)ethyl)-2-amino-2-methylpropanam ide,
**compound 19** *(R)-N*-(1-(4-(3-methoxybenzyl)-5-phenethyl-4*H*-1,2,4-triazol-3-yl)-2-(1 H-indol-3-yl)ethyl)-2-amino-2-methylpropanamide,
**compound 20** *(R)-N-*(1-(4-(4-methoxybenzyl)-5-phenethyl-4*H* 1,2,4-triazol-3-yl)-2-(1*H*-indol-3-yl)ethyl)-2-amino-2-methylpropanamide,
**compound 21** *(R)-N*-(1-(5-(2-(1 H-indol-3-yl)ethyl)-4-(3,5-dimethoxybenzyl)-4H-1,2,4-triazol-3-yl)-2-(1*H*-indol-3-yl) ethyl)-2-amino-2-methylpropanamide,
**compound 22** (R)-*N*-(1-(4-(4-methoxybenzyl)-5-(3-phenylpropyl)-4*H*-1,2,4-triazol-3-yl)-2-(1*H*-indol-3-yl)ethyl)-2-amino-2-methylpropanamide,
**compound 23** (*R*)-*N*-(1-(5-(3-(1*H* indol-3-yl)propyl)-4-(4-methoxybenzyl)-4*H* 1,2 ,4-triazol-3-yl)-2-(1*H*-indol-3-yl) ethyl)-2-amino-2-methylpropanamide,
**compound 24** (*R*)-*N*-(1-(4-(2-(1*H*-indol-3-yl)ethyl)-5-(3-(1*H*-indol-3-yl)propyl)-4*H-*1,2,4-triazol-3-yl)-2-(1*H*-indol-3-yl) ethyl) -2-amino-2-methylpropanamide,
**compound 25** (*R*)-*N*-(1-(5-(2-(1*H*-indol-3-yl)ethyl)-4-(2-methoxy)benzyl)-4*H*-1,2,4-triazol-3-yl)-2-(1*H*-indol-3-yl)ethyl)-2-amino-2-methylpropanamide,
**compound 26** *(R)-N-(* 1-( 4-(2-methoxybenzyl)-5-phenethyl-4*H*-1 ,2,4-triazol-3-yl)-2-(1-*H*-indol-3-yl)ethyl)-2-amino-2-methylpropanamide,
**compound 27** (*R*)-*N*- (2-(1*H*-indol-3-yl)-1-(4-(naphthalen-1-ylmethyl)-5-phenethyl-4*H*-1,2,4-triazol-3-yl)ethyl)-2-amino-2-methylpropanamide,
**compound 28** (*R*)*-N-*(1-(5-(2-(1*H*-indol-3-yl)ethyl)-4-(3,4-dichlorobenzyl)-4*H-*1 ,2 ,4-triazol-3-yl)-2-(1*H*-indol-3-yl)ethyl)-2-amino-2-methylpropanamide,
**compound 29** (*R*)-*N*-(1 -(5-(2-(1 *H*-indol-3-yl)ethyl)-4-(4-fluorobenzyl)-4*H*-1,2,4-triazol-3-yl)-2-(1 *H*-indol-3-yl)ethyl)-2-amino-2-methylpropanamide,
**compound 30** (*R*)*-N-*(1-(4-(4-fluorobenzyl)-5-benzyl-4*H*-1,2,4-triazol-3-yl)-2-(1*H* indol-3-yl)ethyl)-2-amino-2-methylpropanamide,
**compound 31** *(R)-N-*(1-(5-(2-(1*H*-indol-3-yl)ethyl)-4-(2,4-dimethoxybenzyl)-4*H-*1,2,4-triazol-3-yl)-2-(1*H*-indol-3-yl)ethyl)piperidine-4-carboxamide,
**compound 32** *(R)-N*-(1-(5-(2-(1*H*-indol-3-yl)ethyl)-4-(2,4-dimethoxybenzyl)-4*H-*1,2,4-triazol-3-yl)-2-(1*H*-indol-3-yl)ethyl)piperidine-3-carboxamide,
**compound 33** *(R)-N*-(1-(4-(4-methylbenzyl)-5-(3-phenylpropyl)-4*H*-1,2,4-triazol-3-yl)-2-(1*H*-indol-3-yl)ethyl)-2-amino-2-methylpropanamide,
**compound 34** (*R*)-*N*-(1-(5-(2-(1*H*-indol-3-yl)ethyl)-4-(4-methylbenzyl)-4*H*-1,2,4-triazol-3-yl)-2-(1*H*-indol-3-yl)ethyl)-2-amino-2-methylpropanamide,
**compound 36** (*R*)-*N*-(1-(5-(2-(1*H*-indol-3-yl)ethyl)-4-(2,4-dimethoxybenzyl)-4*H-*1,2,4-triazol-3-yl)-2-(1*H*-indol-3-yl)ethyl)piperidine-2-carboxamide,
compound 37 (*R*)-*N*-(1-(4-(4-methylbenzyl)-5-phenethyl-4*H*-1,2,4-triazol-3-yl)-2-(1*H*-indol-3-yl)ethyl)-2-amino-2-methylpropanamide,
compound 38 *(R)-N-*(1-(5-(2-(1*H*-indol-3-yl)ethyl)-4-(2,4-dimethoxybenzyl)-4*H-*1,2,4-triazol-3-yl)-2-(1*H*-indol-3-yl) ethyl)-2-aminobenzamide,
**compound 39** (*R*)-*N*-(1-(5-benzyl-4-(pyridin-2-ylmethyl)-4*H-*1,2,4-triazol-3-yl)-2-(1*H*-indol-3-yl)ethyl)-2-amino-2-methylpropanamide,
**compound 40** (2*S*,4*R*)-N-((R)-1-(4-(4-methoxybenzyl)-5-phenethyl-4*H*-1,2,4-triazol-3-yl)-2-(1*H*-indol-3-yl)ethyl)-4-hydroxypyrrolidine-2-carboxamide,
**compound 41** (*S*)-*N*-((*R*)-1-(4-(4-methoxybenzyl)-5-phenethyl-4*H*-1,2,4-triazol-3-yl)-2-(1*H*- indol-3-yl)ethyl)piperidine-3-carboxamide,
**compound 42** (*R*)-*N*-((*R*)-1-(4-(4-methoxybenzyl)-5-phenethyl-4*H*-1,2,4-triazol-3-yl)-2-(1*H*-indol-3-yl)ethyl)piperidine-3-carboxamide,
**compound 43** (*R*)*-N-*(1-(4-(4-ethylbenzyl)-5-phenethyl-4*H*-1 ,2,4-triazol-3-yl)-2-(1 *H*-indol-3-yl)ethyl)-2-amino-2-methylpropanamide,
**compound 44** (*R*)*-N*-(1-(4-(4-methoxybenzyl)-5-phenethyl-4*H*-1,2,4-triazol-3-yl)-2-(1 *H*-indol-3-yl)ethyl)piperidine-4-carboxamide,
**compound 45** (*R*)-*N*-(1 -(5-(2-(1 *H* indol-3-yl)ethyl)-4-(4-methoxybenzyl)-4*H*-1,2,4-triazol-3-yl)-2-(1 *H*-indol-3-yl)ethyl)piperidine-4-carboxamide,
**compound 46** (*S*)-*N*-((*R*)-1-(4-(4-methoxybenzyl)-5-phenethyl-4*H*-1,2,4-triazol-3-yl)-2-(1*H*-indol-3-yl)ethyl)pyrrolidine-2-carboxamide,
**compound 47** (*R*)-N-((*R*)-1 -(4-(4-methoxybenzyl)-5-phenethyl-4H-1,2,4-triazol-3-yl)-2-(1 1 H-indol-3-yl)ethyl)pyrrolidine-2-carboxamide,
**compound 48** (*S*)-N-((*R*)-1-(4-(4-methoxybenzyl)-5-phenethyl-4H 1,2,4-triazol-3-yl)-2-(1*H*-indol-3-yl)ethyl)piperidine-2-carboxamide,
**compound 49** (*R*)-*N*- ((*R*)-1-(4-(4-methoxybenzyl)-5-phenethyl-4*H* 1,2,4-triazol-3-yl)-2-(1 *H*-indol-3-yl)ethyl)piperidine-2-carboxamide,
**compound 50** *(R)-N-*(1-(4-(4-methoxybenzyl)-5-phenethyl-4*H*-1,2,4-triazol-3-yl)-2-(1 *H*-indol-3-yl)ethyl)-2-aminoacetamide,
**compound 51** (*R*)-*N*-(1-(4-(4-methoxybenzyl)-5-phenethyl-4*H*-1,2,4-triazol-3-yl)-2-(1*H-* indol-3-yl) ethyl) -2-(pyridin -2-yl)acetamide,
**compound 52** (*R*)-*N*-(1 -(4-(4-methoxybenzyl)-5-phenethyl-4*H*-1,2,4-triazol-3-yl)-2-(1*H-*indol-3-yl) ethyl) -2-(pyridin-4-yl)acetamide,
**compound 53** (*R*)-*N*-(1-(4-(4-methoxybenzyl)-5-phenethyl-4*H*-1,2,4-triazol-3-yl)-2-(1*H* indol-3-yl)ethyl)cyclohexanecarboxamide,
**compound 54** (*R*)-*N*-(1-(5-(2-(1*H* indol-3-yl)ethyl)-4-benzyl-4*H*-1,2,4-triazol-3-yl)-2-(1 *H*-indol-3-yl)ethyl)piperidine-4-carboxamide,
**compound 55** (*R*)*-N-*(1-(5-(2-(1*H*-indol-3-yl)ethyl)-4-benzyl-4*H*-1,2,4-triazol-3-yl)-2-(1 *H*-indol-3-yl)ethyl)piperidine-3-carboxam ide,
**compound 56** (*R*)-*N*-(1-(4-(4-methoxybenzyl)-5-phenethyl-4*H*-1,2,4-triazol-3-yl)-2-(1*H*-indol-3-yl)ethyl)-3-aminopropanamide,
**compound 57** (*S*)-((*R*)-1-(4-(4-methoxybenzyl)-5-phenethyl-4*H* 1,2,4-triazol-3-yl)-2-(1*H*-indol-3-yl)ethyl)-2-aminopropanamide,
**compound 58** (*R*)-*N*-(1-(4-(4-methoxybenzyl)-5-phenethyl-4*H*-1,2,4-triazol-3-yl)-2-(1*H*-indol-3-yl)ethyl)-2-(pyridin-3-yl)acetamide,
**compound 59** (*R*)-*N*-(1-(4-(4-methoxybenzyl)-5-phenethyl-4*H*-1,2,4-triazol-3-yl)-2-(1*H*-indol-3-yl)ethyl)-3-(pyridin-3-yl)propanamide,
**compound 60** *(R)-N* (1-(5-(2-(1*H*-indo 1-3-yl) ethyl) -4-benzyl-4*H*- 1,2,4-triazol-3-yl) - 2-(1*H*-indol-3-yl)ethyl)-2-(pyridin-2-yl)acetamide,
compound 61 *(R)-N-(*1-(5-(2-(1*H*-indol-3-yl)ethyl)-4-(2,4-dimethoxybenzyl)-4*H-*1 ,2,4- triazol-3-yl)-2-(1*H*-indol-3-yl)ethyl)-2-(pyridin-2-yl)acetamide,
**compound 62** (*R*)-*N*-(1 -(4-(2,4-d!methoxybenzyl)-5-phenethyl-4H-1,2,4-triazol-3-yl)-2-(1*H*-indol-3-yl)ethyl)piperidine-4-carboxamide,
**compound 63** (*R*)*-N-*((*R*)-1-(4-(2,4-dimethoxybenzyl)-5-phenethyl-4*H*-1,2,4-triazol-3-yl)-2-(1*H*-indol-3-yl)ethyl)piperidine-2-carboxamide,
**compound 64** (*R*)-*N*-(1-(4-(2,4-dimethoxybenzyl)-5-phenethyl-4*H*-1,2,4-triazol-3-yl)-2-(1*H*-indol-3-yl)ethyl)picolinamide,
**compound 65** (*R*)-*N*-(1-(4-(2,4-dimethoxybenzyl)-5-phenethyl-4*H*-1 ,2,4-triazol-3-yl)-2-(1*H-*indol-3-yl)ethyl)isonicotinamide,
**compound 66** (*R*)-*N*-(1-(4-(2,4-dimethoxybenzyl)-5-phenethyl-4*H*-1 ,2,4-triazol-3-yl)-2-(1*H-* indol-3-yl) ethyl) pyrazine-2-carboxamide,
**compound 67** (*R*)-*N*-1-(4-(2,4-dimethoxybenzyl)-5-phenethyl-4*H*-1,2,4-triazol-3-yl)-2-( 1 *H*-indol-3-yl)ethyl)piperazine-2-carboxamide,
**compound 68** (*S*)-*N*-((*R*)-1-(4-(2,4-dimethoxybenzyl)-5-phenethyl-4*H*-1,2,4-triazol-3-yl)-2-(1*H*-indol-3-yl)ethyl)pyrrolidine-2-carboxamide,
**compound 69** (*R*)-*N*-(1 -(5-(2-(1 *H*-indol-3-yl)ethyl)-4-(2,4-dimethoxybenzyl)-4*H-*1,2,4-triazol-3-yl)-2-(1 *H*-indol-3-yl) ethyl) -2-aminoacetamide,
**compound 70** (*S*)-*N*-((*R*)-1-(5-(2-(1*H*-indol-3-yl) ethyl) -4-(2,4-dimethoxybenzyl) - 4*H*-1,2,4-triazol-3-yl)-2-(1*H-* indol-3-yl) ethyl) pyrrolidine-2-carboxamide,
**compound 71** (*R*)-*N*-(1-(5-(2-(1 *H*-indol-3-yl)ethyl)-4-(2,4-dimethoxybenzyl)-4*H-*1,2,4-triazol-3-yl)-2-(1*H*-indol-3-yl) ethyl) pyrazine-2-carboxamide,
**compound 72** (*R*)-*N*-1-(5-(2-(1 H-indol-3-yl)ethyl)-4-(2,4-dimethoxybenzyl)-4H-1 ,2 ,4-triazol-3-yl)-2-(1*H*-indol-3-yl) ethyl) piperazine-2-carboxamide,
**compound 73** (*R*)-*N*-(1-(5-(2-(1 *H*-indol-3-yl)ethyl)-4-(2,4-dimethoxybenzyl)-4*H-*1,2,4-triazol-3-yl)-2-(1 *H*-indol-3-yl)ethyl)picolinamide,
**compound 74** (*R*)-1-(5-(2-(1 *H*-indol-3-yl)ethyl)-4-(2,4-dimethoxybenzyl)-4*H*-1,2,4-triazol-3-yl)-2-(1*H*-indol-3-yl)ethanamine,
**compound 75** (*R*)-*N*-(1-(4-(2,4-dimethoxybenzyl)-5-phenethyl-4*H*-1,2,4-triazol-3-yl)-2-(1*H-* indol-3-yl) ethyl) -2-aminoacetamide,
**compound 76** (*R*)-*N*-(1-(4-(4-methoxybenzyl)-5-phenethyl-4*H*-1,2,4-triazol-3-yl)-2-(1*H*-indol-3-yl)ethyl)pyrazine-2-carboxamide,
**compound 77** *(R)-N-* (1-(4-(4-methoxybenzyl)-5-phenethyl-4H 1,2,4-triazol-3-yl)-2-(1 *H*-indol-3-yl)ethyl)isonicotinamide,
**compound 78** (*R*)-*N*-1 -(4-(4-methoxybenzyl)-5-phenethyl-4H-1,2,4-triazol-3-yl)-2-(1 H-indol-3-yl)ethyl)piperazine-2-carboxamide,
**compound 79** (R)-*N*- (1-(4-(4-methoxybenzyl)-5-phenethyl-4H 1,2,4-triazol-3-yl)-2-(1 *H*- indol-3-yl)ethyl)picolinamide,
**compound 80** *(R)-N-*(1-(5-(2-(1 *H*- indol-3-yl)ethyl)-4-(4-methoxybenzyl)-4H 1,2,4-triazol-3-yl)-2-(1 *H*-indol-3-yl)ethyl)picolinamide,
**compound 81** (R)-N-1-(5-(2-(1 *H*-indol-3-yl)ethyl)-4-(4-methoxybenzyl)-4H- 1,2,4-triazol-3-yl)-2-(1 *H*-indol-3-yl)ethyl)piperazine-2-carboxamide,
**compound 82** (*R*)-*N*-(1-(4-(4-ethylbenzyl)-5-phenethyl-4*H*-1,2,4-triazol-3-yl)-2-(1 H-indol-3-yl)ethyl)-2-(pyridin-2-yl)acetamide,
**compound 83** (*R*)-*N*-(1 -(4-(4-ethylbenzyl)-5-phenethyl-4*H*-1,2,4-triazol-3-yl)-2-(1 H indol-3-yl)ethyl)piperidine-4-carboxamide,
**compound 84** (*R*)-*N*-1-(4-(4-ethylbenzyl)-5-phenethyl-4*H*-1,2,4-triazol-3-yl)-2-(1*H* indol-3-yl)ethyl)piperazine-2-carboxamide,
**compound 85** *(R)-N*-(1-(4-(4-ethylbenzyl)-5-phenethyl-4*H* 1,2,4-triazol-3-yl)-2-(1 H indol-3-yl)ethyl)pyrazine-2-carboxamide,
**compound 86** *(R)-N*-(1-(5-(2-(1 *H*-indol-3-yl)ethyl)-4-(2,4-dimethoxybenzyl)-4*H-*1,2,4-triazol-3-yl)-2-(1 *H* indol-3-yl)ethyl)-2-cis-aminocyclohexanecarboxamide,
compound 87 (S)-IV ((R)-1-(5-(2-(1 *H*-indol-3-yl)ethyl)-4-(4-methoxybenzyl)-4*H* 1,2,4-triazol-3-yl)-2-(1*H*-indol-3-yl)ethyl)piperidine-3-carboxamide,
**compound 88** *(R)-N-*((R)-1-(5-(2-(1-*H*-indol-3-yl)ethyl)-4-(4-methoxybenzyl)-4*H-*1,2,4-triazol-3-yl)-2-(1H-indol-3-yl)ethyl)piperidine-2-carboxamide,
**compound 89** (*S*)-*N*-((*R*)-1-(5-(2-(1*H*-indol-3-yl)ethyl)-4-(4-methoxybenzyl)-4*H-*1,2,4-triazol-3-yl)-2-(1*H*-indol-3-yl) ethyl)pyrrolidine-2-carboxamide,
**compound 90** *(R)-N*-((*R*)-1-(5-(2-(1*H*-indol-3-yl)ethyl)-4-(4-methoxybenzyl)-4*H-*1 ,2 ,4-triazol-3-yl)-2-( 1 H-indol-3-yl)ethyl)pyrrolidine-2-carboxam ide,
**compound 91** *(R)-N-(*1-(5-(2-(1*H*-indol-3-yl)ethyl)-4-(4-methoxybenzyl)-4*H*-1,2,4-triazol-3-yl)-2-(1*H*-indol-3-yl)ethyl)-2-(pyridin-2-yl)acetamide,
**compound 92** *(R)-N*-(1-(5-(2-(1 *H*-indol-3-yl)ethyl)-4-(4-bromobenzyl)-4*H*-1,2,4-triazol-3-yl)-2-(1*H*-indol-3-yl)ethyl)-2-amino-2-methylpropanamide,
**compound 93** (*R*)-*N*-(1-(5-(2-(1*H*-indol-3-yl)ethyl)-4-(4-methoxybenzyl)-4*H*-1,2,4-triazol-3-yl)-2-phenylethyl)-2-amino-2-methylpropanamide,
**compound 94** (*R*)-*N*-(2-(1*H*-indol-3-yl)-1- (5-phen ethyl-4-(thiophen-2-ylmethyl) - 4*H*-1,2,4-triazol-3-yl)ethyl)piperidine-4-carboxamide,
**compound 95** *(R)-N*-(1-(4-(2-(1*H*-indol-3-yl)ethyl)-4*H*-1,2,4-triazol-3-yl)-2-(1*H-*indol-3-yl)ethyl)-2-amino-2-methylpropanamide,
**compound 96** *(R)-N*-(1-(5-((1*H*-indol-3-yl)methyl)-4-methyl-4*H*-1, 2,4-triazol-3-yl) - 2-(1*H*-indol-3-yl)ethyl)-2-amino-2-methylpropanamide,
**compound 97** (R)-N-(1-(5-(2-(1 *H*-indol-3-yl)ethyl)-4-methyl-4*H*-1,2,4-triazol-3-yl)-2-(1 H-indol-3-yl)ethyl)-2-amino-2-methylpropanamide,
**compound 98** *(R)-N*-(1-(5-((1 *H*-indol-3-yl)methyl)-4*H*-1,2,4-triazol-3-yl)-2-(1 *H-*indol-3-yl)ethyl)-2-amino-2-methylpropanamide,
**compound 99** (*R*)-*N*-(1-(5-((1H-indol-3-yl)methyl)-4-(2,4-dimethoxybenzyl)-4H-1 ,2 ,4-triazol-3-yl)-2-( H-indol-3-yl) ethyl) -2-amino-2-methylpropanamide,
**compound 100** (*R*)-*N*-(1 -(4-(2,4-dimethoxybenzyl)-5-methyl-4*H*-1,2,4-triazol-3-yl)-2-(1 H-indol-3-yl)ethyl)-2-amino-2-methylpropanamide,
**compound 101** (*R*)*-N*-(1-(5-((1H-indol-3-yl)methyl)-4-(4-methoxybenzyl)-4*H*-1,2,4-triazol-3-yl)-2-(1*H*-indol-3-yl)ethyl)-2-amino-2-methylpropanamide,
**compound 102** *(R)-N*-(1-(4-(2,4-dimethoxybenzyl)-5-benzyl-4*H*-1,2,4-triazol-3-yl)-2-(1 *H*-indol-3-yl)ethyl)-2-amino-2-methylpropanamide,
**compound 103** *(R)-N-*(1-(5-(3-(1*H*-indol-3-yl)propyl)-4-(2,4-dimethoxybenzyl)-4H-1 ,2 ,4-triazol-3-yl)-2-(1*H*-indol-3-yl) ethyl) -2-amino-2-methylpropanamide,
**compound 104** *(R)-N*-(1-(5-((1*H*-indol-3-yl)methyl)-4-phenethyl-4*H*- 1,2,4-triazol-3-yl)-2-(1*H*-indol-3-yl) ethyl) -2-amino-2-methylpropanamide,
**compound 105** (*R*)-*N*-(1-(5-benzyl-4-phenethyl-4*H*-1,2,4-triazol-3-yl)-2-(1*H*-indol-3-yl)ethyl)-2-amino-2-methylpropanamide,
**compound 106** (*R*)-*N*-(1-(5-benzyl-4-(2,2-diphenylethyl)-4*H*-1 ,2,4-triazol-3-yl)-2-(1*H*-indol-3-yl)ethyl)-2-amino-2-methylpropanamide,
**compound 107** (*R*)-*N*-(1-(5-(2-(1*H*-indol-3-yl)ethyl)-4-(2,2-diphenylethyl)-4*H*-1,2,4-triazol-3-yl)-2-(1*H*-indol-3-yl)ethyl)-2-amino-2-methylpropanamide,
**compound 108** (*R*)-*N*-(1-(4-(3,5-dimethoxybenzyl)-5-benzyl-4*H*-1,2,4-triazol-3-yl)-2-(1*H*-indol-3-yl)ethyl)-2-amino-2-methylpropanamide,
**compound 109** (*R*)-*N*-(1-(4,5-dibenzyl-4*H*-1,2,4-triazol-3-yl)-2-(1*H*-indol-3-yl)ethyl)-2-amino-2-methylpropanamide,
**compound 110** (*R*)-*N*-(5-benzyl-4-hexyl-4*H*-1,2,4-triazol-3-yl)-2-(1*H*-indol-3-yl)ethyl)-2-amino-2-methylpropanamide,
**compound 111** (*R*)-*N*-(1-(4-(2-(1*H*-indol-3-yl)ethyl)-5-benzyl-4*H*-1,2,4-triazol-3-yl)-2-(1*H*-indol-3-yl)ethyl)-2-amino-2-methylpropanamide,
**compound 112** (*S*)-*N*-(1-(4-(2,4-dimethoxybenzyl)-5-benzyl-4*H*-1,2,4-triazol-3-yl)-2-(1*H*-indol-3-yl)ethyl)-2-amino-2-methylpropanamide,
**compound 113** (*R*)-*N*-(1-(4-(3,5-d!methoxybenzyl)-5-phenethyl-4*H*-1,2,4-triazol-3-yl)-2-(1*H*-indol-3-yl) ethyl) -2-amino-2-methylpropanamide,
**compound 114** (*R*)*-N-*(1-(4-(4-bromobenzyl)-5-benzyl-4*H*-1,2,4-triazol-3-yl)-2-(1 *H* indol-3-yl)ethyl)-2-amino-2-methylpropanamide,
**compound 115** (*R*)*-N-*(1-(4-(2-methoxybenzyl)-5-benzyl-4*H*-1,2,4-triazol-3-yl)-2-(1*H*-indol-3-yl)ethyl)-2-amino-2-methylpropanamide,
**compound 116** (*S*)-*N*-(1-(4-(2,4-dimethoxybenzyl)-5-phenethyl-4*H*-1,2,4-triazol-3-yl)-2-(1 H-indol-3-yl)ethyl)-2-amino-2-methylpropanamide,
**compound 117** *(R)-N*-(1-(4,5-diphenethyl-4*H*-1,2,4-triazol-3-yl)-2-(1 *H*-indol-3-yl)ethyl)-2-amino-2-methylpropanamide,
**compound 118** *(R)-N*-(1-(4-(3,4-dichlorobenzyl)-5-benzyl-4*H* 1,2,4-triazol-3-yl)-2-(1 H-indol-3-yl)ethyl)-2-amino-2-methylpropanamide,
**compound 119** *(R)-*1-(5-(2-(1*H*-indol-3-yl)ethyl)-4-(2,4-dimethoxybenzyl)-4*H*-1,2,4-triazol-3-yl)-2-(1H-indol-3-yl)ethanamine,
**compound 120** (*R*)*-N*-(1-(4-(4-methoxybenzyl)-5-benzyl-4*H*-1,2,4-triazol-3-yl)-2-phenylethyl)-2-amino-2-methylpropanamide,
**compound 121** *(R)-N*-(1-(4-(4-fluorobenzyl)-5-phenethyl-4*H* 1,2,4-triazol-3-yl)-2-(1*H*-indol-3-yl)ethyl)-2-amino-2-methylpropanamide,
**compound 122** (*R*)-*N*-(1-(4-(3,4-dichlorobenzyl)-5-phenethyl-4*H*-1,2,4-triazol-3-yl)-2-(1H-indol-3-yl)ethyl)-2-amino-2-methylpropanamide,
**compound 124** *(R)-N*-(1-(4-(4-methylbenzyl)-5-benzyl-4*H*-1,2,4-triazol-3-yl)-2-(1*H* indol-3-yl)ethyl)-2-amino-2-methylpropanamide,
**compound 125** *(S)-N-*(1-(4-(4-methoxybenzyl)-5-(3-phenylpropyl)-4*H*-1,2,4-triazol-3-yl)-2-(1*H*-indol-3-yl)ethyl)-2-amino-2-methylpropanamide,
**compound 126** (*S*)-*N*-(1-(4-(4-methoxybenzyl)-5-benzyl-4*H*-1,2,4-triazol-3-yl)-2-(1 H-indol-3-yl)ethyl)-2-amino-2-methylpropanamide,
**compound 128** *N*-((*R*)-1-(4-(4-nitrobenzyl)-5-phenethyl-4*H*-1,2,4-triazol-3-yl)-2-(1 H-indol-3-yl)ethyl)-2-amino-2-methylpropanamide,
**compound 129** (*S*)-*N*-(1-(4-(4-methoxybenzyl)-5-phenethyl-4*H*-1,2,4-triazol-3-yl)-2-(1*H*-indol-3-yl)ethyl)-2-amino-2-methylpropanamide,
**compound 130** *(R)-N*-(1-(4-(4-methoxyphenethyl)-5-phenethyl-4*H* 1,2,4-triazol-3-yl)-2-(1-*H*-indol-3-yl) ethyl)-2-amino-2-methylpropanamide,
**compound 131** (*R*)-*N*-(2-(1*H*- indol-3-yl)-1-(5-phenethyl-4-(thiophen-2-ylmethyl)-4*H*-1,2,4-triazol-3-yl)ethyl)-2-amino-2-methylpropanamide,
**compound 132** (*R*)-*N*-(2-(1-*H*-indol-3-yl)-1-(5-phenethyl-4-(pyridin-2-ylmethyl)-4*H* 1,2,4-triazol-3-yl)ethyl)-2-amino-2-methylpropanamide,
**compound 133** (*R*)-*N*-(2-(1*H*-indol-3-yl)-1-(5-phenethyl-4-(pyridin-2-ylmethyl)-4*H*-1,2,4-triazol-3-yl)ethyl)piperidine-3-carboxamide,
**compound 134** (*S*)-*N*-((*R*)-1-(4-(4-ethylbenzyl)-5-phenethyl-4*H*-1,2,4-triazol-3-yl)-2-(1 *H*-indol-3-yl)ethyl)pyrrolidine-2-carboxamide,
**compound 135** *N*-((*R*)-1-(4-(4-ethylbenzyl)-5-phenethyl-4*H*-1,2,4-triazol-3-yl)-2-(1*H*-indol-3-yl)ethyl)-2-aminoacetamide,
**compound 136** *N*-((*R*)-1-(5-(2-(1 *H*-indol-3-yl)ethyl)-4-(4-methoxybenzyl)-4*H*-1,2,4-triazol-3-yl)-2-(1 *H*-indol-3-yl)ethyl)-2-(pyridin-4-yl)acetamide,
**compound 137** *(2R)-N-*((*R*)-1-(4-(4-ethylbenzyl)-5-phenethyl-4*H*-1,2,4-triazol-3-yl)-2-(1 *H*-indol-3-yl)ethyl)piperidine-2-carboxam ide,
**compound 138** *N*-((*R*)-1-(4-(4-ethylbenzyl)-5-phenethyl-4*H*-1,2,4-triazol-3-yl)-2-(1*H*-indol-3-yl)ethyl)picolinamide,
**compound 139** *N*-((*R*)-1-(4-(4-ethylbenzyl)-5-phenethyl-4*H*-1,2,4-triazol-3-yl)-2-(1 *H*-indol-3-yl)ethyl)-2-aminopyridine-3-carboxamide,
**compound 140** (2*S*)-*N*-((*R*)-1-(4-(4-ethylbenzyl)-5-phenethyl-4*H*-1,2,4-triazol-3-yl)-2-(1*H*-indol-3-yl)ethyl)-2-aminopropanamide,
**compound 141** N-((*R*)-1-(4-(4-ethylbenzyl)-5-phenethyl-4*H*-1,2,4-triazol-3-yl)-2-(1*H*-indol-3-yl)ethyl)isonicotinamide,
**compound 142** *N*-((*R*)-2-(1*H*-indol-3-yl)-1-(5-phenethyl-4-phenyl-4*H*-1,2,4-triazol-3-yl)ethyl)piperidine-4-carboxamide,
**compound 143** (2*S*)-N-((*R*)-2-(1*H*-indol-3-yl)-1-(5-phenethyl-4-phenyl-4*H*-1,2,4-triazol-3-yl)ethyl)pyrrolidine-2-carboxamide,
**compound 144** *N*-((*R*)-2-(1*H*-indol-3-yl)-1-(5-phenethyl-4-phenyl-4*H*-1,2,4-triazol-3-yl)ethyl)-2-aminoacetamide,
**compound 145** N-((*R*)-2-(1*H*-indol-3-yl)-1-(5-phenethyl-4-phenyl-4H-1,2,4-triazol-3-yl)ethyl)-2-(pyridin-2-yl)acetamide,
**compound 146** N-((*R*)-2-(1*H*-indol-3-yl)-1-(5-phenethyl-4-phenyl-4*H*-1,2,4-triazol-3-yl)ethyl)picolinamide,
**compound 147** *N*-((*R*)-1-(5-(2-(1*H*-indol-3-yl)ethyl)-4-(4-ethylphenyl)-4*H*-1,2,4-triazol-3-yl)-2-(1*H*-indol-3-yl)ethyl)picolinamide,
**compound 148** *N*-((*R*)-1-(5-(2-(1*H*-indol-3-yl)ethyl)-4-(4-ethylphenyl)-4*H*-1,2,4-triazol-3-yl)-2-(1*H*-indol-3-yl)ethyl)-2-(pyridin-2-yl)acetamide,
**compound 149** *N*-((*R*)-1-(5-(2-(1*H* indol-3-yl)ethyl)-4-(4-ethylphenyl)-4*H*-1,2,4-triazol-3-yl)-2-(1*H* indol-3-yl)ethyl)-2-aminoacetamide,
**compound 150** (2*S*)*-N*-((*R*)-1-(5-(2-(1*H*-indol-3-yl)ethyl)-4-(4-ethylphenyl)-4*H-*1,2,4- triazol-3-yl)-2-(1*H*-indol-3-yl)ethyl)pyrrolidine-2-carboxamide,
**compound 152** *N-*((*R*)-1-(5-(2*-*(1*H*-indol-3-yl)ethyl)-4-(4-methoxybenzyl)-4*H*-1,2,4-triazol-3-yl)-2-(1*H*-indol-3-yl)ethyl)-2-aminoacetamide,
**compound 153** *N*-((*R*)-1-(4-(4-methoxybenzyl)-5-phenethyl-4*H*-1,2,4-triazol-3-yl)-2-(1*H*-indol-3-yl)ethyl)-2-trans-aminocyclohexanecarboxamide,
**compound 154** N-((*R*)-1-(4-(4-ethylbenzyl)-5-phenethyl-4*H*-1,2,4-triazol-3-yl)-2-(1*H*-indol-3-yl)ethyl)-2-(pyridin-3-yl)acetamide,
**compound 155** (3*S*)-*N*-((*R*)-1-(4-(4-ethylbenzyl)-5-phenethyl-4*H*-1,2,4-triazol-3-yl)-2-(1*H*-indol-3-yl)ethyl)piperidine-3-carboxamide,
**compound 156** *N*-((*R*)-1-(4-(4-ethylbenzyl)-5-phenethyl-4*H*-1,2,4-triazol-3-yl)-2-(1*H*-indol-3-yl)ethyl)-2-aminobenzamide,
**compound 157** *N-((R)-*1-(5-(2-(1*H-*indol-3-yl)ethyl)-4-phenyl-4*H*-1,2,4-triazol-3-yl)-2-(1*H*-indol-3-yl)ethyl)picolinamide,
**compound 158** *N*-((*R*)-1-(5-(2-(1*H*-indol-3-yl)ethyl)-4-phenyl-4H-1,2,4-triazol-3-yl)-2-(1*H*-indol-3-yl)ethyl)piperidine-4-carboxamide,
**compound 159** *N*-((*R*)-2-(1*H*-indol-3-yl)-1-(4-(2,4-dimethoxyphenyl)-5-phenethyl-4*H*-1,2,4-triazol-3-yl)ethyl)picolinamide,
**compound 160** *N*-((*R*)-2-(1*H*-indol-3-yl)-1-(4-(2,4-dimethoxyphenyl)-5-phenethyl-4*H*-1,2,4-triazol-3-yl)ethyl)-2-(pyridin-2-yl)acetamide,
**compound 161** *N*-((*R*)-2-(1*H*-indol-3-yl)-1-(4-(2,4-dimethoxyphenyl)-5-phenethyl-4*H*-1,2,4-triazol-3-yl)ethyl)pyrazine-2-carboxamide,
**compound 162** *N*-((*R*)-2-(1*H*-indol-3-yl)-1-(4-(2,4-dimethoxyphenyl)-5-phenethyl-4*H*-1,2,4-triazol-3-yl)ethyl)-2-aminoacetamide,
**compound 163** *N*-((*R*)-2-(1*H*-indol-3-yl)-1-(4-(2,4-dimethoxyphenyl)-5-phenethyl-4*H*- 1,2,4-triazol-3-yl)ethyl)piperidine-4-carboxamide,
**compound 164** *N*-((*R*)-1-(5-benzyl-4-((pyridin-2-yl)methyl)-4*H*-1,2,4-triazol-3-yl)-2-(1*H*-indol-3-yl)ethyl)picolinamide,
**compound 165** *N*-((*R*)-1-(5-benzyl-4-((pyridin-2-yl)methyl)-4*H*-1,2,4-triazol-3-yl)-2-(1*H*-indol-3-yl)ethyl)-2-amino-acetamide,
**compound 166** *N*-((*R*)-1-(5-benzyl-4-((pyridin-2-yl)methyl)-4H-1,2,4-triazol-3-yl)-2-(1*H*-indol-3-yl)ethyl)piperidine-4-carboxamide,
**compound 167** *N*-((*R*)-1-(5-benzyl-4-((pyridin-4-yl)methyl)-4*H*-1,2,4-triazol-3-yl)-2-(1*H*-indol-3-yl)ethyl)-2-amino-2-methylpropanamide,
**compound 168** *N*-((*R*)-1-(5-(4-methoxybenzyl)-4-phenethyl-4*H*-1,2,4-triazol-3-yl)-2-(1*H*-indol-3-yl)ethyl)-2-amino-2-methylpropanamide,
**compound 169** *N*-((*R*)-1-(5-benzyl-4-((pyridin-4-yl)methyl)-4*H*-1,2,4-triazol-3-yl)-2-(1*H*-indol-3-yl)ethyl)picolinamide,
**compound 170** *N-(*(*R*)-1-(5-benzyl-4-((pyridin-4-yl)methyl)-4*H*-1 ,2,4-triazol-3-yl)-2-(1*H*-indol-3-yl)ethyl)2-amino-acetamide,
**compound 171** (*R*)-benzyl-3-(2-aminoisobutyramido)-3-(5-(2-(1*H*-indol-3-yl)ethyl)-4-(4-methoxybenzyl)-4*H*-1,2,4-triazol-3-yl)-propanoate,
**compound 172** *N-(*(*R*)-1-(5-benzyl-4-((pyridin-3-yl)methyl)-4*H*-1 ,2,4-triazol-3-yl)-2-(1*H*-indol-3-yl)ethyl)-2-amino-2-methylpropanamide,
**compound 173** *N*-((*R*)-1-(4-benzyl-5-phenethyl-4*H-*1 ,2,4-triazol-3-yl)-2-(1*H*-indol-3-yl)ethyl)-2-amino-2-methylpropanamide,
**compound 174** *N*-((*R*)-2-(1*H*-indol-3-yl)-1-( 4-methyl-5-phenethyl-4*H*-1,2,4-triazol-3-yl)ethyl)picolinamide,
**compound 175** *N*-((*R*)-1-(5-(2-(1*H*-indol-3-yl)ethyl)-4-phenyl-4*H*-1,2,4-triazol-3-yl)-2-(1*H-*indol-3-yl) ethyl) -2-amino-2-methylpropanamide,
**compound 176** *N*-((*R*)-1-(4-(4-methoxybenzyl)-5-phenethyl-4*H*-1,2,4-triazol-3-yl)-2-(1*H*-indol-3-yl)ethyl)benzamide,
**compound 177** (*R*)-1-(4-(2,4-dimethoxybenzyl)-5-phenethyl-4*H*-1,2,4-triazol-3-yl)-2-(1*H*-indol-3-yl)-N-phenylmethanesulfonylamine,
**compound 178** (*R*)-1-(4-(2,4-dimethoxybenzyl)-5-phenethyl-4*H*-1,2,4-triazol-3-yl)-2-(1*H*-indol-3-yl)-N-tosylethanamine,
**compound 179** *N-*((*R*)*-*1-(4-(2,4-dimethoxybenzyl)-5-phenethyl-4*H-*1,2,4-triazol-3-yl)-2-(1 *H*-indol-3-yl)ethyl)-2-amino-2-methylpropanamide,
**compound 180** *N-1-((R)-1-*(4-(2,4-dimethoxybenzyl)-5-phenethyl-4*H*-1,2,4-triazol-3-yl)-2-(1*H*-indol-3-yl)ethyl)ethane-1,2-diamine,
**compound 181** *N-((R)-1-*(4-((furan-2-yl)methyl)-5-phenethyl-4*H*-1,2,4-triazol-3-yl)-2-(1*H*-indol-3-yl)ethyl)-2-amino-2-methylpropanamide,
**compound 182** *N-((R)-1-*(4-((furan-2-yl)methyl)-5-phenethyl-4H-1,2,4-triazol-3-yl)-2-(1*H*-indol-3-yl)ethyl)picolinamide,
**compound 183** *N-((R)-1-*(4-((furan-2-yl)methyl)-5-phenethyl-4*H*-1,2,4-triazol-3-yl)-2-(1*H*-indol-3-yl)ethyl)piperidine-4-carboxamide,
**compound 184** *N-((R)-1-*(4-(4-methoxybenzyl)-5-phenethyl-4*H*-1,2,4-triazol-3-yl)-2-(1*H*-indol-3-yl)ethyl)-tetrahydro-2 H-pyran-4-carboxamide,
**compound 185** *N*- ((*R*)-1-(5-((1*H*- indol-3-yl)methyl)-4-(3-methoxybenzyl)-4H 1,2,4-triazol-3-yl)-2-(1*H*-indol-3-yl)ethyl)-2-amino-2-methylpropanamide,
**compound 186** (*2S*)-*N*-((*R*)-1-(4-(4-methoxybenzyl)-5-phenethyl-4*H*-1,2,4-triazol-3-yl)-2-(1 *H*-indol-3-yl) ethyl) -2-amino-3-phenylpropanamide,
**compound 187** (*R*)-1-(5-(2-(1 H-indol-3-yl)ethyl)-4-(2,4-dimethoxybenzyl)-4H-1,2,4-triazol-3-yl)-2-(1*H*-indol-3-yl)-N-tosylethanamine,
**compound 188** *N*-((*R*)-1-(4-(2,4-dimethoxybenzyl)-5-phenethyl-4*H*-1,2,4-triazol-3-yl)-2-(1 *H*-indol-3-yl)ethyl)-4-azidobenzamide,
**compound 189** *N*-benzyl-(*R*)-1-(4-(2,4-dimethoxybenzyl)-5-phenethyl-4*H*-1,2,4-triazol-3-yl)-2-(1*H*-indol-3-yl)ethanamine,
**compound 190** *(2S)-N-((R)-1* -(5-(2-(1H-indol-3-yl)ethyl)-4-(4-methoxybenzyl)-4*H-*1,2,4-triazol-3-yl)-2-(1*H*-indol-3-yl) ethyl)-2,5-dihydro-1*H*-pyrrole-2-carboxamide.

8. The use of a compound as claimed in any of claims 1 to 7 for the manufacture of a medicament for the treatment or prophylaxis of physiological and/or pathophysiological conditions in mammals.

9. The use as claimed in any of claims 1 to 7, where the treatment is achieved by modulation of one or more G-protein coupled receptors (GPCR).

10. The use as claimed in claim 9, where the compound is a G-protein coupled receptor (GPCR) antagonist.

11. The use as claimed in claim 9, where the compound is a G-protein coupled receptor (GPCR) agonist.

12. The use as claimed in any of claims 1 to 7 and 9 to 11, where the at least one G-protein coupled receptor (GPCR) is selected from the group consisting of: "5-hydroxytryptamine (serotonin) receptor, 5-hydroxytryptamine (serotonin) receptor 1 A, 5-hydroxytryptamine (serotonin) receptor 1 B, 5-hydroxytryptamine (serotonin) receptor 1 D, 5-hydroxytryptamine (serotonin) receptor 1 E, 5-hydroxytryptamine (serotonin) receptor 1F, 5-hydroxytryptamine (serotonin) receptor 2A, 5-hydroxytryptamine (serotonin) receptor 2B, 5-hydroxytryptamine (serotonin) receptor 2C, 5-hydroxytryptamine (serotonin) receptor 4, 5-hydroxytryptamine (serotonin) receptor 5A, 5-hydroxytryptamine (serotonin) receptor 6, 5-hydroxytryptamine (serotonin) receptor 7, muscarinic cholinergic receptor, muscarinic cholinergic receptor 1, muscarinic cholinergic receptor 2, muscarinic cholinergic receptor 3, muscarinic cholinergic receptor 4, muscarinic cholinergic receptor 5, Alpha-adrenergic receptor, Alpha-1-adrenergic receptor, Alpha-1A-adrenergic receptor, Alpha-1 B-adrenergic receptor, Alpha-1 D-adrenergic receptor, Alpha-2-adrenergic receptor, Alpha-2A-adrenergic receptor, Alpha-2B-adrenergic receptor, Alpha-2C-adrenergic receptor, Beta-adrenergic receptor, Beta-1-adrenergic receptor, Beta-2-adrenergic receptor, Beta-3-adrenergic receptor, angiotensin II receptor, angiotensin II receptor type 1, angiotensin II receptor type 2, neuromedin B receptor, gastrin-releasing peptide receptor, bombesin-like receptor 3, calcitonin receptor, calcitonin receptor-like, cannabinoid receptor, cannabinoid receptor 1, cannabinoid receptor 2, cholecystokinin receptor, cholecystokinin A receptor, cholecystokinin B receptor, corticotropin releasing hormone receptor, corticotropin releasing hormone receptor 1, corticotropin releasing hormone receptor 2, dopamine receptor, dopamine receptor D1, dopamine receptor D2, dopamine receptor D3, dopamine receptor D4, dopamine receptor D5, glucagon-like peptide receptor, glucagon-like peptide 1 receptor, glucagon-like peptide 2 receptor, follicle stimulating hormone receptor, histamine receptor, histamine receptor H1, histamine receptor H2, histamine receptor H3, histamine receptor H4, endothelial differentiation lysophosphatidic acid G-protein-coupled receptor, endothelial differentiation lysophosphatidic acid G-protein-coupled receptor 2, endothelial differentiation lysophosphatidic acid G-protein-coupled receptor 4, endothelial differentiation lysophosphatidic acid G-protein-coupled receptor 7, melanin-concentrating hormone receptor, melanin-concentrating hormone receptor 1, melanin-concentrating hormone receptor 2, melanocortin receptor, melanocortin 1 receptor (alpha melanocyte stimulating hormone receptor), melanocortin 2 receptor (adrenocorticotropic hormone receptor), melanocortin 3 receptor, melanocortin 4 receptor, melanocortin 5 receptor, neuropeptide FF receptor, neuropeptide FF receptor 1, neuropeptide FF receptor 2, neuropeptides B/UV receptor, neuropeptides B/UV receptor 1, neuropeptides B/UV receptor 2, neuropeptide Y receptor, neuropeptide Y receptor Y1, neuropeptide Y receptor Y2, neuropeptide Y receptor Y4 (pancreatic polypeptide receptor 1), neuropeptide Y receptor Y5, opioid receptor, opioid receptor delta 1, opioid receptor kappa 1, opioid receptor mu 1, opiate receptor-like 1, hypocretin (orexin) receptor, hypocretin (orexin) receptor 1, hypocretin (orexin) receptor 2, G protein-coupled receptor 103 (GPR103), G protein-coupled receptor 103A (GPR103A), G protein-coupled receptor 103B (GPR103B), prolactin releasing hormone receptor, somatostatin receptor, somatostatin receptor 1, somatostatin receptor 2, somatostatin receptor 3, somatostatin receptor 4, somatostatin receptor 5, galanin receptor, galanin receptor 1, galanin receptor 2, galanin receptor 3, cortistatin receptor, cortistatin receptor MAS-related GPR member X1, cortistatin receptor MAS-related GPR member X2, cortistatin receptor MAS-related GPR member X3, and/or cortistatin receptor MAS-related GPR member X4" and preferably is selected from the group consisting of: "5-hydroxytryptamine (serotonin) receptor 2C, muscarinic cholinergic receptor 3, Beta-3-adrenergic receptor, angiotensin I receptor type 1, angiotensin I receptor type 2, gastrin-releasing peptide receptor, bombesin-like receptor 3, calcitonin receptor, calcitonin receptor-like, cannabinoid receptor 1, cannabinoid receptor 2, cholecystokinin A receptor, cholecystokinin B receptor, corticotropin releasing hormone receptor 1, corticotropin releasing hormone receptor 2, dopamine receptor D1, dopamine receptor D2, dopamine receptor D3, dopamine receptor D4, dopamine receptor D5, glucagon-like peptide 1 receptor, follicle stimulating hormone receptor, histamine receptor H1, histamine receptor H3, endothelial differentiation lysophosphatidic acid G-protein-coupled receptor 2, melanin-concentrating hormone receptor 1, melanocortin 1 receptor (alpha melanocyte stimulating hormone receptor), melanocortin 3 receptor, melanocortin 4 receptor, neuropeptide FF receptor 1, neuropeptide FF receptor 2, neuropeptides B/W receptor 1, neuropeptides B/W receptor 2, neuropeptide Y receptor Y1, neuropeptide Y receptor Y2, neuropeptide Y receptor Y5, opioid receptor kappa 1, hypocretin (orexin) receptor 1, hypocretin (orexin) receptor 2, G protein-coupled receptor 103 (GPR103), G protein-coupled receptor 103A (GPR103A), G protein-coupled receptor 103B (GPR103B), prolactin releasing hormone receptor and/or galanin receptor 1" and most preferably is selected from the group consisting of: "cholecystokinin A receptor, cholecystokinin B receptor, neuropeptide FF receptor 1, neuropeptide FF receptor 2, neuropeptide Y receptor Y1, neuropeptide Y receptor Y2, neuropeptide Y receptor Y5, hypocretin (orexin) receptor 1, hypocretin (orexin) receptor 2, G protein-coupled receptor 103 (GPR103), G protein-coupled receptor 103A (GPR103A), G protein-coupled receptor 103B (GPR103B), galanin receptor 1 and/or cortistatin receptor MAS-related GPR member X2".

13. The use of a compound as claimed in any of claims 1 to 7 and 9 to 12 for the manufacture of a medicament for the treatment or prophylaxis of physiological and/or pathophysiological conditions in mammals, where the physiological and/or pathophysiological conditions are mediated by at least one G-protein coupled receptor (GPCR) according to claim 1 or 12 and at least one other receptor selected from the group consisting of: "GHS receptors, GHS type 1 receptor, GHS-R 1 a, GHS-R 1b, motilin receptor, motilin receptor 1 a, neurotensin receptor, TRH receptor, GPR38 (FM1), GPR39 (FM2), FM3, GHS-R subtype, GHS binding site, cardiac GHS-R, mammary GHS-R".

14. The use as claimed in claim 13, where the treatment is achieved by modulation of one or more of the other receptors.

15. The use as claimed in claim 14, where the compound is an antagonist of the one or more other receptors.

16. The use as claimed in claim 14, where the compound is an agonist of the one or more other receptors.

17. The use as claimed in any of claims 1 to 16, where the physiological and/or pathophysiological conditions are selected from the group consisting of "metabolic disorders, lipid metabolism, disorders of lipid metabolism, adipogenesis, adiposity, anorexia, body weight gain, body weight reduction, bulimia, cachexia, diabetes, diabetes type I, diabetes type II, energy balance, energy homeostasis, food intake, hyperlipidemia, hyperphagia, insulin resistance in the heart, insulin resistance in type 2 diabetic patients, insulin resistance including NIDDM, metabolic homeostasis maintenance, obesity, overweight, short-, medium- and/or long-term regulation of energy balance, short-, medium- and/or long-term regulation (stimulation and/or inhibition) of food intake as well as diseases for which above mentioned physiological and/or pathophysiological conditions are risk factors and which are selected from the group consisting of: "premature death, diabetes, type 2 diabetes, heart disease, heart attack, congestive heart failure, stroke, hypertension (high blood pressure), gallbladder disease, osteoarthritis, sleep apnea, asthma, breathing problems, endometrial cancer, colon cancer, kidney cancer, gallbladder cancer, postmenopausal breast cancer, hypercholesterolemia (high blood cholesterol), insulin resistance, complications of pregnancy, menstrual irregularities, hirsutism, stress incontinence, increased surgical risk, psychological disorders such as depression" and preferably are selected from the group consisting of "adipogenesis, adiposity, cachexia, diabetes, diabetes type I, diabetes type II, energy balance, energy homeostasis, food intake, hyperlipidemia, hyperphagia, obesity, short-, medium- and/or long-term regulation of energy balance, short-, medium- and/or long-term regulation (stimulation and/or inhibition) of food intake".

18. The use as claimed in any of claims 1 to 17, where such medicament comprises at least one additional pharmacologically active substance.

19. The use as claimed in any of claims 1 to 17, where the medicament is applied before and/or during and/or after treatment with at least one additional pharmacologically active substance.

20. The use as claimed in any of claims 18 to 19, where such at least one additional pharmacologically active substance is selected from the group consisting of: "GHS receptor agonist and/or antagonist, GHS type 1 receptor agonist and/or antagonist, GHS-R 1 a agonist and/or antagonist, GHS-R 1 b agonist and/or antagonist, motilin receptor agonist and/or antagonist, motilin receptor 1 a agonist and/or antagonist, neurotensin receptor agonist and/or antagonist, TRH receptor agonist and/or antagonist, GPR38 (FM1) agonist and/or antagonist, GPR39 (FM2) agonist and/or antagonist, FM3 agonist and/or antagonist, GHS-R subtype agonist and/or antagonist, GHS binding site agonist and/or antagonist, cardiac GHS-R agonist and/or antagonist, mammary GHS-R agonist and/or antagonist, endocannabinoid receptor agonist and/or antagonist, CB1 receptor agonist and/or antagonist, rimonabant [1 H-Pyrazole-3-carboxamide, 5-(4-chlorophenyl)-1-(2,4-dichlorophenyl)-4-methyl-N-1-piperidinyl-, monohydrochloride], sibutramine [1-(4-chlorophenyl)-N,N-dimethyl-alpha-(2-methylpropyl)-cyclobutane-methanamine], sibutramide hydrochloride monohydrate".


SEQUENCE LISTING

<110>  Zentaris GmbH
<120>  Novel triazole derivatives as ligands of G-protein coupled re-
ceptors
<130>  07/02 Z
<160>  13
<170>  PatentIn version 3.3

<210>  1
<211>  1296
<212>  DNA
<213>  human
<400>  1
atgcaggcgc ttaacattac cccggagcag ttctctcggc tgctgcggga ccacaacctg   60
acgcgggagc agttcatcgc agttcatcgc ctgcgaccgc tcgtctacac cccagagctg  120
ccgggacgcg ccaagctggc cctcgtgctc accggcgtgc tcatcttcgc cctggcgctc  180
tttggcaatg ctctggtgtt ctacgtggtg acccgcagca aggccatgcg caccgtcacc  240
aacatcttta tctgctcctt ggcgctcagt gacctgctca tcaccttctt ctgcattccc  300
gtcaccatgc tccagaacat ttccgacaac tggctggggg gtgctttcat ttgcaagatg  360
gtgccatttg tccagtctac cgctgttgtg acagaaatcc tcactatgac ctgcattgct  420
gtggaaaggc accagggact tgtgcatcct tttaaaatga agtggcaata caccaaccga  480
agggctttca caatgctagg tgtggtctgg ctggtggcag tcatcgtagg atcacccatg  540
tggcacgtgc aacaacttga gatcaaatat gacttcctat atgaaaagga acacatctgc  600
tgcttagaag agtggaccag ccctgtgcac cagaagatct acaccacctt catccttgtc  660
atcctcttcc tcctgcctct tatggtgatg cttattctgt acagtaaaat tggttatgaa  720
ctttggataa agaaaagagt tggggatggt tcagtgcttc gaactattca tggaaaagaa  780
atgtccaaaa tagccaggaa gaagaaacga gctgtcatta tgatggtgac agtggtggct  840
ctctttgctg tgtgctgggc accattccat gttgtccata tgatgattga atacagtaat  900
tttgaaaagg aatatgatga tgtcacaatc aagatgattt ttgctatcgt gcaaattatt  960
ggattttcca actccatctg taatcccatt gtctatgcat ttatgaatga aaacttcaaa 1020
aaaaatgttt tgtctgcagt ttgttattgc atagtaaata aaaccttctc tccagcacaa 1080

aggcatggaa attcaggaat tacaatgatg cggaagaaag caaagttttc cctcagagag 1140

aatccagtgg aggaaaccaa aggagaagca ttcagtgatg gcaacattga agtcaaattg 1200

tgtgaacaga cagaggagaa gaaaaagctc aaacgacatc ttgctctctt taggtctgaa 1260

ctggctgaga attctccttt agacagtggg cattaa 1296

<210> 2
<211> 1816
<212> DNA
<213> mouse
<400> 2

gccagagcag gaggctggca ggactctgcg cacagcatgc aggcgctcaa catcaccgcg 60

gagcagtttt cccggctgct gagcgcgcac aacctgactc gggaacagtt cattcatcgc 120

tatgggctgc gaccgctggt ctacactccg gagctgcccg cgcgcgctaa actggccttt 180

gcgctggctg gagcactcat ttttgccctg gcgctctttg gcaactctct ggtcatctat 240

gtggtgaccc gcagcaaggc catgcgcacc gtcaccaaca tcttcatctg ctctctggca 300

ctcagtgatc tgctcattgc cttcttctgc atccccgtca cgatgctcca gaacatctcc 360

gacaagtggc tgggtggtgc cttcatctgc aagatggtgc ccttcgtcca gtccactgct 420

gttgtgacgg aaatcctcac catgacttgc atcgctgttg agaggcacca aggactcatc 480

catcctttta aaatgaagtg gcagtacact acccgaaggg ctttcacaat cttgggtgtg 540

gtctggttgg cagccatcat cgtaggatca cccatgtggc acgtacaacg cctcgagatt 600

aagtatgact tcctctatga gaaagaacat gtctgctgtt tggaagagtg ggccagcccc 660

atgcaccaga gaatctacac caccttcatc ctcgtcatcc tcttcctcct gccgcttgtg 720

gtgatgcttg tcctctacag caagattggc tatgaactgt ggatcaagaa gagagttgga 780

gacagttcag cacttcagac tatccacggg aaagaaatgt ccaaaatagc caggaagaag 840

aagcgggctg tcgttatgat ggtgacagtg gtggctctct tcgctgcgtg ctgggcacct 900

ttccatgttg ttcacatgat ggttgagtac agtaactttg aaaaagagta tgatgatgtc 960

acaatcaaga tggtttttgc tgttgcacaa acaattggct ttttcaactc catctgtaat 1020

ccctttgtgt atgcatttat gaatgaaaac ttcaaaaaga attttttgtc tgcggtttgt 1080

tattgcatag taagagaaac cttctcccca ggacagaagc ctggaaattc tgggatttca 1140

atgatgcaaa agagagcaaa gttatcacga tcacagcgtc cagtggcgga agccaaagga 1200

gacttattca gcgatgccaa cgttgatgtc aaattgtgtg agcagccagg ggagaaaagg 1260

caactcaagc gacagcttgc cttctttagt tctgaacttt ctgaaaactc tactttcggc 1320

agtggacatg aactgtaatg atatcctcat agctaatatc atttgtatgg aaagttattt 1380

taagcaaagg tcaggactat tttttttaaa tgacaagaag agaaacaaga catgttttcc 1440

atttaaatga acataataca taacactgta actttgaaaa attattataa cagctttgta 1500

gatgataaaa gtagattttt gaaagtcttc gtacataata aagcagtggt tttggcagca 1560

gttttatcca tgtagtcaat gtaatgtgac ttttatgtat tgctacactg gatgaaaatt 1620

attaaaattg tgtcatcatc cttgaatatt aaacatctga acatcataat gtagtttgta 1680

gtgtgctgta aacgtttgta aaatcagcct ttggaactga catctgtgcc ataattaaaa 1740

aatcaaggag gatgaagaat caggcaagtg acaactaata aaagttaaga gataaatgtc 1800

aaaaaaaaaa aaaaaa 1816

<210> 3
<211> 1772
<212> DNA
<213> mouse
<400> 3

atgtcgtgga acttgaccgc ggagcagctc tcggcgctgc ttcggctgca caacctgacg 60

cgcgctcagt tcatcgcgca ctatggcctg cggccactgg tgctcacccc gcagcttccc 120

gcgcgcgcca ggctggccct cctgctggtc ggcatgctca tctttgccct ggcgctcttc 180

ggcaacgccc tggtagtcta tgtggtgacc cgcagcaagg ccatgcgcac cgtcaccaac 240

atcttcatct gctccctggc actcagcgac ctgctcatcg tcttcttctg catcccggtc 300

accatgctcc agaacgtctc ggacacctgg ctgggggtgg ccttcatttg caaaatggtc 360

ccatttgtcc agtgcactgc cattgtgaca gaaatcctta ctatgacctg cattgctgtg 420

gaaaggcacc agggacttgt ccatcctttt aaaatgaagc ggcagtacac caatcaaaga 480

gctttcacaa tgctaggtgt ggtgtggctg gtggccatca tcataggatc acccatgtgg 540

catgtgcagc gacttgagat taagtatgac ttcctatatg aaaaagaaca catctgctgc 600

ctggaagagt ggagcagccc cgtgcaccag aagatctaca ccaccttcat ccttgtcacc 660

ctcttcctgc taccactgtt gctgctctct gtcctctacg ggaaaatcgg ttatgagctt 720

tggatcaaga aaagaatcgg ggatggctca gtgctccgaa ctattcatgg aaaagaaatg 780

ttcaaaatag ccagaaagaa gaagcgagct gtgatcatga tggtgacagt cgtggttctc 840

tttgctgtgt gctgggcacc tttccacatc gttcacatga tgattgaata cagtaatttt 900

gaaaaggaat atgatgaagt cacaatcaag atgatttttg ctatagtgca aataattgga 960

tttttcaact ccatctgtaa tcccattatt tatgcactta tgaatgaaaa cttcaaaaaa 1020

aactttgtgt ctgccgtttg ctattgcatt gtgaaggaaa caccttcttc agcacggaag 1080

catggaagtt caggagctat ggtgatgcac aggagggcaa agttagctgc aagagagaat 1140

cctgtagaga tcaaaggaga agcatttggg ggcagcaaca tcgatatcaa gtggtgtgaa 1200

cagccagaaa agaagaagag gagatcaaaa gtggcatctt gtcctcttta gttccgaatt 1260

tctgagagct ctgctgtaga cgtgaacact gtaccaatgt cttcagaatg agtatctgtc 1320

atactgtaat cgaaagaaaa tgattttgag aaaaagccag agagctttca tattaaaaat 1380

gttgacaaac actcagaagg cagggacagg ggattcaaga gtttaaagtc atccttagct 1440

gcacgataag tttgaggata acctgggcta caagagaccc tgtctcaaga agccataata 1500

attaaaacaa ccatccttaa ctaatgataa tgacaaagta tttttccatt gaaaatacat 1560

gtaagctgca attttgaaaa attattgaac cacccttgtg attaatagat gaagtttaaa 1620

aaaatttaaa tgtgttttta ttgtatgtat atggttggtt tacctgtgta tatgtctttc 1680

agtaacttgt ataaaactca atgatctcag ctagtaactt tcttctgtgt ggtcaatgtg 1740

atatgatttc ctatatattg ctaaattgaa tg 1772

<210> 4
<211> 1101
<212> DNA
<213> human
<400> 4

atgtggaacg cgacgcccag cgaagagccg gggttcaacc tcacactggc cgacctggac 60

tgggatgctt cccccggcaa cgactcgctg ggcgacgagc tgctgcagct cttccccgcg 120

ccgctgctgg cgggcgtcac agccacctgc gtggcactct tcgtggtggg tatcgctggc 180

aacctgctca ccatgctggt ggtgtcgcgc ttccgcgagc tgcgcaccac caccaacctc 240

tacctgtcca gcatggcctt ctccgatctg ctcatcttcc tctgcatgcc cctggacctc 300

gttcgcctct ggcagtaccg gccctggaac ttcggcgacc tcctctgcaa actcttccaa 360

ttcgtcagtg agagctgcac ctacgccacg gtgctcacca tcacagcgct gagcgtcgag 420

cgctacttcg ccatctgctt cccactccgg gccaaggtgg tggtcaccaa ggggcgggtg 480

aagctggtca tcttcgtcat ctgggccgtg gccttctgca gcgccgggcc catcttcgtg 540

ctagtcgggg tggagcacga gaacggcacc gacccttggg acaccaacga gtgccgcccc 600

accgagtttg cggtgcgctc tggactgctc acggtcatgg tgtgggtgtc cagcatcttc 660

ttcttccttc ctgtcttctg tctcacggtc ctctacagtc tcatcggcag gaagctgtgg 720

cggaggaggc gcggcgatgc tgtcgtgggt gcctcgctca gggaccagaa ccacaagcaa 780

accgtgaaaa tgctggctgt agtggtgttt gccttcatcc tctgctggct ccccttccac 840

gtagggcgat atttattttc caaatccttt gagcctggct ccttggagat tgctcagatc 900

agccagtact gcaacctcgt gtcctttgtc ctcttctacc tcagtgctgc catcaacccc 960

attctgtaca acatcatgtc caagaagtac cgggtggcag tgttcagact tctgggattc 1020

gaacccttct cccagagaaa gctctccact ctgaaagatg aaagttctcg ggcctggaca 1080

gaatctagta ttaatacatg a 1101

<210> 5
<211> 870
<212> DNA
<213> human
<400> 5

atgtggaacg cgacgcccag cgaagagccg gggttcaacc tcacactggc cgacctggac 60

tgggatgctt cccccggcaa cgactcgctg ggcgacgagc tgctgcagct cttccccgcg 120

ccgctgctgg cgggcgtcac agccacctgc gtggcactct tcgtggtggg tatcgctggc 180

aacctgctca ccatgctggt ggtgtcgcgc ttccgcgagc tgcgcaccac caccaacctc 240

tacctgtcca gcatggcctt ctccgatctg ctcatcttcc tctgcatgcc cctggacctc 300

gttcgcctct ggcagtaccg gccctggaac ttcggcgacc tcctctgcaa actcttccaa 360

ttcgtcagtg agagctgcac ctacgccacg gtgctcacca tcacagcgct gagcgtcgag 420

cgctacttcg ccatctgctt cccactccgg gccaaggtgg tggtcaccaa ggggcgggtg 480

aagctggtca tcttcgtcat ctgggccgtg gccttctgca gcgccgggcc catcttcgtg 540

ctagtcgggg tggagcacga gaacggcacc gacccttggg acaccaacga gtgccgcccc 600

accgagtttg cggtgcgctc tggactgctc acggtcatgg tgtgggtgtc cagcatcttc 660

ttcttccttc ctgtcttctg tctcacggtc ctctacagtc tcatcggcag gaagctgtgg 720

cggaggaggc gcggcgatgc tgtcgtgggt gcctcgctca gggaccagaa ccacaagcaa 780

accgtgaaaa tgctgggtgg gtctcagcgc gcgctcaggc tttctctcgc gggtcctatc 840

ctctccctgt gccttctccc ttctctctga 870

<210> 6

<211> 23

<212> DNA

<213> artificial

<220>

<223> artificial

<400> 6

tctttggcaa ctctctggtc atc 23

<210> 7

<211> 22

<212> DNA

<213> artificial

<220>

<223> artificial

<400> 7

cttcgggtag tgtactgcca ct 22

<210> 8

<211> 24

<212> DNA

<213> artificial

<220>

<223> artificial

<400> 8

cgatatcaag tggtgtgaac agcc 24

<210> 9

<211> 20

<212> DNA

<213> artificial

<220>
<223>  artificial
<400>  9
gggtctcttg tagcccaggt                                                    20

<210>  10
<211>  21
<212>  DNA
<213>  artificial
<220>
<223>  artificial
<400>  10
tctgccgtcc ttaccatctc a                                                  21

<210>  11
<211>  22
<212>  DNA
<213>  artificial
<220>
<223>  artificial
<400>  11
tctcaggact gtcccaaagg ag                                                 22

<210>  12
<211>  23
<212>  DNA
<213>  artificial
<220>
<223>  artificial
<400>  12
ggaccagagc gaaagcattt gcc                                                23

<210>  13
<211>  22
<212>  DNA

<213>  artificial

<220>

<223>  artificial

<400>  13

tcaatctcgg gtggctgaac gc                                                22

SEQUENCE LISTING

<110> Zentaris GmbH

<120> Novel triazole derivatives as ligands of G-protein coupled

<130> 07/02 Z

<140> 07102360.0
<141> 2007-02-14

<160> 13

<170> PatentIn version 3.3

<210> 1
<211> 1296
<212> DNA
<213> Homo sapiens

<400> 1
atgcaggcgc ttaacattac cccggagcag ttctctcggc tgctgcggga ccacaacctg 60

acgcgggagc agttcatcgc agttcatcgc ctgcgaccgc tcgtctacac cccagagctg 120

ccgggacgcg ccaagctggc cctcgtgctc accggcgtgc tcatcttcgc cctggcgctc 180

tttggcaatg ctctggtgtt ctacgtggtg acccgcagca aggccatgcg caccgtcacc 240

aacatcttta tctgctcctt ggcgctcagt gacctgctca tcaccttctt ctgcattccc 300

gtcaccatgc tccagaacat ttccgacaac tggctggggg gtgctttcat ttgcaagatg 360

gtgccatttg tccagtctac cgctgttgtg acagaaatcc tcactatgac ctgcattgct 420

gtggaaaggc accagggact tgtgcatcct tttaaaatga agtggcaata caccaaccga 480

agggctttca caatgctagg tgtggtctgg ctggtggcag tcatcgtagg atcacccatg 540

tggcacgtgc aacaacttga gatcaaatat gacttcctat atgaaaagga acacatctgc 600

tgcttagaag agtggaccag ccctgtgcac cagaagatct acaccacctt catccttgtc 660

atcctcttcc tcctgcctct tatggtgatg cttattctgt acagtaaaat tggttatgaa 720

ctttggataa agaaaagagt tggggatggt tcagtgcttc gaactattca tggaaaagaa 780

atgtccaaaa tagccaggaa gaagaaacga gctgtcatta tgatggtgac agtggtggct 840

ctctttgctg tgtgctgggc accattccat gttgtccata tgatgattga atacagtaat 900

tttgaaaagg aatatgatga tgtcacaatc aagatgattt ttgctatcgt gcaaattatt 960

ggattttcca actccatctg taatcccatt gtctatgcat ttatgaatga aaacttcaaa 1020

aaaaatgttt tgtctgcagt ttgttattgc atagtaaata aaaccttctc tccagcacaa 1080

aggcatggaa attcaggaat tacaatgatg cggaagaaag caaagttttc cctcagagag 1140

aatccagtgg aggaaaccaa aggagaagca ttcagtgatg gcaacattga agtcaaattg 1200

tgtgaacaga cagaggagaa gaaaaagctc aaacgacatc ttgctctctt taggtctgaa 1260

ctggctgaga attcctcttt agacagtggg cattaa 1296

<210> 2
<211> 1816

<212>  DNA
<213>  Mus musculus

<400>  2
gccagagcag gaggctggca ggactctgcg cacagcatgc aggcgctcaa catcaccgcg      60
gagcagtttt cccggctgct gagcgcgcac aacctgactc gggaacagtt cattcatcgc     120
tatgggctgc gaccgctggt ctacactccg gagctgcccg cgcgcgctaa actggccttt     180
gcgctggctg gagcactcat ttttgccctg gcgctctttg gcaactctct ggtcatctat     240
gtggtgaccc gcagcaaggc catgcgcacc gtcaccaaca tcttcatctg ctctctggca     300
ctcagtgatc tgctcattgc cttcttctgc atccccgtca cgatgctcca gaacatctcc     360
gacaagtggc tgggtggtgc cttcatctgc aagatggtgc ccttcgtcca gtccactgct     420
gttgtgacgg aaatcctcac catgacttgc atcgctgttg agaggcacca aggactcatc     480
catcctttta aaatgaagtg gcagtacact acccgaaggg ctttcacaat cttgggtgtg     540
gtctggttgg cagccatcat cgtaggatca cccatgtggc acgtacaacg cctcgagatt     600
aagtatgact tcctctatga gaaagaacat gtctgctgtt tggaagagtg ggccagcccc     660
atgcaccaga gaatctacac caccttcatc ctcgtcatcc tcttcctcct gccgcttgtg     720
gtgatgcttg tcctctacag caagattggc tatgaactgt ggatcaagaa gagagttgga     780
gacagttcag cacttcagac tatccacggg aaagaaatgt ccaaaatagc caggaagaag     840
aagcgggctg tcgttatgat ggtgacagtg gtggctctct tcgctgcgtg ctgggcacct     900
ttccatgttg ttcacatgat ggttgagtac agtaactttg aaaaagagta tgatgatgtc     960
acaatcaaga tggtttttgc tgttgcacaa acaattggct tttcaactcc catctgtaat    1020
ccctttgtgt atgcatttat gaatgaaaac ttcaaaaaga attttttgtc tgcggtttgt    1080
tattgcatag taagagaaac cttctcccca ggacagaagc ctggaaattc tgggatttca    1140
atgatgcaaa agagagcaaa gttatcacga tcacagcgtc cagtggcgga agccaaagga    1200
gacttattca gcgatgccaa cgttgatgtc aaattgtgtg agcagccagg ggagaaaagg    1260
caactcaagc gacagcttgc cttctttagt tctgaacttt ctgaaaactc tactttcggc    1320
agtggacatg aactgtaatg atatcctcat agctaatatc atttgtatgg aaagttattt    1380
taagcaaagg tcaggactat tttttttaaa tgacaagaag agaaacaaga catgttttcc    1440
atttaaatga acataataca taacactgta actttgaaaa attattataa cagctttgta    1500
gatgataaaa gtagattttt gaaagtcttc gtacataata aagcagtggt tttggcagca    1560
gttttatcca tgtagtcaat gtaatgtgac ttttatgtat tgctacactg gatgaaaatt    1620
attaaaattg tgtcatcatc cttgaatatt aaacatctga acatcataat gtagtttgta    1680
gtgtgctgta aacgtttgta aaatcagcct ttggaactga catctgtgcc ataattaaaa    1740
aatcaaggag gatgaagaat caggcaagtg acaactaata aaagttaaga gataaatgtc    1800
aaaaaaaaaa aaaaaa                                                   1816

<210>  3

<211>  1772
<212>  DNA
<213>  Mus musculus

<400>  3
atgtcgtgga acttgaccgc ggagcagctc tcggcgctgc ttcggctgca caacctgacg      60

cgcgctcagt tcatcgcgca ctatgggctg cggccactgg tgctcacccc gcagcttccc     120

gcgcgcgcca ggctggccct cctgctggtc ggcatgctca tctttgccct ggcgctcttc     180

ggcaacgccc tggtagtcta tgtggtgacc cgcagcaagg ccatgcgcac cgtcaccaac     240

atcttcatct gctccctggc actcagcgac ctgctcatcg tcttcttctg catcccggtc     300

accatgctcc agaacgtctc ggacacctgg ctggggggtg ccttcatttg caaaatggtc     360

ccatttgtcc agtgcactgc cattgtgaca gaaatcctta ctatgacctg cattgctgtg     420

gaaaggcacc agggacttgt ccatcctttt aaaatgaagc ggcagtacac caatcaaaga     480

gctttcacaa tgctaggtgt ggtgtggctg gtggccatca tcataggatc acccatgtgg     540

catgtgcagc gacttgagat taagtatgac ttcctatatg aaaaagaaca catctgctgc     600

ctggaagagt ggagcagccc cgtgcaccag aagatctaca ccaccttcat ccttgtcacc     660

ctcttcctgc taccactgtt gctgctctct gtcctctacg ggaaaatcgg ttatgagctt     720

tggatcaaga aaagaatcgg ggatggctca gtgctccgaa ctattcatgg aaaagaaatg     780

ttcaaaatag ccagaaagaa gaagcgagct gtgatcatga tggtgacagt cgtggttctc     840

tttgctgtgt gctgggcacc tttccacatc gttcacatga tgattgaata cagtaatttt     900

gaaaaggaat atgatgaagt cacaatcaag atgatttttg ctatagtgca aataattgga     960

tttttcaact ccatctgtaa tcccattatt tatgcactta tgaatgaaaa cttcaaaaaa    1020

aactttgtgt ctgccgtttg ctattgcatt gtgaaggaaa caccttcttc agcacggaag    1080

catggaagtt caggagctat ggtgatgcac aggagggcaa agttagctgc aagagagaat    1140

cctgtagaga tcaaaggaga agcatttggg ggcagcaaca tcgatatcaa gtggtgtgaa    1200

cagccagaaa agaagaagag gagatcaaaa gtggcatctt gtcctcttta gttccgaatt    1260

tctgagagct ctgctgtaga cgtgaacact gtaccaatgt cttcagaatg agtatctgtc    1320

atactgtaat cgaaagaaaa tgattttgag aaaaagccag agagctttca tattaaaaat    1380

gttgacaaac actcagaagg cagggacagg ggattcaaga gtttaaagtc atccttagct    1440

gcacgataag tttgaggata acctgggcta caagagaccc tgtctcaaga agccataata    1500

attaaaacaa ccatccttaa ctaatgataa tgacaaagta tttttccatt gaaaatacat    1560

gtaagctgca attttgaaaa attattgaac cacccttgtg attaatagat gaagtttaaa    1620

aaaatttaaa tgtgttttta ttgtatgtat atggttggtt tacctgtgta tatgtctttc    1680

agtaacttgt ataaaactca atgatctcag ctagtaactt tcttctgtgt ggtcaatgtg    1740

atatgatttc ctatatattg ctaaattgaa tg                                  1772

<210>  4
<211>  1101

<212>  DNA
<213>  Homo sapiens

<400>  4
atgtggaacg cgacgcccag cgaagagccg gggttcaacc tcacactggc cgacctggac      60

tgggatgctt cccccggcaa cgactcgctg ggcgacgagc tgctgcagct cttccccgcg     120

ccgctgctgg cgggcgtcac agccacctgc gtggcactct tcgtggtggg tatcgctggc     180

aacctgctca ccatgctggt ggtgtcgcgc ttccgcgagc tgcgcaccac caccaacctc     240

tacctgtcca gcatggcctt ctccgatctg ctcatcttcc tctgcatgcc cctggacctc     300

gttcgcctct ggcagtaccg gccctggaac ttcggcgacc tcctctgcaa actcttccaa     360

ttcgtcagtg agagctgcac ctacgccacg gtgctcacca tcacagcgct gagcgtcgag     420

cgctacttcg ccatctgctt cccactccgg gccaaggtgg tggtcaccaa ggggcgggtg     480

aagctggtca tcttcgtcat ctgggccgtg gccttctgca gcgccgggcc catcttcgtg     540

ctagtcgggg tggagcacga gaacggcacc gacccttggg acaccaacga gtgccgcccc     600

accgagtttg cggtgcgctc tggactgctc acggtcatgg tgtgggtgtc cagcatcttc     660

ttcttccttc ctgtcttctg tctcacggtc ctctacagtc tcatcggcag gaagctgtgg     720

cggaggaggc gcggcgatgc tgtcgtgggt gcctcgctca gggaccagaa ccacaagcaa     780

accgtgaaaa tgctggctgt agtggtgttt gccttcatcc tctgctggct ccccttccac     840

gtagggcgat atttattttc caaatccttt gagcctggct ccttggagat tgctcagatc     900

agccagtact gcaacctcgt gtcctttgtc ctcttctacc tcagtgctgc catcaacccc     960

attctgtaca acatcatgtc caagaagtac cgggtggcag tgttcagact tctgggattc    1020

gaacccttct cccagagaaa gctctccact ctgaaagatg aaagttctcg ggcctggaca    1080

gaatctagta ttaatacatg a                                              1101

<210>  5
<211>  870
<212>  DNA
<213>  Homo sapiens

<400>  5
atgtggaacg cgacgcccag cgaagagccg gggttcaacc tcacactggc cgacctggac      60

tgggatgctt cccccggcaa cgactcgctg ggcgacgagc tgctgcagct cttccccgcg     120

ccgctgctgg cgggcgtcac agccacctgc gtggcactct tcgtggtggg tatcgctggc     180

aacctgctca ccatgctggt ggtgtcgcgc ttccgcgagc tgcgcaccac caccaacctc     240

tacctgtcca gcatggcctt ctccgatctg ctcatcttcc tctgcatgcc cctggacctc     300

gttcgcctct ggcagtaccg gccctggaac ttcggcgacc tcctctgcaa actcttccaa     360

ttcgtcagtg agagctgcac ctacgccacg gtgctcacca tcacagcgct gagcgtcgag     420

cgctacttcg ccatctgctt cccactccgg gccaaggtgg tggtcaccaa ggggcgggtg     480

aagctggtca tcttcgtcat ctgggccgtg gccttctgca gcgccgggcc catcttcgtg     540

ctagtcgggg tggagcacga gaacggcacc gacccttggg acaccaacga gtgccgcccc     600

accgagtttg cggtgcgctc tggactgctc acggtcatgg tgtgggtgtc cagcatcttc 660

ttcttccttc ctgtcttctg tctcacggtc ctctacagtc tcatcggcag gaagctgtgg 720

cggaggaggc gcggcgatgc tgtcgtgggt gcctcgctca gggaccagaa ccacaagcaa 780

accgtgaaaa tgctgggtgg gtctcagcgc gcgctcaggc tttctctcgc gggtcctatc 840

ctctccctgt gccttctccc ttctctctga 870

<210> 6
<211> 23
<212> DNA
<213> Artificial sequence

<220>
<223> Artificial DNA sequence

<400> 6
tctttggcaa ctctctggtc atc 23

<210> 7
<211> 22
<212> DNA
<213> Artificial sequence

<220>
<223> Artificial DNA sequence

<400> 7
cttcgggtag tgtactgcca ct 22

<210> 8
<211> 24
<212> DNA
<213> Artificial sequence

<220>
<223> Artificial DNA sequence

<400> 8
cgatatcaag tggtgtgaac agcc 24

<210> 9
<211> 20
<212> DNA
<213> Artificial sequence

<220>
<223> Artificial DNA sequence

<400> 9
gggtctcttg tagcccaggt 20

<210> 10
<211> 21
<212> DNA
<213> Artificial sequence

<220>
<223> Artificial DNA sequence

<400>  10
tctgccgtcc ttaccatctc a                                                21


<210>  11
<211>  22
<212>  DNA
<213>  Artificial sequence

<220>
<223>  Artificial DNA sequence

<400>  11
tctcaggact gtcccaaagg ag                                               22


<210>  12
<211>  23
<212>  DNA
<213>  Artificial sequence

<220>
<223>  Artificial DNA sequence

<400>  12
ggaccagagc gaaagcattt gcc                                              23


<210>  13
<211>  22
<212>  DNA
<213>  Artificial sequence

<220>
<223>  Artificial DNA sequence

<400>  13
tcaatctcgg gtggctgaac gc                                               22